# FASCICULE DE BREVET EUROPEEN

(11) **EP 1 940 840 B1**
(45) Date de publication et mention de la délivrance du brevet: **21.07.2010**
(21) Numéro de dépôt: 06830970.7
(22) Date de dépôt: 17.10.2006
(51) Int. Cl.: C07D 471/04, A61K 31/4375, A61P 35/00

(54) **DERIVES DE 6-HETEROARYLPYRIDOINDOLONE, LEUR PREPARATION ET LEUR APPLICATION EN THERAPEUTIQUE**
6-HETEROARYLPYRIDOINDOLON-DERIVATE, IHRE HERSTELLUNG UND IHRE THERAPEUTISCHE ANWENDUNG
6-HETEROARYLPYRIDOINDOLONE DERIVATIVES, THEIR PREPARATION AND THERAPEUTIC USE THEREOF

(30) Priorité: 20.10.2005 FR 0510730
(43) Date de publication de la demande: 09.07.2008
(73) Titulaire: Sanofi-Aventis, 75013 Paris (FR)
(72) Inventeur: MUNEAUX, Yvette, F-34270 Matelles (FR); JEGHAM, Samir, F-34980 Montferrier-sur-lez (FR); BOURRIE, Bernard, F-34980 Saint-gely-du-fesc (FR); CASELLAS, Pierre, F-34090 Montpellier (FR); CIAPETTI, Paola, F-67120 Altorf (FR); DEROCQ, Jean-Marie, F-34570 Murviel-les-montpellier (FR); WERMUTH, Camille-Georges, F-67100 Strasbourg (FR)
(74) Mandataire: Le Pennec, Magali
(86) Numéro de dépôt international: PCT/FR2006/002330
(87) Numéro de publication internationale: WO 2007/045758

(56) Documents cités:
- WO-A-2004/037821
- WO-A-2005/108398
- FR-A- 2 846 329

## Description

La présente invention se rapporte à des dérivés de 6-hétéroarylpyridoindolone, à leur préparation et à leur application en thérapeutique.

Le brevet français n° 97 08409 décrit des composés de formule : 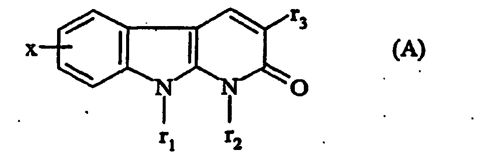 dans laquelle :
- x représente un atome d'hydrogène ou de chlore ou un groupe méthyle ou méthoxy ;
- r₁ représente un atome d'hydrogène ou un groupe méthyle ou éthyle ;
- r₂ représente un groupe méthyle ou éthyle; ou bien
- r₁ et r₂ forment ensemble un groupe (CH₂)₃ ;
- r₃ représente, soit un groupe phényle éventuellement substitué par un atome d'halogène ou un groupe méthyle ou méthoxy, soit un groupe thiényle.

Dans la description de ce brevet, il est mentionné que les composés de formule (A) ayant une affinité pour les sites modulateurs oméga associés aux récepteurs GABA_{A}, peuvent être utilisés dans le traitement d'affections liés aux désordres de la transmission gabaergique associés aux sous-types de récepteurs GABA_{A}, tels que l'anxiété, les troubles du sommeil, l'épilepsie etc...

Les demandes de brevet internationales WO 2002/087574 et WO 2002/087575 décrivent l'utilisation des composés de formule (A) comme agents anticancéreux et leur association avec d'autres agents anticancéreux.

La demande de brevet internationale WO 2004/041817 décrit des composés de formule : 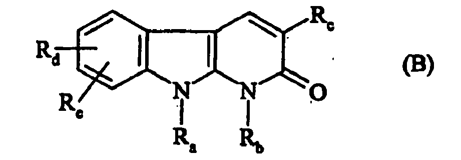 dans laquelle Rₐ, R_{b}, R_{c}, R_{d} et Rₑ ont différentes valeurs. Ces composés présentent une activité anticancéreuse.

La présente invention a pour objet des composés répondant à la formule (I) : 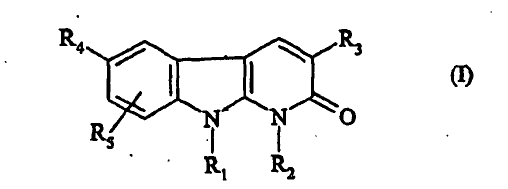 dans laquelle :
- R₁ représente un atome d'hydrogène ou un groupe (C₁-C₄) alkyle, CN, CF3 ou CHF2;
- R₂ représente un atome d'hydrogène ou un groupe (C₁-C₄) alkyle;
- R₃ représente un phényle non substitué ou substitué une ou plusieurs fois par des substituants choisis indépendamment parmi un atome d'halogène, un groupe (C₁-C₄) alkyle, un groupe (C₁-C₄) alkoxy;
- R₄ représente un radical hétérocyclique choisi parmi : 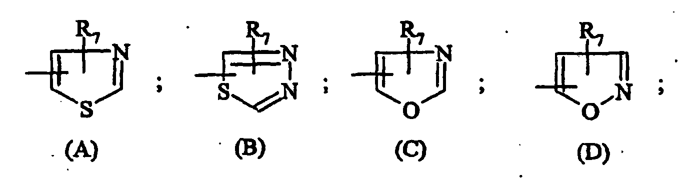 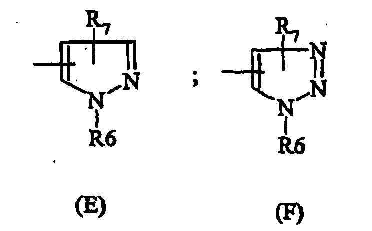
- R₅ représente un atome d'hydrogène, un atome d'halogène, un groupe (C₁-C₄) alkyle, un groupe (C1-C4) alkoxy;
   R6 est choisi parmi l'atome d'hydrogène,
   un groupe -SO2-R12,
   un groupe (C1-C6)alkyle, (C1-C6) mono ou perfluoro alkyle
   un groupe -(CH2)n-NR8R9,
   un groupe -(CH2)n-NH-CO-(C1-C4)alkyle,
   un groupe -(CH2)n-NH-SO2-(C1-C4)alkyle,
   un groupe -(CH2)n-NH-(CH2)m-NR8R9,
   un groupe -(CH2)n-NH-(CH2)m-OR10
   un groupe -(CH2)n-O-(CH2)m -NR8R9
   un groupe -(CH2)n-O-(CH2)m-O-(C1-C4) alkyle
   un groupe -(CH2)nHal,
   un groupe, -(C1-C6)alkyle-O-R10
   un groupe -CO2-(CH2)m-O-R10,
   un groupe -(CH2)n-COOR11;
   un groupe (C1-C6)alkylcarbonyle-, (C1-C6) mono ou perfluoro alkylcarbonyle-,
   -CO-NH-R10, -CO-(C₁-C6) alkyle-O-(C₁-C4) alkyle
   R7 est choisi parmi l'atome d'hydrogène,
   un groupe (C1-C6)alkyle, (C1-C6) mono ou perfluoro alkyle
   un groupe -(CH2)n-NR8R9,
   un groupe -(CH2)n-NH-CO-(C1-C4)alkyle,
   un groupe -(CH2)n-NH-SO2-(C1-C4)alkyle,
   un groupe-(CH2)n-NH-(CH2)m-NR8R9,
   un groupe -(CH2)n -NH-(CH2)m -OR10
   un groupe -(CH2)n-O-(CH2)m -NR8R9
   un groupe -(CH2)n-O-(CH2)m-O-(C1-C4) alkyle
   un groupe -(CH2)nHal,
   un groupe, -(C1-C6)alkyle-O-R10
   un groupe -CO2-(CH2)m -O-R10,
   un groupe -(CH2)n-COOR11;
   un groupe (C1-C6)alkylcarbonyle-, (C1-C6) mono ou perfluoro alkylcarbonyle-, -CO-NH-R10, -CO-(C1-C6) alkyle-O-(C1-C4)alkyle
- R₈ et R₉ représentent chacun indépendamment l'un et l'autre un atonie d'ydrogène ou un groupe (C₁-C₄) alkyle;
- ou bien R₈ et R₉ ensemble avec l'atome d'azote auquel ils sont liés constituent un radical hétérocyclique choisi parmi la pyrrolidin-1-yle, la pipéridin-1-yle, la morpholin-4-yle ou la pipérazinyle éventuellement substituée sur son second atome d'azote,
- R₁₀ représente un atome hydrogène, un groupe (C₁-C₄) alkyle, un groupe (C₁-C₄) alkylrarbonyle-, un groupe (C₃-C₆) cycloalkylcarbonyle-, un groupe (C₃-C₆) cycloalkyl(C₁-C₄) alkylcarbonyle-, un groupe (C3-C6)cycloalkyle, un groupe (C5-C6)hétérocycloalkyle, un groupe (C1-C6) mono ou perfluoro alkyle, un groupe (C3-C6)cycloalkyle (C1-C4)alkyle-
- R₁₁ représente un atome d'hydrogène ou un groupe (C1-C6) alkyle;
- R12 représente un groupe (C1-C6) alkyle, un groupe (C1-C6) mono ou perfluoro alkyle, un groupe cycloalkyle , un groupe cycloalkylalkyle-, un groupe (C₁-C6) alkyle-O-(C₁-C4) alkyle-
- m est 1, 2
- n est 0, 1, 2;
- Hal représente un atome d'halogène ;
   à l'état de base ou de sel addition à un acide, ainsi qu'à l'état d'hydrate ou de solvat.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome, ou un iode ;
   - un groupe (C₁-C6)alkyle: un groupe aliphatique saturé linéaire ou ramifié comportant 1 à 6 atomes de carbone. A titre d'exemple, on peut citer les groupes méthyle, éthyle, propyle, isopropyle, butyle, isobutyle, *sec*-butyle, *tert*-butyle ; pentyle ou hexyle ainsi que leurs isomères
   - un groupe (C1-C6)alcoxy : un radical O-alkyle où le groupe alkyle est tel que précédemment défini ;
- un groupe (C₃-C₆)cycloalkyle : un groupe alkyle cyclique de 3 à 6 atomes de carbone tel que le groupe cyclopropyle, cyclobutyle, cyclopentyle ou cyclohexyle.
- un groupe (C5-C6)hétérocycloalkyle représente notamment les cycles tétrahydropyrane, tétrahydrofurane, morpholinyle, pipéridine, pyrrolidine et pipérazinyle lui-même éventuellement substitué par un radical alkyle.

Parmi les composés de formule (I), objets de l'invention, on distingue :
- les composés de formule (IA) dans laquelle R₄ représente un 1,3-thiazole substitué par R₇ et les substituants R₁ à R₇ sont tels que définis pour les composés de formule (I).
   - les composés de formule (IB) dans laquelle R4 représente un 1,3,4-thiadiazole substitué par R7 et les substituants R1 à R7 sont tels que définis pour les composés de formule (I).
   - les composés de formule (IC) dans laquelle R4 représente un 1,3-oxazole substitué par R7 et les substituants R1 à R7 sont tels que définis pour les composés de formule (I).
   - les composés de formule (ID) dans laquelle R₄ représente un isoxazole substitué par R₇ et les substituants R₁ à R₇ sont tels que définis pour les composés de formule (I).
   - les composés de formule (IE) dans laquelle R₄ représente un 1*H*-pyrazole substitué par R₆ et R₇ et les substituants R₁ à R₇ sont tels que définis pour les composés de formule (I).
   - les composés de formule (IF) dans laquelle R₄ représente un 1,2,3-thiazole substitué par R₆ et R₇ et les substituants R₁ à R₇ sont tels que définis pour les composés de formule (I).

Parmi les composés de formule (I) objets de l'invention, on peut citer les composés préférés qui se définissent comme suit :
- R₁ représente un atome d'hydrogène ou un méthyle; CHF2,
- R₂ représente un méthyle ;
- R₃ représente un 2,4-dichlorophényle ; un 4F phényle ou un 4Cl phényle,
- R₄ représente un radical hétérocyclique choisi parmi : 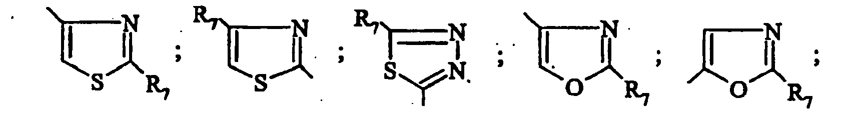 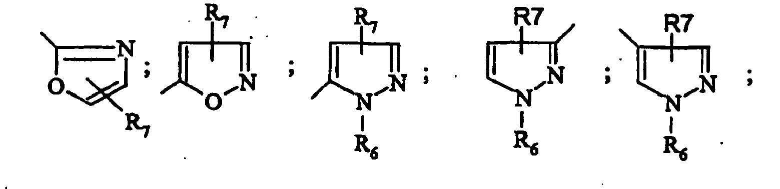 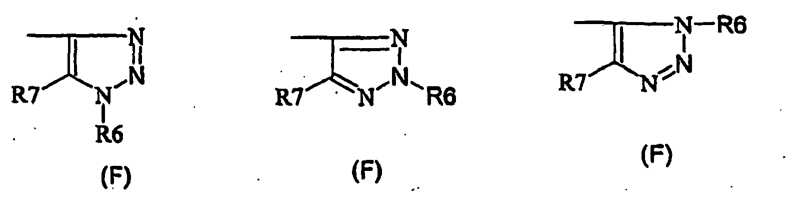
- R₅ représente un atome d'hydrogène ;
   R6 est choisi parmi l'atome d'hydrogène,
   Un groupe -SO2-R12,
   un groupe (C1-C6)alkyle, (C1-C6) mono ou perfluoro alkyle un groupe-(CH2)n-NR8R9,
   un groupe-(CH2)n-NH-CO-(C1-C4)alkyle,
   un groupe -(CH2)n-NH-SO2-(C1-C4)alkyle,
   un groupe -(CH2)n-NH-(CH2)-NR8R9,
   un groupe -(CH2)n-O-(CH2)m -NR8R9
   un groupe -(CH2)n-O-(CH2)m-O-(C1-C4) alkyle
   un groupe -(CH2)nHal,
   un groupe, -(C1-C6)alkyle-O-R10
   un groupe -CO2-(CH2)m-O-R10,
   un groupe -(CH2)n-COOR11;
   un groupe (C1-C6)alkylcarbonyle-, (C1-C6) mono ou perfluoro alkylcarbonyle-, CO-NH-R10,
   R7 est choisi parmi l'atome d'hydrogène,
   un groupe (C1-C6)alkyle, (C1-C6) mono ou perfluoro alkyle
   un groupe -(CH2)n-NR8R9,
   un groupe -(CH2)n-NH-CO-(C1-C4)alkyle,
   un groupe -(CH2)n-NH-SO2-(C1-C4)alkyle,
   un groupe-(CH2)n-NH-(CH2)m-NR8R9,
   un groupe -(CH2)n-O-(CH2)m -NR8R9
   un groupe -(CH2)n-O-(CH2)m-O-(C1-C4) alkyle
   un groupe -(CH2)nHal,
   un groupe, -(C1-C6)alkyle-O-R10
   un groupe -CO2-(CH2)m -O-R10,
   un groupe -(CH2)n-COOR11;
   un groupe (C1-C6)alkylcarbonyle-, (C1-C6) mono ou perfluoro alkylcarbonyle-, CO-NH-R10;
les radicaux R₈, R₉, R₁₀, R₁₁, a et m ayant les définitions ci-dessus,
lesdits produits de formule(I)) étant à l'état de base ou de sel d'addition à un acide, ainsi qu'à l'état d'hydrate ou de solvat.

A titre d'exemples non limitatifs, on peut indiquer les radicaux suivants lorsque R6 et/ou R7 représentent :
- le groupe -(CH2)n-NR8R9, alors ce groupe peut représenter par exemple CH2-NH2, CH2-NHalk, CH2-N(alk)2, CH2-hétérocycloalkyle
- le groupe -(CH2)n-NH-(CH2)m-NR8R9, alors ce groupe peut représenter par exemple (CH2)n-NH (CH2)m-hétérocycloalkyle
- le groupe -(CH2)n-O-(CH2)n-NR8R9, alors ce groupe peut représenter par exemple -(CH2)n-O-(CH2)m hétérocycloalkyle
- le groupe -(CH2)nHal alors ce groupe peut représenter par exemple CH2-Cl,
- le groupe -(C1-C6)alkyle-O-R10, alors ce groupe peut représenter par exemple CH2-OH, CH2-O-CH3 ou CH2-O-C2H5
- le groupe CO-NH-R10 , alors ce groupe peut représenter par exemple -CO-NH-(C1-C4) alkyle.

On peut noter que lorsque R6 ou R7 représente un groupe -(CH2)n-NR8R9, un groupe -(CH2)n-NH-(CH2)m-NR8R9, ou un groupe -(CH2)n-O-(CH2)m-NR8R9, dans ces groupes NR8R9 représente notamment un radical hétérocyclique choisi parmi la pyrrolidin-1-yle, la pipéridin-1-yle ou la morpholin-4-yle ou pipérazinyle éventuellement susbtituée sur son second atome d'azote par un radical alkyle.

La présente invention a ainsi pour objet des composés répondant à la formule : 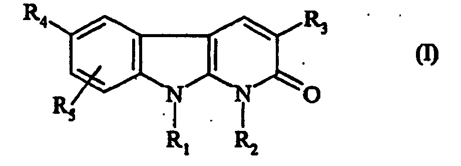 dans laquelle :
R₁ représente un atome d'hydrogène ou un groupe (C₁-C₄) alkyle
   - R₂ représente un atome d'hydrogène ou un groupe (C₁-C₄) alkyle ;
   - R₃ représente un phényle non substitué ou substitué une ou plusieurs fois par des substituants choisis indépendamment parmi un atome d'halogène, un groupe (C₁-C₄) alkyle, un groupe (C₁-C₄) alcoxy;
   - R₄ représente un radical hétérocyclique choisi parmi : 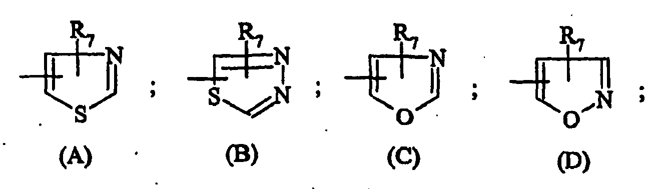 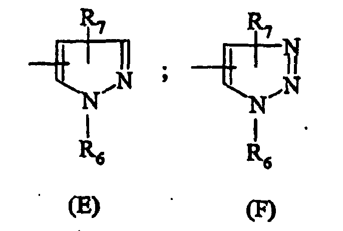
      - R₅ représente un atome d'hydrogène, un atome d'halogène, un groupe (C₁-C₄) alkyle, un groupe (C₁-C₄) alcoxy;
      - R₆ représente un atome d'hydrogène ou un groupe (C₁-C₄) alkyle;
      - R₇ représente un atome d'hydrogène, un groupe (C₁-C₄) alkyle, un groupe
      - (CH₂)ₙ-NR₈R₉, un groupe -(CH₂)ₙHal, un groupe -CH₂-O-R₁₀, un groupe
      - (CH₂)ₙ-COOR₁₁ ;
   - R₈ et R₉ représentent chacun indépendamment l'un et l'autre un atome d'hydrogène ou un groupe (C₁-C₄) alkyle;
   - ou bien R₈ et R₉ ensemble avec l'atome d'azote auquel ils sont liés constituent un radical hétérocyclique choisi parmi la pyrrolidin-1-yle, la pipéridin-1-yle ou la moipholin-4-yle ;
   - R₁₀ représente un atome d'hydrogène, un groupe (C₁-C₄) alkyle, un groupe (C₁-C₄) alkylcarbonyle, un groupe (C₃-C₆) cycloalkylcarbonyle;
   - R₁₁ représente un atome d'hydrogène ou un groupe (C₁-C₄) alkyle;
   - n est 0 ou 1;
      - Hal représente un atome d'halogène.
      à l'état de base ou de sel d'addition à un acide, ainsi qu'à l'état d'hydrate ou de solvat

Parmi les composés de formule (I) objets de l'invention, on peut citer les composés préférés qui se définissent comme suit :
- R₁ représente un atome d'hydrogène ou un méthyle ;
- R₂ représente un méthyle ;
- R₃ représente un 2,4-dichlorophényle ;
- R₄ représente un radical hétérocyclique choisi parmi : 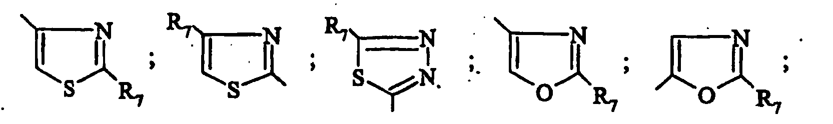 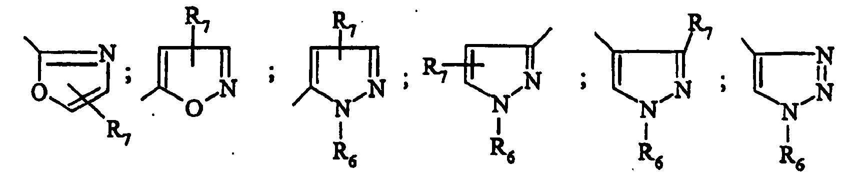
- R₅ représente un atome d'hydrogène;
- R₆ représente un atome d'hydrogène, un méthyle ou un éthyle;
- R₇ représente un atome d'hydrogène, un méthyle, un amino, un méthylamino, un aminométhyle, un (diméthylamino)méthyle, un pyrrolidin-1-ylméthyle, un chlorométhyle, un hydroxyméthyle, un éthoxyméthyle, un. [(2,2-diméthylpropanoyl)oxy]méthyle, un [(cyclopropylcarbonyl)oxy]méthyle, un méthoxycarbonyle, un 2-méthoxy-2-oxo éthyle, un carboxyméthyle; à l'état de base ou de sel d'addition à un acide, ainsi qu'à l'état d'hydrate ou de solvat.

Parmi les composés de ce dernier groupe, on peut citer les composés de formule (I) pour lesquels :
- R₁ représente un atome d'hydrogène ou un méthyle;
- R₂ représente un méthyle ;
- R₃ représente un 2,4-dichlorophényle ;
- R₄ représente :
   un 2-méthyl-1,3-thiazol-4-yle, un 2-amino-1,3-thiazol-4-yle, un 2-(méthylamino)-1,3-thiazol-4-yle, un 2-(hydroxyméthyl)-1,3-thiazol-4-yle, un 2-(éthoxyméthyl)-1,3-thiazol-4-yle, un 2-[[(2,2-diméthylpropanoyl)oxy]méthyl)-1,3-thiazol-4-yle, un 2-[(cyclopropylcarbonyl)oxy]méthyle ;
   un 1,3-thiazol-2-yle, un 4-méthyl-1,3-thiazol-2-yle, un 4-amino-1,3-thiazol-2-yle,
   un 4-(aminométhyl)-1,3-thiazol-2-yle, un 4-[(diméthylamino)méthyl]-1,3-thiazol-2-yle, un 4-(pyrrolidin-1-ylméthyl)-1,3-thiazol-2-yie, un 4-(chlorométhyl)-1,3-thiazol-2-yle, un 4-(2-méthoxy-2-oxoéthyl)-1,3-thiazol-2-yle, un 4-(carboxyméthyl)-1,3-thiazol-2-yle;
   un 1,3-oxazol-4-yle, un 2-méthyl-1,3-oxazol-4-yle, un 2-amino-1,3-oxazol-4-yle, un 2-(hydroxyméthyl)-1,3-oxazol-4-yle, un 2-(éthoxyméthyl)-1,3-oxazol-4-yle, un 2-[[(2,2-diméthylpropanoyl)oxy]méthyl]-1,3-oxazol-4-yle;
   un 1,3-oxazol-5-yle, un 2-méthyl-1,3-oxazol-5-yle, un 2-(éthoxyméthyl)-1,3-oxazol-5-yle;
   un 1,3-oxazol-2-yle, un 4-méthyl-1,3-oxazol-2-yle, un 5-méthyl-1,3-oxazol-2-yle;
   un isoxazol-5-yle, un 4-méthylisoxazol-5-yle, un 3-(méthoxycabonyl)isoxazol-5-yle;
   un 1*H*-pyrazol-5-yle, un 1-éthyl-1*H*-pyrazol-5-yle, un 3-(méthoxycarbonyl)-1*H-*pyrazol-5-yle, un 3-méthyl-1*H*-pyrazol-5-yle, un 3-amino-1*H*-pyrazol-5-yle ;
   un 1-mtéthyl-1*H*-pymol-3-yle, un 1-éthyl-1*H*-pyrazol-3-yle;
   un 3-amino-1*H*-pyrazol-4-yle ;
   un 5-amino-1,3,4-thiadiazol-2-yle;
   un 1*H*-1,2,3-triazol-4-yle ;
- R₅ représente un atome d'hydrogène;
   à l'état de base ou de sel d'addition à un acide, ainsi qu'à l'état d'hydrate ou de solvat.

Parmi les composés de formula (I), objets de l'invention, on peut notamment citer les composés suivants :
- 6-(2-Amino-1,3-thiazol-4-yl)-3-(2,4-dichlorophényl)-1-méthyl-1,9-dihydro-2*H-*pyrido[2,3-*b*]indol-2-one ;
- 3-(2,4-Dichlorophényl)-6-[2-(hydroxyméthyl)-1,3-thiazol-4-yl]-1-méthyl-1,9-dihydro-2*H*-pyrido[2,3-*b*]indol-2-one ;
- 3-(2,4-Dichlorophényl)-1,9-diméthyl-6-(2-méthyl-1,3-thiazol-4-yl)-1,9-dihydro-2*H*-pyrido[2,3-*b*]indol-2-one ;
- Pivalate de [4-[3-(2,4-dichlorophényl)-1,9-diméthyl-2-oxo-2,9-dihydro-1*H-*pyrido[2,3-*b*]indol-6-yl]-1,3-thiazol-2-yl]méthyle ;
- 3-(2,4-Dichlorophényl)-6-[2-(éthoxyméthyl)-1,3-thiazol-4-yl]-1,9-diméthyl-1,9-dihydro-2*H*-pyrido[2,3-*b*]indol-2-one ;
- 3-(2,4-Dichlorophényl)-1,9-diméthyl-6-(2-méthyl-1,3-oxazol-4-yl)-1,9-dihydro-2*H*-pyrido[2,3-*b*]indol-2-one ;
- 3-(2,4-Dichlorophényl)-1,9-diméthyl-6-(2-méthyl-1,3-oxazol-5-yl)-1,9-dihydro-2*H*-pyrido[2,3-*b*]indol-2-one ;
- 6-(3-Amino-1*H*-pyrazol-5-yl)-3-(2,4-dichlorophényl)-1,9-diméthyl-1,9-dihydro-2*H*-pyrido[2,3-*b*]indol-2-one ;
- 6-[4-(Aminométhyl)-1,3-thiazol-2-yl]-3-(2,4-dichlorophényl)-1,9-diméthyl-1,9-dihydro-2*H*-pyrido[2,3-*b*]indol-2-one ;
- 6-(3-Amino-1*H*-pyrazol-4-yl)-3-(2,4-dichlorophényl)-1,9-diméthyl-1,9-dihydro-2*H*-pyrido[2,3-*b*]indol-2-one ;
- 3-(2,4-Dichlorophényl)-1,9-diméthyl-6-(1*H*-pyrazol-5-yl)-1,9-dihydro-2*H-*pyrido[2,3-b]indol-2-one ;
   à l'état de base ou de sel d'addition à un acide, ainsi qu'à l'état d'hydrate ou de solvat.

Parmi les composés de formule (I), objets de l'invention, on peut notamment citer les composés suivants :
- 3-(2,4-Dichloro-phenyl)-6-(1-methoxymethyl-1H-pyrazol-3-yl)-1,9-dimethyl-1,9-dihydro-pyrido[2,3-b]indol-2-one
- 3-(4-bromo-phenyl)-6-(1-ethyl-1H-pyrazol-3-yl)-1,9-dimethyl-1,9-hydropyrido[2,3-b]indol-2-one
- 6-(2-Amino-thiazol-5-yl)-3-(2-chloro-4-fluoro-phenyl)-1,9-dimethyl-1,9-dihydropyrido[2,3-b]indol-2-one
- 3-(2,4-Dichloro-phenyl)-6-(2-methoxymethyl-thiazol-4-yl)-1,9-dimethyl-1,9-dihydro-pyrido[2,3-b]indol-2-one
- 3-(2,4-Dichloro-phenyl)-6-[1-(2,2-dimethyl-propionyl)-4-methyl-1H-pyrazol-3-yl]-1,9-dimethyl-1,9-dihydro-pyrido[2,3-b]indol-2-one
- 3-(2,4-Dichloro-phenyl)-6-(5-ethoxymethyl-1-methyl-1H-pyrazol-3-yl)-1,9-dimethyl-1,9-dihydro-pyrido[2,3-b]indol-2-one
- 3-(4-fluoro-phenyl)-6-(1-methoxymethyl-1H-pyrazol-3-yl)-1,9-dimethyl-1,9-dihydro-pyrido[2,3-b]indol-2-one
- 3-(4-Fluoro-phenyl)-6-(1-methoxymethyl-4-methyl-1H-pyrazol-3-yl)-1,9-dimethyl-1,9-dihydro-pyrido[2,3-b]indol-2-one
- 3-(4-Chloro-phenyl)-6-(2-ethoxymethyl-thiazol-4-yl)-1,9-dimethyl-1,9-dihydropyrido[2,3-b]indol-2-one
- 3-(2,4-Dichloro-phenyl)-6-[1-(2,2-dimethyl-propionyl)-1H-pyrazol-3-yl]-1,9-dimethyl-1,9-dihydro-pyrido[2,3-b]indol-2-one
- 3-(2,4-Dichloro-phenyl)-6-(1-méthoxymethyl-1H-[1,2,3]triazol-4-yl)-1,9-dimethyl-1,9-dihydro-pyrido[2,3-b]indol-2-one
   à l'état de base ou de sel d'addition à un acide, ainsi qu'à l'état d'hydrate ou de solvat.

Dans ce qui suit, on entend par groupe protecteur Gp ou G'p un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York, 1991).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc Des exemples de groupes partants ainsi que des références pour leur préparation sont donnés dans « Advanced Organic Chemistry », J. March, 3rd Edition, Wiley Interscience, p. 310-316,1985.

Conformément à l'invention, on peut préparer les composés de formule générale (I) selon des procédés bien connus de l'Homme de l'art tels que ceux décrits ci-après.

Conformément à l'invention, on peut préparer les composés de formule (IA) dans laquelle 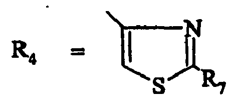 selon un procédé qui est **caractérisé en ce que :**
on fait réagir un composé de formule : 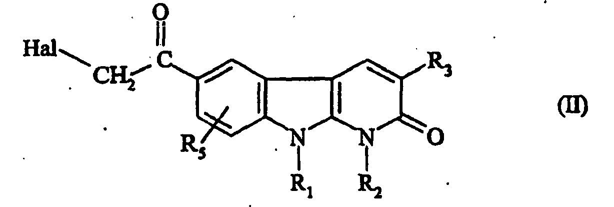 dans laquelle R₁, R₂, R₃ et R₅ sont tels que définis pour un composé de formule (I) et Hal représente un atome d'halogène avec un composé de formule : 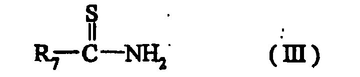 dans laquelle R₇ est tel que défini pour un composé de formule (I).

La réaction s'effectue dans un solvant tel que la N-méthylpyrrolidin-2-one, le méthanol ou l'éthanol, à une température comprise entre la température ambiante et la température de reflux du solvant.

Conformément à l'invention, on peut préparer les composés de formule (IA) dans laquelle 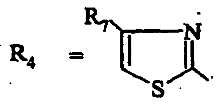 selon un procédé qui est **caractérisé en ce que** :
on fait réagir un composé de formule : 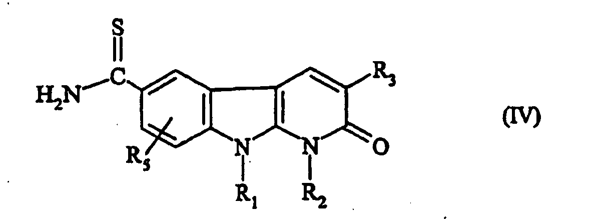 dans laquelle R₁, R₂, R₃ et R₅ sont tels que définis pour un composé de formule (I) avec un composé de formule : 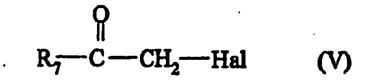 dans laquelle R₇ est tel que défini pour un composé de formule (I) et Hal représente un atome d'halogène.

La réaction s'effectue sans solvant ou dans un solvant tel que l'acétonitrile ou le 1,4-dioxane, à une température comprise entre la température ambiante et la température de reflux du solvant.

De façon particulière on peut préparer un composé de formule (IA) dans laquelle : 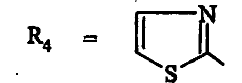 par réaction d'un composé de formule : 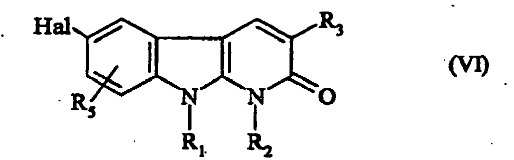 dans laquelle R₁, R₂, R₃ et R₅ sont tels que définis pour un composé de formule (I) et Hal représente un atome d'halogène, avec du 2-(tributylstannyl)-1,3-thiazole, en présence de Cu₂O, d'acétate de palladium (II) et de 1,3-bis (diphénylphosphino) propane, dans un solvant tel que la N-méthylpyrrolidon-2-one et à une température comprise entre la température ambiante et 100°C.

Conformément à l'invention, on peut préparer les composés de formule (IB) dans laquelle 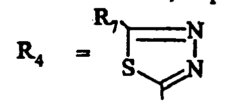 selon un procédé qui est **caractérisé en ce que :**
on fait réagir un composé de formule : 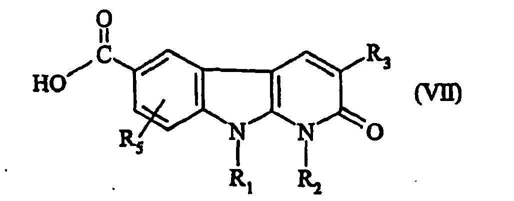 dans laquelle R₁, R₂, R₃ et R₅ sont tels que définis pour un composé de formule (I), avec un composé de formule : 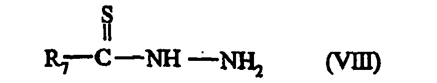 dans laquelle R₇ est tel que défini pour un composé de formule (I).

La réaction s'effectue en présence de POCl₃, à une température comprise entre la température ambiante et la température de reflux du mélange réactionnel.

Conformément à l'invention, on peut préparer les composés de formule (IC) dans laquelle 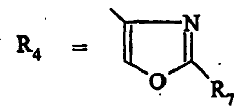 selon un procédé qui est **caractérisé en ce que :**
on fait réagir un composé de formule : 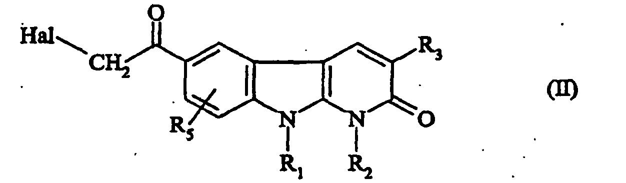 dans laquelle R₁, R₂, R₃ et R₅ sont tels que définis pour un composé de formule (I), et Hal représente un atome d'halogène, avec un composé de formule : 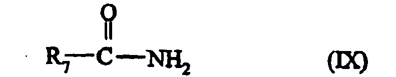 dans laquelle R₇ est tel que défini pour un composé de formule (I).

La réaction s'effectue sans solvant ou dans un solvant tel que le N-méthylpyrrolidin-2-one, le méthanol, ou l'éthanol à une température comprise entre la température ambiante et 160°C.

Conformément à l'invention, on peut préparer les composés de formule (IC) dans laquelle 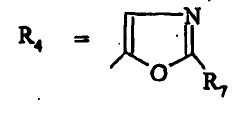 selon un procédé qui est **caractérisé en ce que :**
A) on fait réagir un composé de formule : 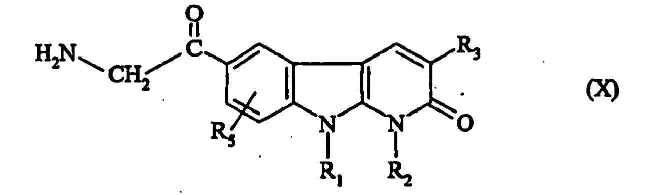 dans laquelle R₁, R₂, R₃ et R₅ sont tels que définis pour un composé de formule (I), avec un dérivé fonctionnel d'un acide de formule : 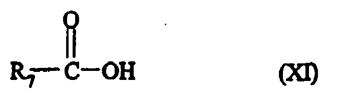 dans laquelle R₇ est tel que défini pour un composé de formule (I) pour obtenir un composé de formule : 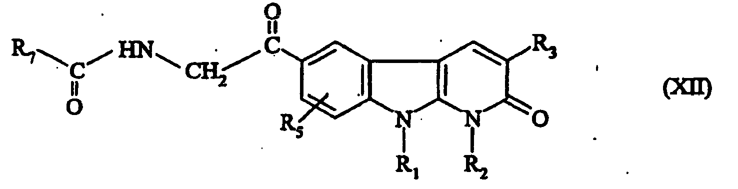
B) On cyclise le composé de formule (XII) ainsi obtenu par action d'un acide.

A l'étape A), lorsqu'on traite un composé de formule (X) avec l'acide de formule (XI) lui-même, on opère en présence d'un agent de couplage utilisé en chimie peptidique tel que le 1,3-dicyclohexylcarbodiimide ou l'hexafluorophosphate de benzotriazol-1-yloxytris(diméthylamino) phosphonium ou l'hexafluorophosphate de benzotriazol-1-yloxytris(pyrrolidino) phosphonium ou le tétrafluoroborate de 2-(1*H-*benzotriazol-1-yl)-1,1,3,3-tétraméthyl uronium, en présence d'une base telle que la triéthylamine, la N,N-diisopropyléthylamine ou la 4-diméthylaminopyridine, dans un solvant tel que le dichlorométhane, le dichloroéthane, le N-N-diméthylformamide ou le tétrahydrofurane à une température comprise entre -10°C et la température de reflux du solvant.

Comme dérivé fonctionnel de l'acide (XI) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle.

Ainsi dans le procédé selon l'invention, on peut aussi faire réagir le chlorure de l'acide obtenu par réaction du chlorure de thionyle ou du chlorure d'oxalyle sur l'acide de formule (XI), avec le composé de formule (X), dans un solvant, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préparer anhydride mixte de l'acide de formule (XI) par réaction du chloroformiate d'éthyle avec l'acide de formule (XI), en présence d'une base telle que la triéthylamine, et à le faire réagir avec le composé de formule (X), dans un solvant tel que le dichlorométhane, sous une atmosphère inerte, à la température ambiante, en présence d'une base telle que la triéthylamine.

A l'étape B), la réaction de cyclisation s'effectue par action d'un acide tel que l'acide sulfurique, à une température comprise entre 0°C et la température ambiante.

De façon particulière, on peut préparer un composé de formule (IC) dans laquelle : 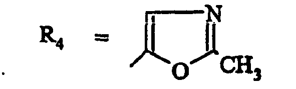 par réaction d'un composé de formule (X) avec l'orthoacétate de triéthyle, en présence d'une quantité catalytique d'un acide tel que l'acide toluène-4-sulfonique, à une température comprise entre la température ambiante et 150°C.

De façon particulière aussi, on peut préparer un composé de formule (IC) dans laquelle 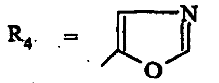 par réaction d'un composé de formule : 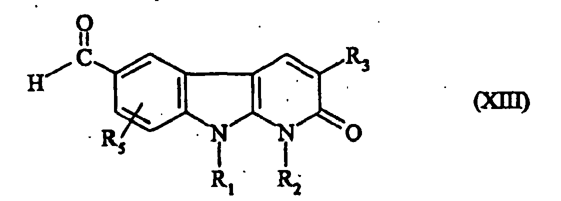 dans laquelle R₁, R₂, R₃ et R₅ sont tels que définis pour un composé de formule (I), avec le 1-[(isocyanométhyl)sulfonyl]-4-méthylbenzène, en présence d'une base telle que le carbonate de potassium, dans un solvant tel que le méthanol et à une température comprise entre la température ambiante et la température de reflux du solvant.

Conformément à l'invention, on peut préparer les composés de formule (IC) dans laquelle 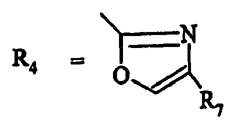 selon un procédé qui est **caractérisé en ce que :**
on fait réagir un composé de formule : 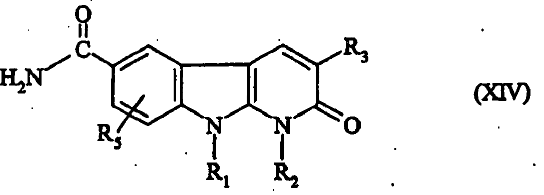 dans laquelle R₁, R₂, R₃, et R₅ sont tels que définis pour un composé de formule (I), avec un composé de formule : 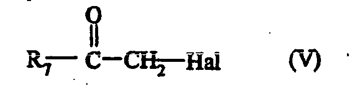 dans laquelle R₇ est tel que défini pour un composé de formule (I) et Hal représente un atome d'halogène. La réaction s'effectue selon les conditions opératoires décrites dans Chem. Ber., 1982,115 (7),2494-2507.

Conformément à l'invention, on peut préparer les composés de formule (IC) dans laquelle 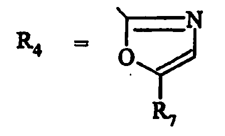 selon un procédé qui est **caractérisé en ce que :**
on fait réagir un composé de formule (XIV) avec un composé de formule (V) selon les conditions opératoires décrites dans Tetrahedron, 1989,45(19),6249-6262.

De façon particulière, on peut préparer un composé de formule (IC) dans laquelle : 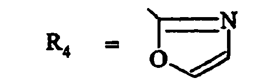 par réaction d'un composé de formule (XIV) avec le 1,3-dioxol-2-one, en présence d'un acide tel que H₃PO₄, à une température comprise entre la température ambiante et 170°C.

De façon particulière aussi, on peut préparer un composé de formule (IC) dans laquelle 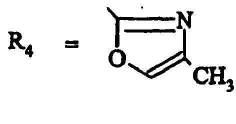 par réaction d'un composé de formule : 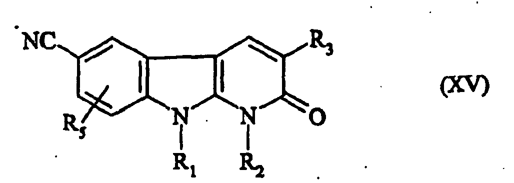 dans laquelle R₁, R₂, R₃ et R₅ sont tels que définis pour un composé de formule (I), avec l'alcool propargylique, en présence d'un acide tel que l'acide sulfurique, à une température comprise entre -20°C et la température ambiante. De façon particulière, enfin, on peut préparer un composé de formule (IC) dans laquelle 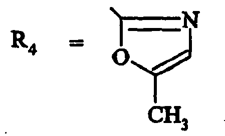 par cyclisation d'un composé de formule : 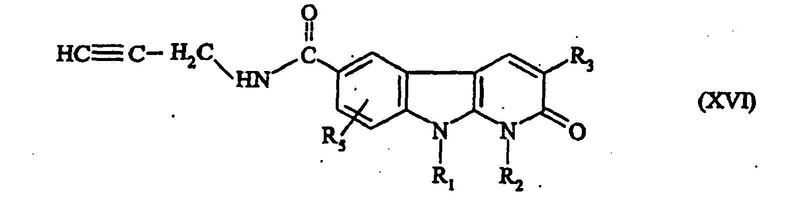 dans laquelle R₁, R₂, R₃ et R₅ sont tels que définis pour un composé de formule (I). La réaction de cyclisation s'effectue en présence d'acétate de mercure et d'un acide tel que l'acide acétique, à une température comprise entre la tempéraure ambiante et 120°C.

Conformément à l'invention, on peut préparer les composés de formule (ID) dans laquelle 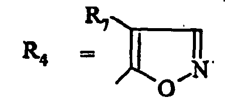 selon un procédé qui est **caractérisé en ce que :**
on fait réagir un composé de formule : 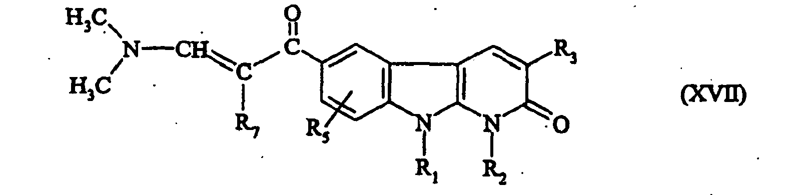 dans laquelle R₁, R₂, R₃, R₅ et R₇ sont tels que définis pour un composé de formule (I), avec l'hydroxylamine. La réaction s'effectue dans un solvant tel que le méthanol et à une température comprise entre la température ambiante et la température de reflux du solvant.

Conformément à l'invention, on peut préparer les composés de formule (ID) dans laquelle 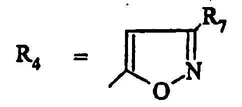 selon un procédé qui est **caractérisé en ce que :**
on fait réagir un composé de formule : 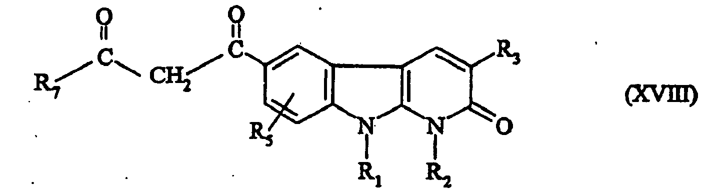 dans laquelle R₁, R₂, R₃, R₅ et R₇ sont tels que définis pour un composé de formule (I), avec l'hydroxylamine. La réaction s'effectue dans un solvant tel que le méthanol et à une température comprise entre la température ambiante et la température de reflux du solvant.

Conformément à l'invention, on peut préparer les composés de formule (ID) dans laquelle 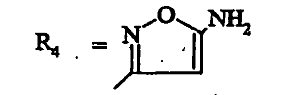 selon un procédé **caractérisé en ce que** l'on fait réagir un composé de formule (XX) : 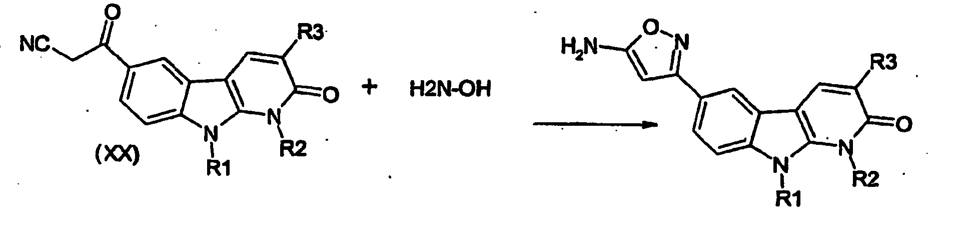 dans laquelle R₁, R₂ et R₃ sont tels que définis pour un composé de formule (I), avec l'hydroxylamine. La réaction s'effectue dans un solvant tel que le méthanol et à une température comprise entre la température ambiante et la température de reflux du solvant

Conformément à l'invention, on peut préparer un composé de formule (IE) dans laquelle 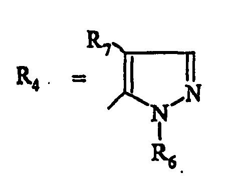 selon un procédé qui est **caractérisé en ce que :**
on fait réagir un composé de formule : 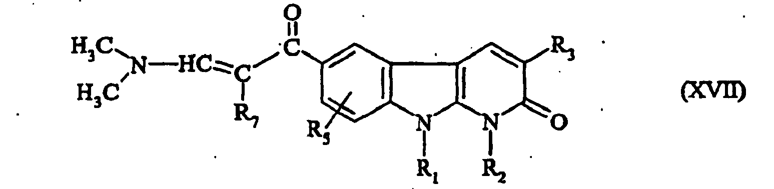 dans laquelle R₁, R₂, R₃, R₅ et R₇ sont tels que définis pour un composé de formule (I), avec un composé de formule :

   NH₂-NH-R₆ (XIX)

   dans laquelle R₆ est tel que défini pour un composé de formule (I). La réaction s'effectue dans un solvant tel que le méthanol et à une température comprise entre la température ambiante et la température de reflux du solvant.

Conformément à invention, on peut préparer un composé de formule (IE) dans laquelle 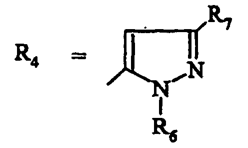 selon un procédé qui est **caractérisé en ce que :**
on fait réagir un composé de formule : 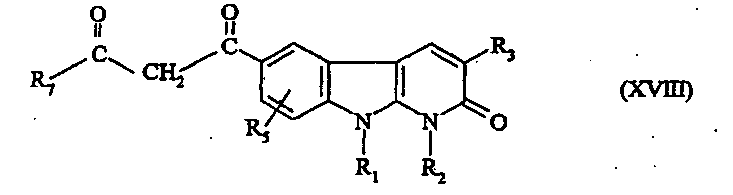 dans laquelle R₁, R₂, R₃, R₅ et R₇ sont tels que définis pour un composé de formule (I), avec une hydrazine de formule (XIX). La réaction s'effectue dans un solvant tel que le méthanol ou l'éthanol et à une température comprise entre la température ambiante et la température de reflux du solvant.

De façon particulière, on peut préparer un composé de formule (IE) dans laquelle 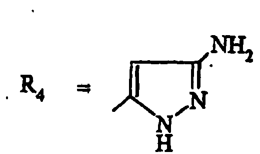 par réaction d'un composé de formule: 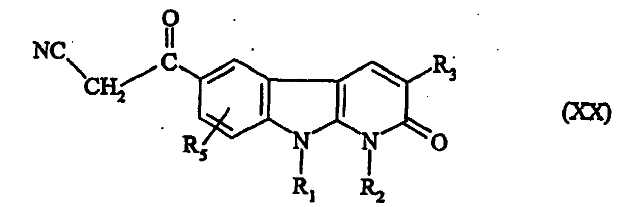 dans laquelle R₁, R₂, R₃, et R₅ sont tels que définis pour un composé de formule (I), avec l'hydrazine. La réaction s'effectue dans un solvant tel que l'éthanol et à une température comprise entre la température ambiante et la température de réflux du solvant.

De façon particulière, on peut préparer un composé de formule (IE) dans laquelle 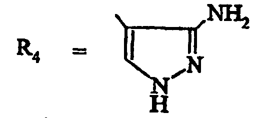 par réaction d'un composé de formule : 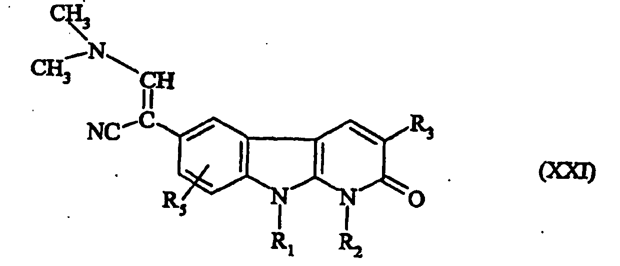 dans laquelle R₁, R₂, R₃, et R₅ sont tels que définis pour un composé de formule (I), avec l'hydrazine. La réaction s'effectue en présence d'un acide tel que l'acide acétique, dans un solvant tel que le MeOH et à une température comprise entre la température ambiante et la température de reflux du solvant.

Conformément à l'invention, on peut préparer un composé de formule (IF) dans laquelle 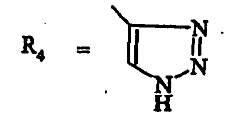 selon un procédé qui est **caractérisé en ce que :**
on fait réagir un composé de formule : 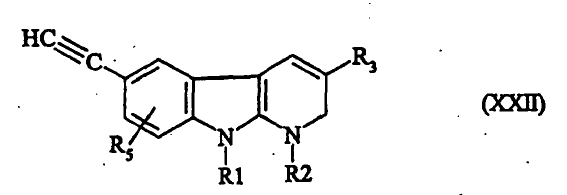 dans laquelle R₁, R₂, R₃ et R₅ sont tels que définis pour un composé de formule (I), avec l'azidure de sodium. La réaction s'effectue en présence de chlorure d'ammonium, dans un solvant tel que le N,N-diméthylformamide, à une température comprise entre la température ambiante et 200°C.

Un composé de formule (I) dans laquelle R₁ et/ou R₂ représentent un groupe (C₁-C₄)alkyle peut également être préparé par réaction d'un composé de formule (I) dans laquelle R₁ et/ou R₂ représentent un atome d'hydrogène avec un halogénure de (C₁-C₄)alkyle, en présence d'une base telle que l'hydrure de sodium, dans un solvant tel que le N,N-diméthylformamide et à une température comprise entre la température ambiante et la température de reflux du solvant.

Les composés de formule (I) dans laquelle R₄ représente un hétérocycle substitué par R₇ = -(CH₂)ₙ-NR₈R₉ peuvent également se préparer par réaction d'un composé de formule (I) correspondant dans lequel R₇ = -(CH₂)ₙ-Hal avec une amine de formule HNR₈R₉, en présence d'une base telle qu'un carbonate de métal alcalin, dans un solvant tel que le diméthylsulfoxyde ou le N,N-diméthylformamide et à une température comprise entre la température ambiante et la température de reflux du solvant.

Les composés de formule (I) dans laquelle R₄ représente un hétérocycle substitué par R₇ = -CH₂-O-R₁₀, avec R₁₀ = (C₁-C₄)alkyle peuvent également se préparer par réaction d'un composé de formule (I) correspondant dans lequel R₇ = - CH₂-OH avec un halogènure de (C₁-C₄)alkyle, en présence d'une base telle que l'hydrure de sodium, dans un solvant tel que le N,N-diméthylformamide et à une température comprise entre la température ambiante et la température de reflux du solvant.

Les composés de formule (I) dans laquelle R₄ représente un hétérocycle substitué par R₇ = -CH₂-O-R₁₀, avec R₁₀ = (C₁-C₄) alkylcarbonyle ou (C₃-C₆) cycloalkylcarbonyle peuvent également se préparer par réaction d'un composé de formule (I) correspondant dans lequel R₇ = -CH₂-OH avec un halogènure de (C₁-C₄) alkylcarbonyle ou de (C₃-C₆) cycloalkylcarbonyle, en présence d'une base telle que la pyridine, à une température comprise entre 0°C et la température ambiante.

Les composés de formule (I) dans laquelle R₄ représente un hétérocycle subtitué par R₆ = (C₁-C₄)alkyle peuvent également se préparer par réaction d'un composé de formule (I) correspondant dans lequel R₆ = H avec un halogènure de (C₁-C₄)alkyle, en présence d'une base telle que l'hydrure de sodium, dans un solvant tel que le N, N-diméthylformamide et à une température comprise entre 0°C et la température de reflux du solvant.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Les composés de formule (II) se préparent par réaction d'un composé de formule : 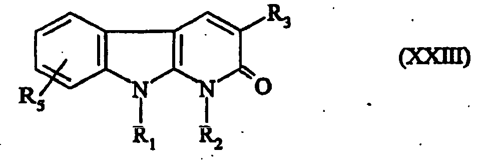 dans laquelle R₁, R₂, R₃ et R₅ sont tels que définis pour un composé de formule (I), avec un composé de formule : 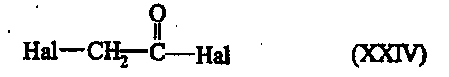 dans laquelle Hal représente un atome d'halogène, selon les conditions de la réaction de Friedels et Craft. Ainsi la réaction s'effectue en présence d'un acide de Lewis tel que le chlorure d'aluminium, dans un solvant tel que le dichlorométhane et à une température comprise entre 0°C et la température ambiante.

Les composés de formule (III) sont connus ou se préparent selon des méthodes connues.

Les composés de formule (IV) se préparent par réaction d'un composé de formule : 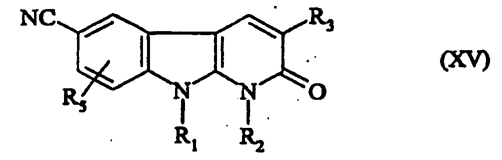 dans laquelle R₁, R₂, R₃, et R₅ sont tels que définis pour un composé de formule (I), avec le O,O'-diéthyldithiophosphate, dans un mélange de N-méthylpyrrolidin-2-one et d'eau, à une température comprise entre la température ambiante et 100°C.

Les composés de formule (V) sont connus ou se préparent selon des méthodes connues.

Les composés de formule (VI) sont connus et se préparent selon les méthodes décrites dans WO 2004/041817.

Les composés de formule (VII) se préparent par hydrolyse classique des composés de formule: 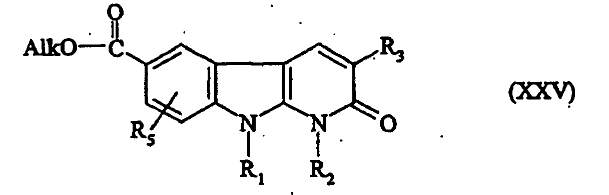 dans laquelle R₁, R₂, R₃, et R₅ sont tels que définis pour un composé de formule (I), et Alk représente un (C₁-C₂) alkyle. La réaction s'effectue par action d'une base telle que hydroxyde de potassium, dans un solvant tel que le méthanol, l'éthanol, en mélange avec de l'eau, à une température comprise entre la température ambiante et la température de reflux du solvant.

Les composés de formule (VIII) et (IX) sont connus ou se préparent selon des méthodes connues.

Les composés de formule (X) se préparent par réaction des composés de formule (II) avec l'hexaméthylènetétramine, dans un solvant tel que le chloroforme, à la température ambiante, suivi d'une hydrolyse acide.

Les composés de formule (XI) sont connus ou se prépaient selon des méthodes connues.

Les composés de formule (XIII) se préparent par réaction des composés de formule (XV) avec NaH₂PO₂, en présence d'une base telle que la pyridine, d'un acide tel que l'acide acétique et d'un catalyseur tel que le nickel de Raney^{®} à une température comprise entre la température ambiante et la température de reflux du mélange réactionnel.

Les composés de formule (XIV) se préparent par réaction des composés de formule (VI) avec le 1,1,1,3,3,3-hexaméthyldisilazane, en présence de PdCl₂(PPh₃)₂, dans un solvant tel que le N,N-diméthylformamide, à une température comprise entre la température ambiante et la température de reflux du solvant.

Les composés de formule (XV) sont connus et se préparent selon les méthodes décrites dans WO 2004/041817.

Les composés de formule (XVI) se préparent par réaction des composés de formule (VII) avec la propargylamine selon les méthodes du couplage peptidique précédemment décrites.

Les composés de formule (XVII) se préparent par réaction des composés de formule : 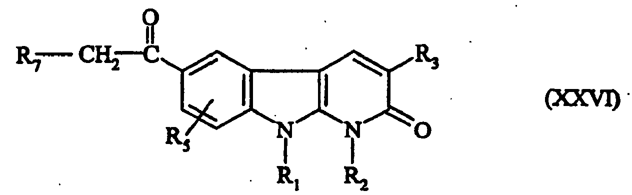 dans laquelle R₁, R₂, R₃, R₅ et R₇ sont tels que définis pour un composé de formule (I), avec le réactif de Bredereck (tertbutoxybis(diméthylamino)méthane) à chaud.

Les composés de formule (XVIII) se préparent par réaction des composés de formule (XXVI) dans laquelle R₇ = H avec un composé de formule : 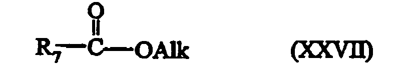 dans laquelle R₇ est tel que défini pour un composé de formule (I), et Alk représente un (C₁-C₄)alkyle en présence d'une base telle que l'hydrure de sodium, dans solvant tel que le N,N-diméthylformamide et à une température comprise entre 0°C et la température de reflux du solvant.

Les composés de formule (XIX) sont connus ou se préparent selon les méthodes connues.

Les composés de formule (XX) se préparent par réaction d'un composé de formule (II) avec le cyanure de potassium, dans un solvant tel que l'éthanol en mélange avec de l'eau et à une température comprise entre la température ambiante et la température de reflux du solvant

Les composés de formule (XXI) se préparent par réaction d'un composé de formule: 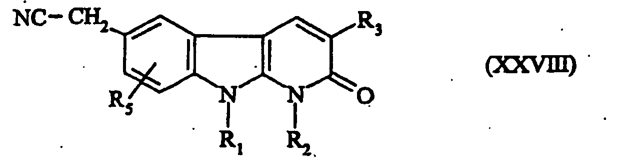 dans laquelle R₁, R₂, R₃, et R₅ sont tels que définis pour un composé de formule (I), avec le réactif de Bredereck à chaud.

Les composés de formule (XXII) se préparent par réaction d'un composé de formule (VI) avec le (triméthylsilyl)acétylène, en présence de Pd(PPh3)4, dans un solvant tel que la pyrrolidine, à une température de 150°C, suivi d'une hydrolyse basique.

Les composés de formule (XXIII) et (XXV) sont connus et se préparent selon les méthodes décrite dans WO2004/041817.

Les composés de formule (XXIV) et (XXVII) sont connus ou se préparent selon des méthodes connues.

Les composés de formule (XXVI) se préparent par réaction des composés de formule (XXIII) avec des composés de formule : 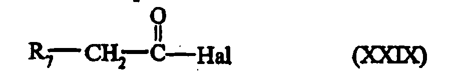 dans laquelle R₇ est tel que défini pour un composé de formule (I) et Hal représente un atome d'halogène, en présence d'un acide de Lewis tel que le chlorure d'aluminium, dans un solvant tel que le dichlorométhane et à une température comprise entre 0°C et la température ambiante.

Les composés de formule (XXVIII) se préparent selon le SCHEMA I ci-aprés :

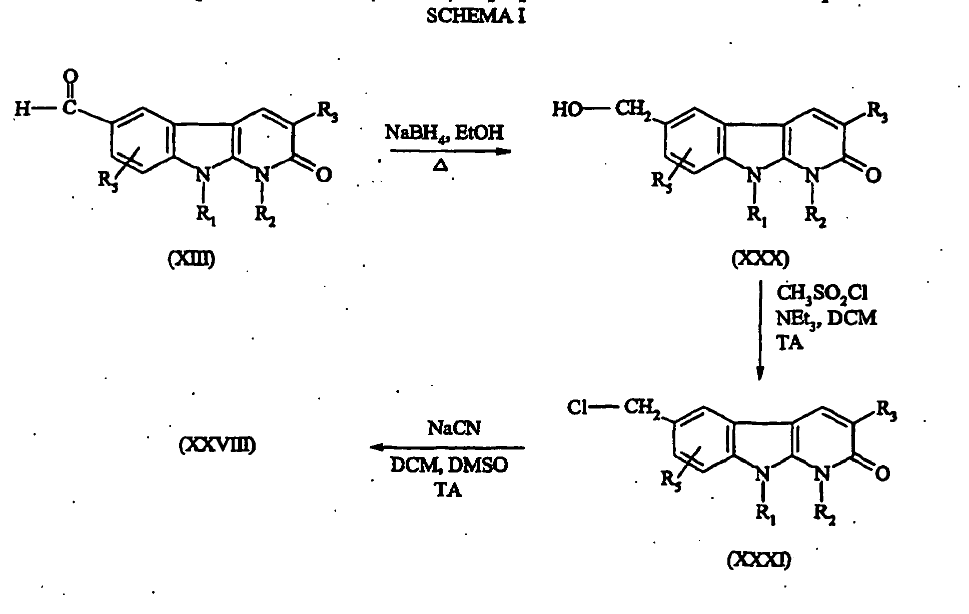

Sauf indication contraire, les spectres de résonance magnétique nucléaire (RMN¹H) du proton sont enregistrés dans DMSO-d₆, la référence est placée dans le DMSO-d₆ qui se situe à 2,50 ppm du tétraméthylsilane.

Les signaux observés en RMN sont exprimés ainsi : s: singulet ; se : singulet élargi ; d : doublet ; d.d : doublet dédoublé ; t : triplet ; td : triplet dédoublé ; q : quadruplet ; m : massif ; mt : multiplet.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau I ci-après, qui illustre les structures chimiques et les propriétés physiques de quelques composés selon l'invention.

Dans les préparations et exemples qui vont suivre, les abréviations suivantes sont utilisées :
DIPEA : diisopropyléthylamine
TEA : triéthylamine
DMF : diméthylformamide
EP : éther de pétrole
NMP : N-Me pyrrolidin-2-one
LAH : hydrure de lithium et d'aluminium
THF : tétrahydrofurane
Ether : éther diéthylique
DCM : dichlorométhane
AcOEt : acétate d'éthyle
AcOH : acide acétique
dppp : 1,3-bis (diphénylphosphino)propane
iPrOH : propan-2-ol réactif de Bredereck : *tert*butoxybis(diméthylamino)méthane
PyBOP : (benzotriazol-1-yloxy)tripyrrolidinophosphoniumhexafluorophosphate
BrCN : Bromure de cyanogène
TA : température ambiante
F : point de fusion

### PREPARATIONS

### Préparation 1.1

6-(Bromoacétyl)-3-(2,4-dichlorophényl)-1-méthyl-1,9-dihydro-2*H*-pyrido[2,3-b] indol-2-one.

### A) N'-Phénylformic hydrazide.

A une solution de 20 g de chlorhydrate de phénylhydrazine dans 150 ml de formiate de méthyle et 60 ml d'eau, on ajoute 25 g de K₂CO₃, chauffe à reflux pendant 1 heure et laisse 12 heures sous agitation à TA. On essore le précipité formé, le lave avec un mélange propan-2-ol/éther de pétrole. On obtient 18,8 g du composé attendu.

### B) N-Méthyl-N'-phénylacétohydrazide.

On chauffe à reflux une solution de 170 ml de LiAlH₄ 2M dans le THF, ajoute lentement une suspension de 18,8 g du composé de l'étape précédente dans 120 ml de THF et poursuit le reflux pendant 15 heures. On refroidit le mélange réactionnel au bain de glace, ajoute, goutte à goutte, 6 ml d'eau puis 20 ml de NaOH 1N, filtre les sels minéraux, les lave à l'AcOEt et concentre le filtrat sous vide. On reprend le résidu dans 150 ml d'AcOEt, ajoute une solution de 48 g de K₂CO₃ dans 100 ml d'eau puis 11 ml d'anhydride acétique et laisse 2 heures sous agitation à TA. Après décantation, on sèche la phase organique sur MgSO₄ et évapore le solvant sous vide. On reprend le résidu à l'éther de pétrole et essore le produit cristallisé formé. On obtient 19,9 g du composé attendu.

### C) Chlorhydrate de N-[1,3-dihydro-2H-indol-2-ylidène]méthanamine.

On chauffe à 80°C pendant 16 heures une solution de 19,9 g du composé de l'étape précédente dans 80 ml de POCl₃. Après refroidissement à TA, on ajouté de l'éther, essore le précipité formé et le lave à l'éther. On reprend le précipité dans 60 ml d'EtOH, chauffe à reflux pendant 4 heures, essore le précipité et le lave à l'éther. On obtient 12 g du composé attendu.

### D) 3-(2,4-Dichlorophényl)-1-méthyl-1,9-dihydro-2H-pyrido[2,3b]indol-2-one.

On chauffe à 110°C pendant 15 minutes une solution de 5 g du composé de l'étape précédente dans 25 ml d'AcOH, ajoute 7,5 g de 2-(2,4-dichlorophényl)-3-(diméthylamino)acrylate de méthyle et chauffe à 110°C pendant 16 heures. On ajoute de l'eau au mélange réactionnel, essore le précipité formé et le lave par un mélange propan-2-ol/éther de pétrole (50/50 ; v/v). On obtient 7,8 g du composé attendu.

### E) 6-(Bromoacétyl)-3-(2,4-dichlorophényl)-1-méthyl-1,9-dihydro-2H-pyrido[2,3-b] indol-2-one.

On refroidit à 0°C une solution de 2 g du composé de l'étape précédente dans 60 ml de DCM, ajoute 3,1 g d'AlCl₃ puis 1 ml de chlorure de bromoacétyle et laisse 1 heure sous agitation à 0°C. On verse le mélange réactionnel sur de la glace, extrait à l'AcOEt et chromatographie sur gel de silice en éluant par le mélange AcOEt/cyclohexane (50/50 ; v/v) puis à l'AcOEt. On obtient 1,2 g du composé attendu.

RMN¹H : DMSO-d₆ (300 MHz) : δ (ppm) : 3,71 : s : 3H ; 4,94 : s : 2H ; 7,44-7,48 : m : 2H ; 7,59 : d : 1H ; 7,70 : s : 1H ; 7,90 : d : 1H ; 8,32 : s : 1H ; 8,68 : s : 1H ; 12,61 : s : 1H.

### Préparation 1.2

### 6-(Bromoacétyl)-3-(2,4-dichlorophényl)-1,9-diméthyl-1,9-dihydro-2H-pyrido[2,3-b]indol-2-one.

### A) N,1-Diméthyl-1H-indol-2-amine.

On prépare ce composé selon les modes opératoires décrits dans WO 2004/041817, F = 249°C.

### B) 3-(2,4-Dichlorophényl)-1,9-diméthyl-1,9-dihydro-2H-pyrido[2,3-b]indol-2-one.

On chauffe à reflux pendant 15 minutes une solution de 3 g du composé de l'étape précédente dans 15 ml d'AcOH, ajoute 4,2 g de 2-(2,4-dichlorophényl)-3-(diméthylamino)acrylate de méthyle et chauffe à reflux pendant 16 heures. On ajoute de l'eau au mélange réactionnel, essore le précipité formé et le lave au MeOH puis à l'AcOEt. On obtient 4,1 g du composé attendu.

### C) 6-(Bromoacétyl)-3-(2,4-dichlorophényl)-1,9-diméthyl-1,9-dihydro-2H-pyrido[2,3-b]indol-2-one.

On refroidit à 0°C une solution de 1,2 g du composé de l'étape précédente dans 40 ml de DCM, ajoute 1,8 g d'AlCl₃ puis, goutte à goutte, 0,6 ml de chlorure de bromoacétyle et laisse 1 heure sous agitation à TA. On verse le mélange réactionnel sur de la glace, extrait au DCM, sèche la phase organique sur Na₂SO₄ et évapore le solvant sous vide. On chromatographie le résidu sur gel de silice en éluant par le mélange AcOEt/cyclohexane (75/25 ; v/v) puis à l'AcOEt. On obtient 1 g du composé attendu.

RMN¹H : DMSO-d₆ (300 MHz) : δ (ppm) : 4,01 : s : 3H ; 4,19 : s : 3H ; 4,93 : s : 2H ; 7,41-7,49 : m : 2H ; 7,69 : d : 1H ; 7,75 : d : 1H ; 7,91 : dd : 1H ; 8,34 : s : 1H ; 8,67 : d : 1H.

### Préparation 1.3

### 6-(Chloroacétyl)-3-(2,4-dichlorophényl)-1,9-diméthyl-1,9-dihydro-2H-pyrido[2,3-b]indol-2-one.

On refroidit à 0°C une solution de 0,7 g du composé de l'étape B de la Préparation 1.2 dans 25 ml de DCM, ajoute 1,04 g de AlCl₃ puis, goutte à goutte, 0,19 ml de chlorure de chloroacétyle et laisse 1 heure sous agitation à TA. On verse le mélange réactionnel sur de la glace, extrait au DCM, sèche la phase organique sur Na₂SO₄ et évapore le solvant sous vide. On chromatographie le résidu sur gel de silice en éluant par le mélange AcOEt/cyclohexane (75/25 ; v/v) puis à l'AcOEt et enfin par le mélange AcOEt/MeOH/NH₃ (90/10/1 ; v/v/v). On reprend le produit obtenu dans un mélange AcOEt/cyclohexane (25/75 ; v/v) et essore le précipité formé. On obtient 0,56 g du composé attendu.

RMN¹H : DMSO-d₆ (300 MHz): δ (ppm): 4,03 : s : 3H ; 4,21 : s : 3H ; 5,22 : s : 2H ; 7,43-7,51 : m : 2H ; 7,70 : d : 1H ; 7,76 : d : 1H ; 7,91 : dd : 1H ; 8,35 : s : 1H ; 8,66 : s : 1H.

### Préparation 1.4

### 6-Bromo-3-(2,4-dichlorophényl)-1-méthyl-1,9-dihydro-2H-pyrido[2,3-b]indol-2-one.

On prépare ce composé selon le mode opératoire décrit à l'Exemple 114 dans WO 2004/041817, F = 386°C (déc.).

### Préparation 1.5

### 3-(2,4-Dichlorophényl)-1,9-diméthyl-2-oxo-2,9-dihydro-1H-pyrido[2,3-b]indole-6-carbonitrile.

### A) N'-(4-Cyanophényl)acétohydrazide.

A une solution de 5 g de chlorhydrate de 4-cyanophénylhydrazine dans 40 ml d'acide acétique on ajoute 2,7 g d'acétate de sodium puis chauffe à 80°C pendant 20 heures. On concentre sous vide le mélange réactionnel, reprend le résidu à l'eau, extrait à l'AcOEt, lave la phase organique par une solution saturée de NaCl, sèche sur MgSO₄ et évapore le solvant sous vide. On triture le résidu dans un mélange EP/AcOEt (90/10 ; v/v) et essore le précipité formé. On obtient 4,8 g du composé attendu.

RMN¹H : CDCl₃ (300 MHz) : δ (ppm) 1,92 : s : 3H ; 6,75 : d : 2H ; 7,53 : d : 2H ; 8,25 : s : 1H ; 9,76 : s : 1H.

### B) N'-(4-Cyanophényl)-N,N'-diméthylacétohydrazide.

A une suspension de 2,8 g de NaH à 60 % dans l'huile dans 50 ml de DMF on ajoute, en goutte à goutte et à TA, une solution de 4,8 g du composé obtenu à l'étape précédente dans 20 ml de DMF et laisse sous agitation jusqu'à la fin du dégagement gazeux. On ajoute ensuite 6,8 ml d'iodure de méthyle puis laisse sous agitation pendant 2 heures à TA. On verse le mélange réactionnel sur une solution saturée de NH₄Cl, extrait à l'AcOEt, lave la phase organique par une solution saturée de NaCl, sèche sur MgSO₄ et évapore le solvant sous vide. On reprend le résidu dans l'EP et essore le précipité formé. On obtient 4,5 g du composé attendu.

RMN¹H : CDCl₃ (300 MHz) : δ (ppm) : 2,04 : s : 3H ; 3,03 : s : 3H ; 3,21 : s : 3H ; 6,70 : d : 3H ; 7,57 : d : 2H.

### C) 1-Méthyl-2-(méthylamino)-1H-indole-5-carbonitrile.

On chauffe à 75°C pendant 1 heure 30 minutes une solution de 4,0 g du composé obtenu à l'étape précédente dans 30 ml de POCl₃. Après refroidissement à TA on essore le précipité formé et le lave à l'éther. On obtient 3,4 g du composé attendu.

### D) 3-(2,4-Dichlorophényl)-1,9-diméthyl-2-oxo-2,9-dihydro-1H-pyrido[2,3-b]indole-6-carbonitrile.

On chauffe à 100°C pendant 15 minutes un mélange de 0,4 g du composé obtenu à l'étape précédente dans 4 ml d'acide acétique puis ajoute 0,43 g de 2-(2,4-dichlorophényl)-3-(diméthylamino)acrylate de méthyle et chauffe à 100°C pendant 3 heures. Après refroidissement à TA, on ajoute de l'eau au mélange réactionnel, essore le précipité beige formé et le lave à l'eau. On reprend le précipité dans du MeOH et concentre sous vide le solvant. On obtient 0,5 g du composé attendu.

RMN¹H : DMSO-d₆ (300 MHz) : δ (ppm) : 4,02 : s : 3H ; 4,21 : s : 3H ; 7,42-7,51 : m : 2H ; 7,66-7,70 : m : 2H ; 7,83 : d : 1H ; 8,31 : s : 1H ; 8,42 : s : 1H.

### Préparation 1.6

### 3-(2,4-Dichlorophényl)-1,9-diméthyl-2-oxo-2,9-dihydro-1H-pyrido[2,3-b]indole-6-carbothioamide.

A une solution de 0,5 g du composé de la Préparation 1.5 dans 10 ml de NMP et 1,5 ml d'eau on ajoute 0,4 ml de O,O'-diéthyldithiophosphate et chauffe à 90°C pendant une nuit. Après refroidissement à TA, on verse le mélange réactionnel sur une solution saturée de NH₄Cl, essore le précipité formé, le lave à l'eau puis avec le mélange propan-2-ol/EP (50/50 ; v/v). On reprend le précipité au MeOH et concentre le solvant sous vide. On chromatographie le résidu sur gel de silice en éluant par le mélange DCM/MeOH de (99/1 ; v/v) à (98/2 ; v/v). On obtient 0,41 g du composé attendu.

### Préparation 1.7

### 3-(2,4-Dichlorophényl)-1,9-diméthyl-2-oxo-2,9-dihydro-1H-pyrido[2,3-b]indole-6-carbaldéhyde.

A une solution de 0,45 g du composé de la Préparation 1.5 dans 30 ml de pyridine, 10 ml d'acide acétique et 6 ml d'eau, on ajoute 3,4 g de NaH₂PO₂ et 1,7 ml de nickel de Raney^{®} et chauffe à 65°C pendant 36 heures. On filtre le catalyseur, lave au MeOH et concentre sous vide le filtrat. On reprend le résidu à l'eau, extrait à l'AcOEt, lave la phase organique par une solution saturée de NaCl, sèche sur MgSO₄ et évapore le solvant sous vide. On reprend le résidu dans un mélange AcOEt/EP (50/50 ; v/v) et essore le précipité formé. On obtient 0,2 g du composé attendu, F = 284°C.

### Préparation 1.8

### 2-[3-(2,4-Dichlorophényl)-1,9-diméthyl-2-oxo-2,9-dihydro-1H-pyrido[2,3-b] indol-6-yl]-3-(diméthylamino)acrylonitrile.

### A) 3-(2,4-Dichlorophényl)-6-(hydroxyméthyl)-1,9-diméthyl-1,9-dihydro-2H-pyrido[2,3-b]indol-2-one.

A une solution de 0,5 g du composé de la Préparation 1.7 dans 15 ml d'EtOH on ajoute 0,06 g de NaBH₄ et chauffe à reflux pendant 5 heures. Après refroidissement à TA, on ajoute 1 ml de NaOH 6N et concentre sous vide. On triture le résidu dans l'eau, essore le précipité formé et le lave à l'eau. On reprend le précipité dans du MeOH et évapore le solvant sous vide. On obtient 0,49 g du composé attendu.

### B) 6-(Chlorométhyl)-3-(2,4-dichlorophényl)-1,9-diméthyl-1,9-dihydro-2H-pyrido[2,3-b]indol-2-one.

A une solution de 0,49 g du composé de l'étape précédente et 0,53 ml de triéthylamine dans 10 ml de DCM on ajoute 0,27 ml de chlorure de méthanesulfonyle et laisse 3 jours sous agitation à TA. On chromatographie le mélange réactionnel sur gel de silice en éluant par le mélange AcOEt/cyclohexane (75/25 ; v/v). On obtient 0,4 g du composé attendu.

### C) [3-(2,4-Dichlorophényl)-1,9-diméthyl-2-oxo-2,9-dihydro-1H-pyrido[2,3-b]indol-6-yl]acétonitrile.

A un mélange de 0,2 g du composé de l'étape précédente et 0,049 g de NaCN dans 2 ml de DCM on ajoute, goutte à goutte, 3 ml de DMSO et laisse une nuit sous agitation à TA. On ajoute de l'eau et de l'AcOEt, décante, lave la phase organique à l'eau, sèche sur Na₂SO₄ et évapore le solvant sous vide. On reprend le résidu au MeOH et évapore sous vide. On obtient 0,18 g du composé attendu.

### D) 2-[3-(2,4-Dichlorophényl)-1,9-diméthyl-2-oxo-2,9-dihydro-1H-pyrido[2,3-b] indol-6-yl]-3-(diméthylamino)acrylonitrile.

On chauffe à 100°C pendant une nuit un mélange de 0,18 g du composé de l'étape précédente et 5 ml de réactif de Bredereck. Après refroidissement à TA, on ajoute de l'EP et de l'eau, essore le précipité formé, le lave à l'eau puis avec le mélange EP/propan-2-ol (50/50 ; v/v). On reprend le précipité dans du MeOH et évapore sous vide. On obtient 0,15 g du composé attendu.

RMN¹H : DMSO-d₆ (300 MHz): δ (ppm) : 3,19 : s : 6H ; 4,00 : s : 3H ; 4,13 : s : 3H ; 7,22-7,26 : dd : 1H ; 7,39-7,46 : m : 3H ; 7,54 : d : 1H ; 7,68 : s : 1H ; 7,80 : s : 1H ; 8,18 : s : 1H.

### Préparation 1.9

### 3-(2,4-Dichlorophényl)-6-[3-(dimémylamino)prop-2-ènoyl]-1-méthyl-9-[[2-(triméthylsilyl)éthoxy]méthyl]-1,9-dihydro-2H-pyrido[2,3-b]indol-2-one.

### A) 6-Acétyl-3-(2,4-dichlorophényl)-1-méthyl-1,9-dihydro-2H-pyrido[2,3-b]indol-2-one.

On refroidit à 0°C une solution de 1,5 g du composé de l'étape D de la Préparation 1.1 dans 60 ml de DCM, ajoute 2,33 g d'AlCl₃ puis 0,624 ml de chlorure d'acétyle et laisse 2 heures sous agitation à TA. On verse le mélange réactionnel sur de la glace, extrait au DCM, essore le précipité formé, extrait les jus d'essorage au DCM, sèche la phase organique sur MgSO₄ et évapore sous vide. On rassemble le résidu et le précipité, triture dans le mélange MeOH/éther et essore. On obtient 1,36 g du composé attendu.

### B) 6-Acétyl-3-(2,4-dichlorophényl)-1-méthyl-[[2-(triméthylsilyl)éthoxy]méthyl]-1,9-dihydro-2H-pyrido[2,3-b]indol-2-one.

A une solution de 1 g du composé de l'étape précédente dans 13 ml de DMF on ajoute 0,311 g de NaH à 60 % dans l'huile puis 0,69 ml de chlorure de 2-(triméthylsilyl)éthoxyméthyle et laisse 5 heures sous agitation à TA. On ajoute une solution saturée de NaCl, extrait à l'AcOEt, lave la phase organique par une solution saturée de NaCl, sèche sur MgSO₄ et évapore le solvant sous vide. On chromatographie le résidu sur gel de silice en éluant au cyclohexane puis par le gradient du mélange cyclohexane/AcOEt de (90/10 ; v/v) à (50/50 ; v/v). On obtient 0,639 g du composé attendu.

### C) 3-(2,4-Dichlomphényl)-6-[3-(diméthylamino)prop-2-ènoyl]-1-méthyl-9-[[2-(triméthylsilyl)éthoxy]méthyl]-1,9-dihydro-2H-pyrido[2,3-b]indol-2-one.

On chauffe à 100°C pendant une nuit une solution de 0,25 g du composé de l'étape précédente dans 5 ml de réactif de Bredereck. Après refroidissement à TA, on ajoute de l'EP, essore le précipité formé, le lave à l'EP et le sèche. On obtient 0,236 g du composé attendu.

### Préparation 1.10

### 3-(2,4-Dichlorophényl)-6-[3-(diméthylamino)prop-2-ènoyl]-1,9-diméthyl-1,9-dihydro-2H-pyrido[2,3-b]indol-2-one.

### A) N'-(4-Iodophényl)acétohydrazide.

On chauffe à reflux pendant 5 heures un mélange de 10 g de 4-iodophénylhydrazine dans 80 ml d'acide acétique. On concentre le mélange réactionnel sous vide, reprend le résidu à l'eau, essore le précipité formé et le lave par un mélange propan-2-ol/EP (50/50 ; v/v). On obtient 9 g du composé attendu.

### B) N'-(4-Iodophényl)-N,N'-diméthylacétohydrazide.

A une suspension de 3,9 g de NaH à 60 % dans l'huile dans 50 ml de DMF, on ajoute une solution de 9 g du composé de l'étape précédente dans 30 ml de DMF et laisse 5 minutes sous agitation à TA. On ajoute ensuite 6,1 ml d'iodure de méthyle et laisse 1 heure sous agitation à TA. On verse le mélange réactionnel sur une solution saturée de NH₄Cl, extrait à l'AcOEt, lave la phase organique par une solution saturée de NaCl, sèche sur MgSO₄ et évapore le solvant sous vide. On triture le résidu huileux dans l'EP et essore le précipité formé. On obtient 6,7 g du composé attendu.

### C) Chlorhydrate de N-[5-iodo-1-méthyl-1,3-dihydro-2H-indol-2-ylidène) méthanamine.

On chauffe à 80°C pendant 16 heures un mélange de 6,7 g du composé de l'étape précédente et 50 ml de POCl₃. Après refroidissement à TA, on ajoute de l'éther, essore le précipité formé et le lave à l'éther. On reprend le précipité dans 20 ml d'EtOH et chauffe à reflux pendant 5 heures. On essore le précipité formé et le lave à l'éther. On obtient 4,4 g du composé attendu.

### D) 3-(2,4-Dichlorophényl)-6-iodo-1,9-diméthyl-1,9-dihydro-2H-pyrido[2,3-b]indol-2-one.

On chauffe à reflux pendant 15 minutes une solution de 1,4 g du composé de l'étape précédente dans 8 ml d'acide acétique, ajoute 1,2 g de 2-(2,4-dichlorophényl)-3-(diméthylamino)acrylate de méthyle puis chauffe à reflux pendant 16 heures. On verse le mélange réactionnel dans l'eau, extrait à l'AcOEt, lave la phase organique par une solution saturée de NaCl, sèche sur MgSO₄ et évapore le solvant sous vide. On chromatographie le résidu sur gel de silice en éluant par le mélange AcOEt/cyclohexane (75/25 ; v/v). On reprend le produit obtenu par un mélange AcOEt/cyclohexane (25/75 ; v/v) et essore le précipité formé. On obtient 1,3 g du composé attendu, F = 223-225°C.

### E) 6-Acétyl-3-(2,4-dichlorophényl)-1,9-diméthyl-1,9-dihydro-2H-pyrido[2,3-b]indol-2-one.

A une solution de 0,4 g du composé de l'étape précédente dans 15 ml de NMP on ajoute 0,5 g du Cu₂O puis dégaze le mélange réactionnel par barbotage d'argon pendant 30 minutes. On ajoute ensuite 0,075 g d'acétate de Palladium (II), 0,273 g de 1,3-bis(diphénylphosphino)propane et 0,64 ml de tributyl(1-éthoxyvinyl)stannane puis chauffe à 80°C pendant 8 heures. On verse le mélange réactionnel sur 100 ml d'HCl 1N, laisse 10 minutes sous agitation et essore le précipité formé. On chromatographie le précipité sur gel de silice en éluant par le mélange AcOEt/cyclohexane (75/25 ; v/v). On triture le produit obtenu dans un mélange AcOEt / cyclohexane (75/25 ; v/v) et essore le précipité formé. On obtient 0,15 g du composé attendu, F = 254-255°C.

### F) 3-(2,4-Dichlorophényl)-6-[3-(diméthylamino)prop-2-ènoyl]-1,9-diméthyl-1,9-dihydro-2H-pyrido[2,3-b]indol-2-one.

On chauffe à 100°C pendant une nuit une solution de 0,5 g du composé de l'étape précédente dans 5 ml de réactif de Bredereck. Après refroidissement à température ambiante, on ajoute de l'EP, essore le précipité formé et le lave à l'EP. On obtient 0,575 g du composé attendu.

### Préparation 1.11

### 3-(2,4-Dichlorophényl)-1,9-diméthyl-2-oxo-2,9-dihydro-1H-pyrido[2,3-b]indol-6-carboxamide.

On fait barboter pendant 5 minutes du CO dans une solution de 1,28 g du composé de l'étape D de la Préparation 1.10, 0,055 g de PdCl₂(PPh₃)₂ et 2,24 ml de 1,1,1,3,3,3-hexaméthyl disilazane dans 8,5 ml de DMF puis chauffe à 80°C pendant 12 heures sous atmosphère de CO. Après refroidissement à TA, on ajoute 1,39 ml de MeOH et laisse 10 minutes sous agitation. On verse le mélange réactionnel dans 56 ml d'H₂SO₄ 2N, essore le précipité formé et le lave à l'eau puis à l'AcOEt. On extrait le filtrat à l'AcOEt, lave la phase organique par une solution saturée de NaHCO₃, par une solution saturée de NaCl, sèche sur MgSO₄ et évapore le solvant sous vide. On chromatographie le précipité et le résidu sur gel de silice en éluant par le mélange AcOEt/cyclohexane (80/20 ; v/v). On obtient 0,6 g du composé attendu.

RMN¹H : CDCl₃ (300 MHz) : δ (ppm) : 4,09 : s : 3H ; 4,17 : s : 3H ; 7.28-7,45 : m : 3H ; 7,50 : d : 1H ; 7,79 : dd : 1H ; 8,02 : s : 1H ; 8,25 : d : 1H.

### Préparation 1.12

### 4-[3-(2,4-Dichlorophényl)-1,9-diméthyl-2-oxo-2,9-dihydro-1H-pyrido[2,3-b] indol-6-yl]-2,4-dioxobutanoate de méthyle.

On refroidit à 5°C une solution de 0,3 g du compose de l'étape E de la Préparation 1.10 dans 4 ml de DMF, ajoute 0,036 g de NaH à 60 % dans l'huile et 0,1 g d'oxalate de diméthyle, laisse 1 heure sous agitation à TA puis chauffe à 50°C pendant 1 heure. Après refroidissement à TA, on ajoute une solution d'HCl 2,4N, extrait à l'AcOEt, lave la phase organique à l'eau, sèche sur MgSO₄ et évapore le solvant sous vide. On obtient 0,46 g du composé attendu que l'on utilise tel quel.

### Préparation 1.13

### 3-(2,4-Dichlorophényl)-6-[3-(diméthylamino)-2-méthylprop-2-ènoyl]-1,9-diméthyl-1,9-dihydro-2H-pyrido[2,3-b]indol-2-one.

### A) 3-(2,4-Dichlorophényl)-1,9-diméthyl-6-propionyl-1,9-dihydro-2H-pyrido[2,3-b] indol-2-one.

On refroidit à 0°C une solution de 0,9 g du composé de l'étape B de la Préparation 1.2 dans 50 ml de DCM, ajoute, par petites portions, 1,34 g de AlCl₃ puis, goutte à goutte, 0,5 ml de chlorure de propionyle et laisse 2 heures sous agitation à 0°C. On verse le mélange réactionnel sur de la glace, extrait au DCM, sèche la phase organique sur Na₂SO₄ et évapore le solvant sous vide. On reprend le résidu dans un mélange EP/AcOEt (90/10 ; v/v) et essore le précipité formé On obtient 0,65 g du composé attendu.

### B) 3-(2,4-Dichlorophényl)-6-[3-(diméthylamino)-2-méthylprop-2-ènoyl]-1,9-diméthyl-1,9-dihydro-2H-pyrido[2,3-b]indol-2-one.

On chauffe à 100°C pendant 16 heures un mélange de 0,6 g du composé de l'étape précédente et 2 ml de réactif de Bredereck. Après refroidissement à TA, on rajoute de l'EP et essore le précipité formé. On obtient 0,63 g du composé attendu.

RMN¹H : DMSO-d₆ (300 MHz) : δ (ppm) : 2,04 : s : 3H ; 2,98 : s : 6H ; 4,02 : s : 3H ; 4,17 : s : 3H ; 6,89 : s : 1H ; 7,26 : d : 1H ; 7,44 : m : 2H ; 7,59 : d : 1H ; 7,68 : s : 1H ; 7,89 : s : 1H ; 8,29 : s : 1H.

### Préparation 1.14

### 1-[3-(2,4-Dichlorophényl)-1,9-diméthyl-2-oxo-2,9-dihydro-1H-pyrido[2,3-b] indol-6-yl]butane-1,3-dione.

On laisse 10 minutes sous agitation un mélange de 0,052 g de NaH à 60 % dans l'huile et 0,12 ml d'AcOEt dans 2 ml de THF puis ajoute une solution de 0,246 g du compose de l'étape E de la Préparation 1.10, 2 gouttes d'EtOH et 0,0035 g de dibenzo-18-crown-6 dans 2 ml de THF et chauffe à 80°C pendant 3 heures. Après refroidissement à TA, on ajoute une solution d'HCl 1N, extrait à l'AcOEt et évapore le solvant sous vide. On reprend le résidu au MeOH, essore le précipité formé et le lave au MeOH. On obtient 0,242 g du composé attendu.

RMN¹H : DMSO-d₆ (300 MHz) : δ (ppm) : 2,17 : s : 2H ; 2,25 : s : 1H ; 4,02 : s : 3H ; 4,20 : s : 3H ; 6,63 : s : 1H ; 7,40-7,52 : m : 2H ; 7,67-7,80 : m : 2H ; 7,85-7,95 : m : 1H ; 8,36 : d : 1H ; 8,62 : d : 1H.

### Préparation 1.15

### 3-(2,4-Dichlorophényl)-6-éthynyl-1,9-diméthyl-1,9-dihydro-2H-pyrido[2,3-b] indol-2-one.

### A) 3-(2,4-Dichlorophényl)-1,9-diméthyl-6-[(triméthylsilyl)éthynyl]-1,9-dihydro-2H-pyrido[2,3-b] indol-2-one.

On fait barboter de l'argon pendant 5 minutes dans une solution de 0,5g du composé de l'étape D de la Préparation 1.10, 0,161 ml de (triméthylsilyl)acétylène et 0,06,g de Pd (PPh₃)₄ dans 5 ml de pyrrolidine puis chauffe à 150°C dans un micro-ondes. On chromatographie le mélange réactionnel sur gel de silice en éluant par le mélange AcOEt/cyclohexane (70/30 ; v/v). On obtient 0,292 g du composé attendu.

### B) 3-(2,4-Dichlorophényl)-6-éthynyl-1,9-diméthyl-1,9-dihydro-2H-pyrido[2,3-b] indol-2-one.

A une solution de 0,29 g du composé de l'étape précédente dans 6,7 ml de THF, 1,7 ml de MeOH et 0,3 ml d'eau on ajoute 0,079 g de KOH et laisse 3 heures sous agitation à TA. On concentre sous vide, et chromatographie le résidu sur gel de silice en éluant par le mélange AcOEt / cychohexane (80/20 ; v/v). On triture le produit obtenu dans le MeOH, essore le précipité formé et le lave au MeOH. On obtiens 0,2 g du composé attendu, F =_{.} 184-185°C.

### Préparation 1.16

### Acide 3-(2,4-Dichlorophényl)-1,9-dimétbyl-2-oxo-2,9-dihydro-1H-pyrido[2,3-b] indole-6-carboxylique.

### A) 4-(2-Acétylhydrazino)benzoate de méthyle.

On dissout 5,5 g de 4-hydrazinobenzoate de méthyle dans 38,2 ml d'AcOH contenant 2,4 g d'acétate de sodium, on chauffe 18 heures à 80°C. On essore le minéral puis on évapore et on reprend par un minimum d'Et₂O. On essore pour obtenir 7,97 g du composé attendu.

### B) 4-(2-Acétyl-1,2-diméthylhydrazino)benzoate de méthyle.

On met en suspension 2,95 g de NaH à 95 % dans 90 ml de DMF et on ajoute goutte à goutte 8,135 g du composé de l'étape précédente en solution dans le minimum de DMF, puis après quelques minutes on ajoute goutte à goutte 9,75 ml d'iodure de méthyle. On agite 1 heure à TA. On verse le milieu sur une solution saturée de NH₄Cl et on extrait par AcOEt La phase organique est lavée par NaCl, séchée, évaporée pour donner 5,4 g du composé attendu.

### C) 1-Méthyl-2-(méthylamino)-1H-indole-5-carboxylate de méthyle.

On mélange 5,4 g du composé de l'étape précédente et 62 ml d'oxychlorure de phosphore et on chauffe 2 heures et demie à 80°C. On évapore le milieu et on reprend par AcOEt. Le solide formé est essoré, lavé par AcOEt, séché pour donner 4 g du composé attendu.

RMN MeOD (250 MHz) : 3,2 ppm : s : 3H ; 3,6 ppm : s : 3H ; 3,9 ppm : s : 3H ; 7,3-7,4 ppm : m : 2H ; 8,1-8,2 ppm : m : 2H.

### D) 3-(2,4-Dichlorophényl)-1,9-diméthyl-2-oxo-2,9-dihydro-1H-pyrido[2,3-b]indole-6-carboxylate de méthyle.

On chauffe pendant 2 heures à 100°C un mélange de 25,47 g du composé obtenu à l'étape précédente et 27,4 g de 2-(2,4-dichlorophényl)-3-(diméthylamino)acrylate de méthyle dans 100 ml d'acide acétique. On verse le mélange réactionnel chaud dans 500 ml d'un mélange eau/glace, essore le précipité formé, le lave à l'eau puis à l'éther et le sèche. On obtient 22 g du composé attendu.

### E) Acide 3-(2,4-dichlorophényl)-1,9-diméthyl-2-oxo-2,9-dihydro-1H-pyrido[2,3-b] indole-6-carboxylique.

A un mélange de 9,5 g du composé obtenu à la l'étape précédente dans 100 ml de MeOH on ajoute une solution de 5,14 g de KOH en pastilles dans 90 ml d'eau et chauffe à reflux pendant 18 heures. Après refroidissement à TA puis au bain de glace, on acidifie le mélange réactionnel à pH = 1 par ajout d'une solution d'HCl 5N, en diluant à l'eau pour permettre l'agitation. On essore le précipité formé, le lave à l'eau et le sèche. On obtient 9,1 g du composé attendu.

### Préparation 1.17

### 3-(2,4-Dichlorophényl)-1,9-diméthyl-2-oxo-N-prop-2-yn-1-yl-2,9-dihydro-1H-pyrido[2,3-b] indole-6-carboxamide.

On laisse une nuit sous agitation à TA un mélange de 0,2 g du composé de la Préparation 1.16, 0,038ml de propargylamine, 0,285 g de PyBOP et 0,096 ml de DIPEA dans 7 ml de DCM et 1,3 ml de DMF. On concentre le mélange réactionnel sous vide, reprend le résidu par une solution saturée de NH₄Cl extrait à l'ACOEt, sèche la phase organique sur MgSO₄ et évapore le solvant sous vide. On reprend le résidu dans un mélange MeOH / DCM et essore le précipité formé. On obtient 0,21 g du composé attendu.

### EXEMPLE 1 : Composé N° 1

### 3-(2,4-Dichlorophényl)-1-méthyl-6-(2-méthyl-1,3-thiazol-4-yl)-1,9-dihydro-2H-pyrido[2,3-b]indol-2-one.

A une solution de 0,5 g du composé de la Préparation 1.1 dans 3 ml de NMP on ajoute 0,08 g de thioacétamide puis chauffe à 150°C pendant 30 minutes. Après refroidissement à TA, on rajouta de l'eau et de l'AcOBt, décante, lave la phase organique par une solution saturée de NH₄Cl, par une solution saturée de NaCl, sèche sur Na₂SO₄ et évapore le solvant sous vide. On reprend le résidu dans un mélange

MeOH/EP (50/50 ; v/v), essore le précipité formé et le lave à l'AcOEt On obtient 0,21 g du composé attendu, F > 350°C (déc.).

RMN¹H : DMSO-d₆ (300 MHz) : δ (ppm) : 2,72 : s: 3H ; 3,70 : s : 3H ; 7,46-7,52 : m : 3H ; 7,69 : s : 1H ; 7,77-7,85 : m : 2H ; 8,25 : s : 1H ; 8,44 : s : 1H ; 12,21 : s : 1H.

### EXEMPLE 2 : Composé N° 2

### 6-(2-Amino-1,3-thiazol-4-yl)-3-(2,4-dichlorophényl)-1-méthyl-1,9-dihydro-2H-pyrido[2,3-b]indol-2-one.

A une solution de 0,2 g du composé de la Préparation 1.1 dans 7 ml de MeOH, on ajoute 0,04 g de thiourée puis chauffe à reflux pendant 18 heures. On essore le précipité formé, le triture dans une solution saturée de K₂CO₃, l'essore, le lave à l'eau puis par le mélange propan-2-ol/EP (50/50 ; v/v). On obtient 0,17 g du composé attendu.

RMN¹H : DMSO-d⁶ (300 MHz) : δ (ppm) : 3,71 : s : 3H ; 7,06 : s : 1H ; 7,43= 7,50 : m : 2H ; 7,57 : s : 2H ; 7,70 : s : 1H ; 8,19 : s : 1H ; 8,25 : s : 1H ; 8,60-8,85 : sb : 2H ; 12,40 : s : 1H.

### EXEMPLE 3 : Composé N° 3

### 3-(2,4-Dichlorophényl)-6-[2-(hydroxyméthyl)-1,3-thiazol-4-yl]-1-méthyl-1,9-dihydro-2H-pyrido[2,3-b]indol-2-one.

### A) Pivalate de [4-[3-(2,4-dichlorophényl)-1-méthyl-2-oxo-2,9-dihydro-1H-pyrido [2,3-b]indol-6-yl]1,3-thiazol-2-yl]méthyle.

A une solution de 0,33 g du composé de la Préparation 1.1 dans 15 ml d'EtOH, on ajoute 0,12 g de pivalate de 2-amino-2-thioxoéthyle et chauffe à reflux pendant 3 jours. On reprend le mélange réactionnel dans du DCM et du MeOH et chromatographie sur gel de silice en éluant par le mélange AcoEt/cyclohexane (75/25 ; v/v). On obtient 0,18 g du composé attendu.

### B) 3-(2,4-Dichlorophényl)-6-[2-(hydroxyméthyl)-1,3-thiazol-4-yl]-1-méthyl-1,9-dihydro-2H-pyrido[2,3-b]indol-2-one.

A une solution de 0,016 g. de sodium dans 10 ml de MeOH on ajoute une suspension de 0,18 g du composé de l'étape précédente dans 5 ml de MeOH et laisse 16 heures sous agitation à TA. On ajoute de l'eau, extrait à l'AcOEt, sèche la phase organique sur MgSO₄ et évapore le solvant sous vide. On chromatographie le résidu sur gel de silice en éluant par le mélange AcOEt/cyclohexane (90/10 ; v/v). On obtient 0,06 g du composé attendu.

RMN¹H : DMSO-d₆ (300 MHz) : δ (ppm) : 3,69 : s : 3H ; 4,80 : s : 2H ; 6,11 : m : 1H ; 7,46-7,52 : m : 3H ; 7,69 : s : 1H ; 7,82 : d : 1H ; 7,88 : s : 1H ; 8,25 : s : 1H ; 8,44 : s : 1H ; 12,20 : s : 1H.

### EXEMPLE 4 : Composé N° 4

### 3-(2,4-Dichlorophényl)-1-méthyl-6-(2-méthyl-1,3-oxazol-4-yl)-1,9-dihydro-2H-pyrido[2,3-b]indol-2-one.

A une solution de 0,2 g du composé de la Préparation 1.1 dans 2 ml de NMP on ajoute 0,064 g d'acétamide puis chauffe à 150°C pendant 2 heures. On ajoute de l'eau, extrait à l'AcOEt, sèche la phase organique sur Na₂SO₄ et évapore le solvant sous vide. On chromatographie le résidu sur gel de silice en éluant par le mélange AcOEt/cyclohexane (75/25 ; v/v). On reprend le produit obtenu dans 5 ml de MeOH, le triture, essore le précipité formé et le lave à l'EP. On obtient 0,12 g du composé attendu.

RMN¹H : DMSO-d₆ (300 MHz : δ (ppm) : 2,45 : s : 3H ; 3,67 : s : 3H ; 7,44-7,52 : m : 3H ; 7,60 : d :1H ; 7,67 : s : 1H ; 8,20-8,34 : m : 3H ; 12,18 : s : 1H.

### EXEMPLE 5 : Composé N° 5

### 3-(2,4-Dichlorophényl)-1-méthyl-6-(2-méthyl-1,3-oxazol-5-yl)-1,9-dihydro-2H pyrido[2,3-b]indol-2-one.

### A) Chlorhydrate de 6-(aminoacétyl)-3-(2,4-dichlorophényl)-1-méthyl-1,9-dihydro-2H-pyrido[2,3-b]indol-2-one.

On laisse 20 heures sous agitation à TA un mélange de 0,845 g du composé de la Préparation 1.1 et 0,255 g d'hexaméthylènetétramine dans 13 ml de chloroforme. On essore le précipité formé, le lave au DCM puis à l'éther. On reprend le précipité dans 3,5 ml d'EtOH, ajoute 0,395 ml d'HCl à 37 %, et chauffe à 50°C pendant 16 heures. On essore le précipité formé, le lave à l'eau et le sèche sous vide. On obtient 0,547 g du composé attendu.

### B) 3-(2,4-Dichlorophényl)-1-méthyl-6-(2-méthyl-1,3-oxazol-5-yl)-1,9-dihydro-2H pyrido[2,3-b]indol-2-one.

A une solution de 0,5 g du composté de l'étape précédente dans 10 ml d'orthoacétate de triéthyle on ajoute 6 mg d'acide *para*toluène sulfonique monohydrate puis chauffe à 140°C pendant 45 minutes. Après refroidissement à TA, on dissout le mélange réactionnel avec du DCM et chromatographie sur gel de silice en éluant à l'AcOEt puis par le mélange AcOEt/MeOH (97/3 ; v/v). On obtient 0,045 g du composé attendu, F = 338-340°C.

### EXEMPLE 6 : Composé N°6

### 3-(2,4-Dichlorophényl)-1,9-diméthyl-6-(2-méthyl-1,3-thiazol-4-yl)-1,9-dihydro-2H-pyrido[2,3-b]indol-2-one.

A une solution de 0,2 g du composé de la Préparation 1.2 dans 20 ml de MeOH on ajoute 0,047 g de thioacétamide puis chauffe à reflux pendant 48 heures. On essore le précipité formé et le lave à l'EP. On dissout le précipité dans du DCM et chromatographie sur gel de silice en éluant par le mélange DCM/MeOH (99/1 ; v/v). On obtient 0,13 g du composé attendu, F = 253-254°C.

RMN¹H : DMSO-d₆ (300 MHz) : δ (ppm) : 2,70 : s : 3H ; 3,99 : s : 3H ; 4,15 : s : 3H ; 7,44 : m : 2H ; 7,62-7,67 : m : 2H ; 7,79 : s : 1H ; 7,86 : d : 1H ; 8,26 : s : 1H ; 8,43 : s : 1H.

### EXEMPLE 7 : Composé N°7

### 3-(2,4-Dichlorophényl)-1,9-diméthyl-6-[2-(méthylamino)-1,3-thiazol-4-yl)-1,9-dihydro-2H-pyrido[2,3-b]indol-2-one.

A une solution de 0,2 g du composé de la Préparation 1.2 dans 10 ml de MeOH on ajoute 0,045 g de N-méthylthiourée puis chauffe à reflux pendant 16 heures. On essore le précipité formé et le lave par un mélange propan-2-ol/EP (50/50 ; v/v). On obtient 0,19 g du composé attendu.

RMN¹H : DMSO-d₆ (300 MHz) : δ (ppm) : 3,03 : s : 3H ; 4,03: s : 3H ; 4,20 : s : 3H ; 7,08 : s : 1H ; 7,42-7,48 : m : 2H ; 7,70-7,72 : m : 3H ; 8,23 : s : 1H ; 8,30 : s : 1H.

### EXEMPLE 8 : Composé N° 8

### Pivalate de [4-[3-(2,4-dichlorophényl)-1,9-diméthyl-2-oxo-2,9-dihydro-1H-pyrido[2,3-b]indol-6-yl]-1,3-thiazol-2-yl]méthyle.

A une solution de 0,45 g du composé de la Préparation 1.2 dans 20 ml d'EtOH on ajoute 0,25 g de pivalate de 2-amino-2-thioxoéthyle et chauffe à reflux pendant 3 jours. On essore le précipité formé et le lave par un mélange MeOH/EP (50/50 ; v/v). On dissout le précipité dans du DCM et chromatographie sur gel de silice en éluant par le mélange DCM/MeOH (99/1 ; v/v). On reprend le produit obtenu dans EP et essore le précipité. On obtient 0,4 g du composé attendu, F = 193-195°C.

RMN¹H : DMSO-d₆ (300 MHz) : δ (ppm) :1,23 ; s : 9H ; 4,02: s : 3H ; 4,18 : s : 3H ; 5,46 : s : 2H ; 7,47 : s : 2H; 7,68 : s : 2H ; 7,89 : m : 1H ; 8,04 : s : 1H ; 8,28 : s : 1H ; 8,48 : s : 1H.

### EXEMPLE 9 : Composé N° 9

### 3-(2,4-Dichlorophényl)-6-[2-(hydroxyméthyl)-1,3-thiazol-4-yl]-1,9-diméthyl-1,9-dihydro-2H-pyrido[2,3-b]indol-2-one.

A une solution de 0,017 g de sodium dans 20 ml de MeOH on ajoute 0,2 g du composé N° 8 et laisse 6 heures sous agitation à TA. On essore le précipité formé, le lave à l'eau, par un mélange propan-2-ol/EP (50/50 ; v/v) puis au DCM. On obtient 0,16 g du composé attendu, F = 261-262°C.

RMN¹H : DMSO-d₆ (300 MHz) : δ (ppm) : 4,01 : s : 3H ; 4,17 : s : 3H ; 4,80 : s : 2H ; 6,12 : s : 1H ; 7,46 : s : 2H ; 7,64-7,69 :m : 2H ; 7,88-7,92 : m : 2H ; 8,28 : s : 1H ; 8,46 : s : 1H.

### EXEMPLE 10 : Composé N° 10

### 3-(2,4-Dichlorophényl)-6-[2-(éthoxyméthyl)-1,3-thiazol-4-yl]-1,9-diméthyl-1,9-dihyro-2H-pyrido[2,3-b]indol-2-one.

A une solution de 0,13 g du composé N° 9 dans 10 ml de DMF on ajoute 0,02 g de NaH à 60 % dans l'huile et laisse 30 minutes sous agitation à TA. On ajoute ensuite 0,05 ml d'iodoéthane et laisse 1 heure sous agitation à TA On verse le mélange réactionnel sur une solution saturée de NH₄Cl, extrait à l'AcOEt, lave la phase organique par une solution saturée de NaCl, sèche sur Na₂SO₄ et évapore le solvant sous vide. On chromatographie le résidu sur gel de silice en éluant à l'AcOEt. On reprend le produit obtenu à l'EP et essore le précipité formé. On obtient 0,1 g du composé attendu.

RMN¹H : DMSO-d₆ (300 MHz) : δ (ppm) : 1,20 : t : 3H ; 3,64 : q : 2H ; 4,02 : s : 3H ; 4,18 : s : 3H ; 4,81 : s : 2H ; 7,46 : s : 2H ; 7,65-7,69 : m : 2H ; 7.89 : d : 1H ; 7,99: s : 1H ; 8,28 : s: 1H; 8,47 : s: 1H.

### ExEMPLE 11 : Composé N° 11

### Cyclopropanecarboxylate de [4-[3-(2,4-dichlrophényl)-1,9-diméthyl-2-oxo-2,9-dihydro-1H-pyrido[2,3-b]indol-6-yl]-1,3-thiazol-2-yl]méthyle.

A une solution de 0,1 g du composé N° 9 dans 5 ml de pyridine on ajoute 0,1 ml de chlorure de cyclopropanecarbonyle et laisse 48 heures sous agitation à TA. On verse le mélange réactionnel dans l'eau, extrait à l'AcOEt, lave la phase organique par une solution saturée de NH₄Cl, sèche sur Na₂SO₄ et évapore le solvant sous vide. On chromatographie le résidu sur gel de silice en éluant par le mélange AcOEt/cyclohexane (90/10 ; v/v). On reprend le produit obtenu dans un mélange AcOEt/cyclohexane (10/90 ; v/v) et essore le précipité formé. On obtient 0,105 g du composé attendu.

RMN¹H : DMSO-d₆ (300 MHz) : δ (ppm) : 0,91-0,99 : m : 4H ; 1,77 : m : 1H ; 4,03 : s : 3H ; 4,18 : s : 3H ; 5,44 : s : 2H ; 7,47 : m : 2H ; 7,67-7,70 ; m : : 2H ; 7,90 : d :1H; 8,05 : s : 1H ; 8,29 : s : 1H ; 8,49 : s : 1H.

### EXEMPLE 12 : Composé N° 12

### 3-(2,4-Dichlorophényl)-1,9-diméthyl-6-(1,3-oxazol-4-yl)-1,9-dihydro-2H-pyrido[2,3-b]indol-2-one.

On chauffe à 160°C pendant 35 minutes une solution de 0,1 g du composé de la Préparation 1.2 dans 1 ml de formamide. Après refroidissement, on verse le mélange réactionnel dans une solution saturée de NaHCO₃, extrait au DCM, sèche la phase organique sur MgSO₄ et évapore le solvant sous vide. On chromatographie le résidu sur gel de silice en éluant à l'AcOEt On obtient 0,042 g du composé attendu, F = 221-222°C.

RMN¹H : DMSO-d₆ (300 MHz) : δ (ppm) : 4,01 : s : 3H ; 4,17 : s : 3H ; 7,43-7,50 : m : 2H ; 7,67-7,75 : m : 3H ; 8,25 : s : 1H ; 8,32 : s : 1H ; 8,47 : d : 1H : 8,55 : s : 1H.

### EXEMPLE 13 : Composé N° 13

### 3-(2,4-Dichlorophényl)-1,9-diméthyl-6-(2-méthyl-1,3-oxazoly-4-yl)-1,9-dihydro-2H-pyrido[2,3-b]indol-2-one.

A une solution de 0,3 g du composé de la Préparation 1.2 dans 2 ml de NMP on ajoute 0,22 g d'acétamide et chauffe à 150°C pendant 30 minutes. Après refroidissement, on extrait à l'AcOEt, lava la phase organique par une solution saturée de NH₄Cl, par une solution d'HCl 1N, sèche sur Na₂SO₄ et évapore le solvant sous vide. On chromatographie le résidu sur gel de silice en éluant par le mélange AcOEt/cyclohexane (90/10 ; v/v). On obtient 0,11 g du composé attendu, F.= 222-223°C.

RMN¹H : DMSO-d₆ (300 MHz) : δ (ppm) : 2,47 : s : 3H ; 4,01 : s : 3H ; 4,16 : s : 3H ; 7,46 : m : 2H ; 7,62-7,69 : m : 3H ; 8,25 : s : 1H ; 8,26 : s : 1H ; 8,38 : s : 1H.

### EXEMPLE 14 : Composé N° 14

### 6-(2-Amino-1,3-oxazol-4-yl)-3-(2,4-dichlorphényl)-1,9-diméthyl-1,9-dihydro-2H-pyrido[2,3-b]indol-2-one.

A une solution de 0,3 g du composé de la Préparation 1.2 dans 2 ml de NMP on ajoute 0,22 g d'urée et chauffe à 150°C pendant 30 minutes. Après refroidissement, on ajoute de l'eau et essore le précipité formé. On chromatographie le précipité dissout dans du MeOH sur gel de silice en éluant par le mélange AcOEt/cyclohexane (75/25 ; v/v). On obtient 0,04 g du composé attendu, F = 295-297°C.

RMN¹H : DMSO-d₆ (300 MHz) : δ (ppm) : 3,95 : s : 3H ; 4,10 : s : 3H ; 6,62 : s : 2H ; 7,37-7,43 : m : 2H ; 7,51 : s : 2H ; 7,63 : s : 1H ; 7,75 : s : 1H ; 8,06 : s : 1H ; 8,13 : s : 1H

### EXEMPLE 15 : Composé N° 15

### 3-(2,4-Dichlorophényl)-6-[2-(hydroxyméthy)-1,3-oxazol-4-yl]-1,9-diméthyl-1,9-dihydro-2H-pyrido[2,3-b]indol-2-one.

A une solution de 0,2 g du composé de la Préparation 1.2 dans 2 ml de NMP on ajoute 0,39 g de 2-(tétrahydro-2*H*-pyran-2-yloxy)acétamida et chauffe à 150°C pendant 1 heure 30 minutes. On verse le mélange réactionnel dans l'eau et essore le précipité formé. On chromatographie le précipité dissout dans un mélange AcOEt/DCM sur gel de silice en éluant à l'AcOEt On reprend le produit obtenu dans le mélange AcOEt/cyclohexane (10/90 ; v/v) et essore le précipité. On obtient 0,035 g du composé attendu.

RMN¹H : DMSO-d₆ (300 MHz) : δ (ppm) : 4,02 : s : 3H ; 4,18 : s : 3H ; 4,56 : d : 2H ; 5,72 : t : 1H ; 7,47 : m : 1H ; 7,69 : m : 3H ; 8,26 : s : 1H ; 8,30 : s : 1H ; 8,49 : s : 1H.

### EXEMPLE 16 : Composé N° 16

### 3-(2,4-Dichlorophényl)-6-[2-(éthoxyméthyl)-1,3-oxazol-4-yl]-1,9-diméthyl-1,9-dihydro-2H-pyrido[2,3-b]indol-2-one.

A une solution de 0,12 g du composé N° 15 dans 4 ml de DMF on ajoute 0,032 g de NaH à 60 % dans l'huile et laisse 30 minutes sous agitation à TA. On ajoute ensuite 0,06 ml d'iodoéthane et laisse 1 heure sous agitation à TA. On verse le mélange réactionnel dans l'eau, extrait à l'AcOEt, lave la phase organique par une solution saturée de NH₄Cl, sèche sur Na₂SO₄ et évapore le solvant sous vide. On reprend le résidu dans un mélange AcOEt/EP (20/80 ; v/v) et essore le précipité formé. On obtient 0,07 g du composé attendu.

RMN¹H : DMSO-d₆ (300 MHz) : δ (ppm) : 1,15 : t : 3H ; 3,56 : q : 2H ; 4,02 : s : 3H ; 4,17: s : 3H ; 4,58 : s : 2H ; 7,43-7,50: m : 2H ; 7,67 : m : 3H ; 8,27 : s : 1H ; 8,31 : s : 1H ; 8,53 : s : 1H.

### EXEMPLE 17 : Composé N° 17

### Pivalate de [4-[3-(2,4-dichlorophényl)-1,9-diméthyl-2-oxo-2,9-dihydro-1H-pyrido[2,3-b]indol-6-yl]-1,3-oxazol-2-yl]méthyle.

A une solution de 0,1 g du composé N° 15 dans 4 ml de pyridine on ajoute 0,14 ml de chlorure de pivaloyle et laisse 2 heures sous agitation à TA. On ajoute de l'eau, extrait à l'AcOEt, lave la phase organique par une solution saturée de NH₄Cl,sèche sur Na₂SO₄ et évapore le solvant sous vide. On chromatographie le résidu sur gel de silice en éluant par le mélange AcOEt/cyclohexane (75/25 ; v/v). On reprend le produit obtenu dans un mélange éther/EP et essore le précipité formé. On obtient 0,07 g du composé attendu.

RMN¹H : DMSO-d₆ (300 MHz) : δ (ppm) : 1,19 : s : 9H ; 4,02 : s : 3H ; 4,18 : s : 3H ; 5,24 : s : 2H ; 7,43-7,50 : m : 2H ; 7,69 : s : 3H ; 8,26 : s : 1H ; 8,56 : s : 1H.

### EXEMPLE 18 : Composé N° 18

### 3-(2,4-Dichlorophényl)-1,9-diméthyl-6-(2-méthyl-1,3-oxazol-5-yl)-1,9-dihydro-2H-pyrido[2,3-b]indol-2-one.

### A) Chlorhydrate de 6-(aminoacétyl)-3-(2,4-dichlorophényl)-1,9-diméthyl-1,9-dihydro-2H-pyrido[2,3-b]indol-2-one.

On prépare ce composé selon le mode opératoire décrit à l'étape A de l'Exemple 5 à partir du composé de la Préparation 1.2.

### B) 3-(2,4-Dichlorophényl)-1,9-diméthyl-6-(2-méthyl-1,3-oxazol-5-yl)-1,9-dihydro-2H-pyrido[2,3-b]indol-2-one.

On prépare ce composé selon le mode opératoire décrit à l'étape B de l'Exemple 5, F = 239-240°C.

### EXEMPLE 19 : Composé N° 19

### 3-(2,4-Dichlorophényl)-6-[2-(éthoxyméthyl-1,3-oxazol-5-yl)-1,9-diméthyl-1,9-dihydro-2H-pyrido[2,3-b]indol-2-one.

### A) N-[2-[3-(2,4-Dichlorophényl)-1,9-diméthyl-2-oxo-2,9-dihydro-1H-pyrido[2,3-b]indol-6-yl]-2-oxoéthyl]-2-éthoxyacétamide.

On laisse 16 heures sous agitation à TA un mélange de 0,25 g du composé de l'étape A de l'Exemple 18, 0,058 ml d'acide éthoxyacétique, 0,318 g de PyBOP et 0,29 ml de DIPEA dans 3 ml de DCM. On ajoute une solution saturée de NH₄Cl, extrait an DCM, sèche la phase organique sur MgSO₄ et évapore le solvant sous vide. On chromatographie le résidu sur gel de silice en éluant à l'AcOEt puis par le mélange AcOEt/MoOH (95/5 ; v/v). On obtient 0,268 g du composé attendu.

### B) 3-(2,4-Dichlorophényl)-6-[2-(éthoxyméthyl)-1,3-oxazol-5-yl)-1,9-diméthyl-1,9-dihydro-2H-pyzido[2,3-b]indol-2-one.

On laisse 20 heures sous agitation à TA une solution de 0,268 g du composé de l'étape précédente dans 0,5 ml d'H₂SO₄ concentré. On ajoute de l'eau, extrait à I'AcOEt, sèche la phase organique sur MgSO₄ et évapore le solvant sous vide. On chromatographie sur gel de silice en éluant par le mélange AcOEt/cyclohexane (50/50 ; v/v) puis à l'AcoEt On obtient 0,04 g du composé attendu, F = 179-180°C.

### EXEMPLE 20 : Composé N° 20

### 6-(3-Amino-1H-pyrazol-5-yl)-3-(2,4-dichlorophényl)-1,9-diméthyl-1,9-dihydro-2H-pyrido[2,3-b]indol-2-one.

### A) 3-[3-(2,4-Dichlorophényl)-1,9-diméthyl-2-oxo-2,9-dihydro-1H-pyrido[2,3-b] indol-6-yl]-2-oxopropanenitrile.

A une solution de 0,7 g du composé de la Préparation 1.2 dans 11 ml d'EtOH et 2 ml d'eau on ajoute 0,19 g de KCN et chauffe à reflux pendant 4 heures. On essore le précipité formé, le dissout dans un mélange MeOH/AcOEt et chromatographie sur gel de silice en éluant par le mélange AcOEt/cyclohexane (75/25 ; v/v) puis à l'AcOEt. On obtient 0,2 g du composé attendu.

### B) 6-(3-Amino-1H-pyrazol-5-yl)-3-(2,4-dichlorophényl)-1,9-diméthyl-1,9-dihydro-2H-pyrido[2,3-b]indol-2-one.

A une solution de 0,2 g du composé de l'étape précédente dans 10 ml d'EtOH on ajoute 0,2 ml d'hydrazine monohydrate et chauffe à reflux pendant 16 heures. On chromatographie le mélange réactionnel sur gel de silice en éluant à l'AcOEt puis par le mélange AcOBt/MeOH/NH₃ (90/10/0,2 ; v/v/v). On obtient 0,05 g du composé attendu, F = 284-286°C.

### EXEMPLE 21 : Composé N° 21

### 6-(2-Amino-1,3-thiazol-4-yl)-3-(2,4-dichlorophényl)-1,9-diméthyl-1,9-dihydro-2H-pyrido[2,3-b]indol-2-one.

A une solution de 0,25 g du composé de la Préparation 1.3 dans 12 ml de MeOH on ajoute 0,053 g de thiourée et chauffe à reflux pendant 16 heures. On essore le précipité formé, le lave avec une solution saturée de K₂CO₃, au MeOH, avec un mélange AcOEt/EP (50/50 ; v/v). On obtient 0,18 g du composé attendu, F = 272-273°C.

### EXEMPLE 22 : Composé N° 22

### 3-(2,4-Dichlorophényl)-1-méthyl-6-(1,3-thiazol-2-yl)-1,9-dihydro-2H-pyrido[2,3-b]indol-2-one.

A une solution de 0,25 g du composé de la Préparation 1.4 dans 10 ml de NMP on ajoute 0,37 g de Cu₂O puis dégaze le mélange réactionnel pendant 10 minutes. On ajoute ensuite 0,055 g d'acétate de palladium (II), 0,2 g de 1,3-bis(diphénylphosphino) propane et 1 g de 2-(tributylstannyl)-1,3-thiazole puis chauffe à 100°C pendant 16 heures. Après refroidissement à TA, on verse le mélange réactionnel sur une solution d'AcOH à 30 %, extrait à l'AcOEt, sèche la phase organique sur MgSO₄ et évapore le solvant sous vide. On chromatographie le résidu sur gel de silice en éluant à l'AcOEt. On reprend le produit obtenu dans l'AcOEt et essore le précipité formé. On obtient 0,06 g du composé attendu.

RMN¹H : DMSO-d₆ (300 MHz) : δ (ppm) : 3,71 : s : 3H ; 7,46-7,88 : m : 7H ; 8,37 : s : 1H ; 8,50 : s : 1H ; 12,37 : s : 1H.

### EXEMPLE 23 : Composé N° 23

### 3-(2,4-Dichlorophényl)-1,9-diméthyl-6-(1,3-thiazol-2-yl)-1,9-dihyro-2H-pyrido[2,3-b]indol-2-one.

A une suspension de 0,35 g de NaH à 60 % dans l'huile dans 10 ml de DMF on ajoute 0,25 g du composé N° 22 et laisse sous agitation à TA jusqu'à arrêt du dégagement gazeux. On ajoute ensuite 0,07 ml d'iodure de méthyle et laisse 1 heure sous agitation à TA. On ajoute de l'eau, extrait à l'AcOEt, sèche la phase organique sur MgSO₄ et évapore le solvant sous vide. On chromatographie le résidu sur gel de silice en éluant à l'AcOEt. On reprend le produit obtenu dans un mélange AcOEt/cyclohexane (25/75 ; v/v), essore le précipité formé et le lave à l'HP.

RMN¹H : DMSO-d₆ (300 MHz) : 8 (ppm) : 4,04 : s : 3H ; 4,20 : s : 3H ; 7,43-7,51 : m : 2H ; 7,69-7,75 : m : 3H ; 7,88-7,94 : m : 2H ; 8,41 : s : 1H ; 8,51 : s : 1H.

### EXEMPLE 24 : Composé N° 24

### 3-(2,4-Dichlorophényl)-1,9-diméthyl-6-(4-méthyl-1,3-oxazol-2-yl)-1,9-dihydro-2H-pyrido[2,3-b]indol-2-one.

On refroidit à -15°C 10 ml d'H₂SO₄ concentré, ajoute 0,6 g du composé de la Préparation 1.5 et 0,092 ml d'alcool propargylique et laisse 3 heures sous agitation à 0°C puis une nuit à TA. On verse le mélange réactionnel sur un mélange glace/H₂O, ajoute Na₂CO₃ jusqu'à pH = 7, extrait à l'AcOEt, lave la phase organique par une solution saturée de NaCl, sèche sur MgSO₄ et évapore le solvant sous vide. On chromatographie le résidu sur gel de silice en éluant à l'AcOEt puis par le mélange AcOEt/MeOH (99/1 ; v/v). On reprend le produit obtenu dans le mélange propan-2-ol/EP (50/50 ; v/v) et essore le précipité formé. On obtient 0,015 g du composé attendu, F = 258-261°C.

### EXEMPLE 25 : Composé N° 25

### 3-(2,4-Dichlorophényl)-1,9-diméthyl-6-(4-méthyl-1,3-thiazol-2-yl)-1,9-dihydro-2H-pyrido[2,3-b]indol-2-one.

Dans un tube scellé, on chauffe à 80°C pendant une nuit un mélange de 0,2 g du composé de la Préparation 1.6 et 3 ml de chloroacétone. Après refroidissement à TA, on verse le mélange réactionnel sur une solution saturée de NaHCO₃, essore le précipité formé, le lave à l'eau puis avec le mélange propan-2-ol/EP (50/50 ; v/v). On obtient 0,175 g du composé attendu, F = 255-257°C.

### EXEMPLE 26 : Composé N° 26

### 6-(4-Amino-1,3-thiazol-2-yl)-3-(2,4-dichlorophényl)-1,9-diméthyl-1,9-dihydro-2H-pyrido[2,3-b]indol-2-one.

On chauffe à reflux pendant une nuit un mélange de 0,2 g du composé de la Préparation 1.6 et 0,04 ml de bromoacétonitrile dans 5 ml d'acétonitrile. Après refroidissement à TA, on ajoute du MeOH et concentre sous vide. On chromatographie le résidu sur gel de silice en éluant par le mélange AcOEt/cyclohexane (90/10 ; v/v). On reprend le produit obtenu dans un mélange EP/MeOH/éther et essore le précipité formé. On obtient 0,06 g du composé attendu, F > 300°C (déc.).

RMN¹H : DMSO-d₆ (300 MHz) : δ (ppm) : 4,02 : s : 3H ; 4,18 : s : 3H ; 5,39 : s : 2H ; 5,87 : s : 1H ; 7,43-7,50 : m : 2H ; 7,60-7,70 : m : 3H ; 8,35-8,39 : m : 2H.

### EXEMPLE 27 : Composé N° 27

### 6-[4-(Chlorométhyl)-1,3-thiazol-2-yl]-3-(2,4-dichlorophényl)-1,9-diméthyl-1,9-dihydro-2H-pyrido[2,3-b]indol-2-one.

A une suspension de 0,2 g du composé de la Préparation 1.6 dans 6 ml de 1,4-dioxane on ajoute 0,073 g de 1,3-dichloroacétone et chauffe à reflux pendant une nuit. Après refroidissement à TA, on concentre sous vide, reprend le résidu dans l'eau, puis avec un mélange éther/MeOH (50/50 ; v/v), à l'éther et essore le précipité formé. On chromatographie le précipité sur gel de silice en éluant par le mélange DCM/MeOH (99/1 ; v/v). On reprend le produit obtenu dans l'éther et essore. On obtient 0,145 g du composé attendu, F = 277-281°C (déc.).

### EXEMPLE 28 : Composé N° 28

### 6-[4-(Aminométhyl)-1,3-thiazol-2-yl]-3-(2,4-dichlorophényl)-1,9-diméthyl-1,9-dihydro-2H-pyrido[2,3-b]indol-2-one.

### A) 6-[4-(Azidométhyl)-1,3-thiazol-2-yl]-3-(2,4-dichlorophényl)-1,9-diméthyl-1,9-dihydro-2H-pyrido[2,3-b]indol-2-one.

A une solution de 0,4 g du composé N° 27 dans 15 ml de DMSO on ajoute 0,16 g de NaN₃ et chauffe à 50°C pendant une nuit. On verse le mélange réactionnel dans l'eau, et essore le précipité formé. On reprend le précipité dans un mélange AcOEt/MeOH et concentre les solvants sous vide. On triture le résidu dans un mélange éther/MeOH et essore le précipité formé. On obtient 0,33 g du composé attendu.

### B) 6-[4-(Aminométhyl)-1,3-thiazol-2-yl]-3-(2,4-dichlorophényl)-1,9-diméthyl-1,9-dihydro-2H-pyrido[2,3-b]indol-2-one.

On hydrogène pendant 24 heures, à TA et sous 4 bars de pression, un mélange de 0,33 g du composé de l'étape précédente et 0,033 g de Pd/C à 10 % dans 10 ml de MeOH et 40 ml d'acide acétique. On filtre le catalyseur, lave au MeOH et concentre sous vide le filtrat. On reprend le résidu par une solution saturée de K₂CO₃, extrait à l'AcOEt, sèche la phase organique sur Na₂SO₄ et évapore le solvant sous vide. On chromatographie le résidu sur gel de silice en éluant par 18 mélange DCM/MeOH/triéthylamine (95/5/1 ; v/v/v). On triture le produit obtenu dans l'éther et essore le précipité formé. On obtient 0,12 g du composé attendu, F = 261-266°C.

### EXEMPLE 29 : Composé N° 29

### 3-(2,4-Dichlorophényl)-1,9-diméthyl-6-[4-(pyrrolidin-1-ylméthyl)-1,3-thiazol-2-yl]-1,9-dihydro-2H-pyrido[2,3-b]indol-2-one.

A une solution de 0,1 g du composé N° 27 dans 3 ml de DMF on ajoute 0,068 g de K₂CO₃ et 0,021 ml de pyrrolidine et chauffe à 80°C pendant une nuit. Après refroidissement à TA, on verse le mélange réactionnel sur une solution d'HCl 1N, lave la phase aqueuse acide à l'AcOEt, alcalinise par ajout d'une solution de NaOH 2N, extrait à l'AcOEt, sèche la phase organique sur Na₂SO₄ et évapore le solvant sous vide. On triture le résidu dans l'eau, essore le précipité formé, le reprend dans du MeOH et concentre le solvant sous vide. On dissout le résidu dans un mélange MeOH/pyridine et chromatographie sur gel de silice en éluant par le mélange AcOEt/MoOH/NH₄OH 28 % (89/10/1 ; v/v/v). On triture le produit obtenu dans l'éther et essore le précipité formé. On obtient 0,03 g du composé attendu, F = 222-226°C.

### EXEMPLE 30 : Composé N° 31

### [2-[3-(2,4-Dicblorophényl)-1,9-diméthyl-2-oxo-2,9-dihydro-1H-pyrido[2,3-b] indol-6-yl]-1,3-thiazol-4-yl]acétate de méthyle.

A une suspension de 0,16 g du composé de la Préparation 1.6 dans 6 ml de 1,4-dioxane on ajoute 0,07 g de 4-chloro-3-oxobutanoate de méthyle et chauffe à reflux pendant une nuit. Après refroidissement à TA, on concentre le mélange réactionnel sous vide, triture le résidu à l'eau, puis avec un mélange EP/propan-2-ol (50/50 ; v/v), puis avec un mélange éther/MeOH (50/50 ; v/v), puis à l'éther et essore le précipité formé. On obtient 0,145 g du composé attendu, F = 198-201°C.

### EXEMPLE 31 : Composé N° 32

### Acide [2-[3-(2,4-dichlorophényl)-1,9-diméthyl-2-oxo-2,9-dihydro-1H-pyrido[2,3-b]indol-6-yl]-1,3-thiazol-4-yl]acétique.

A une solution de 0,345 g du composé N° 31 dans 10 ml de MeOH 3,5 ml d'eau on ajoute 0,168 g d'hydroxyde de lithium monohydrate et chauffe à reflux pendant 48 heures. Après refroidissement à TA, on verse le mélange réactionnel sur une solution de NaOH 2N, lave la phase aqueuse à l'AcOEt, acidifie par ajout d'une solution d'HCl 6N, extrait à l'AcOEt, sèche la phase organique sur Na₂SO₄ et évapore le solvant sous vide. On chromatographie le résidu sur gel de silice en éluant par le mélange AcOEt/MeOH/AcOH (94,5/5/0,5 ; v/v/v) puis (89/10/1 ; v/v/v). On obtient 0,09 g du composé attendu, F = 234-239°C.

### EXEMPLE 32 : Composé N° 33

### 3-(2,4-Dichlorophényl)-1,9-diméthyl-6-(1,3-oxazol-5-yl)-1,9-dihydro-2H-pyrido[2,3-b]indol-2-one.

A une solution de 0,145 g du composé de la Préparation 1.7 dans 4 ml de MeOH on ajoute 0,073 g de 1-[(isocyanométhyl)sulfonyl]-4-méthylbenzène et 0,062 g de K₂CO₃ puis chauffe à reflux pendant 3 heures. On concentre le mélange réactionnel sous vide, reprend le résidu dans l'eau, essore le précipité formé et le lave à l'eau. On chromatographie le précipité sur gel de silice en éluant par le mélange AcOEt/cyclohexane (80/20 ; v/v) puis à l'AcOEt On reprend le produit obtenu dans un mélange MeOH/DCM, essore le précipité formé et le lave au MeOH. On obtient 0,048 g du composé attendu, F = 170-174°C.

### EXPMPLE 33 : Composé N° 34

### 6-(3-Amino-1H-pyrazol-4-yl)-3-(2,4-dichlorophényl)-1,9-diméthyl-1,9-dihydro-2H-pyrido[2,3-b]indol-2-one.

A une solution de 0,15 g du composé de la Préparation 1.8 dans 5 ml de MeOH, on ajoute 0,044 ml d'hydrazine monohydrate puis 0,029 ml d'acide acétique et chauffe à reflux pendant une nuit. On concentre le mélange réactionnel sous vide, dissout le résidu dans un mélange DCM/AcOEt et chromatographie sur gel de silice en éluant à l'AcOEt, par le mélange AcOEt/MeOH (95/5 ; v/v) puis par le mélange AcOEt/MeOH/triéthylamine (90/10/2 ; v/v/v). On triture le produit obtenu dans l'éther et essore le précipité formé. On obtient 0,05 g du composé attendu, F = 240-245°C**.**

### EXEMPLE 34 : Composé N° 35

### 3-(2,4-Dichlorophényl)-6-isoxazol-5-yl-1-méthyl-1,9-dihydro-2H-pyrido[2,3-b] indol-2-one.

### A) 3-(2,4-Dichlorophényl)-6-isoxazol-5-yl-1-méthyl-9-[[2-(triméthylsilyl)éthoxy] méthyl]-1,9-dihydro-2H-pyrido[2,3-b]indol-2-one.

On chauffe à reflux pendant 3 heures une solution de 0,236 g du composé de la Préparation 1.9 et 0,043 g de chlorhydrate d'hydroxylamine dans 3 ml de MeOH. Après refroidissement à TA, on ajoute de l'EP, essore le précipité formé, le lave à l'éther et le sèche. On obtient 0,147 g du composé attendu.

### B) 3-(2,4-Dichlorophényl)-6-isoxazol-5-yl-1-méthyl-1,9-dihydro-2H-pyrido[2,3-b] indol-2-one.

On chauffe à 100°C pendant 3 jours un mélange de 0,147 g du composé de l'étape précédente, 10 ml de HCl 3M et 5 ml de THF. Après refroidissement à TA, on alcalinise par ajout d'une solution de NaOH 1N, extrait à l'AcOEt, et concentre la phase organique sous vide. On chromatographie le résidu sur gel de silice en éluant par le mélange cyclohexane/AcOEt (50/50 ; v/v), à l'AcOEt puis par le mélange AcOEt/MeOH (95/5 ; v/v). On reprend le produit obtenu dans le MeOH, essore le précipité formé et le lave du MeOH et à l'éther. On obtient 0,053 g du composé attendu, F = 320-323°C.

### EXEMPLE 35: Composé N° 36

### 3-(2,4-Dichlorophényl)-1-méthyl-6-(1H-pyrazol-5-yl)-1,9-dihydro-2H-pyrido [2,3-b]indol-2-one.

### A) 3-(2,4-Dichlorophényl)-1-méthyl-6-(1H-pyrazol-5-yl)-9-[[2-(triméthylsilyl) éthoxy]méthyl]-1,9-dihydro-2H-pyrido [2,3-b]indol-2-one.

On chauffe à reflux pendant 24 heures une solution de 0,208 g du composé de la Préparation 1.9 et 0,068 ml d'hydrazine monohydrate dans 4 ml de MeOH. Après refroidissement à TA, on ajoute de l'EP, essore le précipité formé, le lave à l'EP et le sèche. On obtient 0,149 g du composé attendu.

### B) 3-(2,4-Dichlorophényl)-1-méthyl-6-(1H-pyrazol-5-yl)-1,9-dihydro-2H-pyrido [2,3-b]indol-2-one.

On chauffe à 100°C pendant 2 jours un mélange de 0,098 g du composé de l'étape précédente, 6,7 ml de HCl 3M et 3,3 ml de THF. Après refroidissement à TA, on alcalinise par ajout d'une solution de NaOH 1N, extrait à l'AcOEt et concentre la phase organique sous vide. On reprend le résidu au MeOH, essore le précipité formé, le lave an MeOH puis à l'éther. On obtient 0,02 g du composé attendu, F = 246-250°C.

### EXEMPLE 36 : Composé N° 37

### 3-(2,4-Dichlorophényl)-1-méthyl-6-(1-méthyl-1H-pyrazol-3-yl)-1,9-dihydro-2H-pyrido [2,3-b]indol-2-one

### A) 3-(2,4-Dichlorophényl)-1-méthyl-6-(1-méthyl-1H-pyrazol-3-yl)-9-[[2-(triméthylsilyl)éthoxy]méthyl]-1,9-dihydro-2H-pyrido [2,3-b]indol-2-one.

A une solution de 0,099 g du composé de l'étape A de l'Exemple 35 dans 2 ml de DMF on ajoute 0,011 g de NaH à 60 % dans l'huile puis 0,017 ml de iodométhane et laisse une nuit sous agitation à TA. On ajoute une solution saturée de NaHCO₃, extrait à l'AcOBt, sèche la phase organique sur MgSO₄ et évapore le solvant sous vide. On chromatographie le résidu sur gel de silice en éluant par le mélange AcOEt/cyclohexane (60/40 ; v/v) puis à l'AcOEt. On obtient 0,111 g du composé attendu.

### B) 3-(2,4-Dichlorophényl)-1-méthyl-6-(1-méthyl-1H-pyrazol-3-yl)-1,9-dihydro-2H-pyrido [2,3-b]indol-2-one.

On chauffe à 100°C pendant 4 jours un mélange de 0,111 g du composé de l'étape précédente, 7,4 ml de HCl 3M et 3,7 ml de THF. Après refroidissement à TA, on alcalinise par ajout de NaOH 1N, extrait à l'AcOEt, sèche la phase organique sur MgSO₄ et évapore le solvant sous vide. On chromatographie le résidu sur gel de silice en éluant par le mélange AcOEt/cyclohexane (80/20 ; v/v) puis à l'AcOEt. On obtient 0,028 g du composé attendu.

RMN¹H: DMS0-d₆ (400 MHz) : 3,7 s : 3H ; 3,95 : s : 3H ; 6,65 : s : 1H ; 7,40-7,50 : m : 2H ; 7,55-7,70 ; m : 3H ; 7,75 : d : 1H ; 8,15 : s : 1H ; 8,25 : s : 1H ; 10,70 : se :1H.

### EXEMPLE 37 : Composé N° 38

### 3-(2,4-Dichlorophényl)-6-(1-éthyl-1H-pyrazol-5-yl)-1-méthyl-1,9-dihydro-2H-pyrido [2,3-b]indol-2-ono.

### A) 3-(2,4-Dichlorophényl)-6-(1-éthyl-1H-pyrazol-5-yl)-1-méthyl-9-[[2-(triméthylsilyl)éthoxy]méthyl]-1,9-dihydro-2H-pyrido [2,3-b]indol-2-one.

On chauffe à reflux pendant une nuit une solution de 0,15 g du composé de la Préparation 1.9 et 0,118 g d'oxalate d'éthylhydrazine dans 5 ml de MeOH. Après refroidissement à TA, on ajoute une solution saturée de NaHCO₃, extrait au DCM, sèche la phase organique sur MgSO₄ et évapore le solvant sous vide. On obtient 0,158 g du composé attendu.

### B) 3-(2,4-Dichlorophényl)-6-(1-éthyl-1H-pyrazol-5-yl)-1-méthyl-1,9-dihydro-2H-pyrido [2,3-b]indol-2-one.

On chauffe à 100°C pendant 3 jours un mélange de 0,158 g du composé de l'étape précédente, 10 ml de HCl 3M et 5 ml de THF. Après refroidissement à TA, on alcalinise par ajout de NaOH 1N, extrait à l'AcOEt, sèche la phase organique sur MgSO₄ et évapore le solvant sous vide. On chromatographie le résidu sur gel de silice en éluant par le mélange AcOEt/cyclohexane (80/20 ; v/v). On reprend le produit obtenu dans du MeOH et laisse une nuit en cristallisation. On essore le produit cristallisé formé, le lave au MeOH puis à l'éther. On obtient 0,045 g du composé attendu, F = 255-258°C.

### EXEMPLE 38 : Composé N° 39

### 3-(2,4-Dichlorophényl)-6-isoxazol-5-yl-1,9-diméthyl-1,9-dihydro-2H-pyrido[2,3-b]indol-2-one.

On laisse 48 heures sous agitation à TA un mélange de 0,221 g du composé de la Préparation 1.10, 0,042 ml d'acide acétique et 0,051 g de chlorhydrate d'hydroxylamine dans 3 ml de MeOH. On concentre sous vide, extrait le résidu au DCM, lave la phase organique par une solution de NaHCO₃, à l'eau, par une solution saturée de NaCl, sèche sur MgSO₄ et évapore le solvant sous vide. On chromatographie le résidu sur gel de silice en éluant à l'AcOEt. On triture le produit obtenu dans un mélange MeOH/DCM/AcOEt et essore le précipité formé, F = 241-242°C.

### EXEMPLE 39 : Composé N° 40

### 3-(2,4-Dichlorophényl)-1,9-diméthyl-6-(1H-pyrazol-5-yl)-1,9-dihydro-2H-pyrido[2,3-b]indol-2-one.

On laisse 48 heures sous agitation à TA un mélange de 0,114 g du composé de la Préparation 1.10, 0,021 ml d'acide acétique et 0,018 ml d'hydrazine monohydrate dans 1 ml de MeOH. On concentre sous vide, extrait le résidu au DCM, lave la phase organique par une solution saturée de NaHCO₃, à l'eau par une solution saturée de NaCl, sèche sur MgSO₄ et évapore le solvant sous vide. On chromatographie le résidu sur gel de silice en éluant à l'AcOEt. On triture le produit obtenu dans du MeOH et essore le précipité formé. On obtient 0,027 g du composé attendu, F = 281-282°C.

### EXEMPLE 40 : Composé N° 41

### 3-(2,4-Dichlorophényl)-1,9-diméthyl-6-(1-méthyl-1H-pyrazol-3-yl)-1,9-dihydro-2H-pyrido[2,3-b]indol-2-one.

A une solution de 0,23 g du composé N° 40 dans 8 ml de DMF, on ajoute 0,033 g de NaH à 60 % dans l'huile et laisse 5 minutes sous agitation à TA. On ajoute ensuite 0,051 ml de iodométhane et laisse une nuit sous agitation à TA. On ajoute une solution saturée de NaHCO₃, extrait à l'AcOEt, sèche la phase organique sur MgSO₄ et évapore le solvant sous vide. On chromatographie le résidu sur gel de silice en éluant à l'AcOEt. On reprend le produit obtenu dans du MeOH, essore le précipité formé et le lave au MeOH puis à l'éther. On obtient 0,103 g du composé attendu.

RMN¹H : DMSO-d₆ (300 MHz) : δ (ppm) : 3,88 : s : 3H ; 4,01 : s : 3H ; 4,19 : s : 3H ; 6,68 : d : 1H ; 7,46 : m : 2H ; 7,61 : m : 1H ; 7,71 : m : 3H ; 8,28 : s : 2H.

### EXEMPLE 41 : Composé N° 42

### 3-(2,4-Dichlorophényl)-6-(1-éthyl-1H-pyrazol-3-yl)-1,9-diméthyl-1,9-dihydro-2H-pyrido[2,3-b]indol-2-one.

A une solution de 0,23 g du composé N° 40 dans 8 ml de DMF on ajoute 0,033 g de NaH à 60 % dans l'huile et laisse 5 minutes sous agitation à TA. On ajoute ensuite 0,065 ml d'iodoéthane et laisse une nuit sous agitation à TA. On ajoute une solution saturée de NaHCO₃, extrait à l'AcOEt, sèche la phase organique sur MgSO₄ et évapore le solvant sous vide. On chromatographie le résidu sur gel de silice en éluant à l'AcOEt On reprend le produit obtenu dans du MeOH, essore le précipité formé et le lave au MeOH puis à l'éther.

RMN¹H : DMSO-d₆ (300 MHz): δ (ppm) : 1,41 : t : 3H ; 4,02 : s : 3H ; 4,16 : m : 5H ; 6,68 : d : 1H ; 7,46 : m : 2H ; 7,61 : d : 1H ; 7,68 : d : 1H ; 7,76 : m : 2H ; 8,29 : d:2H.

### EXEMPLE 42 : Composé N° 43.

### 3-(2,4-Dichlorophényl)-1,9-diméthyl-6-(1,3-oxazol-2-yl)-1,9-dihydro-2H-pyrido[2,3-b]indol-2-one.

On chauffe à 170°C pendant une nuit un mélange de 0,2 g du composé de la Préparation 1.11, 0,047 g de 1,3-dioxol-2-one et 1 g d'H₃POO₄. Après refroidissement à TA, on ajoute de l'eau et de l'AcOEt et laisse 10 minutes sous agitation. Après décantation, on sèche la phase organique sur MgSO₄ et évapore le solvant sous vide. On chromatographie le résidu sur gel de silice en éluant à l'AcOEt. On obtient 0,062 g du composé attendu, F = 232-233°C.

### EXEMPLE 43 : Composé N°44

### 5-[3-(2,4-Dichlorophényl)-1,9-diméthyl-2-oxo-2,9-dihydro-1H-pyrido[2,3-b] indol-6-yl]-1H-pyrazole-3-carboxylate de méthyle.

### A) Acide 5-[3-(2,4-dichlorophényl)-1,9-diméthyl-2-oxo-2,9-dihydro-1H-pyrido[2,3-b]indol-6-yl]-1H-pyrazolo-3-carboxylique.

A une solution de 0,364 g du composé de la Préparation 1.12 dans 10 ml d'EtOH on ajoute 0,04 ml d'hydrazine monohydrate puis chauffe à reflux pendant 5 heures. On concentre le mélange réactionnel sous vide et chromatographie le résidu sur gel de silice en éluant à l'AcOEt, puis par le gradient du mélange AcOEt/MeOH/triéthylamine (45/45/10 ; v/v/v). On obtient 0,27 g du composé attendu que l'on utilise tel quel.

### B) 5-[3-(2,4-Dichlorophényl)-1,9-diméthyl-2-oxo-2,9-dihyaro-1H-pyrido[2,3-b] indol-6-yl]-1H-pyrazole-3-carboxylate de méthyle.

A une solution de 0,27 g du composé de l'étape précédente dans 5 ml de MeOH on ajoute 0,1 ml d'H₂SO₄ et chauffe à reflux pendant 12 heures. On concentre le mélange réactionnel sous vide, reprend le résidu dans un mélange pyridine/MeOH, triture, essore l'insoluble et le lave au MeOH. On chromatographie le filtrat sur gel de silice en éluant à l'AcOEt. On obtient 0,068 g du composé attendu, F = 290-292°C.

### EXEMPLE 44 : Composé N° 45

### 5-[3-(2,4-Dichlorophényl)-1,9-diméthyl-2-oxo-2,9-dihydro-1H-pyrido[2,3-b] indol-6-yl]isoxazole-3-catboxylate de méthyle.

A une solution de 0,364 g du composé de la Préparation 1.12 dans 5 ml de MeOH on ajoute 0,156 g de chlorhydrate d'hydroxylamine puis chauffe à reflux pendant 4 heures. On concentre le mélange réactionnel sous vide et chromatographie le résidu sur gel de silice en éluant à l'AcOEt. On reprend le résidu au MeOH, essore le précipité formé et le lave au MeOH puis à l'éther. On obtient 0,037 g du composé attendu, F = 280-281°C.

### EXEMPLE 45 : Composé N° 46

### 3-(2,4-Dichlorophényl)-1,9-diméthyl-6-(4-méthylisoxazol-5-yl)-1,9-dihydro-2H-pyrido[2,3-b]indol-2-one.

A une suspension de 0,3 g du composé de la Préparation 1.13 dans 7 ml de MeOH on ajoute 0,09 g de chlorhydrate d'hydroxylamine et laisse 48 heures sous agitation à TA. On essore le précipité formé et le lave par un mélange cyclohexane/AcoEt (75/25 ; v/v). On obtient 0,25 g du composé attendu, F = 233-234°C.

### EXEMPLE 46 : Composé N° 47

### 3-(2,4-Dichlorophényl)-1,9-diméthyl-6-(3-méthyl-1H-pyrazol-5-yl)-1,9-dihydro 2H-pyrido[2,3-b]indol-2-one.

On laisse une nuit sous agitation à TA une solution de 0,12 g du composé de la Préparation 1.14 et 0,109 g d'hydrazine monohydrate dans 3 ml de MeOH. On dissout le mélange réactionnel par ajout de DCM et de MeOH et chromatographie sur gel de silice en éluant à l'AcOEt puis par le mélange AcOEt/MeOH (95/5 ; v/v). On obtient 0,09 g du composé attendu, F = 309-310°C.

### EXEMPLE 47 : Composé N° 48

### 3-(2,4-Dichlorophényl)-1,9-diméthyl-6-(1H-1,2,3-triazol-4-yl)-1,9-dihydro-2H-pyrido[2,3-b]indol-2-one.

On chauffe au micro-ondes à 190°C pendant 30 minutes un mélange de 0,178 g du composé de la Préparation 1.15, 0,410 g de NaN₃ et 0,337 g de NH₄Cl dans 5 ml de DMF. On ajoute une solution saturée de NaHCO₃, extrait à l'AcOEt, sèche la phase organique sur MgSO₄ et évapore le solvant sous vide. On chromatographie le résidu sur gel de silice en éluant par le mélange AcOEt / cyclohexane (70/30;v/v). On reprend le produit obtenu dans un mélange DCM/MeOH et essore le précipité formé. On obtient 0,031 g du composé attendu, F = 274-275°C.

### EXEMPLE 48 : Composé N° 49

### 6-(5-Amino-1,3,4-thiadiazol-2yl)-3-(2,4-dichlorophényl)-1,9-diméthyl-1,9-dihydro-2H-pyrido[2,3-b]indol-2-one.

A une solution de 0,6 g du composé de la Préparation 1.16 dans 6 ml de POCl₃ on ajoute 0,15 g d'hydrazine carbothioamide puis chauffe à reflux pendant 3 heures. On refroidit le mélange réactionnel, ajoute goutte à goutte de l'eau jusqu'à formation d'un précipité puis chauffe à reflux pendant 2 heures. Après refroidissement à TA, on alcalinise le mélange par ajout de NaOH 1 N , essore le précipité, le reprend dans un mélange DCM / MeOH et chromatographie sur gel de silice en éluant par le mélange AcOEt / MeOH / NH₄OH (95/ 5/ 0,5;v/v/v). On obtient 0,065 g du composé attendu, F = 321-323°C.

### EXEMPLE 49 : Composé N° 50

### 3-(2,4-Dichlorophényl)-1,9-diméthyl-6-(5-méthyl-1,3-oxazol-2-yl)-1,9-dihydro-2H-pyrido[2,3-b]indol-2-one.

On chauffe à 120°C pendant 4 heures un mélange de 0,21 g du composé de la Préparation 1.17, 0,017 g d'acétate de mercure et 5,5 ml d'acide acétique. On concentre sous vide, reprend le résidu par une solution saturée de K₂CO₃ , extrait au DCM, sèche la phase organique sur MgSO₄ et évapore le solvant sous vide. On chromatographie le résidu sur gel de silice en éluant à l'AcOEt, puis par le mélange AcOEt / MeOH (99/1 ; v/v). On triture le produit obtenu dans du MeOH et essore le précipité formé. On obtient 0,076 g du composé attendu, F = 289-292°C.

### EXEMPLE 50: Composé N°30: le composé 30 du tableau est préparé comme indiqué pour l'exemple 29

### EXEMPLE 51: 3-(2,4-Dichloro-phenyl)-6-[1-(2-hydroxy-ethyl)-1H-pyrazol-3-yl]-1,9-dimethyl-1,9-dihydro-pyrido[2,3-b]indol-2-one

### A) composé A: 3-(2,4-Dichloro-phenyl)-1,9-dimethyl-6-{1-[2-(tetrahydro-pyran-2-yloxy)-ethyl]-1H-pyrazol-3-yl}-1,9-dibydro-pyrido[2,3-b]indol-2-one

On procède comme indiqué à l'exemple 36 ci-dessus à partir de 3-(2,4-Dichlorophenyl)-1,9-dimethyl-6-(5-methyl-2H-pyrazol-3-yl)-1,9- dihydro-pyrido[2,3-b]indol-2-one décrit ex 39 et de 2-(2-Bromo-ethoxy)-tetrahydro-pyrane (Collect. Czech. Chem. Commun.; EN; 69; 10; 2004; 1843 -1876).

RMN 1H DMSO d6 (300 MHz) : 1.30 - 1.51 (m, 4H); 1.51 - 1.75 (m, 2H); 3.60 (m, 1H); 3.78 (m, 1H) ; 3.95 - 4.10 (m, 4H) ; 4.15 (s, 3H); 4.20 (s, 1H); 4.32 (m, 2H) ; 4.56 (m, 1H); 7.40 - 7.50 (m, 3H); 7.57 - 7.65 (d, J = 8.63, 1H) ; 7.70 (d, J = 1.69, 1H); 7.75 - 7.85 (m, 2H); 8.29 (m, 2H).

### B) 3-(2,4-Dichloro-phenyl)-6-[1-(2-hydroxy-ethyl)-1H-pyrazol-3-yl]-1,9-dimethyl-1,9-dihydro-pyrido[2,3-b]indol-2-one

Chauffer à 45°C pendant 16h une solution contenant 311 mg (0.56mmol) de composé A 3-(2,4-Dichloro-phenyl)-1,9-dimethyl-6-{1-[2-(tetrahydro-pyran-2-yloxy)-ethyl]-1H-pyrazol-3-yl}-1,9-dihydro-pyrido[2,3-b]indol-2-one , 2.7 mL d'acide acétique, 1.3 mL de THF et 0.7 mL de H2O. Laisser refroidir le milieu réactionnel, adsorber sur silice et purifier par flash chromatographie éluée avec AcOEt/cyclohexane 20% puis AcOEt 100%, et enfin AcOEt/MeOH 5%. Reprendre le résidu dans MeOH, filtrer puis laver avec MeOH.

Obtention de 147 mg de poudre blanche.
Tf° : 239-244°C

RMN 1H DMSO d6 (300 MHz) : 3.80 (d, J = 5.08, 2H); 4.02 (s, 3H); 4.19 (m, 5H); 4.95 (m 1H); 6.70 (d, J = 2.16, 1H); 7.46 (m, 2H); 7.62 (d, J = 8.64, 1H); 7.70 (d, J = 1.62, 1H); 7.71 - 7.81 (m, 2H); 8.30 (s, 2H).

### EXEMPLE 52 : 3-(2,4-Dichloro-phenyl)-6-(2-ethyl-4-methyl-2H-pyrazol-3-yl)-1,9-dimethyl-1,9-dihydro-pyrido[2,3-b]indol-2-one

On procède comme indiqué à l'exemple 37 ci-dessus à partir de 3-(2,4-Dichlorophenyl)-6-(3-dimethylamino-2-methyl-acryloyl)-1,9-dimethyl-1,9-dihydro-pyrido[2,3-b]indol-2-one de la préparation 1.13 ci-dessus et d'oxalate d'éthyl hydrazine RMN DMSO d6 1H (300 MHz) : 1,20 (t, J=7,1, 3H), 1,94 (s, 3H); 3,97-7,04 (m, 5H); 4,20 (s, 3H); 7,25 (d, J=8,4, 1H); 7,35 (s, 1H); 7,42-7,49 (m, 2H); 7,69 (s, 1H); 7,74 (d, J=8,4, 1H); 7,91 (s, 1H); 8,32 (s,1H).

### EXEMPLE 53 : 3-(2,4-Dichloro-phenyl)-1,9-dimethyl-6-(4-methyl-2H-pyrazol-3-yl)-1,9- dihydro-pyrido[2,3-b]indol-2-one

On procède comme indiqué à l'exemple 40 ci-dessus à partie de 3-(2,4-Dichlorophenyl)-6-(3-dimethylamino-2-methyl-acryloyl)-1,9-dimethyl-1,9-dihydro-pyrido[2,3-b]indol-2-one de la prép.1.13 ci-dessus et d'hydrate d'hydrazine RMN DMSO d6 1H (300 MHz) : 2,23 (s, 3H); 4,03 (s, 3H); 4,18 (s, 3H); 7,43-7,57 (m, 4H); 7,67-7,70 (m, 2H); 8,09 (s, 1H); 8,31 (s, 1H); 12 67 (s,1H).

### EXEMPLE 54 : 3-(2,4-Dichloro-phenyl)-1,9-dimethyl-6-(3-methyl-isoxazol-5-yl)-1,9-dihydro-pyrido[2,3-b]indol-2-one

On procède comme indiqué à l'exemple 44 ci-dessus à partir de 1-[3-(2,4-Dichlorophenyl)-1,9-dimethyl-2-oxo-2,9-dihydro-1H-pyrido[2,3-b]indol-6-yl]-butane-1,3-dione de la prep. 1.14 et de chlorhydrate d'hydroxylamine
T°f : 288-289°C

RMN 1H DMSO d6 (300 MHz) : 2.29 (s, 3H) ; 4.02 (s, 3H); 4.20 (s, 3H) ; 6.80 (s, 1H) ; 7.40 - 7.51 (m, 2H) ; 7.70 (d, J = 1.93, 1H) ; 7.75 (d, J = 1.69, 2H) ; 8.34 (s, 1H) ; 8.42 (s, 1H).

### EXEMPLE 55 : 6-(5-Amino-isoxazol-3-yl)-3-(2,4-dichloro-phenyl)1,9-dimethyl-1,9-dihydro-pyrido[2,3-b]indol-2-one

Agiter à 50°C pendant 24h puis à température ambiente pendant 18h une solution contenant 300 mg (0.707 mmol) de composé 3-[3-(2,4-Dichloro-phenyl)-1,9-dimethyl-2-oxo-2,9-dihydro-1H-pyrido[2,3-b]indol-6-yl]-3-oxo-propionitrile de l'exemple 20A , 238 mg (2.9 mmol) de sodium acetate et 152 mg (2.2 mmol) de chlorhydrate d'hydroxylamine dans 4 mL de CH2Cl2 et 4 mL de MeOH. Concentrer le milieu réactionnel en l'adsorbant sur silice, purifier par colonne de silice éluée avec AcoEt/cycyclohexane 20% puis AcOEt 100%.

Obtention de 67 mg de poudre beige.
Tf° : 234-235°C

RMN 1H DMSO d6 (300 MHz) : 4.01 (s, 3H); 4.18 (s, 3H); 5.44 (s, 1H); 6.74 (s, 2H); 7.40 - 7.50 (m, 2H); 7.68 (m, 3H); 8.27 (s, 1H); 8.31 (s, 1H).

### EXEMPLE 56 : 3-(2,4-Dichloro-phenyl)-6-(2-ethoxymethyl-oxazol-5-yl)-1-methyl-1,9-dihydro-pyrido[2,3-b]indol-2-one

### A) Composé A : N-{2-[3-(2,4-Dichloro-phenyl)-1-methyl-2-oxo-2,9-dihydro-1H-pyrido[2,3-b]indol-6-yl]-2-oxo-ethyl}-2-ethoxy-acetamide

Dissoudre 756 mg (1.73 mmol) de composé Chlorhydrate de 6-(aminoacétyl)-3-(2,4-dichlorophényl)-1-méthyl-1,9-dihydro-2H-pyrido[2,3-b]indol-2-one de l'exemple 5A ci-dessus, 180 µL (1.90 mmol) d' acide éthoxyacétique, 991 mg (1.90 mmol) de PyBOP et 905 µL (5.2 mmol) de diisopropylethylamine dans 9 mL de CH2Cl2. Agiter à température ambiante pendant 16h. Ajouter une solution saturée de NH4Cl puis extraire avec CH2Cl2, sécher sur MgSO4, filtrer et concentrer sous vide. Purifier par chromatographie sur colonne de silice éluée avec AcOEt puis avec AcOEt/MeOH 3%. Obtention de 841 mg d'une huile marron.

RMN DMSO d6 1H (300 MHz) : 1.17 - 1.35 (m, 3H); 3.58 (m, 2H); 3.70 (s, 3H); 3.94 (s, 2H); 4.74. (d, J = 5.34, 2H); 7.40 - 7.52 (m, 2H); 7.59 (d, J = 8.54, 1H); 7.70 (s, 1H); 7.90 (d, J = 8.51, 1H); 7.95 (m, 1H); 8.37 (s, 1H); 8.71 (s, 1H).

### B) : 3-(2,4-Dichloro-phenyl)-6-(2-ethoxymethyl-oxazol-5-yl)-1-methyl-1,9-dihydropyrido[2,3-b]indol-2-one

Agiter à température ambiante pendant 20h une solution de composé A N-{2-[3-(2,4-Dichloro-phenyl)-1-methyl-2-oxo-2,9-dihydro-1H-pyrido[2,3-b]indol-6-yl]-2-oxoethyl}-2-ethoxy-acetamide (841 mg, 1.73 mmol) dans 2 mL de H2SO4. Ajouter H2O, extraire avec EtOAc et CH2Cl2, concentrer sans sécher. Adsorber sur silice et purifier par chromatographie sur colonne de silice éluée avec AcOEt. Reprendre le résidu dans Et20, filtrer et laver avec une minimum d' Et2O.

Obtention de 372 mg de poudre beige.
T°f :dec. 320°C

RMN DMSO d6 1H (300 MHz) :1.16 (t, J = 6.99, 3H); 3.52 - 3.62 (q, J = 3.72, 2H); 3.70 (s, 3H); 4.58 (s, 2H); 7.40 - 7.50 (m, 2H); 7.55 (m, 1H); 7.58 (m, 2H); 7.70 (d, J = 1.62, 1H); 8.26 (s, 1H); 8.30 (s, 1H); 12.35 (s, 1H).

### EXEMPLE 57 : 3-(2,4-Dichloro-phenyl)-6-(1,5-dimethyl-1H-pyrazol-3-yl)-1,9-dimethyl-1,9-dihydro-pyrido[2,3-b]indol-2-one

On procède comme à l'exemple 36 ci-dessus à partir de 3-(2,4-dchlorophényl)-1,9-diméthyl-6-(3-méthyl-1H-pyrazol-5-yl)-1,9-dihydro-2H-pyrido[2,3-b]indol-2-one décrit ex46 ci-dessus et de iodomethane.
Tf° : 286-287°C

RMN 1H DMSO d6 (300 MHz) : 2.28 (s, 3H); 3.75 (s, 3H); 4.00 (s, 3H); 4.15 (s, 3H); 6.47 (s, 1H); 7.40 - 7.50 (m, 2H); 7.58 (d, J = 8.61, 1H); 7.69 (m, 2H); 8.23 (d, J = 1.24, 1H); 8.27 (s, 1H).

### EXEMPLE 58 : 3-(2,4-Dichloro-phenyl)-6-(1-ethyl-5-methyl-1H-pyrazol-3-yl)-1,9-dimethyl-1,9-dihydro-pyrido[2,3-b]indol-2-one

On procède comme indiqué à l'exemple 36 ci-dessus à partir de 3-(2,4-dchlorophényl)-1,9-diméthyl-6-(3-méthyl-1H-pyrazol-5-yl)-1,9-dihydro-2H-pyrido[2,3-b]indol-2-one décrit ex46 ci-dessus de iodoethane.
Tf° : 270-272°C

RMN 1H DMSO d6 (300 MHz) : 1.21 - 1.40 (2t, J = 7.18, 3H); 2.20 - 2.32 (2s, 3H); 4.00 (s, 3H); 4.10 (q, J = 7.20, 2H); 4.15 (s, 3H); 6.11 - 6.46 (2s, 1H); 7.42 (m, 2H); 7.60 (d, J = 8.62, 1H); 7.70 (2d, J = 1.65, 2H); 8.20 - 8.40 (m, 2H

### EXEMPLE 59 5-[3-(2,4-dichloro-phenyl)-1,9-dimethyl-2-oxo-2,9-dihydro-1H-pyrido[2,3-b]indol-6-yl]-1-ethyl-1H-pyrazole-3-carboxylic acid ethyl ester

### A) composé A : 4-[3-(2,4-dichloro-phenyl)-1,9-dimethyl-2-oxo-2,9-dihydro-1H-pyrido[2,3-b]indol-6-yl]-2,4-dioxo-butyric acid ethyl ester

Dissoudre 940 mg (6,4 mmol) de diethyl oxalate dans 5 mL de THF anhydre. Ajouter 273 mg (6,8 mmol) de NaH 60% puis agiter à température ambiante pendant 10 minutes. Ajouter une solution de 1,3 g (3,2 mmol) de composé 6-(Bromoacétyl)-3-(2,4-dichloraphényl)-1,9-diméthyl-1,9-dihydro-2H-pyrido[2,3-b]indol-2-one. de la prép. 1.2 ci-dessus et 18 mg (0,052 mmol) de dibenzo 18 crown 6 dissous dans 20 mL de THF anhydre et quelques gouttes d'EtOH anhydre. Chauffer à 70°C sous argon pendant 3 heures. Revenir à température ambiante. Ajouter de l'AcOEt au milieu réactionnel et laver la phase organique avec une solution de HCl 1N. Evaporer à sec la phase organique, triturer dans l'eau le produit obtenu, filtrer. Reprendre dans de l'EtOH et évaporer à sec.

Obtention de 1,85 g de poudre orange.

RMN DMSO d6 1H (300 MHz) : 1,32 (t, J=7,08, 3H); 4,03 (s, 3H), 4,21 (s, 3H); 4,29-4,36 (q, J=7,07, 2H); 7,30 (s, 1H), 7,43-7,52 (m, 2H); 7,71 (s, 1H); 7,80 (d, J=8,85, 1H); 8,00-8,04 (dd, J=1,74, J=8,77, 1H); 8,48 (s, 1H); 8,83 (s, 1H).

### B) 5-[3-(2,4-dichloro-phenyl)-1,9-dimethyl-2-oxo-2,9-dihydro-1H-pyrido[2,3-b]indol-6-yl]-1-ethyl-1H-pyrazole-3-carboxylic acid ethyl ester.

Dissoudre 930 mg (1,85 mmol) de composé A 4-[3-(2,4-dichloro-phenyl)-1,9-dimethyl-2-oxo-2,9-dihydro-1H-pyrido[2,3-b]indol-6-yl]-2,4-dioxo-butyric acid ethyl ester dans 20 mL d'éthanol absolu. Ajouter 583 mg (5,56 mmol) d'éthylhydrazine oxalate et chauffer à reflux pendant une nuit. Evaporer à sec le milieu réactionnel et triturer le résidu dans une solution de K2CO3 saturée. Filtrer et laver à l'eau. Purification sur colonne de silice éluée avec de l'AcOEt.

Obtention de 510 mg de mousse blanche.

RMN DMSO d6 1H (300 MHz) : 1,28-1,33 (m, 6H); 4,03 (s, 3H); 4,18-4,32 (m, 7H), 6,83 (s, 1H); 7,39-7,50 (m, 3H); 7,69 (d, J=1,93, 1H); 7,76 (d, J=8,57, 1H); 8,07 (d, J=1,33, 1H); 8,32 (s, 1H).

### EXEMPLE 60 : 3-(4-bromo-phenyl)-1,9-dimethyl-6-(2H-pyrazol-3-yl)-1,9-dihydropyrido[2,3-b]indol-2-one

### A) Composé A: 3-(4-bromo-phenyl)-1,9-dimethyl-1,9-dihydro-1H-pyrido[2,3-b]indol-2-one

On procède comme indiqué à la preparation 1.2B ci-dessus avec du chlorhydrate de Mefhyl-(1-methyl-1H-indol-2-yl)-amino (préparé selon les modes opératoires décrits dans WO 2004/041817) et du 3-Dimethylamino-2-(4-bromo-phenyl)-acrylic acid methyl ester de la prep.2.1 0 WO2005/108398

RMN DMSO d6 1H (300 MHz) : 4,01 (s, 3H) ; 4,12 (s, 3H); 7,24 (m, 2H) ; 7,58 (m, 3H); 7,75 (dd, J=1.91, J=6,69, 2H); 7,93 (d, J=7,24, 1H); 8,45 (s, 1H).

### B) Composé B : 6-Acetyl-3-(4-bromo-phonyl)-1,9-dimethyl-1,9-dihydro-pyrido[2,3-b]indol-2-one

On procède comme indiqué à la préparation 1.9A ci-dessus avec le Composé A 3-(4-bromo-phenyl)-1,9-dimethyl-1,9-dihydro-1H-pyrido[2,3-b]indol-2-one du acetyl chloride

RMN DMSO d6 1H (300 MHz) : 2,65 (s, 3H); 4,03 (s, 3H); 4,18 (s, 3H); 7,59 (d, J=8,61, 2H); 7,69-7,72 (d, J=8,71, 1H); 7,79 (d, J=8,62, 2H); 7,90 (dd, J=1,70, J=8,67, 1H); 8,65 (s, 1H); 8,69 (d, J=1,51, 1H).

### C) Composé C : 3-(4-bromo-phenyl)-6-(3-dimethylamino-acryloyl)-1,9-dimethyl-1,9-dihydro-pyrido[2,3-b]indol-2-one

On procède comme indiqué à la preparation 1.9C ci-dessus avec du composé B 6-Acetyl-3-(4-bromo-phenyl)-1,9-dimethyl-1,9-dihydro-pyrido[2,3-b]indol-2-one et du réactif de Bredereck.

RMN DMSO d6 1H (300 MHz) : 2,96 (bs, 3H); 3,14 (bs, 3H); 4,02 (s, 3H); 4,17 (s, 3H); 6,00 (d, J=12,27, 1H); 7,60 (t, J=9,11, 3H); 7,72 (d, J=12,25, 1H); 7,79 (d, J=6,71, 2H); 7,89 (dd, J=1,61, J=8,64, 1H); 8,57 (d, J=1,34, 1H); 8,59 (s, 1H).

### D). 3-(4-bmromo-phenyl)-1,9-dimethyl-6-(2H-pyrazol-3-yl)-1,9-dihydro-pyrido[2,3-b]indol-2-one

On procède comme indiqué à l'ex36 ci-dessus avec du composé 3-(4-bromophenyl)-6-(3-dimethylamino-acryloyl)-1,9-dimethyl-1,9-dihydro-pyrido[2,3-b]indol-2-one et de l'hydrazine monohydrate.
T°f : 347°C

RMN DMSO d6 1H (300 MHz) : 4,01 (s, 3H); 4,12 (s, 3H); 6,74 (m, 1H); 7,54-7,80 (m, 7H); 8,39-8,54 (m, 2H); 12,80 (s, 1H).

### EXEMPLE 61 : 3-(2,4-dichloro-phenyl)-6-(2-ethyl-5-hydroxymethyl-2H-pyrazol-3-yl)1,9-dimethyl-1,9-dihydro-pyrido[2,3-b]indol-2-one

Dissoudre 450 mg (0,86 mmol) de composé de l'ex 59, 5-[3-(2,4-dichloro-phenyl)-1 ,9-dimethyl-2-oxo-2,9-dihydro-1H-pyrido[2,3-b]indol-6-yl]-1-ethy-1H-pyrazole-3-carboxylie acid ethyl ester dans 10 mL d'éthanol absolu, ajouter 42 mg (1,12 mmol) de NaBH4. Chauffer à reflux pendant une nuit. Revenir à température ambiante, ajouter du CH2Cl2 et du MeOH au milieu réactionnel et adsorber sur silice. Purifier sur colonne de silice éluée avec de l'AaOEt, puis avec un mélange AcOEt/MeOH 95 /05. Triturer l'huile obtenu dans de l'éther.

Obtention de 50 mg de poudre blanche.
T°f : 235°C

RMN DMSO d6 1H (300 MHz) : 1,27 (t, J=7,13, 3H); 4,03 (s, 3H); 4,05-4,13 (q, J=7,18, 2H); 4,20 (s, 3H) ; 4,42 (d, J=5,43, 2H); 5,03 (t, J=5,68, 1H); 6,27 (s, 1H); 7,33 (dd, J=1,63, J=8,46, 1H); 7,42-750 (m, 2H); 7,69 (d, J=1,95, 1H); 7,73 (d, J=8,55, 1H); 7,98 (d, J=1,40, 1H); 8,32 (s, 1H);

### EXEMPLE 62 : 3-(2,4-Dichloro-phenyl)-6-(thiazol-4-yl)-1,9-dimethyl-1,9-dihydropyrido[2,3-b]indol-2-one

Dissoudre 150 mg (0,3 mmol) de composé 3-(2,4-Dichlorophényl)-6-[2-(hydroxyméthyl)-1,3-thiazol-4-yl]-1,9-dimémyl-1,9-dihydro-2H-pyrido[2,3-b]indol-2-one. ex 9 ci-dessus dans 10 mL de DMF. Rajouter 50 mg de NaH 60% (0,96 mmol) puis agiter à température ambiante pendant 30 min. Rajouter 0,05 mL (0,6 mmol) de bromure de cyclopropyl puis agiter à température ambiante pendant 3 heures. Rajouter de l'eau, extraire à l'AcoEt Laver avec une solution de NaCl saturée puis adsorber sur silice. Purifier par chromatographie sur colonne de silice éluée avec de l'AcOEt. Obtention de 60 mg de poudre blanche. T°f : 289°C
MS : 440,08

RMN DMSO d6 1H (300 MHz) : 4,02 (s, 3H) ; 4,18 (s, 3H); 7,46 (m, 2H); 7,69 (m, 2H); 7,96 (d, J=8,6, 1H); 8,06 (s, 1H); 8,28 (s, 1H); 8,53 (s, 1H); 9,20 (s, 1H). EXEMPLE 63 : 3-(4-Fluoro-phenyl)-6-(2-hydroxymethyl-thiazol-4-yl)-1,9-dimethyl-1,9-dihydro-pyrido[2,3-b]indol-2-one

### A) Composé A : 3-(4-fluoro-phenyl)-1,9-dimethyl-1,9-dihydro-pyrido[2,3-b]indol-2-one

On procède comme indiqué à la preparation 1.2B ci-dessus avec du chlorhydrate de Methyl-(1-methyl-1H-indol-2-yl)-amine (préparé selon les modes opératoires décrits dans WO 2004/041817) et du 2-(4-fluoro-phenyl)-3-dimethylamino-acrylic acid methyl ester, Prep.2.12 WO5108398

### B) Composé B : 6-(2-Bromo-acetyl)-3-(4-fluoro-phenyl)-1,9-dimethyl-1,9-dihydropyrido [2,3-b]indol-2-one

On procède comme indiqué à la preparation 1.1E ci-dessus avec composé A 3-(4-fluoro-phenyl)-1,9-dimethyl-1,9-dihydro-pyrido[2,3-b]indol-2-et du bromoacetyl chloride.

### C) Composé C : 2,2-Dimethyl-propionic acid 4-[3-(4-fluoro-phenyl)-1,9-dimethyl-2-oxo-2,9-dihydro-1H-pyrido[2,3-b]indol-6-yl]-thiazol-2-ylmethyl ester

On procède comme indiqué à l'ex3 ci-dessus avec du composé B 6-(2-Bromoacetyl)-3-(4-fluoro-phenyl)-1,9-dimethyl-1,9-dihydro-pyrido [2,3-b]indol-2-one et du 2-amino-2-thioxoethyl pivalate

RMN 1H DMSO-d6 (300 MHz) : 1.16 (s, 3H); 1.22 (s, 6H); 4.01 (s, 3H); 4.15 (s, 3H); 5.46 (s, 2H); 7.16 - 7.26 (t, J = 8.96, 2H) ; 7.63 (d, J = 8.67, 1H) ; 7.78 - 7.90 (m, 3H) ; 8.05 (s, 1H) ; 8.48 (s, 1H) ; 8.53 (d, J = 1.48, 1H).

### D) : 3-(4-Fluoro-phenyl)-6-(2-hydroxymethyl-thiazol-4-yl)-1,9-dimethyl-1,9-dihydropyrido[2,3-b]indol-2-one

On procède comme indiqué à l'ex3 ci-dessus avec le composé C 2,2-Dimethyl-propionic acid 4-[3-(4-fluoro-phenyl)-1,9-dimethyl-2-oxo- 2,9-dihydro-1H-pyrido[2,3-b]indol-6-yl]-thiazol-2-ylmethyl ester
T°f : 162-166°C

RMN 1H DMSO-d6 (300 MHz) : 4.01 (s, 3H); 4.15 (s, 3H); 4.82 (s, 2H); 7.16 - 7.28 (t, J = 8.96, 2H); 7.63 (d, J = 8.64, 1H); 7.80 - 7.90 (m, 3H); 7.94 (s, 1H); 8.48 (s, 1H); 8.52 (d, J = 1.45).

### EXEMPLE 64 : 3-(2,4-Dichloro-phenyl)-6-(2-hydroxymethyl-oxazol-5-yl)-1,9-dimethyl-1,9-dihydro-pyrido[2,3-b]indol-2-one

### A) Composé A : 2-Benzyloxy-N-{2-[3-(2,4-dichloro-phenyl)-1,9-dimethyl-2-oxo-2,9-dihydro-1H-pyrido[2,3-b]indol-6-yl]-2-oxo-ethyl}-acetamide

Dissoudre 500 mg (1.11 mmol) de Chlorhydrate de 6-(aminoacétyl)-3-(2,4-dichlorophényl)-1,9-diméthyl-1,9-dihydro-2H-pyrido[2,3-b]indol-2-one décrit ex 18A ci-dessus, 175 µL (1.22 mmol) de benzyloxyacetic acid, 635 mg (1.22 mmol) de PyBop et 580 µL (3.33 mmol) de diisopropylethylamine dans 6 mL de CH2Cl2. Agiter à température ambiante pendant 16h. Ajouter une solution saturée de NH4Cl puis extraire avec CH2Cl2, sécher sur MgSO4, filtrer et concentrer sous vide. Purifier par chromatographie sur colonne de silice éluée avec AcOEt puis avec AcOEt/MeOH 5%.

Obtention de 454 mg de cristaux jaune.

### B) : 3-(2,4-Dichloro-phenyl)-6-(2-hydroxymethyl-oxazol-5-yl)-1,9-dimethyl-1,9-dihydro-pyrido[2,3-b]indol-2-one

Agiter à température ambiante pendant 20h une solution de composé A 2-Benzyloxy-N-{2-[3-(2,4-dichloro-phenyl)-1,9-dimethyl-2-oxo-2,9-dihydro-1H-pyrido[2,3-b]indol-6-yl]-2-oxo-ethyl}-acetamide (454 mg, 0.81 mmol) dans 2 mL de H2SO4. Ajouter un mélange d'H2O et d'AcOEt, filtrer le solide vert obtenu, reprendre le filtrat et l'extraire avec EtOAc , concentrer ces phases organiques combinées avec le précipité sans sécher. Adsorber sur silice avec un minimum de pyridine et purifier par chromatographie sur colonne de silice éluée avec AcOEt puis AcOEt/MeOH 5%. Reprendre le résidu dans MeOH, filtrer et laver avec un minimum de MeOH puis d' Et2O.

Obtention de 221mg de poudre beige.
T°f :296-297°C

RMN 1H DMSO-d6 (300 MHz): 4.01 (s, 3H); 4.18 (s, 3H); 4.54 (bs, 2H); 5.70 (bs, 1H); 7.40 - 7.48 (m, 2H); 7.52 (m, 1H); 7.60 - 7.76 (m, 3H); 8.25 (s, 1H); 8.31 (s, 1H).

### EXEMPLE 65 : 3-(2-Chloro-4-hydroxy-phenyl)-1,9-dimethyl-6-(2H-pyrazol-3-yl)-1,9-dihydro-pyrido[2,3-b]indol-2-one

### A) Composé A: 3-(2-Chloro-4-fluoro-phenyl)-1,9-dimethyl-1,9-dihydro-pyrido[2,3-b]indol-2-one

On procède comme indiqué à l'exemple 60 ci-dessus à partir de 3-Dimethylamino-2-(2-Chloro-4-fluoro -phenyl)-acrylic acid methyl ester. RMN 1H DMSO-d6 (300 MHz): 3.99 (s, 3H); 4.13 (s, 3H); 7.15 - 7.35 (m, 3H); 7.40 - 7.55 (m, 2H); 7.55 - 7.64 (d, J = 8.10, 1H); 7.82 - 7.90 (d, J = 7.4, 1H); 8.19 (s,1H).

### B) Composé B 3-(2-Chloro-4-methoxy-phenyl)-1,9-dimethyl-1,9-dihydro-pyrido[2,3-b]indol-2-one

Dans 300 mL de DMF et 9.6 mL de MeOH, ajouter 3.25 g (81.1 mmol) de NaH 60%. Laisser agiter 5 minutes puis ajouter 9.22 g (27.0 mmol) de composé A 3-(2-Chloro-4-fluoro-phcnyl)-1,9-dimethyl-1,9-dihydro-pyrido[2,3-b]indol-2-one. Agiter à température ambiante puis, dès la fin du dégagement gazeux, chauffer à 80°C pendant 16h. Laisser refroidir, verser le milieu réactionnel dans H2O filtrer le précipité jaune, laver avec H2O, reprendre le résidu dans MeOH et concentrer à sec.

Obtention de 10.6 g de poudre jaune.

RMN 1H DMSO-d6 (300 MHz) : 3.81 (s, 3H) ; 4.00 (s, 3H) ; 4.15 (s, 3H) ; 6.95 (dd, J = 8.53 , j = 2.1, 1H) ; 7.10 (d, J = 2.57, 1H) ; 7.17 - 7.26 (m, 1H) ; 7.27 - 7.32 (m, 2H) ; 7.60 (d, J = 8.06, 1H) ; 7.87 (d, J = 6.97, 1H) ; 8.14 (s, 1H).

### C) Composé C : 6-Acetyl-3-(2-chloro-4-methoxy-phenyl)-1,9-dimethyl-1,9-dihydropyrido[2,3-b]indol-2-one

On procède comme indiqué à l'exemple 60 ci-dessus à partir de Composé B :3-(2-Chloro-4-methoxy-phenyl)-1,9-dimethyl-1,9-dihydro-pyrido[2,3-b]indol-2-one et d'acetyl chloride.

### D) Composé D : 3-(2-Chloro-4-methoxy-phenyl)-6-(3-dimethylamino-acryloyl)-1,9-dimethyl-1,9-dihydro-pyrido[2,3-b]indol-2-one

On procède comme indiqué à l'exemple 60 ci-dessus à partir de composé C 3-(2-Chloro-4-methoxy-phenyl)-6-(3-dimethylamino-acryloyl)-1,9-dimethyl-1,9-dihydropyrido[2,3-b]indol-2-one et de réactif de Bredereck

### E) Composé E : 3-(2-Chloro-4-methoxy-phenyl)-1,9-dimethyl-6-(2H-pyrazol-3-yl)-1,9-dihydro-pyrido[2,3-b]indol-2-one

On procède comme indiqué à l'exemple 60 ci-dessus à partir de Composé D 3-(2-Chloro-4-methoxy-phenyl)-6-(3-dimethylamino-acryloyl)-1,9-dimethyl-1,9-dihydropyrido[2,3-b]indol-2-one et de hydrate d'hydrazine

### F) 3-(2-Chloro-4-hydroxy-phenyl)-1,9-dimethyl-6-(2H-pyrazol-3-yl)-1,9-dihydropyrido[2,3-b]indol-2-one

A -78°C, ajouter une solution 1M de BBr3. dans le CH2Cl2 (2.19 mL, 2.19 mmol) à une solution de composé E 3-(2-Chloro-4-methoxy-phenyl)-1,9-dimethyl-6-(2H-pyraml-3-yl)-1,9-dihydro-pyrido[2,3-b]indol-2-one (306 mg, 0.73 mmol) dans 3 mL de CH2Cl2. Agiter à TA pendant 16h. Ajouter H2O, filtrer le précipité. Reprendre le solide dans MeOH/CH2Cl2 et l'adsorber sur silice. Purifier par chromatographie sur colonne éluée avec CH2Cl2/MeOH 5%. Reprendre le résidu de MeOH, filtrer et laver avec MeOH.

Obtention de 199 mg de poudre blanche. T°f: 319-320°C

RMN 1H DMSO-d6 (300 MHz) : 4.00 (s, 3H); 4.16 (s, 3H); 6.70 (bs, 1H); 6.75 - 6.82 (dd, J = 8.35, j = 2.42, 1H); 6.90 (d, J = 2.39, 1H); 7.20 (d, J = 8.36, 1H); 7.45 - 7.82 (m, 3H); 8.05 - 8.20 (m, 1H); 8.30 (bs, 1H); 9.88 (bs, 1H).

### EXEMPLE 66 : 3-(2,4-Dichloro-phenyl)-6-(1-methoxymethyl-1H-pyrazol-3-yl)-1,9-dimethyl-1,9-dihydro-pyrido[2,3-b]indol-2-one

On procède comme indiqué à l'exemple 36 ci-dessus à partir de 3-(2,4-Dichlorophényl)-1,9-diméthyl-6-(1H-pyrazol-5-yl)-1,9-dihydro-2H-pyrido[2,3-b]indol-2-one ex39 ci-dessus et de bromomethyl methylether.
Tf° : 232-234°C

RMN 1H DMSO-d6 (300 MHz) : 3.28 (s, 3H); 4.01(s, 3H); 4.16 (s, 3H); 5.40 (s, 2H); 6.81 (d, J = 2.34, 1H); 7.41 - 7.51 (m, 2H); 7.60 - 7.71 (m, 2H); 7.80 (dd, 1H); 7.94 (d, J = 2.34, 1H); 8.28 (s, 1H); 8.34 (d, 1H)

### EXEMPLE 67 : 3-(2,4-Dichloro-phenyl)-6-[1-(2-methoxyethyl)-1H-pyazol-3-yl]-1,9-dimethyl-1,9-dihydro-pyrido[2,3-b]indol-2-one

On procède comme à l'exemple 10 ci-dessus à partir de 3-(2,4-Dichloro-phenyl)-6-[1-(2-hydroxy-ethyl)-1H-pyrazol-3-yl]-1,9-dimethyl-1,9-dihydro-pyrido[2,3-b]indol-2-one ex 51 et de iodomethane.
Tf°: 206-208°C

RMN 1H DMSO-d6 (300 MHz) : 3.25 (s, 3H); 3.70 - 3.79 (t, J = 5.31, 2H); 4.01 (s, 3H); 4.16 (s, 3H); 4.24 - 4.32 (t, J = 5.26, 2H); 6.68 (d, J = 2.25, 1H); 7.40 - 7.50 (m, 2H); 7.59 - 7.64 (d, J = 8.62, 1H); 7.69 (d, J = 1.65, 1H); 7.73 - 7.81 (m, 2H); 8.29 (d, J = 2.56, 2H).

### EXEMPLE 68 : 3-(4-bromo-phenyl)-6-(2-ethyl-2H-pyrazol-3-yl)-1,9-dimethyl-1,9-dihydro-pyrido[2,3-b]indol-2-one

On procède comme à l'exemple 37 ci-dessus avec le composé de l'ex60C 3-(4-bromo-phenyl)-6-(3-dimethylamino-acryloyl)-1,9-dimethyl-1,9-dihydro-pyrido[2,3-b]indole-2-one. et de l'éthyl hydrazine oxalate.

### RMN DMSO d6 1H (300 MHz) : 1,29 (t, J=7,20, 3H); 4,03 (s, 3H); 4,18 (m, 5H); 6,36 (s, 1H); 7,35 (dd, J=1,71, J=8,47, 1H); 7,50 (d, J=1,86, 1H); 7,59 (m, 2H); 7,78 (m, 3H); 8,07 (d, J=1,53, 1H); 8,57 (s, 1H). T°f: 200-203°C

### EXEMPLE 69 : 6-(2-Ethoxymethyl-thiazol-4-yl)-3-(4-fluoro-phenyl)-1,9-dimethyl-1,9-dihydro-pyrido[2,3-b]indol-2-one

On procède comme à l'exemple 10 ci-dessus à partir de 3-(4-Fluoro-phenyl)-6-(2-hydroxymethyl-thiazol-4-yl)-1,9-dimethyl-1,9-dihydro-pyrido[2,3-b]indol-2-one, composé de 1' Ex 63 et de iodoethane.
T°f : 86°C - 90°C
MS : 448.15

RMN 1H DMSO-d6 (300 MHz) : 1.21 (t, J = 6.98, 3H); 3.60 - 3.70 (q, J = 7.0, 2H); 4.02 (s, 3H); 4.15 (s, 3H); 4.82 (s, 2H); 7.18 - 7.28 (t, J = 8.96, 2H); 7.64 (d, J = 8.62, 1H); 7.80 - 7.92 (m, 3H); 8.00 (s, 1H); 8.48 (s, 1H); 8.52 (d, J = 1.33, 1H).

### EXEMPLE 70 : 2,2-Dimethyl-propionic acid 4-[3-(4-fluoro-phenyl)-1,9-dimethyl-2-oxo-2,9-dihydro-1H-pyrido[2,3-b]indol-6-yl]-thiazol-2-ylmethyl ester

Dissoudre 200 mg (0.48 mmol) de composé 3-(4-Fluoro-phenyl)-6-(2-hydroxymethyl-thiazol-4-yl)-1,9-dimethyl-1,9-dihydro-pyrido[2,3-b]indol-2-one ex63 dans 3 mL de pyridine. Rajouter 294 µL (2.38 mmol) de pivaloylchloride puis agiter à température ambiante pendant 2 heures. Rajouter H2O, extraire à l'AcOEt, laver la phase organique avec une solution saturée de NH4Cl, puis la sécher sur MgSO4, filtrer et concentrer. Purifier par chromatographie sur colonne de silice éluée avec de l'AcOEt. Obtention de 209 mg de poudre jaune.
T°f : 90°C - 92°C
MS : 504.19

RMN 1H DMSO-d6 (300 MHz) : 1.23 (s, 9H); 4.02 (s, 3H); 4.15 (s, 3H); 5.47 (s, 2H); 7.18 - 7.29 (t, J = 8.95, 2H); 7.65 (d, J = 8.67, 1H); 7.80 - 7.92 (m, 3H); 8.05 (s, 1H); 8.48 (s, 1H); 8.53 (d, J = 1.41, 1H).

### EXEMPLE 71 : 3-(4-Fluoro-phenyl)-1,9-dimethyl-6-thiazol-4-yl-1,9-dihydropyrido[2,3-b]indol-2-one

On procède comme à l'exemple 62 ci-dessus avec le composé de l'Ex 63, 3-(4-Fluoro-phenyl)-6-(2-hydroxymethyl-thiazol-4-yl)-1,9-dimehyl-1,9-dihydropyrido[2,3-b]indol-2-one
T°f : 280°C - 282°C
MS: 390.13

RMN 1H DMSO-d6 (300 MHz) : 4.02 (s, 3H); 4.16 (s, 3H); 7.15 - 7.30 (t, J = 8.72, 2H); 7.66 (d, J = 8.53, 1H); 7.76 - 7.89 (q, J = 5.95, 2H); 7.94 (d, J = 8.33, 1H); 8.08 (s, 1H); 8.49 (s, 1H); 8.59 (s, 1H); 9.21 (s, 1H).

### EXEMPLE 72 : 3-(4-Fluoro-phenyl)-1,9-dimethyl-6-(2H-pyrazol-3-yl)-1,9-dihydropyrido[2,3-b]indol-2-one

### A) composé A : 6-Acetyl-3-(4-fluoro-phenyl)-1,9-dimethyl-1,9-dihydro-pyrido[2,3-b]indol-2-one

On procède comme à la préparation. 1.9B ci-dessus à partir de 3-(4-Fluoro-phenyl)-1,9-dimethyl-1,9-dihydro-pyrido[2,3-b]indol-2-one de l'Ex 63A ci-dessus et de chlorure d'acétyle

RMN 1H DMSO d6 (300 MHz) : 2,64 (s, 3H) ; 4,01 (s, 3H) ; 4,16 (s, 3H) ; 7,24 (t, J=8,9, 2H) ; 7,67 (d ; J=8,7, 1H); 7,80-7,89 (m, 3H) ; 8,57 (s, 1H) ; 8,66 (s, 1H).

### B) composé B : 6-(3-Dimethylamino-acryloyl)-3-(4-fluoro-phenyl)-1,9-dimethyl-1,9-dihydro-pyrido[2,3-b]indol-2-one

On procède comme à la préparation 1.9C ci-dessus à partir de composé A, 6-Acetyl-3-(4-fluoro-phenyl)-1,9-dimethyl-1,9-dihydro-pyrido[2,3-b]indol-2-one RMN 1H DMSO d6 (300 MHz) : 2.95 (s, 3H) ; 3,14 (s, 3H) ; 4,01 (s, 3H) ; 4,15 (s, 3H) ; 6,00 (d, J=12,3, 1H) ; 7 ;23 (t, J=8,9, 2H) ; 7,59 (d, J=8,7, 1H); 7,72 (d, J=12,3, 1H); 7,80-7,95 (m, 3H); 8,55 (s, 1H); 8,56 (s, 1H).

### C) 3-(4-Fluoro-phenyl)-1,9-dimethyl-6-(2H-pyrazol-3-yl)-1,9-dihydro-pyrido[2,3-b]indol-2-one

On procède comme à l'exemple 40 ci-dessus à partir de composé B 6-(3-Dimethylamino-acryloyl)-3-(4-fluoro-phenyl)-1,9-dimethyl-1,9-dihydro-pyrido[2,3-b]indol-2-one et d'hydrate d'hydrazine PF 318°

RMN 1H DMSO d6 (300 MHz) : 4,02 (s, 3H) ; 4,14 (s, 3H) ; 6,75 (s, 1H) ; 7,23 (m, 2H) ; 7,61-7,83 (m, 5H) ; 8,40 (s, 1H) ; 8,49 (s, 1H) ; 8,80 (s, 1H).

### EXEMPLE 73 : 3-(2,4-Dichloro-phenyl)-1,9-dimethyl-6-[1-(2-morpholin-4-yl-ethyl)-1H-pyrazol-3-yl]-1,9-dihydro-pyrido[2,3-b]indol-2-one

On procède comme à l'exemple 36 ci-dessus à partir de de composé 3-(2,4-Dichlorophényl)-1,9-diméthyl-6-(1H-pyrazol-5-yl)-1,9-dihydro-2H-pyrido[2,3-b]indol-2-one ex 39 ci-dessus et de chloroethyl morpholine

RMN 1H CHCl3-d (300 MHz) : 2,49-2,52 (m, 4H) ; 2,87 (t, J=6,4, 2H); 3,69-3.72 (m, 4H) ; 4,06 (s, 3H) ; 4,11 (s, 3H) ; 4,30 (t, J=6,6, 2H) ; 6,57 (s, 1H) ; 7,30 (d, J=2,0, 1H) ; 7,36 (t, J=8,2, 2H) ; 7,49-7,52 (m, 2H) ; 7,76 (dd; J=1,6, J=8,5, 1H) ; 8,04 (s, 1H); 8,16 (d, J=1,3, 1H).

### EXEMPLE 74: 3-(2,4-Dichloro-phenyl)-1,9-dimethyl-6-[1-(2-pyrrolidin-1-yl-ethyl)-1H-pyrazol-3-yl]-1,9-dihydro-pyrido[2,3-b]indol-2-one

On procède comme à l'exemple 73 ci-dessus en utilisant la chloroéthylpyrrolidine. RMN 1H CHCl3-d (300 MHz) : 1,76-1,80 (m, 4H) ; 2,54-2,58 (m, 4H) ; 3,00 (t, J=7,0, 2H) ; 4,06 (s, 3H) ; 4,10 (s, 3H) ; 4,32 (t, J=7,0, 2H) ; 6,57 (s, 1H) ; 7,30 (d, J=2,1, 1H) ; 7,36 (t, J=8,2, 2H) ; 7,50 (s, 2H) ; 7,76 (dd, 1=1,6, J=8,5, 1H) ; 8,04 (s, 1H) ; 8,16 (d, J=1,3, 1H).

### EXEMPLE 75 : 6-(4-Aminomethyl-thiazol-2-yl)-3-(4-bromo-phenyl)-1,9-dimethyl-1,9-dihydro-pyrido[2,3-b]indol-2-one

### A) Composé A : 3-(4-Bromo-phenyl)-6-(4-chloromethyl-thiazol-2-yl)-1,9-dimethyl-1,9-dihydro-pyrido[23-b]indol-2-one

Dissoudre 3,3 g (7.7 mmol) de 3-(4-Bromo-phenyl)-1,9-dimethyl-2-oxo-2,9-dihydro-1H-pyrido[2,3-b]indole-6-carbothioic acid amide [ préparé selon la preparation 1.6 ci-dessus (RMN 1H DMSO-d6 (300 MHz) : 4.03 (s, 3H); 4.20 (s, 3H); 7.60 (m, 3H); 7.77 (m, 2H); 7.90 (d, J=1.8, 1H); 8.48 (s, 1H); 8.58 (s, 1H); 9.42 (s, 1H), 9.75 (s, 1H))], dans 40 mL de dioxane.

Ajouter 1.2 g (9.3 mmol) de dichloroacétone. Chauffer à reflux pendant une nuit. Revenir à température ambiante. Ajouter du méthanol et de l'AcOEt au milieu réactionnel et adsorber sur silice. Purifier sur colonne de silice éluée avec un mélange CH2Cl2/MeOH 99/01. Obtention de 2.5 g de poudre jaune.

RMN 1H DMSO-d6 (300 MHz) : 4.03 (s, 3H); 4.18 (s, 3H); 4.89 (s, 2H); 7.58 (d, J=8.4, 2H); 7.80 (m, 5H), 8.58 (d, J=1.5, 1H); 8.7.1 (s, 1H).

### B) Composé B : 6-(4-Azidomethyl-thiazol-2-yl)-3-(4-bromo-phenyl)-1,9-dimethyl-1,9-dihydro-pyrido[2,3-b]indol-2-one

Dissoudre 400 mg (0.8 mmol) de composé A 3-(4-Bromo-phenyl)-6-(4-chloromethyl-thiazol-2-yl)-1,9-dimethyl-1,9-dihydro-pyrido[2,3-b]indol-2-one dans 10 mL de DMSO. Ajouter 160 mg (2.4 mmol) de NaN3. Chauffer à 45°C pendant une nuit. Revenir à température ambiante. Verser le milieu réactionnel sur de l'eau, filtrer le précipité et laver à l'eau. Reprendre dans de l'éthanol et évaporer à sec. Obtention de 370 mg de poudre jaune.
MS: 504.96

### C) : 6-(4-Aminomethyl-thiazol-2-yl)-3-(4-bromo-phenyl)-1,9-dimethyl-1,9-dihydropyrido[2,3-b]indol-2-one

Dissoudre 370 mg (0.73 mmol) de composé B 6-(4-Azidomethyl-thiazol-2-yl)-3-(4-bromo-phenyl)-1,9-dimethyl-1,9-dihydro-pyrido[2,3-b]indol-2-one dans 40 mL de méthanol et 40 mL d'acide acétique glacial. Ajouter 40 mg de Pd/C. Hydrogéner sous 60psi à 25°C pendant 48 heures. Filtrer le milieu réactionnel sur célite. Laver à l'éthanol. Evaporer à sec le filtrat. Dissoudre le résidu dans un mélange CH2Cl2/MeOH et adsorber sur silice. Purifier sur colonne de silice éluée avec CH2Cl2/MeOH/NH4OH 97/02/01, puis avec CH2Cl2/MeOH/NH4OH 93/05/02. Obtention de 110 mg de poudre jaune pâle.

RMN 1H DMSO-d6 (300 MHz) : 1.94 (bs, 2H); 3.86 (s, 2H); 4.03 (s, 3H); 4.17 (s, 3H); 7.78 (s, 1H); 7.58 (d, J=8.7, 2H); 7.70 (d, J=8.7, 1H); 7.85 (m, 3H); 8.56 (s; 1H); 8.70 (s, 1H).

### EXEMPLE 76 : 3-(4-bromo-phenyl)-6-(1-ethyl-1H-pyrazol-3-yl)-1,9-dimethyl-1,9-dihydro-pyrido[2,3-b]indol-2-one

On procède comme indiqué à l'exemple 36 ci-dessus à partir de 3-(4-bromo-phenyl)-1,9-dimethyl-6-(2H-pyrazol-3-yl)-1,9-dihydm-pyrido[2,3-b]indol-2-one de l'ex 60 ci-dessus et de iodoéthane.

RMN 1H DMSO-d6 (300 MHz) : 1.29/1.43 (t, m/M, J=7.2, 3H); 4.03 (s, 3H); 4.18 (m, 5H); 6.34/6.71 (d, m/M, J=2.1, 1H); 7.60 (m, 3H); 7.80 (m, 4H); 8.07/8.37 (s, m/M, 1H); 8.57 (s, 1H).

### EXEMPLE 77 : 6-(2-Amino-thiazol-5-yl)-3-(2-chloro-4-fluoro-phenyl)-1,9-dimethyl-1,9-dihydro-pyrido[2,3-b]indol-2-one

On procède comme indiqué à l'exemple 21 ci-dessus à partir de 6-(2-Bromo-acetyl)-3-(2-chloro-4-fluoro-phenyl)-1,9-dimethyl-1,9-dihydro-pyrido[2,3-b]indol-2-one [préparé selon selon la préparation 1.1 ci-dessus à partir du composé de l'Ex 65 A ci-dessus, 3-(2-Chloro-4-fluoro-phenyl)-1,9-dimethyl-1,9-dihydro-pyrido[2,3-b]indol-2-one, ] et de thiourée
Tf : 294°C-295°C
MS : 438.8

RMN 1H DMSO-d6 (300 MHz) : 4.00 (s, 3H); 4.14 (s, 3H); 6.89 (s, 1H); 6.99 (s, 2H); 7.22 - 7.29 (td, J = 8.4, J = 2.7, 1H); 7.43 - 7.59 (m, 2H); 7.57 (d, J = 8.7, 1H); 7.73 - 7.77 (dd, J = 8.4, J = 1.5, 1H); 8.17 (s, 1H); 8.27 (d, J = 1.5, 1H).

### EXEMPLE 78 : 6-(2-Aminomethyl-thiazol-4-yl)-3-(4-bromo-phenyl)-1,9-dimethyl-1,9-dihydro-pyrido[2,3-b]indol-2-one

### A) Composé A : {4-[3-(4-Bromo-phenyl)-1,9-dimethyl-2-oxo-2,9-dihydro-1H-pyrido[2,3-b]indol-6-yl]-thiazol-2-ylmethyl}-carbamic acid benzyl ester

On procède comme indiqué à l'exemple 3 ci-dessus à partir de 6-(2-Bromo-acetyl)-3-(-4-bromo-phenyl)-1,9-dimethyl-1,9-dihydro-pyrido[2,3-b]indol-2-one [préparé selon selon la préparation 1.1 ci-dessus à partir de 3-( -4-bromo-phenyl)-1,9-dimethyl-1,9-dihydro-pyrido[2,3-b]indol-2-one, RMN 1H DMSO-d6 (300 MHz): 3.97 (s, 3H); 4.17 (s, 3H); 4.93 (s, 2H); 7.56 (dd, J = 6.9, J = 2.1, 2H); 7.70 - 7.81 (m, 3H); 7.90 - 7.98 (m, 1H); 8.58 (s, 1H); 8.71 (d, J = 1.5, 1H) ] et de N-benzyloxycarbonyl glycine thioamide Tf : 238-240°C MS : 613.18

RMN 1H DMSO-d6 (300 MHz) : 4.01 (s, 3H); 4.15 (s, 3H); 4.57 (d, J = 6.3, 2H); 5.11 (s, 2H); 7.20 - 7.41 (m, 5H); 7.52 - 7.68 (m, 3H); 7.75 - 7.82 (m, 2H); 7.87(d, J = 8.7, 1H) ; 7.93 (s, 1H) ; 8.28 (t, J = 6.0, 1H); 8.52 (s, 2H).

### B) : 6-(2-Aminomethyl-thiazol-4-yl)-3-(4-bromo-phenyl)-1,9-dimethyl-1,9-dihydro-pyrido[2,3-b]indol-2-one

Laisser agiter pendant une nuit à température ambiante une solution de composé A {4-[3-(4-Bromo-phenyl)-1,9-dimethyl-2-oxo-2,9-dihydro-1H-pyrido[2,3-b]indol-6-yl]-thiazol-2-ylmethyl}-carbamic acid benzyl ester (200 mg, 0.32 mmol) et de thioanisole (308 µL, 2.60 mmol) dans 2 mL de TFA. Ajouter H2O, extraire avec AcOEt, laver les phases organiques avec H2O, sécher sur MgSO4, filtrer et concentrer. Purifier par chromatographie sur colonne de silice éluée avec 100% AcOEt puis avec un mélange AcOEt/MeOH 5% puis AcOEt/MeOH 5%/TEA 1%. Reprendre le résidu dans un mélange AcOEt/Et2D 1/1, filtrer et laver avec un minimum d'Et2O.
Obtention de 82 mg de poudre jaune ocre.
T°f : 195°C - 201°C
MS : 480.8
RMN 1H DMSO-d6 (300 MHz) : 4.00 (s, 3H); 4.14 (s, 3H); 4.41 (s, 2H); 7.57 (d, J = 8.1, 2H); 7.66 (d, J = 8.7, 1H); 7.74 (d, J = 8.1, 2H); 7.80 - 7.98 (m, 3H); 8.04 (s, 1H); 8.42 (s, 1H); 8.54 (s, 1H).

### EXEMPLE 79 : 3-(2,4-Dichloro-phenyl)-6-(2-ethyl-2H-pyrazol-3-yl)-1,9-dimethyl-1,9-dihydro-pyrido[2,3-b]indol-2-one

On procède comme à l'exemple 37 ci-dessus avec le composé de la prep. 1.13B , 3-(2,4-Dichlorophényl)-6-[3-(diméthylamino)prop-2-ènoyl]-1,9-diméthyl-1,9-dihydro-2H-pyrido[2,3-b]indol-2-one
T°F : 232°C - 235°C

RMN 1H DMSO-d6 (300 MHz): 1.25 (t, J = 6.9, 2H); 4.00 (s, 3H); 4.02 - 4.30 (m, 6H); 5.72 (s, 1H); 6.31 (s, 1H); 7.25 - 7.35 (d, J = 8.1, 1H); 7.36 - 7.50 (m, 2H); 7.60 - 7.75 (m, 2H); 7.96 (s, 1H); 8.28 (s, 1H).

### EXEMPLE 80 : 3-(2,4-dichloro-phenyl)-6-(5-ethoxymethyl-2H-pyrazol-3-yl)-1,9-dimethyl-1,9-dihydro-pyrido[2,3-b]indol-2-one

### A) composé A : 1-[3-(2,4-dichloro-phenyl)-1,9-dimethyl-2-oxo-2,9-dihydro-1H-pyrido[2,3-b]indol-6-yl]-4-ethoxy-butane-1,3-dione

Dissoudre 200 mg (0.5 mmol) de 6-Acétyl-3-(2,4-dichlorophényl)-1,9-diméthyl-1,9-dihydro-2H-pyrido[2,3-b]dol-2-one, préparation.1.10E dans 5 mL de THF anhydre. Ajouter une pointe de spatule de dibenzo-18-crown-6 et quelques gouttes d'EtOH anhydre. Ajouter une solution de 132 mg (1 mmol) d'éthyléthoxy acetate et 42 mg (1.05 mmol) de NaH 60% dans 5 mL de THF anhydre. Chauffer à reflux pendant 3 heures. Revenir à température ambiante et ajouter de l'AcOEt au milieu réactionnel. Laver la phase organique avec une solution d' HCl 1N. Sécher la phase organique sur Na2SO4, filtrer et évaporer à sec.

Obtention de 280 mg de poudre orange.
Rendement : quantitatif
MS: 485.13

### B) 3-(2,4-dichloro-phenyl)-6-(5-ethoxymethyl-2H-pyrazol-3-yl)-1,9-dimethyl-1,9-dihydro-pyrido[2,3-b]indol-2-one

On procède comme à l'exemple 43 ci-dessus avec le composé A 1-[3-(2,4-dichlorophenyl)-1,9-dimethyl-2-oxo-2,9-dihydro-1H-pyrido[2,3-b]indol-6-yl]-4-ethoxy-butane-1,3- dione et l'hydrate d'hydrazine
T°F : 246°C

RMN 1H DMSO-d6 (300 MHz) : 1:14 (t, J=1.9, 3H); 3.49 (q, J=6.9, 2H); 4.02 (s, 3H); 4.17 (s, 3H); 4.45 (d, J=21, 2H); 6.66 (s, 1H); 7.47 (m, 2H); 7.69 (m, 3H); 8.23 (m, 2H); 13.0 (m, 1H).

### EXEMPLE 81 : 3-(4-bromo-phenyl)-6-(5-hydroxymethyl-1-methyl-1H-pyrazol-3-yl)-1,9-dimethyl-1,9-dihydro-pyrido[2,3-b]indol-2-one

### A) Composé A :4-[3-(4-bromo-phenyl)-1,9-dimethyl-2-oxo-2,9-dihydro-1H-pyrido[2,3-b]indol-6-yl]-2,4-dioxo-butyric acid ethyl ester

On procède comme à l'exemple 80 ci-dessus avec le composé de 1' Ex60B, 6-Acetyl-3- (4-bromo-phenyl)-1,9-dimethyl-1,9-dihydro-pyrido[2,3-b]indol-2-one et le diethyl oxalate

RMN 1H DMSO-d6 (300 MHz) : 1.31 (t, J=7.1, 3H); 4.03 (s, 3H); 4.18 (s, 3H); 4.35 (q, J=7:1, 2H); 7.30 (s, 1H); 7.60 (d, J=8.6, 2H); 7.80 (d, J=8.7, 3H); 8.00 (dd, J=1.7, J=8.8, 1H); 8.72 (s, 1H); 8.86 (s, 1H).

### B) Composé B: 5-[3-(4-bromo-phenyl)-1,9-dimethyl-2-oxo-2,9-dihydro-1H-pyrido[2,3-b]indol-6-yl]-1H-pyrazole-3-caboxylic acid ethyl ester

On procède comme à l'exemple 43 ci-dessus avec le composé A, 4-[3-(4-bromo-phenyl)-1,9-dimethyl-2-oxo-2,9-dihydro-1H-pyrido[2,3-b]indol-6-yl]-2,4-dioxo-butyric acid ethyl ester et l'hydrazine monohydrate.

RMN 1H DMSO-d6 (300 MHz) : 1.30 (t, J=7.20, 3H); 3.98 (s, 3H); 4.12 (s, 3H); 4.30 (q, J= 7.20, 2H); 7.20 (s, 1H); 7.66 (m, 6H); 8.44 (s, 2H); 13.86 (s, 1H).

### C) Composé C : 5-[3-(4-bromo-phenyl)-1,9-dimethyl-2-oxo-2,9-dihydro-1H-pyrido [2,3-b]indol-6-yl]-2-methyl-2H-pyrazole-3-carboxylic acid ethyl ester

On procède comme à l'exemple 36 ci-dessus avec le composé B 5-[3-(4-bromo-phenyl)-1,9-dimethyl-2-oxo-2,9-dihydro-1H-pyrido[2,3-b]indol-6-yl]-1H-pyrazole-3-carboxylic acid ethyl ester et l'iodométhane

RMN 1H DMSO-d6 (300 MHz) : 1.32 (t, J=6.90, 3H); 3.99 (s, 3H); 4.12 (s, 6H); 4.32 (q, J=6.90, 2H); 7.34 (s, 1H); 7.59 (m, 3H); 7.76 (d, J=8.4, 3H); 8.46 (s, 1H); 8.57 (s, 1H).

### D) 3-(4-bromo-phenyl)-6-(5-hydroxymethyl-1-methyl-1H-pyrazol-3-yl)-1,9-dimethyl-1,9-dihydro-pyrido[2,3-b]indol-2-one

Dissoudre 100 mg (0.2 mmol) de composé C : 5-[3-(4-bmmo-phenyl)-1,9-dimethyl-2-oxo-2,9-dihydro-1H-pyrido[2,3-b]indol-C-yl]-2-methyl-2H-pyrazole-3-carboxylic acid ethyl ester dans 5 mL de CH2Cl2 anhydre. Chauffer à reflux puis ajouter 0.2 mL de LiBH4 1M dans THF. Chauffer à reflux pendant 20 heures en rajoutant 3 fois 0.2mL de LiBH4. Revenir à température ambiante et ajouter 1 mL de NaOH 2N. Evaporer à sec le milieu réactionnel. Ajouter de l'eau, acidifier avec HCl 1N et extraire à l'AcOEt. Evaporer à sec la phase organique. Triturer dans l'eau, filtrer, laver à l'eau.
Reprendre dans du méthanol et évaporer à sec.
Obtention de 65 mg de poudre blanche. '

RMN 1H DMSO-d6 (300 MHz) : 3.84 (s, 3H); 4.02 (s, 3H); 4.14 (s, 3H); 4.55 (d, J=5.1, 2H); 5.33 (t, J=5.4, 1H); 6.64 (s, 1H); 7.59 (m, 3H); 7.71 (dd, J=1.5, J=9.2, 1H); 7.81 (d, J=8.4, 2H); 8.38 (s, 1H); 8.58 (s, 1H).

### EXEMPLE 82 : 3-(2,4-Dichloro-phenyl)-6-(5-hydroxymethyl-1-methyl-1H-pyrazol-3-yl)-1,9-dimethyl-1,9-dihydropyrido[2,3-b]indol-2-one

### A) Composé A : 5-[3-(2,4-Dichloro-phényl)-1,9-dimethyl-2-oxo-2,9-dihydro-1H-pyrido[2,3-b]indol-6-yl]-2-methyl-2H-pyrazole-3-carboxylic acid ethyl ester

On procède comme à l'exemple 36 ci-dessus avec le 5-[3-(2,4-Dichloro-phenyl)-1,9-dimethyl-2-oxo-2,9-dihydro-1H-pyrido[2,3-b]indol-6-yl]-2H-pyrazole-3-carboxylic acid ethyl ester ex 43 ci-dessus et l'iodométhane.

### B) 3-(2,4-Dichloro-phenyl)-6-(5-hydroxymethyl-1-methyl-1H-pyrazol-3-yl)-1,9-dimethyl-1,9-dihydro-pyrido[2,3-b]indol-2-one

On procède comme à l'exemple 81D ci-dessus avec le composé A 5-[3-(2,4-Dichloro-phenyl)-1,9-dimethyl-2-oxo-2,9-dihydro-1H-pyrido[2,3-b]indol-6-yl]-2-methyl-2H-pyrazole-3-carboxylic acid ethyl ester

RMN 1H DMSO-d6 (300 MHz) : 3.82 (s, 3H); 4.01 (s, 3H); 4.16 (s, 3H); 4.52 (s, 2H); 5.34 (bs, 1H); 6.62 (s, 1H); 7.47 (m, 2H); 7.62 (m,1H); 7.71 (m, 2H); 8.28 (m, 2H).

### EXEMPLE 83 : 3-(2,4-Dichloro-phenyl)-6-(1-methoxymethyl-4-methyl-1H-pyrazol-3-yl)-1,9-dimethyl-1,9-dihydro-pyrido[2,3-b]indol-2-one

On procède comme à l'exemple 36 ci-dessus avec le composé de l'ex53 ci-dessus, 3-(2,4-Dichloro-phenyl)-1,9-dimethyl-6-(4-methyl-2H-pyrazol-3-y)-1,9-ddrydro-pyrido[2,3-b]indol-2-one

RMN 1H DMSO-d6 (300 MHz) : 224 (s, 3H) ; 3.27 (s, 3H) ; 4.02 (s, 3H) ; 4.18 (s, 3H) ; 5.34 (s, 2H); 7.46-7.49 (m, 2H) ; 7.62-7.74 (m, 4H) ; 8.12 (d, J=0.9, 1H) ; 8.35 (s,1H).
Tf= 208-212 °C

### EXEMPLE 84 : Chlorhydrate du 3-(4-Bromo-phenyl)-6-(5-ethoxymethyl-2H-pyrazol-3-yl)-1,9-dimethyl-1,9-dihydro-pyrido[2,3-b]indol-2-one

### A) Composé A : 1-[3-(4-Bromo-phenyl)-1,9-dimethyl-2-oxo-2,9-dihydro-1H-pyrido[2,3-b]indol-6-yl]-4-ethoxy-butane-1,3-dione

On procède comme à l'exemple 80 ci-dessns avec le composé de l'ex 60B ci-dessus, 6-Acetyl-3-(4-bromo-phenyl)-1,9-dimethyl-1,9-dihydro-pyrido[2,3-b]indo 1-2-one ex et l'éthylethoxy acétate
MS: 495.10

### B) Chlorhydrate du 3-(4-Bromo-phenyl)-6-(5-ethoxymethyl-2H-pyrazol-3-yl)-1,9-dimethyl-1,9-dihydro-pyrido[2,3-b]indol-2-one

On procède comme à l'exemple 43 ci-dessus avec le composé A 1-[3-(4-Bromo-phenyl)-1,9-dimethyl-2-oxo-2,9-dihydro-1H-pyrido[2,3-b]indol-6-yl]-4-ethoxy-butane-1,3-dione et l'hydrazine monohydrate
T°f: >340°C (dec.)

RMN 1H DMSO-d6 (300 MHz) : 1.16 (t, J=6.9, 3H); 3.50. (q, J=6.9, 2H); 4.02 (s, 3H); 4.15 (s, 3H); 4.49 (s, 2H); 6.73 (s, 1H); 7.60 (m, 3H); 7.76 (m, 3H); 8.40 (s, 1H); 8.50 (s, 1H).

### EXEMPLE 85 : N-{2-[3-(4-bromo-phenyl)-1,9-dimethyl-2-oxo-2,9-dihydro-1H-pyrido[2,3-b]indol-6-yl]-thiazol-4-ylmethyl}-2,2-dimethyl-propionamide

Dissoudre 70 mg (0.15 mmol) de composé de 1' ex75 ci-dessus 6-(4-aminomethyl-thiazol-2-yl)-3-4-bromophenyl)-1,9-dimethyl-1,9-dihydro-pyrido[2,3-b]indol-2-orie dans 4 mL de pyridine anhydre. Ajouter 90 µL (0.73 mmol) de chlorure de pivaloyle. Agiter à température ambiante sous argon pendant une nuit. Evaporer à sec le milieu réactionnel, triturer dans une solution de NH4Cl saturée, filtrer et laver à l'eau. Triturer dans un mélange AcOEt/MeOH et filtrer.
Obtention de 30 mg de poudre jaune pâle.
T°f : 250-255°C

RMN 1H DMSO-d6 (300 MHz) : 1.17 (s, 9H); 4.03 (s, 3H); 4.18 (s, 3H); 4.42 (d, J=6.0, 2H); 7.18 (s, 1H); 7.60 (d, J=8.4,2H); 7.72 (d, J=8.1, 1H); 7.86 (m, 3H); 8.16 (m,1H); 8.57 (s, 1H); 8.71 (s, 1H).

### EXEMPLE 86 3-(4-Bromo-phenyl)-1,9-dimethyl-6-[5-(tetrahydro-pyran-2-yloxy methyl)-2H-pyrazol-3-yl]-1,9-dihydro-pyrido[2,3-b]indol-2-one

### A) Composé A : 1-[3-(4-Bromo-phenyl)-1,9-dimethyl-2-oxo-2p-dihydro-1H pyrido[2,3-b]indol-6-yl]-4-(tetrahydro-pyran-2-yloxy)-butane-1,3-dione

On procède comme à l'exemple 80 ci-dessus avec le composé de l'ex60B ci-dessus, 6-Acetyl-3-(4-bromo-phenyl)-1,9-dimethyl-1,9-dihydro-pyrido[2,3-b]indol-2-one et le (tetrahydropyran-2-yloxy)-acetic acid ethyl ester
MS: 55.1.16

### B) : 3-(4-Bromo-phenyl)-1,9-dimethyl-6-[5-(tetrahydro-pyran-2-yloxymethyl)-2H-pyrazol-3-yl]-1,9-dihydro-pyrido[2,3-b]indol-2-one

On procède comme à l'exemple 43 ci-dessus avec le composé A, 1-[3-(4-Bromo-phenyl)-1,9-dimethyl-2-oxo-2,9-dihydro-1H-pyrido[2,3-b]indol-6-y1]-4-(tetrahydro-pyran-2-yloxy)-butane-1,3-dione et l'hydrazine monohydrate
T°f : 240-245°C

RMN 1H DMSO-d6 (300 MHz) : 1.49 (m, 6H); 3.48 (m, 1H); 4.03 (s, 3H); 4.15 (s, 3H); 4.46 (m, 1H); 4.67 (m, 2H); 6.68 (d, J=6.50, 1H); 7.69 (m, 6H); 8.40 (m, 2H); 12.90 (m, 1H).

### EXEMPLE 87 : 3-(4-Bromo-phenyl)-6-(5-hydroxymethyl-2H-pyrazol-3-yl)-1,9-dimethyl-1,9-dihydro-pyrido[2,3-b]indol-2-one

Dissoudre 110 mg (0.2 mol) de composé de exemple 86 ci-dessus, 3-(4-Bromo-phenyl)-1,9-dimethyl-6-[5-(tetrahydro-pyran-2-loxymethyl)-2H-pyrazol-3-yl]-1,9-dihydro-pyrido[2,3-b]indol-2-one dans 6 mL de MeOH. Ajouter une pointe de spatule d'acide p-toluène sulfonique et chauffer à reflux pendant une nuit. Evaporer à sec le milieu réactionnel. Ajouter une solution de NaHCO3 saturée et extraire à l'AcOEt. Evaporer à se la phase organique. Triturer dans l'eau le produit obtenu, filtrer, laver à l'eau. Reprendre dans du méthanol et évaporer à sec.
Obtention de 80 mg de poudre beige.
T°f : 287-290°C

RMN 1H DMSO-d6 (300 MHz) : 4.03 (s, 3H); 4.15 (s, 3H); 4.47/4.53 (s/S, 2H); 5.0/5.29 (bs/BS, 1H); 6.61 (s,1H); 7.67 (m, 6H); 8.46 (m, 2m; 12.67/12.91 (S/s, 1H).

### EXEMPLE 88 3-(2,4-dichloro-phenyl)-1,9-dimethyl-6-[5-(tetrahydro-pyran-2-yloxymethyl)-2H-pyrazol-3-yl]-1,9-dihydro-pyrido[2,3 b]indol-2-one

### A) Composé A :1-[3-(2,4-dichloro-phenyl)-1,9-dimethyl-2-oxo-2,9-dihydro-1H-pyrido[2,3-b]indol-6-yl]-4-(tetrahydro-pyran-2-yloxy)-butano-1,3-dione

On procède comme à l'exemple 80 ci-dessus avec le composé de la prep. 1.10E, 6-Acétyl-3-(2,4-dichlorophényl)-1,9-diméthyl-1,9-dihydro-2H-pyrido[2,3-b]indol-2-one et le (tetrahydropyran-2-yloxy)-acetic acid ethyl ester.
MS: 541.19

### B) 3-(2,4-dichloro-phenyl)-1,9-dimethyl-6-[5-(tetrahydro-pyran-2-yloxymethyl)-2H-pyrazol-3-yl]-1,9-dihydro-pyrido[2,3-b]indol-2-one

On procède comme à l'exemple 80 ci-dessus avec le Composé A , 1-[3-(2,4-dichlorophenyl)-1,9-dimethyl-2-oxo-2,9-dihydro-1H-pyrido[2,3-b]indol-6-yl]-4-(tetrahydropyran-2-yloxy)-butane-1,3-dione et l'hydrazine monohydrate
T°f : 185-190°C

RMN 1H DMSO-d6 (300 MHz) : 1.39 (m, 6H); 3.32 (m, 1H); 3.82 (m, 1H); 4.02 (s, 3H); 4.17 (s, 3H); 4.47 (bs, 1H); 4.63 (m, 2H); 6.67 (s, 1H); 7.47 (m, 2H); 7.69 (m, 3H); 8.26 (m, 2H);12.90 (m, 1H).

### EXEMPLE 89 : 3-(2,4-dichloro-phenyl)-6-(5-hydroxymethyl-2H-pyrazol-3-yl)-1,9-dimethyl-1,9-dihydro-pyrido[2,3-b]indol-2-one

On procède comme à l'exemple 87 ci-dessus avec le composé de l'exemple 88B, 3-(2,4-dichloro-pheyl)-1,9-dimethyl-6-[5-(tetrahydro-pyran-2-yloxymethyl)-2H-pyrazol-3-yl]-1,9-dihydro-pyrido[2,3-b]indol-2-one
T°f : 283-289°C

RMN 1H DMSO-d6 (300 MHz) 4.02 (s, 3H); 4.17 (s, 3H); 4.50 (m, 2H); 4.99/5.28 (s/S, 1H); 6.59 (s, 1H); 7.48 (m, 2H); 7.69 (m, 3H); 8.28 (m, 2H); 12.65/12.90 (S/s, 1H).

### EXEMPLE 90 : 3-(2,4-Dichloro-phenyl)-1-methyl-6-(1-methyl-1H-pyrazol-3-yl)-2-oxo-1,2-dihydro-pyrido[2,3-b]indole-9-carbonitrile

Dissoudre 460 mg (1.09 mmol) de composé de l'ex 37 ci-dessus, 3-(2,4-Dichlorophényl)-1-méthyl-6-(1-méthyl-1H-pyrazol-3-yl)-1,9-dihydro-2H-pyrido [2,3-b]indol-2-one dans 50 mL de CH2Cl2. Ajouter 576 mg (5.43 mmol) de BrCN, 4.4 mg (0.36 mmol) de DMAP puis 308 µL (2.17 mmol) de triethylamine. Agiter à température ambiente pendant 18h. Ajouter H2O, extraire avec CH2Cl2, sécher sur MgSO4, filtrer, concentrer et purifier par colonne de silice éluée avec AcOEt/cyclohexane 50%.
Obtention de 128 mg de poudre blanche.
Tf : 263°C- 266°C
MS: 448.1

RMN 1H DMSO-d6 (300 MHz) : 3.89 (s, 3H); 3.99 / 4.00 (s, M/m, 3H); 6.46 /.6.72 (s, m/M, 1H); 7.40 - 7.56 (m, 2H); 7.57 - 7.70 (m, 1H); 7.70 - 7.87 (m, 2H); 7.88 - 8.98 (d, J = 8.07,1 1H); 8.19 / 8.42 (s, m/M, 2H).

### EXEMPLE 91 : 3-(2,4-Dichloro-phenyl)-6-(5-ethoxymethyl-2-ethyl-2H-pyrazol-3-yl)-1,9-dimethyl-1,9-dihydro-pyrido[2,3-b]indol-2-one

On procède comme à l'exemple 37 ci-dessus avec le composé de l'Ex80A, 1-[3-(2,4-dichloro-phenyl)-1,9-dimethyl-2-oxo-2,9-dihydro-1H-pyrido[2,3-b]indol-6-yl]-4-etboxy-butane-1,3-dione et l'ethylhydrazine oxalate.

RMN 1H DMSO-d6 (300 MHz) : 1.14 (t, J=6.99, 3H); 1.28 (t, J=7.2, 3H); 3.50 (q, J=6.99, 2H); 4.03 (s, 3H); 4.11 (q, J=7.2, 2H); 4.2 (s, 3H); 4.40 (s, 2H); 6.30 (s, 1H); 7.35 (dd, J=1.5, J=7.5,1H); 7.45 (m, 2H); 7.70 (m, 2H); 8.00 (s, 1H); 8.32 (s, 1H).
T°f=163°C-167°C

### EXEMPLE 92 : 3-(4-fluoro-phenyl)-6-(5-ethoxymethyl-2-ethyl-2H-pyrazol-3-yl)-1,9-dimethyl-1,9-dihydro-pyrido[2,3-b]indol-2-one

### A) ComposéA :1-[3-(4-fluoro-phenyl)-1,9-dimethyl-2-oxo-2,9-dihydro-1H-pyrido[2,3-b]indol-6-yl]-4-ethoxy-butane-1,3-dione

On procède comme à l'exemple 80 ci-dessus avec le composé de l'Ex72B ci-dessus, 6-Acetyl-3-(4-fluoro-phenyl)-1,9-dimethyl-1,9-dihydro-pyrido[2,3-b]indol-2-one et l'éthylethoxy acetate

### B) 3-(4-fluoro-phenyl)-6-(5-ethoxymethyl-2-ethyl-2H-pyrazol-3-yl)-1,9-dimethyl-1,9-dihydro-pyrido[2,3-b]indol-2-one

On procède comme à l'exemple 37 ci-dessus avec le composé A, 1-[3-(4-fluoro-phenyl)-1,9-dimethyl-2-oxo-2,9-dihydro-1H-pyrido[2,3-b]indol-6-yl]-4-ethoxy butane-1,3-dione et l'ethylhydrazine oxalate.

RMN 1H DMSO-d6 (300 MHz) : 1.15 (t, J=7.02, 3H); 1.29 (t, J=7.2, 3H); 3.50 (q, J=6.99, 2H); 4.04 (s, 3H); 4.13 (m, 5H); 4.41 (s, 2H); 6.32 (s, 1H); 7.23 (t, J=8.9, 2H); 7.33 (dd, J=1.5, J=8.4, 1H); 7.70 (d, J=8.7, 1H); 7.80 (m, 2H); 8.07 (s, 1H); 8.52 (s, 1H).
T°f=77°C-83°C

### EXEMPLE 93: 3-(4-Bromo-phenyl)-6-(5-ethoxymethyl-1-methyl-1H-pyrazol-3-yl)-1,9-dimethyl-1,9-dihydro-pyrido[2,3-b]indol-2-one

On procède comme à l'exemple 36 ci-dessus avec le composé de 1' ex84, 3-(4-Bromo-phenyl)-6-(5-ethoxyneothyl-1H-pyrazol-3-yl)-1,9-dimethyl-1,9-dihydro-pyrido [2,3-b]indol-2-one et l'iodométhane.

RMN 1H DMSO-d6 (300 MHz) : 1.16 (t, J=6.99, 3H) ; 3.53 (q, J=6.99, 2H) ; 3.84/3.86 (S/s, 3H) ; 4.02/4.04 (S/s, 3H) ; 4.14/4.17 (S/s, 3H) ; 4.39/4.54 (s/S, 2H); 6.38/6.72 (s/S, 1H) ; 7.59 (m, 3H); 7.74 (m, 3H); 8.18/8.36 (s/S, 1H); 8.57 (s, 1H).
T°f= 220°C-225°C

### EXEMPLE 94 : 3-(2,4-Dichloro-phenyl)-6-(5-dimethylaminomethyl-2H-pyrazol-3-yl)-1,9-dimethyl-1,9-dihydro-pyrido[2,3-b]indol-2-one

### A) composé A : 1-[3-(2,4-Dichloro-phenyl)-1,9-dimethyl-2-oxo-2,9-dihydro-1H-pyrido[2,3-b]indol-6-yl]-4-dimethylamino-butane-1,3-dione

On procède comme à l'exemple 80 ci-dessus avec le composé de la prep.1.10E, 6-Acétyl-3-(2,4-dichlorophényl)-1,9-diméthyl-1,9-dihydro-2H-pyrido[2,3-b]indol-2-one et le dimethylglycine methyl ester
MS:484.17.

### B) 3-(2,4-Dichloro-phenyl)-6-(5-dimethylaminomethyl-2H-pyrazol-3-yl)-1,9-dimethyl-1,9-dihydro-pryido[2,3-b]indol-2-one

On procède comme à l'exemple 43 ci-dessus avec le composé A 1-[3-(2,4-Dichloro-phenyl)-1,9-dimethyl-2-oxo-2,9-dihydro-1H-pyrido[2,3-b]indol-6-yl]-4-dimethylamino-butane-1,3-dione et l'hydrazine monohydrate

RMN 1H DMSO-d6 (300 MHz) : 2.18 (s, 6H) ; 3.47 (s, 2H) ; 4.02 (s, 3H) ; 4.17 (s, 3H) ; 6.58 (s, 1H) ; 7.46 (m, 2H) ; 7.69 (m, 3H) ; 8.29 (m, 2H) ; 12.66/12.90 (S/s, 1H).
T°f= 238°C-243°C

### EXEMPLE 95 3-(2,4-Dichloro-phenyl)-6-(4-hydroxymethyl-thiazol-2-yl)-1,9-dimethyl-1,9-dihydro-pyrido[2,3-b]indol-2-one

### A) Composé A : 3-[3-(2,4-Dichloro-phenyl)-1,9-dimethyl-2-oxo-2,9-dihydro-1H-pyrido[2,3-b]indole-6-carboximidoylsulfanyl]-2-oxo-propionic acid ethyl ester

Agiter à température ambiante pendant 2h30 une solution contenant 200 mg (0.48 mmol) de composé 3-(2,4-Dichloro-phenyl)-1,9-dimethyl-2-oxo-2,9-dihydro-1H-pyrido[2,3-b]indole-6-carbothioic acid amide de la prep.1.6 ci-dessus et 91 µL (0.72 mmol) d'ethylbromopyruvate dans 3 mL de DMF. Ajouter 5 mL de H2O, filtrer le précipité formé, laver avec H2O puis avec un minimum d'Et2O (poudre hygroscopique devenue pâteuse). Reprendre le résidu pâteux dans CH2Cl2 et MeOH et concentrer à sec. L'utiliser tel quel pour l'étape B.
MS : 529.98

### B) Composé B : 2-[3-(2,4-Dichloro-phenyl)-1,9-dimethyl-2-oxo-2,9-dihydro-1H-pyrido[2,3-b]indol-6-yl]-thiazole-4-carboxylic acid ethyl ester

Ajouter 3 mL de H2SO4 au composé A, 3-[3-(2,4-Dichloro-phenyl)-1,9-dimethyl-2-oxo-2,9-dihydro-1H-pyrido[2,3-b]indole-6-carboximidoylsulfanyl]-2-oxo-propionic acid ethyl ester obtenu précédemment et agiter à température ambiante pendant 16h. Ajouter 15 mL d'H2O et 5 mL de NaOH 1M. Extraire avec EtOAC et concentrer sans sécher sur MgSO4.
Obtention de 218 mg de poudre jaune.
MS : 512.03

RMN 1H DMSO-d6 (300 MHz) 1.31 (t, J = 7.11, 3H); 4.00 (s, 3H); 4.18 (s, 3H); 4.32 (q, J = 7.05, 2H); 7.45 (m, 2H); 7.67 (d, J =1.8, 1H); 7.73 (d, J = 8.67, 1H); 7.90 (dd, J = 8.61, J = 1.74, 1H); 8.42 (s, 1H); 8.49 (s, 1H); 8.51 (d, J = 1.53, 1H).

### C) 3-(2,4-Dichloro-phenyl)-6-(4-hydroxymethyl-thiazol-2-yl)1,9-dimethyl-1,9-dihydro-pyrido[2,3-b]indol-2-one

Chauffer à reflux une solution contenant 218 mg (0.43 mmol) de composé B, 2-[3-(2,4-Dichloro-phenyl)-1,9-dimethyl-2-oxo-2,9-dihydro-1H-pyrido [2,3-b]indol-6-yl]-thiazole-4-carboxylic acid ethyl ester dans 8 mL de CH2Cl2. Ajouter 430 µL (0.85 mmol) de LiBH4 2M dans THF. Chauffer à reflux pendant 16h. Laisser refroidir, ajouter 10 mL de NaOH 1M et concentrer le milieu réactionnel à sec. Reprendre le résidu dans H2O, le filtrer, le laver avec H2O puis avec MeOH.
Obtention de 177 mg de poudre blanche.
T°f : 240-243°C
MS : 470.3

RMN 1H DMSO-d6 (300 MHz) : 3.99 (s, 3H); 4.16 (s, 3H); 4.59 (s, 2H); 5.36 (bs, 1H); 7.36 (s, 1H); 7.39 - 7.47 (m, 2H); 7.65 (d, J = 1.8, 1H); 7.68 (d, J = 8.88, 1H); 7.85 (dd, J = 8.61, J = 1.59, 1H); 8.38 (s, 1H); 8.44 (d, J = 1.41, 1H).

### EXEMPLE 96 2,2-Dimethyl-propionic acid 4-[3-(2,4-dichloro-phenyl)-1-methyl-2-oxo-2,9-dihydro-1H-pyrido[2,3-b]indol-6-yl]-thiazol-2-ylmethyl ester

### Décrit dans l'exemple 3A

RMN 1H DMSO-d6 (300 MHz) : 1.22 (s, 9H); 3.70 (s, 3H); 5.45 (s, 2H); 7.46 (s, 2H); 7.52 (d, J = 8.43, 1H); 7.68 (m, 1H); 7.85 (dd, J = 8.46, J = 1.53, 1H); 7.99 (s, 1H); 8.25 (s,1H); 8.46 (s,1H); 12.24 (s, 1H).

### EXEMPLE 97 : 2,2-Dimethyl-propionic acid 4-[9-cyano-3-(2,4-dichloro-phenyl)-1-methyl-2-oxo-2,9-dihydro-1H-pyrido[2,3-b]indol-6-yl]-thiazol-2-ylmethyl ester

On procède comme à l'exemple 90 ci-dessus avec le composé de l'Ex96, 2,2-Dimethyl-propionic acid 4-[3-(2,4-dichloro-phenyl)-1-methyl-2-oxo-2,9-dihydro-1H-pyrido[2,3-b]indol-6-yl]-thiazol-2-ylmethyl ester
T°f : 141-143 °C
MS : 565.1

RMN 1H DMSO-d6 (300 MHz) : 1.19 (s, 9H); 3.97 (s, 3H); 5.44 (s, 2H); 7.35 - 7.55 (m, 2H); 7.70 (m, 2H); 8.00 (m, 1H); 8.12 (d, J = 4.50, 1H); 8.39 (d, J = 4.50, 1H); 8.57 (s, 1H).

### EXEMPLE 98 3-(2,4-Dichloro-phenyl)-6-(2-methoxymethyl-thiazol-4-yl)-1,9-dimethyl-1,9-dihydro-pyrido[2,3-b]indol-2-one

On procède comme à l'exemple 10 ci-dessus avec le composé de l'ex 9, 3-(2,4-Dichloro-phenyl)-6-(2-hydroxymethyl-thiazol-4-yl)-1,9-dimethyl-1,9-dihydropyrido[2,3-b]indol-2-one et l'iodométhane

RMN 1H DMSO-d6 (300 MHz) : 3.42 (s, 3H) ; 4.00 (s, 3H) ; 4.16 (s, 3H) ; 4.7.5 (s, 2H) ; 7.41-7.46 (m, 2H) ; 7.63-7.66 (m, 2H) ; 7.88 (dd, J=4.4, J=8.5, 1H); 7.97 (s, 1H) ; 8.25 (s, 1H) ; 8.45 (s, 1H).
Tf= 180-183 °C

### EXEMPLE 99 3-(2,4-Dichloro-phenyl)-6-[1-(2,2-dimethyl-propionyl)-4-methyl-1H-pyrazol-3-yl]-1,9-dimethyl-1,9-dihydro-pyrido[2,3-b]indol-2-one

Dissoudre 0.14 g (0.32 mmol) de composé de l'ex 53, 3-(2,4-Dichloro-phenyl)-1,9-dimethyl-6-(4-methyl-2H-pyrazol-3-yl)-1,9- dihydro-pyrido[2,3-b]indol-2-one dans 5 mL de pyridine. Rajouter 0.2 mL (1.6 mmol) de chlorure de pivaloyle. Agiter à température ambiante pandant 2 heures. Verser le milieu réactionnel sur de l'eau, extraire à l'AcOEt. Laver avec une solution de NaCl saturée, sécher sur Na2SO4 puis adsorber sur silice. Purifier par chromatographie sur colonne de silice éluée avec un mélange AcOEt/Cyclohexane 75/25 puis AcOEt pur.
Obtention de 100 mg de poudre blanche.
RMN 1H DMSO-d6 (300 MHz) : 1.50 (s, 9H) ; 2.28 (s, 3H) ; 4.03 (s, 3H) ; 4.20 (s, 3H) ; 4.10 (s, 2H) ; 7.42-7.50 (m, 2H) ; 7.67-7.75 (m, 3H) ; 8.22 (s, 2H) ; 8.39 (s, 1H). Tf= 239-240 °C

### EXEMPLE 100 3-(4-fluoro-phenyl)-6-(4-hydroxymethyl-thiazol-2-yl)-1,9-dimethyl-1,9-dihydro-pyrido[2,3-b]indol-2-one

### A) Composé A : 3.(4-Fluoro-phenyl)-1,9-dimethyl-2-oxo-2,9-dihydro-1H-pyrido[2,3-b]indole-6-carbonitrile

On procède comme indiqué à la préparation 1.2B ci-dessus avec du chlorhydrate de 1-Méthyl-2-(méthylamino)-1H-indole-5-carbonitrile prep1.5C ci-dessus et du 2-(4-fluoro-phenyl)-3-dimethylamino-acrylic acid methyl ester, Prep.2.12 WO5108398

RMN 1H DMSO-d6 (300 MHz) : 3.96 (s, 3H); 4.12 (s, 3H); 7.20 (t, J = 8.94, 2H); 7.60 (dd, J = 8.52, J = 1.53,1H); 7.75 (m, 3H); 8.39 (d, J =1.26, 1H); 8.44 (s, 1H). B) Composé B : 3-(4-fluoro-phenyl)-1,9-dimethyl-2-oxo-2,9-dihydro-1H-pyrido[2,3-b ]indole-6-carbothioic acid amide

On procède comme indiqué à la préparation 1.6 ci-dessus avec le composé A, 3-(4-Fluoro-phenyl)-1,9-dimethyl-2-oxo-2,9-dihydro-1H-pyrido[2,3-b]indole-6-carbonitrile et du O,O'-diéthyldithiophosphate
MS : 366.16

RMN 1H DMSO-d6 (300 MHz) : 4.02 (s, 3H); 4.16 (s, 3H); 7.22 (t, J = 8.85, 2H); 7.61 (d, J = 8.76, 1H); 7.82 (m, 2H); 7.91 (dd, J = 8.79, J = 1.32, 1H); 8.41 (s, 1H); 8.56 (s, 1H); 9.39 (s, 1H); 9.72 (s, 1H).

### C) Composé C : 3-[3-(4-fluoro-phenyl)-1,9-dimethyl-2-oxo-2,9-dihydro-1H-pyrido[2, 3-b]indole-6-carboximidoylsulfanyl]-2-oxo-propionic acid ethyl ester

On procède comme indiqué dans l'ex95 ci-dessus avec le composé B, 3-(4-fluoro-phenyl)-1,9-dimethyl-2-oxo-2,9-dihydro-1H-pyrido[2,3-b]indole-6-carbothioic acid amide et l'éthylbromopyruvate
MS : 480.4

### D) Composé D : 2.[3-(4-fluoro-phenyl)-1,9-dimethyl-2-oxo-2,9-dihydro-1H-pyrido[2,3-b]indol-6-yl]-thiazole-4-carboxylic acid ethyl ester

On procède comme indiqué dans l'ex95 ci-dessus avec le composé C, 3-[3-(4-fluoro-phenyl)-1,9-dimethyl-2-oxo-2,9-dihydro-1H-pyrido[2, 3'-b]indole-6-carboximidoyl sulfanyl]-2-oxo-propionic acid ethyl ester obtenu précédemment MS : 462.4

RMN 1H DMSO-d6 (300 MHz) : 1.34 (t, J = 7.11, 3H); 4.03 (s, 3H); 4.18 (s, 3H); 4.35 (q, J = 7.05, 2H); 7.23 (t, J = 8.76, 2H); 7.74 (d, J = 8.67, 1H); 7.82 - 7.93 (m, 3H); 8.52 (s, 1H); 8.60 (s, 1H); 8.65 (s, 1H).

### E) 3-(4-fluoro-phenyl)-6-(4-hydroxymethyl-thiazol-2-yl)-1,9-dimethyl-1,9-dihydropyrido[2,3-b]indol-2-one

On procède comme indiqué dans l'ex95 ci-dessus avec le composé D, 2-[3-(4-fluoro-phenyl)-1,9-dimethyl-2-oxo-2,9-dihydro-1H-pyrido[2,3-b]indol-6-yl]-thiazole-4-carboxylic acid ethyl ester
T°f : 154-155°C
MS : 420.19

RMN 1H DMSO-d6 (300 MHz) : 4.02 (s, 3H); 4.16 (s, 3H); 4.64 (d, J = 5.4, 2H); 5.36 (t, J = 5.64, 1H); 7.22 (t, J = 8.91, 2H); 7.40 (s, 1H); 7.69 (d, J = 8.64, 1H); 7.80 - 7.90 (m, 3H); 8.54 (d, J = 1.32, 1H); 8.62 (s, 1H).

### EXEMPLE 101 3-(4-fluoro-phenyl)-6-(4-methoxymethyl-thiazol-2-yl)-1,9-dimethyl-1,9-dihydro-pyrido[2,3-b]indol-2-one

Dissoudre 120 mg (0.286 mmol) de composé de l'ex 100 ci-dessus, 3-(4-fluoro-phenyl)-6-(4-hydroxymethyl-thiazol-2-yl)-1,9-dimethyl-1,9-dihydro-pyrido[2,3-b]indol-2-one, dans 3 mL de DMF. Ajouter 17mg (0.429 mmol) de NaH 60% ainsi qu'une spatulée de dibenzo-18-crown-6. Après 5 minutes d'agitation, ajouter 27 µL (0.429 mmol) d'iodométhane. Laisser agiter 4h à température ambiante. Ajouter une solution saturée de NaHCO3, extraire avec AcOEt puis avec CH2Cl2 et concentrer à sec sans sécher. Reprendre le résidu dans MeOH, filtrer le précipité blanc, laver avec MeOH puis avec un minimum d'Et2O
Obtention de 94 mg de poudre blanche.
T°f : 222-223°C
MS : 434.11

RMN 1H DMSO-d6 (300 MHz) : 3.37 (s, 3H); 4.01 (s, 3H); 4.16 (s, 3H); 4.54 (s, 2H); 7.22 (t, J = 8.61, 2H); 7.55 (s, 1H); 7.69 (d, J = 8.61, 1H); 7.82 - 7.90 (m, 3H); 8.55 (s, 1H); 8.63 (s, 1H).

### EXEMPLE 102 3-(2,4-Dichloro-phenyl)-6-(4-methoxymethyl-thiazol-2-yl)-1,9-dimethyl-1,9-dihydro-pyrido[2,3-b]indol-2-one

On procède comme indiqué dans l'ex101 ci-dessus avec le composé de l'Ex 95 ci-dessus, 3-(2,4-Dichloro-phenyl)-6-(4-hydroxymethyl-thiazol-2-yl)-1,9-dimethyl-1,9-dihydro-pyrido[2,3-b]iadol-2-one et l'iodométhane
T°f :318-322°C
MS : 484.07

RMN 1H DMSO-d6 (300 MHz) : 3.35 (s, 3H); 4.00 (s, 3H); 4.18 (s, 3H); 4.52 (s, 2H); 7.40 - 7.50 (m, 2H); 7.52 (s, 1H); 6.67 (d, J = 1.8, 1H); 7.71 (d, J = 8.64, 1H); 7.86 (dd, J = 8.61, J = 1.74, 1H); 8.42 (s, 1H); 8.47 (d, J = 1.56, 1H).

### EXEMPLE 103 3-(2,4-Dichloro-phenyl)-6-(4-ethoxymethyl-thiazol-2-yl)-1,9-dimethyl-1,9-dihydro-pyrido[2,3-b]indol-2-one

On procède comme indiqué dans l'ex101 ci-dessus avec le composé de l'Ex 95 ci-dessus , 3-(2,4-Dichloro-phenyl)-6-(4-hydroxymethyl-thiazol-2-yl)1,9-dimethyl-1,9-dihydm-pyrido[2,3-b]indol-2-one et l'iodoéthane
T°f:248-250°C
MS : 498.2

RMN 1H DMSO-d6 (300 MHz) : 1.17 (t, J = 6.96, 3H); 3.56 (q, J = 6.96, 2H); 4.02 (s, 3H); 4.19 (s, 3H); 4.56 (s, 2H); 7.40 - 7.56 (m, 3H); 7.72 (d, J =10.5, 2H); 7.88 (d, J = 7.71, 1H); 8.43 (s, 1H); 8.49 (s, 1H).

### EXEMPLE 104 3-(4-Chloro phenyl)-1,9-dimethyl-6-(2H-pyrazol-3-yl)-1,9-dihydropyrido[2,3-b]indol-2-one

### A) Composé A : 3-(4-Chloro-phenyl)-1,9-dimethyl-1,9-dihydro-pyrido[2,3-b]indol-2-one

On procède comme indiqué à la preparation 1.2B ci-dessus avec du chlorhydrate de Methyl-(1-methyl-1H-indol-2-yl)-amino (préparé selon les modes opératoires décrits dans WO 2004/041817) et du 3-Dimethylamino-2-(4-chloro-phenyl)-acrylic acid methyl ester de la prep.2.8 WO2005/108398

RMN 1H DMSO-d6 (300 MHz) : 3.99 (s, 3H); 4.10 (s, 3H); 7.15 - 7.30 (m, 2H); 7.41 (m, 2H); 7.57 (d, J = 7.86, 1H); 7.79 (m, 2H); 7.90 (dd, J = 7.74, J = 1.05, 1H); 8.42 (s, 1H).

### B) composé B : 6-Acetyl-3-(4-chloro-phenyl)-1,9-dimethyl-1,9-dihydro-pyrido[2,3-b]indol-2-one

On procède comme indiqué à a preparation 1.9B ci-dessus avec le Composé A 3-(4-chloro-phenyl)-1,9-dimethyl-1,9-dihydro-1H-pyrido[2,3-b]indol-2-one et du acetyl chloride

### C) composé C : (4-Chloro-phenyl)-6-(3-dimethylamino-acryloyl)-1,9-dimethyl-1,9-dihydro-pyrido[2,3-b]indol-2-one

On procède comme indiqué à la preparation 1.9C ci-dessus avec du composé B 6-Acetyl-3-(4-chloro-phenyl)-1,9-dimethyl-1,9-dihydro-pyrido[2,3-b]indol-2-one et du réactif de Bredereck.

### D) 3-(4-Chloro phenyl)-1,9-dimethyl-6-(2H-pyrazol-3-yl)-1,9-dihydro-pyrido[2,3-b]indol-2-one

On procède comme indiqué à l'ex 36 ci-dessus avec du composé 3-(4-chlorophenyl)-6-(3-dimethylamino-acryloyl)-1,9-dimethyl-1,9-dihydro-pyrido[2,3-b]indol-2-one et de l'hydrazine monohydrate.
T°f : 340-341 °C
MS : 389.17

RMN 1H DMSO-d6 (300 MHz) : 4.01 (s, 3H); 4.14 (s, 3H); 6.73 (s, 1H); 7.45 (d, J = 8.52, 2H); 7.62 (m, 1H); 7.77 (m, 2H); 7.85 (d, J = 8.31, 2H); 8.38 (s, 1H); 8.51 (m, 1H), 12.80/13.20 (bs, M/m, 1H).

### EXEMPLE 105 3-(2,4-Dichloro-phenyl)-1,9-dimethyl-6-(5-piperidin-1-ylmethyl-2H-pyrazol-3-yl)-1,9-dihydro-pyrido[2,3-b]indol-2-one

### A) Composé A : 3-(2,4-Dichloro-phenyl)-1,9-dimethyl-2-oxo-2,9-dihydro-1H-pyrido[2,3-b]indol-6-yl]-4-piperidin-1-yl-butane-1,3-dione

On procède comme à l'exemple 37 ci-dessus avec le composé de la prep. 1.10E 6-Acétyl-3-(2,4-dichlorophényl)-1,9-diméthyl-1,9-dihydro-2*H*-pyrido[2,3-b]indol-2-one et le piperidin-1-yl-acetic acid methyl ester
MS: 524.19

### B) 3-(2,4-Dichloro-phenyl)-1,9-dimethyl-6-(5-piperidin-1-ylmethyl-2H-pyrazol-3-yl)-1,9-dihydro-pyrido[2,3-b]indol-2-one

On procède comme à l'exemple 80 ci-dessus avec le composé A, 3-(2,4-Dichlorophenyl)-1,9-dimethyl-2-oxo-2,9-dihydro-1H-pyrido[2,3-b]indol-6-yl]-4-piperidin-1-yl-butane-1,3-dione et l'hydrazine monohydrate

RMN 1H DMSO-d6 (300 MHz) : 1.50 (m, 6H) ; 2.37 (bs, 4H) ; 3.50 (bs, 2H) ; 4.02 (s, 3H) ; 4.17 (s, 3H); 6.58 (s, 1H) ; 7.47 (m, 2H) ; 7.69 (m, 3H) ; 8.30 (m, 2H) ; 12.62/12.89 (S/s, 1H).
T°f= 228°C-235°C

### EXEMPLE 106 Chlorhydrate de 3-(2,4-Dichloro-phenyl)-1,9-dimethyl-6-(1-methyl-5-piperidin-1-ylmethyl-1H-pyrazol-3-yl)-1,9-dihydro-pyrido[2,3-b]indol-2-one

On procède comme à l'exemple 36 ci-dessus avec le composé de l'ex 105, 3-(2,4-Dichloro-phenyl)-1,9-dimethyl-6-(5-piperidin-1-ylmethyl-2H-pyrazol-3-yl)-1,9-dihydro-pyrido[2,3-b]indol-2-one

RMN 1H DMSO-d6 (300 MHz) : 1.78 (m, 6H) ; 2.90 (m, 2H) ; 3.42 (m, 2H) ; 4.03 (s, 3H) ; 4.19 (s, 3H); 4.26 (m, 2H); 6.89 (s, 1H) ; 7.47 (m, 2H) ; 7.70 (m, 3H) ; 8.20 (s, 1H) ; 8.30 (s,1H); 10.42.(s, 1H).
T°f= 203°C-208°C

### EXEMPLE 107 3-(2,4-Dichloro-phenyl)-6-(5-ethoxymethyl-1-methyl-1H-pyrazol-3-yl)-1,9-dimethyl-1,9-dihydro-pyrido[2,3-b]indol-2-one

On procède comme à l'exemple 101 ci-dessus avec le composé de 1' Ex82, 3-(2,4-Dichloro-phenyl)-6-(5-hydroxymethyl-1-methyl-1H-pyrazol-3-yl)-1,9-dimethyl-1,9-dihydro-pynrido[2,3-b]indol-2-one et l'iodoéthane

RMN 1H DMSO-d6 (300 MHz) : 1.16 (t, J=6.99, 3H); 3.50 (q, J=6.99, 2H) ; 3.83 (s, 3H) ; 4.02 (s, 3H) ; 4.16 (s, 3H) ; 4.53 (s, 2H); 6.69 (s, 1H) ; 7.46 (m, 2H); 7.60 (d, J=8.64, 1H); 7.72 (m, 2H); 8.28 (s, 2H).
T°f= 137°C-142°C

### EXEMPLE 108 Chlorhydrate du 3-(2,4-Dichloro-phenyl)-6-[2-ethyl-5-(2-pyrrolidin-1-yl-ethoxymethyl)-2H-pyrazol-3-yl]-1,9-dimethyl-1,9-dihydro-pyrido[2,3-b]indol-2-one

On procède comme à l'exemple 101 ci-dessus avec le composé de l'Ex 61 3-(2,4-Dichloro-phenyl)-6-(2-ethyl-5-hydroxymethyl-2H-pyrazol-3-yl)-1,9-dimethyl-1,9-dihydro-pyrido[2,3-b]indol-2-one et la chloroethylpyrrolidine.

RMN 1H DMSO-d6 (300 MHz) : 1.27 (t, J=4.17, 3H) ; 1.87 (m, 4H) ; 3.02 (m, 2H) ; 3.35 (m, 2H) ; 3.50 (m, 2H) ; 3.78 (m, 4H); 4.04 (s, 3H) ; 4.13 (q, J=7.23, 2H); 4.21 (s, 3H); 4.52 (s, 2H); 6.40 (s, 1H); 7.35 (dd, J=1.5, J=8.5, 1H); 7.45 (m, 2H); 7.73 (m, 2H); 7.99 (s, 1H); 8.32 (s, 1H).
T°f= 125°C-130°C

### EXEMPLE 109 : 6-(4-Aminomethyl-thiazol-2-yl)-3-(2,4-dichloro-phenyl)-1-methyl-1,9-dihydro-pyrido[2,3-b]indol-2-one

### A) Composé A 3-(2,4-Dichloro-phenyl)-1-methyl-2-oxo-2,9-dihydro-1H-pyrido[2,3-b]indole-6-carbothioic acid amide

On procède comme pour la prep.1.6 ci-dessus avec le 3-(2,4-Dichloro-phenyl)-1-methyl-2-oxo-2,9-dihydro-1H-pyrido[2,3-b]indole-6-carbonitrile ( RMN 1H DMSO-d6 (300 MHz) : 3.70 (s, 3H); 7.47 (m, 2H); 7.63 (m, 3H); 8.27 (s, 1H); 8.40 (s, 1H); 12.69 (s, 1H)) et le O,O'-diéthyldithiophosphate

RMN 1H DMSO-d6 (300 MHz) : 3.70 (s, 3H) ; 7.46 (m, 3H) ; 7.69 (s, 1H) ; 7.87 (d, J=8.52, 1H) ; 8.19 (s,1H) ; 8.46 (s,1H); 9.38/9.69 (2s, 2H); 12.39 (s, 1H).

### B) Composé B: 6-(4-Chloromethyl-thiazol-2-yl)-3-(2,4-dichloro-phenyl)-1-methyl-1,9-dihydro-pyrido[2,3-b]indol-2-one

On procède comme dans l'ex75 avec le Composé A : 3-(2,4-Dichloro-phenyl)-1-methyl-2-oxo-2,9-dihydro-1H-pyrido[2,3-b]indole-6-carbothioic acid amide et la 1,3-dichloroacétone. RMN 1H DMSO-d6 (300 MHz) : 3.71 (s, 3H ; 4.87 (s, 2H) ; 7.47 (m, 2H) ; 7.57 (d, J=8.4, 1H); 7.70 (m, 2H) ; 7.85 (dd, J=1.8, J=8.4, 1H); 8.40 (s, 1H); 8.49 (s, 1H); 12.39 (s, 1H).

### C) Composé C : 6-(4-Azidomethyl-thiazol-2-yl)-3-(2,4-dichloro-phenyl)-1-methyl-1,9-dihydro-pyrido[2,3-b]indol-2-one

On procède comme dans l'ex75 avec le Composé B : 6-(4-Chloromethyl-thiazol-2-yl)-3-(2,4-dichloro-phenyl)-1-methyl-1,9-dihydro-pyrido[2,3-b]indol-2-one MS: 481.2.

### D) 6-(4-Aminomethyl-thiazol-2-yl)-3-(2,4-dichloro-phenyl)-1-methyl-1,9-dihydropyrido[2,3-b]indol-2-one

On procède comme dans l'ex75 avec le Composé C 6-(4-Azidomethyl-thiazol-2-yl)-3-(2,4-dichloro-phenyl)-1-methyl-1,9-dihydro-pyrido[2,3-b]indol-2-one RMN 1H DMSO-d6 (300 MHz) : 3.70 (s, 3H) ; 3.89 (s, 2H) ; 7.37 (s, 1H) ; 7.53 (m, 3H) ; 7.68 (s,1H) ; 7.82 (d, J=8.4, 1H) ; 8.34 (s,1H); 8.45 (s, 1H).
T°f= 205°C-210°C

### EXEMPLE 110: 3-(4-fluoro-phenyl)-6-(1-methoxymethyl-1H-pyrazol-3-yl)-1,9-dimethyl-1,9-dihydro-pyrido[2,3-b]indol-2-one

### A) composé A : 1 (amino-acryloyl)-3-(4-fluoro-phenyl)-1,9-dimethyl-1,9-dihydro - pyrido[2,3-b]indol-2-one

On procède comme à la prep. 1.9C ci-dessus à partir de composé de l'Ex 72B, 6-Acetyl-3-(4-fluoro-phenyl)-1,9-dimethyl-1,9-dihydro-pyrido[2,3-b]indol-2-one et de réactif de Bredereck.

### B) composé B : 3-(4-fluoro-phenyl)-1,9-dimethyl-6-(1H-pyrazol-3-yl)-1,9-dihydropyrido[2,3-b]indol-2-one

On procède comme à l'exemple 40 ci-dessus à partir de composé A, 1 amino-acryloyl)-3-(4-fluoro-phenyl)-1,9-dimethyl-1,9-dihydro-pyrido[2,3-b]indol-2-one et l'hydrate d'hydrazine

### C) 3-(4-fluoro-phenyl)-6-(1-methoxymethyl-1H-pyrazol-3-yl)-1,9-dimethyl-1,9-dihydro-pyrido[2,3-b]indol-2-one

Dissoudre 303 mg (0.81 mmol) de composé B 3-(4-fluoro-phenyl)-1,9-dimethyl-6-(1H-pyrazol-3-yl)-1,9-dihydro-pyrido[2,3-b]indol-2-one dans 8 mL de DMF. Ajouter 50 mg (1.22 mmol) de NaH 60% ainsi qu'une spatulée de dibenzo-18-crown-6. Après 5 minutes d'agitation, ajouter 73 µL (0.89 mmol) de bromomethyl methylether. Laisser agiter 4h à température ambiante. Ajouter une solution saturée de NaHC03, extraire avec AcOEt, sécher sur MgSO4, filtrer et concentrer à sec en adsorbant sur silice. Enrichir le mélange en isomère □ par chromatographie sur colonne de silice éluée avec AcOEt/cyclohexane 70/30 (ratio obtenu: 62/38 □□ puis par une deuxième purification par chromatographie sur colonne de silice éluée avec AcOEt/cyclohexane 60/40
Obtention de 215 mg de cristaux jaune (67/33 beta/alpha)
MS: 417.2

RMN 1H DMSO-d6 (300 MHz) : 3.29/3.30 (s, m/M, 3H); 4.01/4.02 (s, M/m, 3H); 4.14/4.17 (s, M/m, 3H); 5.42/5.43 (s, M/m, 2H); 6.54/6.83 (d, m/M, J =1.71/ J = 2.34, 1H); 7.22 (m, 2H); 7.50 (m, 1/3H); 7.61 (m, 1H); 7.72 (d, J = 8.55, 1/3H); 7.76 - 7.90 (m, 8/3H); 7.96 (d, M, J = 2.31, 2/3H); 8.14/8.40 (m, m/M, 1H); 8.42/8.50 (s, m/M, 1H

Une deuxième chromatographie permet d'obtenir le dérivé beta pur.

### EXEMPLE 111 3-(4-Chloro-phenyl)-6-(1-methoxymethyl-1H-pyrazol-3-yl)-1,9-dimethyl-1,9-dihydro-pyrido[2,3-b]indol-2-one

On procède comme à l'exemple 110 ci-dessus à partir de composé de l'ex 105, 3-(4-Chloro phenyl)-1,9-dimethyl-6-(1H-pyrazol-3-yl)-1,9-dihydro-pyrido[2,3-b]indol-2-one et de bromomethyl methylether.
T°f : 245-247°C
MS : 433.25

RMN 1H DMSO-d6 (300 MHz) : 3.30 (s, 3H); 4.02 (s, 3H); 4.14 (s, 3H); 5.42 (s, 2H); 6.83 (d, J = 1.83, 1H); 7.44 (d, J = 8.4, 2H); 7.62 (d, J = 8.52, 1H); 7.78 (d, J = 8.79, 1H); 7.87 (d, J = 8.43, 2H); 7.96 (d, J =1.89, 1H); 8.42 (s, 1H); 8.56 (s, 1H).

### EXEMPLE 112 : 3-(4-Fluoro-phenyl)-1,9-dimethyl-6-(4-methyl-2H-pyrazol-3-yl)-1,9-dihydro-pyrido[2,3-b]indol-2-one

### A) Composé A : 3-(4-Fluoro-phenyl)-1,9-dimethyl-6-propionyl-1,9-dihydropyrido[2,3-b]indol-2-one

On procède comme indiqué à la preparation 1.1 E ci-dessus avec composé de l'ex 63A, 3-(4-fluoro-phenyl)-1,9-dimethyl-1,9-dihydro-pyrido[2,3-b]indol-2-one et le propionyl chloride

RMN 1H DMSO-d6 (300 MHz) : 1.13 (t, J=7.2, 3H); 3.10 (q, J=7.2, 2H) ; 4.00 (s, 3H) ; 4.15 (s, 3H) ; 7.23 (t, J=8.9,2H) ; 7.66 (d , J=8.7, 1H) ; 7.80-7.90 (m, 3H) ; 8.56 (s, 1H); 8.66 (s, 1H).

### B) Composé B : 6-(3-Dimethylamino-2-methyl-acryloyl)-3-(4-fluoro-phenyl)-1,9-dimethyl-1,9-dihydro-pyrido[2,3-b]indol-2-one

On procède comme indiqué à la preparation 1.9C ci-dessus avec composé A 3-(4-Fluoro-phenyl)-1,9-dimethyl-6-propionyl-1,9-dihydro-pyrido[2,3-b]indol-2-one et le réactif de Bredereck.

RMN 1H DMSO-d6 (300 MHz) : 2.05 (s, 3H) ; 2.98 (s, 6H); 4.01 (s, 3H) ; 4.14 (s, 3H); 6.91 (s, 1H); 7.17-1.28 (m, 3H); 7.55 (d, J=8.4, 1H); 7.78-7.83 (m, 2H); 7.96 (s, 1H); 8.50 (s, 1H).

### C) 3-(4-Fluoro-phenyl)-1,9-dimethyl-6-(4-methyl-2H-pyrazol-3-yl)-1,9-dihydropyrido[2,3-b]indol-2-one

On procède comme à l'exemple 40 ci-dessus à partir de composé B, 6-(3-Dimethylamino-2-methyl-acryloyl)-3-(4-fluoro-phenyl)-1,9-dimethyl-1,9-dihydropyrido[2,3-b]indol-2-one et l'hydrate d'hydrazine

RMN 1H DMSO-d6 (300 MHz) : 2.24 (s, 3H); 4.02 (s, 3H); 4.15 (s, 3H); 7.21 (t, J=8.9, 2H); 7.48-7.55 (m, 2H) ; 7.65 (d, J=8.6, 1H); 7.78-7.83 (m, 2H) ; 8.14 (s, 1H) ; 8.47 (s, 1H).
Tf = 311-312 °C

### EXEMPLE 113 : 3-(4-Fluoro-phenyl)-6-(1-methoxymethyl-4-methyl-1H-pyrazol-3-yl)-1,9-dimethyl-1,9-dihydro-pyrido[2,3-b]indol-2-one

On procède comme à l'exemple 36 ci-dessus à partir de composé de l' ex 112, 3-(4-Fluoro-phenyl)-1,9-dimethyl-6-(4-methyl-2H-pyrazol-3-yl)-1,9-dihydro-pyrido[2,3-b]indol-2-one et de bromomethylmethylether.

RMN 1H DMSO-d6 (300 MHz) : 4.00 (s, 3H); 4.14 (s, 3H); 4.49 (d, J=5.6, 2H); 5.35 (t, J=5.6, 1H) ; 7.41-7.49 (m, 3H) ; 7.56 (d, J=8.6, 1H) ; 7.68-7.71 (m, 2H) ; 8.19 (s, 1H) ; 8.23 (s, 1H).
Tf= 166-168 °C.

### EXEMPLE 114 : 2,2-Dimethyl-propionic acid 4-[3-(4-chloro-phenyl)-1,9-dimethyl-2-oxo-2,9-dihydro-1H-pyrido[2,3-b]indol-6-yl]-thiazol-2-yl methyl ester

### A) Composé A : 6-(2-Bromo-acetyl)-3-(4-chloro-phenyl)-1,9-dimethyl-1,9-dihydropyrido[2,3-b]indol-2-one

On procède comme à la prep. 1.1 ci-dessus à partir du composé de l'Ex 104A, 3-(4-Chloro-phenyl)-1,9-dimethyl-1,9-dihydro-pyrido[2,3-b]indol-2-one et de chlorure de bromoacétyle

RMN 1H DMSO-d6 (300 MHz) : 4.03 (s, 3H); 4.20 (s, 3H); 4.96 (s, 2H); 7.47 (m, 2H); 7.74(d, J=8.7, 1H); 7.84 (m, 2H); 7.94 (dd, J=1.7, J=8.7, 1H); 8.62 (s, 1H); 8.75 (s,1H).

### B) 2,2-Dimethyl-propionic acid 4-[3-(4-chloro-phenyl)-1,9-dimethyl-2-oxo-2,9-dihydro-1H-pyrido[2,3-b]indol-6-yl]-thiazol-2-ylmethyl ester

On procède comme pour l'ex 8 ci-dessus à partir du composé A, 6-(2-Bromo-acetyl)-3-(4-chloro-phenyl)-1,9-dimethyl-1,9-dihydro-pyrido[2,3-b]indol-2-one et du 2-amino-2-thioxoethylpivalate.

RMN 1H DMSO-d6 (300 MHz) : 1.23 (s, 9H) ; 4.02 (s, 3H) ; 4.15 (s, 3H); 5.47 (s, 2H); 7.45 (d, J=8.7, 2H); 7.65 (d, J=8.7, 1H); 7.87 (m, 3H); 8.05 (s, 1H); 8.54 (m, 2H).
T°f= 225°C-228°C

### EXEMPLE 115 3-(4-Chloro-phenyl)-6-(2-hydroxymethyl-thiazol-4-yl)-1,9-dimethyl-1,9-dihydro-pyrido[2,3-b]indol-2-one

On procède comme pour l'ex 9 ci-dessus à partir du composé de l'ex 114, 2,2-Dimethyl-propionic acid 4-[3-(4-chloro-phenyl)-1,9-dimethyl-2-oxo-2,9-dihydro-1H-pyrido[2,3-b]indol-6-yl]-thiazol-2-ylmethyl ester

RMN 1H DMSO-d6 (300 MHz) : 4.03 (s, 3H) ; 4.16 (s, 3H); 4.82 (d, J=4.53, 2H); 6.10 (bs, 1H); 7.44(d, 1=8.7, 2H); 7.65 (d, J=8.7, 1H); 7.88 (m, 3H); 7.94 (s, 1H); 8.53
T°f= 256°C-260°C

### EXEMPLE 116 6-(2-Amino-thiazol-4-yl)-3-(4-chloro-phenyl)-1,9-dimethyl-1,9-dihydro-pyrido[2,3-b]indol-2-one

On procède comme pour l'ex 21 ci-dessus à partir du composé de l'exemple 114A 6-(2-Bromo-acetyl)-3-(4-chloro-phenyl)-1,9-dimethyl-1,9-dihlydro-pyrido[2,3-b]indol-2-one et de la thiourée.

RMN 1H DMSO-d6 (300 MHz) : 4.02 (s, 3H) ; 4.14 (s, 3H); 6.93 (s, 1H); 6.99 (bs, 2H); 7.45 (d, J=8.4, 2H); 7.57 (d, J=8.7, 1H); 7.76 (d, J=8.4, 1H); 7.85 (d, J=8.7, 2H); 8.36 (s, 1H); 8.44 (s, 1H);
T°f= 320°C-323°C (dec.)

### EXEMPLE 117 6-(5-Ethoxymethyl-2H-pyrazol-3-yl)-3-(4-fluoro-phenyl)-1,9-dimethyl-1,9-dihydro-pyrido[2,3-b]indol-2-one

### A) composé A : 3-(4-fluoro-phenyl)-1,9-dimethyl-2-oxo-2,9-dihydro-1H-pyrido[2,3-b]indol-6-yl]-butane-1,3-dione

On procède comme pour l'ex 59 ci-dessus à partir du composé de l'exemple 72A, 6-Acetyl-3-(4-fluoro-phenyl)-1,9-dimethyl-1,9-dihydro-pyrido[2,3-b]indol-2-one et de l'ethyl ethoxy acetate
MS: 435.24

### B) 6-(5-Ethoxymethyl-2H-pyrazol-3-yl)-3-(4-fluoro-phenyl)-1,9-dimethyl-1,9-dihydro-pyrido[2,3-b]indol-2-one

On procède comme à l'exemple 43 ci-dessus avec le composé A, 3-(4-fluoro-phenyl)-1,9-dimethyl-2-oxo-2,9-dihydro-1H-pyrido[2,3-b]indol-6-yl]-butane-1,3-dione et l'hydrazine monohydrate

RMN 1H DMSO-d6 (300 MHz) : 1.15 (t, J=7.0, 3H) ; 3.51 (q, J=7.0, 2H); 3.99 (s, 3H); 4.15 (s, 3H); 4.45 (d, J=19.4, 2H); 6.68 (s, 1H); 7.23 (t, J=8.85, 2H); 7.67 (m, 4H); 8.37 (m, 2H); 12.82/13.03 (2s, 1H).

### EXEMPLE 118 : 6-(2-Amino-thiazol-4-yl)-3-(4-fluoro-phenyl)-1,9-dimethyl-1,9-dihydro-pyrido[2,3-b]indol-2-one

### A) composé A : 6-(2-Bromo-acetyl)-3-(4-fluoro-phenyl)-1,9-dimethyl-1,9-dihydropyrido[2,3-b]indol-2-one

On procède comme à la prep. 1.1 ci-dessus à partir du composé de l'Ex 72A, 3-(4-Fluoro-phenyl)-1,9-dimethyl-1,9-dihydro-pyrido[2,3-b]indol-2-one et de chlorure de bromoacetyle

RMN 1H DMSO-d6 (300 MHz) : 4.04 (s, 3H); 4.20 (s, 3H); 4.96 (s, 2H); 7.25 (t, J=8.7, 2H); 7.75(d, J=8.7, 1H); 7.82 (m, 2H); 7.94 (dd, J=1.8, J=8.7, 1H); 8.57 (s, 1H); 8.75 (s, 1H).

### B) 6-(2-Amino-thiazol-4-yl)-3-(4-fluoro-phenyl)-1,9-dimethyl-1,9-dihydropyrido[2,3-b]indol-2-one

On procède comme pour l'ex 21 ci-dessus à partir du composé A, 6-(2-Bromoacetyl)-3-(4-fluoro-phenyl)-1,9-dimethyl-1,9-dihydro-pyrido[2,3-b]indol-2-one et de la thiourée.

RMN 1H DMSO-d6 (300 MHz) : 4.01 (s, 3H) ; 4.13 (s, 3H); 6.92 (s, 1H); 6.99 (bs, 2H); 7.22 (t, J=9.0, 2H); 7.55 (d, J=8.4, 1H); 7.75 (d, J=8.4, 1H); 7.83 (m, 2H); 8.35 (s, 1H); 8.38 (s, 1H);
T°f= 265°C-268°C (dec.)

### EXEMPLE 119 : 3-(2,4-Dichloro-phenyl)-1,9-dimethyl-6-(2-methyl-2H-pyrazol-3-yl)-1,9-dihydro-pyrido[2,3-b]indol-2-one

On procède comme à l'exemple 37 ci-dessus avec le composé de la prep. 1.10F, 3-(2,4-Dichloro-phenyl)-6-(3-dimethylamino-acryloyl)-1,9-dimethyl-1,9-dihydropyrido[2,3-b]indol-2-one et la methylhydrazine
T°f : 134-136°C
MS : 437.2

RMN 1H DMSO-d6 (300 MHz) : 3.87 (s, 3H); 4.03 (s, 3H); 4.19 (s, 3H); 6.39 (d, J = 1.77, 1H); 7.48 - 7.52 (m, 4H); 7.68 - 7.80 (m, 2H); 8.06 (s, 1H); 8.31 (s, 1H).

### EXEMPLE 120 : 6-(5-Ethoxymethyl-1-methyl-1H-pyrazol-3-yl)-3-(4-fluoro-phenyl)-1,9-dimethyl-1,9-dihydro-pyrido[2,3-b]indol-2-one

On procède comme à l'exemple 36 ci-dessus avec le composé de l'ex 117, 6-(5-Ethoxymethyl-1H-pyrazol-3-yl)-3-(4-fluoro-phenyl)-1,9-dimethyl-1,9-dihydropyrido[2,3-b]indol-2-one et l'iodure de méthyle

RMN 1H DMSO-d6 (300 MHz) : 1.16 (t, J=7.0, 3H) ; 3.52 (q, J=7.0, 2H); 3.84 (s, 3H); 4.02 (s, 3H); 4.14 (s, 3H); 4.54 (s, 2H); 6.71 (s, 1H); 7.23 (t, J=9.0, 2H); 7.56 (d, J=8.7, 1H); 7.72 (d, J=8.7, 1H); 7.85 (m, 2H); 8.35 (s, 1H); 8.50 (s, 1H).
T°f= 148°C-15°C

### EXEMPLE 121 : 3-(4-Chloro-phenyl)-6-(2-ethoxymethyl-thiazol-4-yl)-1,9-dimethyl-1,9-dihydro-pyrido[2,3-b]indol-2-one

On procède comme à l'exemple 10 ci-dessus avec le composé de l'ex 115, 3-(4-Chloro-phenyl)-6-(2-hydroxymethyl-thiazol-4-yl)-,9-dimethyl-1,9-dihydropyrido[2,3-b]indol-2-et l'iodoéthane.

RMN 1H DMSO-d6 (300 MHz) : 1.21 (t, J=6.9, 3H) ; 3.67 (q, J=6.9, 2H) ; 4.02 (s, 3H) ; 4.16 (s, 3H); 4.83 (s, 2H); 7.45 (d, J=8.7, 2H); 7.65 (d, J=8.7, 1H); 7.88 (m, 3H); 8.0 (s,1H); 8.54 (s, 2H).
T°f= 202°C-206°

### EXEMPLE 122 : Chlorhydrate de 3-(2,4-Dichloro-phenyl)-1,9-dimethyl-6-(1-methyl-5-morpholin-4-ylmethyl-1H-pyrazol-3-yl)-1,9-dihydro-pyrido[2,3-b]indol-2-one

### A) Composé A : 6-(5-Chloromethyl-1-methyl-1H-pyrazol-3-yl)-3-(2,4-dichlorophenyl)-1,9-dimethyl-1,9-dihydro-pyrido[2,3-b]indol-2-one

Dissoudre 690 mg (1.48 mmol) de composé de l'Ex82, 3-(2,4-Dichloro-phenyl)-6-(5-hydroxymethyl-1-methyl-1H-pyrazol-3-yl)-1,9-dimethyl-1,9-dihydro-pyrido[2,3-b]indol-2-one dans 20 mL de CH2Cl2 anhydre. Ajouter 0.62 mL (4.44 mmol) de Et3N et 0.32 mL de chlorure de methane sulfonyl. Agiter à température ambiante pendant 3 jours. Adsorber le milieu réactionnel sur silice et purifier sur colonne de silice éluée avec un mélange AcOEt/cyclohexane 75/25, puis à l'AcOEt.
Obtention de 400 mg de poudre blanche.
RMN 1H DMSO-d6 (300 MHz) : 3.89 (s, 3H) ; 4.02 (s, 3H) ; 4.77 (s, 3H) ; 4.97 (s, 2H) ; 6.80 (s, 1H) ; 7.50 (m, 2H); 7.70 (m, 3H); 8.29 (s, 2H).

### B) Chlorhydrate de 3-(2,4-Dichloro-phenyl)-1,9-dimethyl-6-(1-methyl-5-morpholin-4-ylmethyl-1H-pyrazol-3-yl)-1,9-dihydro-pyrido[2,3-b]indol-2-one

Dissoudre 100 mg (0.2 mmol) de Composé A, 6-(5-Chloromethyl-1-methyl-1H-pyrazol-3-yl)-3-(2,4-dichloro-phenyl)-1,9-dimethyl-1,9-dihydro-pyrido[2,3-b]indol-2-one dans 5 mL de DMF anhydre. Ajouter 55 mg (0.4 mmol) de K2CO3 et 22 µL (0.25 mmol) de morpholine. Chauffer à 80°C pendant une nuit Revenir à température ambiante et verser sur de l'eau. Extraire à l'AcOEt. Laver la phase organique avec une solution saturée en NaCl. Sécher sur Na2SO4, filtrer et évaporer à sec. Purifier sur colonne de silice éluée avec un mélange AcOEt/MeOH 95/05. Dissoudre dans de l'AcOEt le produit obtenu et ajouter 0.5 mL d'une solution saturée de HCl dans Et2O/éther de pétrole. Filtrer le précipité formé et laver à l'éther. Reprendre dans du MeOH et évaporer à sec.
Obtention de 40 mg de poudre blanche.

RMN 1H Pyridine-d6 (300 MHz) : 2.28 (t, J=4.5, 4H) ; 3.35 (s, 2H); 3.55 (t, J=4.5, 4H); 3.73 (s, 3H) ; 3.77 (s, 3H) ; 3.85 (s, 3H); 6.73 (s, 1H) ; 7.30 (m, 1H); 7.50 (m, 2H); 7.98 (s, 1H); 8.09 (dd, J=1.6, J=8.5, 1H).
T°f: 225°C-230°C (dec.)

### EXEMPLE 123 : Chlorhydrate de 3-(2,4-Dichloro-phenyl)-1,9-dimethyl-6-[1-mehyl-5-(4-methyl-piperazin-1-ylmethyl)-1H-pyrazol-3-yl]-1,9-dihydro-pyrido[2,3-b]indol-2-one

Dissoudre 100 mg (0.2 mmol) de composé de l'Ex 122A : 6-(5-Chloromethyl-1-methyl-1H-pyrazol-3-yl)-3-(2,4-dichloro-phenyl)-1,9-dimethyl-1,9-dihydiopyrido[2,3-b] indol -2-one dans 6 mL de THF anhydre et 1 mL de NMP. Ajouter 55 mg (0.4 mmol) de K2CO3 et 28 µL (0.25 mmol) de N-methylpipérazine. Chauffer à reflux pendant une nuit. Revenir à température ambiante et verser sur de l'eau. Extraire à l'AcOEt. Sécher sur Na2SO4, filtrer et évaporer à sec. Dissoudre le produit obtenu dans 5 mL d'AcOEt et ajouter 0.5 mL d'une solution saturée de HCl dans Et2O/éther de pétrole. Filtrer le précipité formé et laver à l'éther. Reprendre dans du MeOH et évaporer à sec.
Obtention de 65 mg de poudre beige.

RMN 1H Pyridine-d6 (300 MHz) : 2.55 (s, 3H) ; 2.83 (bs, 4H); 3 (bs, 4H); 3.39 (s, 2H) ; 3.74 (s, 3H) ; 3.77 (s, 3H); 3.80 (s, 3H); 6.65 (s, 1H) ; 7.29 (m, 1H); 7.50 (m, 2H); 8 (s, 1H); 8.08 (dd, J=1.5, J=8.5, 1H).
T°f: 235°C-241°C (dec.)

### EXEMPLE 124 : Chlorhydrate de 3-(2,4-Dichloro-phenyl)-1,9-dimethyl-6-{1-methyl-5-[(2-morpholin-4-yl-ethylamino)-methyl]-1H-pyrazol-3-yl}-1,9-dihydro-pyrido[2,3-b]indol-2-one

On procède comme à l'exemple 122 ci-dessus avec le composé de l'ex 122A : 6-(5-Chloromethyl-1-methyl-1H-pyrazol-3-yl)-3-(2,-4-dichloro-phenyl)-1,9-dimethyl-1,9-dihydro-pyrido[2,3-b]indol-2-one et l'aminoethylmorpholine.

RMN 1H Pyridine-d6 (300 MHz) : 2.38 (bs, 4H) ; 2.85 (t, J=5.9, 2H); 3.28 (t, J=5.9, 2H); 3.46 (t, J=4.5, 4H) ; 3.71 (s, 3H) ; 3.75 (s, 3H); 4.05 (s, 3H); 4.50 (s, 2H); 7.30 (m, 3H) ; 7.50 (m, 2H); 7.88 (s, 1H); 7.98 (m, 1H); 8.50 (s, 1H). T°f: 233°C-237°C (dec.)

### EXEMPLE 125 : 6-[1-(2-Cyclopropylmethoxy-ethyl)-1H-pyrazol-3-yl]-3-(2,4-dichloro-phenyl)-1,9-dimethyl-1,9-dihydro-pyrido[2,3-b]indol-2-one

On procède comme à l'exemple 101 ci-dessus avec le composé de l'ex 51, 3-(2,4-Dichloro-phenyl)-6-[1-(2-hydroxy-ethyl)-1H-pyrazol-3-yl]-1,9-dimethyl-1,9-dihydropyrido[2,3-b]indol-2-one et le (bromomethyl)cyclopropane.
T°f : 164-165°C
MS : 521.11

RMN 1H DMSO-d6 (300 MHz) : 0.15 (m, 2H); 0.43 (m, 2H); 0.97 (m, 1H); 3.24 (d, J = 6.78, 2H); 3.79 (t, J = 5.43, 2H); 4.01 (s, 3H); 4.16 (s, 3H); 4.29 (t, J = 5.4, 2H); 6.69 (d, J = 2.25, 1H); 7.40 - 7.51 (m, 2H); 7.61 (d, J = 8.61, 1H); 7.68 (d, J = 1.62, 1H); 7.73 (m, 2H); 8.28 (m, 2H).

### EXEMPLE 126 : 3-(2,4-Dichloro-phenyl)-6-{1-[2-(2-ethoxy-ethoxy)-ethyl]-1H-pyrazol-3-yl}-1,9-dimethyl-1,9-dihydro-pyrido[2,3-b]indol-2-one

On procède comme à l'exemple 101 ci-dessus avec le composé de l'ex 51, 3-(2,4-Dichloro-phenyl)-6-[1-(2-hydroxy-ethyl)-1H-pyrazol-3-yl]-1,9-dimethyl-1,9-dihydropyrido[2,3-b]indol-2-one et le 2-bromoethylethylether.
T°f : 165-170°C
MS : 539.04

RMN 1H DMSO-d6 (300 MHz) : 1.06 (t, J = 6.99, 3H); 3.34 - 3.42 (m, 2H) ; 3.43 - 3.50 (m, 2H) ; 3.50 - 3.55 (m, 2H) ; 3.81 (t, J = 5.31, 2H); 4.00 (s, 3H); 4.13 (s, 3H); 4.29 (t, J = 5.25, 2H); 6.69 (m, 1H); 7.40 - 7.51 (m, 2H); 7.60 (d, J = 8.61, 1H); 7.68 (d, J = 1.5, 1H); 7.70 - 7.80 (m, 2H); 8.27 (m, 2H).

### EXEMPLE 127 : 3-(2,4-Dichloro-phenyl)-6-[5-(2-ethoxy-ethoxymethyl)-1-methyl-1H-pyrazol-3-yl]-1,9-dimethyl-1,9-dihydro-pyrido[2,3-b]indol-2-one

On procède comme à l'exemple 101 ci-dessus avec le composé de l'ex 82, 3-(2,4-Dichloro-phenyl)-6-(5-hydroxymethyl-1-methyl-1H-pyrazol-3-yl)-1,9-dimethyl-1,9-dihydro-pyrido[2,3-b]indol-2-one et le 2-chloroethyl ethyl ether

RMN 1H DMSO-d6 (300 MHz) : 1.10 (t, J=6.99, 3H); 3.45 (q, J=6.99, 2H) ; 3.53 (m, 4H) ; 4.83 (s, 3H) ; 4.02 (s, 3H); 4.16 (s, 3H); 4.56 (s, 2H); 6.70 (s, 1H) ; 7.47 (m, 2H); 7.61 (d, J=8.7, 1H); 7.72 (m, 2H); 8.28 (s, 2H).
T°f: 82°C-86°C

### EXEMPLE 128 : Chlorhydrate du 3-(2,4-Dichloro-phenyl)-1,9-dimethyl-6-{2-[(2-morpholin-4-yl-ethylamino)-methyl]-thiazol-4-yl}-1,9-dihydro-pyrido[2,3-b]indol-2-one

### A) Composé A : 6-(2-Chlorométhyl-thiazol-4-yl)-3-(2,4-dichloro-phenyl)-1,9-dimethyl-1,9-dihydro-pyrido[2,3-b]indol-2-one

On procède comme pour l'ex122A avec le composé de l'Ex 9 , 3-(2,4-dichlorophenyl)-6-(2-hydroxymethyl-thiazol-4-yl)-1,9-dimethyl-1,9-dihydro-pyrido[2,3-b]indol-2-one RMN 1H DMSO-d6 (300 MHz) : 4.01 (s, 3H) ; 4.18 (s, 3H) ; 5.16 (s, 2H) ; 7.45 (m, 2H) ; 7.66-7.69 (m, 2H) ; 7.90 (dd, J=1.7, J=8.6, 1H) ; 8.07 (s, 1H) ; 8.28 (s, 1H) ; 8.47 (d, J=1.5, 1H).

### B) Chlorhydrate du 3-(2,4-Dichloro-phenyl)-1,9-dimethyl-6-{2-[(2-morpholin-4-yl-ethylamino)-methyl]-thiazol-4-yl}-1,9-dihydro-pyrido[2,3-b]indol-2-one

On procède comme à l'exemple 122 ci-dessus avec le composé A : 6-(2-Chloromethyl-thiazol-4-yl)-3-(2,4-dichloro-phenyl)-1,9-dimethyl-1,9-dihydropyrido[2,3-b]indol-2-one et l'aminoethylmorpholine.
Tf = 205-210 °C (déc.)

RMN 1H DMSO-d6 (300 MHz) : 3.53-3.64 (m, 8H) ; 3.75-3.92 (m, 4H) ; 4.02 (s, 3H) ; 4.19 (s, 3H) ; 4.70 (s, 2H) ; 7.43-7.46 (m, 2H) ; 7.68-7.71 (m, 2H) ; 7.98 (d, J=8.9, 1H) ; 8.11 (s, 1H) ; 8.25 (s,1H) ; 8.62 (s, 1H) ; 10.05 (m, 1H).

### EXEMPLE 129 : 3-(2,4-Dichloro-phenyl)-6-[1-(2-methoxy-ethoxymethyl)-1H-pyrazol-3-yl]-1,9-dimethyl-1,9-dihydro-pyrido[2,3-b]indol-2-one

On procède comme à l'exemple 10 ci-dessus avec le composé de l' ex39 3-(2,4-Dichlorophényl) 6-(1H-pyrazol-5-yl)-1,9-dihydro-2H-pyrido[2,3-b]indol-2-one et le méthoxy éthoxymethyl chloride.

RMN 1H DMSO-d6 (300 MHz) : 3.18 (s, 3H) ; 3.37-3.40 (m, 2H) ; 3.57-3.61 (m, 2H) ; 3.98 (s, 3H) ; 4.14 (s, 3H) ; 5.44 (s, 2H) ; 6.77 (s, 1H) ; 7.40-7.43 (m, 2H) ; 7.59 (d, J=8.6, 1H) ; 7.65 (s, 1H) ; 7.75 (d, J=8.5, 1H) ; 7.90 (d, J=2.2, 1H) ; 8.26 (s, 1H) ; 8.30 (s, 1H).
Tf = 162-163 °C

### EXEMPLE 130 : 3-(2,4-Dichloro-phenyl)-1,9-dimethyl-6-(2-morpholin-4-ylmethyl-thiazol-4-yl)-1,9-dihydro-pyrido[2,3-b]indol-2-one

On procède comme à l'exemple 122 ci-dessus avec le composé de 1' ex 128A chloromethyl-thiazol-4-yl)-3-(2,4-dichloro-phenyl)-1,9-dimethyl-1,9-dihydropyrido[2,3-b]indol-2-one et la morpholine.

RMN 1H DMSO-d6 (300 MHz) : 2.54 (m, 4H) ; 3.60-3.63 (m, 4H) ; 3.88 (s, 2H) ; 4.01 (s, 3H) ; 4.17 (s, 3H) ; 7.42-7.49 (m, 2H) ; 7.63-7.68 (m, 2H) ; 7.87 (d, J=8.6, 1H) ; 7.93 (s, 1H) ; 8.27 (s, 1H) ; 8.44 (s, 1H).
Tf= 213-214 °C

### EXEMPLE 131 : 3-(4-Chloro-phenyl)-1,9-dimethyl-6-(4-methyl-2H-pyrazol-3-yl)-1,9-dihydro-pyrido[2,3-b]indol-2-one

### A) composé A : 3-(4-Chloro-phenyl)-1.9-bimethyl-6-propionyl-1,9-dihydropyrido[2,3-b]indol-2-one

On procède comme indiqué à la préparation 1.9B ci-dessus avec le Composé de l'ex 104A 3-(4-chlom-phenyl)-1,9-dimethyl-1,9-dihydro-1H-pyrido[2,3-b]indol-2-one et du propionyl chloride

RMN 1H DMSO-d6 (300 MHz) : 1.13 (t, J=7.2, 3H) ; 3.10 (q, J=7.2, 2H) ; 4.00 (s, 3H) ; 4.15 (s, 3H) ; 7.41 (d, J=8.5, 2H) ; 7.65 (d , J=8.7, 1H) ; 7.83-7.90 (m, 3H) ; 8.60 (s, 1H) ; 8.66 (s, 1H).

### B) composé B : 6-(-3-Dimethylamino-2-methyl-acryloyl)-3-(4-chloro-phenyl)-1,9-dimethyl-1,9-dihydro-pyrido[2,3-b]indol-2-one

On procède comme indiqué à la preparation 1.9C ci-dessus avec du composé A, 3-(4-Chloro-phenyl)-1,9-dimethyl-6-propionyl-1,9-diydro-pyrido[2,3-b]indol-2-one et du réactif de Bredereck.

RMN 1H DMSO-d6 (300 MHz) : 2.03 (s, 3H) ; 2.97 (s, 6H) ; 3.99 (s, 3H) ; 4.12 (s, 3H) ; 6.99 (s, 1H) ; 7.25 (dd, J=1.5, J=8.4, 1H) ; 7.40 (d, J=8.6, 2H) ; 7.54 (d, J=8.5, 1H) ; 7.80 (d, J=8.6, 2H) ; 7.95 (d, J=1.2, 1H) ; 8.54 (s, 1H).

### C) 3-(4-Chloro-phenyl)-1,9-dimethyl-6-(4-methyl-2H-pyrazol-3-yl)-1,9-dihydropyrido[2,3-b]indol-2-one

On procède comme indiqué à l'ex36 ci-dessus avec du composé B , 6-(-3-Dimethylamino-2-methyl-acryloyl)-3-(4-chloro-phenyl)-1,9-dimethyl-1,9-dihydropyrido[2,3-b]indol-2-one et l'hydrate d'hydrazine

RMN 1H DMSO-d6 (300 MHz) : 2.25 (s, 3H) ; 4.02 (s, 3H) ; 4.15 (s, 3H) ; 7.21-7.56 (m, 4H) ; 7.65 (d, J=8.6, 1H) ; 7.83 (d, 1=8.6, 2H); 8.15 (s, 1H) ; 8.53 (s, 1H). Tf = 271-273 °C

### EXEMPLE 132 : 3-(4-Chloro-phenyl)-6-(1-methoxymethyl-4-methyl-1H-pyrazol-3-yl)-1,9-dimethyl-1,9-dihydro-pyrido[2,3-b]indol-2-one

On procède comme indiqué à l'ex36 ci-dessus avec le composé de 1' Ex131, 3-(4-Chloro-phenyl)-1,9-dimethyl-6-(4-methyl-2H-pyrazol-3-yl)-1,9-dihydro-pyrido[2,3-b]indol-2-one et le bromomethylmethylether.

RMN 1H DMSO-d6 (300 MHz) : 2.27 (s, 3H) ; 3.29 (s, 3H) ; 4.03 (s, 3H) ; 4.16 (s, 3H) ; 5.35 (s, 2H) ; 7.43 (d, J=8.6, 2H) ; 7.58-7.66 (m, 2H) ; 7.76 (s, 1H) , 7.85 (d, J=8.6, 2H) ; 8.21 (s, 1H) ; 8.58 (s, 1H).
Tf = 205 - 207 °C

### EXEMPLE 133 : 6-(1-Ethyl-1H-pyrazol-3-yl)-3-(4-fluoro-phenyl)-1,9-dimethyl-1,9-dihydro-pyrido[2,3-b]indol-2-one

On procède comme indiqué à l'ex36 ci-dessus avec le composé de l'ex 72, 3-(4-Fluoro-phenyl)-1,9-dimethyl-6-(2H-pyrazol-3-yl)-1,9-dihydro-pyrido[2,3-b]indol-2-one et l'iodoethane.

RMN 1H DMSO-d6 (300 MHz) : 1.40 (t J=7.2,3H) ; 3.99 (s, 3H) ; 4.11-4.18 (m, 5H) ; 6.67 (d, J=2.1, 1H) ; 7.16-7.22 (m, 2H) ; 7.55 (d, J=8.6, 1H) ; 7.70-7.84 (m, 4H),; 8.33 (s, 1H) ; 8.47 (s, 1H).
Tf= 178-182 °C

### EXEMPLE 134 :3-[3-(2,4-Dichloro-phenyl)-1,9-dimethyl-2-oxo-2,9-dihydro-1H-pyrido[2,3-b]indol-6-yl]-pyrazole-1-carboxylic acid 2-methoxy-ethyl ester

On procède comme indiqué à l'ex99 ci-dessus avec le composé de l'Ex 39, 3-(2,4-Dichloro-phenyl)-1,9-dimethyl-6-(2H-pyrazol-3-yl)-1,9-dihydro-pyrido[2,3-b]indol-2-one et le 2-methoxyethylchloroformate
T°f : 151-153°C
MS : 524.95

RMN 1H DMSO-d6 (300 MHz) : 3.31 (s, 3H); 3.70 (t, J = 4.5, 2H); 4.02 (s, 3H); 4.19 (s, 3H); 4.55 (t, J = 4.2, 2H); 7.11 (d, J = 2.79, 1H); 7.40 - 7.50 (m, 2H); 7.70 (m, 2H); 7.87 (d, J = 8.7, 1H); 8.32 (s, 1H); 8.40 (d, J = 2.76, 1H); 8.46 (s, 1H).

### EXEMPLE 135 : 3-[3-(2,4-Dichloro-phenyl)-1,9-dimethyl-2-oxo-2,9-dihydro-1H-pyrido[2,3-b]indol-6-yl]-pyrazole-1-carboxylic acid isopropylamide

Dissoudre 100 mg (0.24 mmol) de composé de l'Ex 39, 3-(2,4-Dichloro-phenyl)-1,9-dimethyl-6-(2H-pyrazol-3-yl)-1,9-dihydro-pyrido[2,3-b]indol-2-one dans 4 mL de chloroforme. Rajouter 232 µL (2.36 mmol) d'isopropylisocyanate puis chauffer à reflux pendant 16 heures. Laisser refroidir puis ajouter Et2O, filtrer et laver avec un minimum d'Et2O.
Obtention de 96 mg de poudre blanche.
T°f : 251-253°C
MS : 508.044

RMN 1H DMSO-d6 (300 MHz) : 1.25 (d, J = 6.51, 6H); 4.02 (s, 4H); 4.19 (s, 3H); 7.01 (d, J = 2.49, 1H); 7.40 - 7.55 (m, 2H); 7.60 - 7.75 (m, 2H); 7.96 (d, J = 7.83, 1H); 8.09 (d, J = 8.25, 1H); 8.23 (s, 1H); 8.32 (d, J = 2.49, 1H); 8.47 (s, 1H).

### EXEMPLE 136 3-[3-(2,4-Dichloro-phenyl)-1,9-dimethyl-2-oxo-2,9-dihydro-1H-pyrido[23-b]indol-6-yl]-pyrazole-1-carboxylic acid 2,2-dimethyl-propyl ester

On procède comme indiqué à l'ex99 ci-dessus avec le composé de l'Ex 39, 3-(2,4-Dichloro-phenyl)-1,9-dimethyl-6-(2H-pyrazol-3-yl)-1,9-dihydro-pyrido[2,3-b]indol-2-one et le neopentylchloroformate
T°f : 170-172°C
MS : 537.19

RMN 1H DMSO-d6 (300 MHz) : 1.02 (s, 9H); 4.02 (s, 3H); 4.13 (s, 2H); 4.19 (s, 3H); 7.12 (d, J = 2.88, 1H); 7.40 - 7.51 (m, 2H); 7.65 - 7.75 (m, 2H); 7.89 (dd, J = 8.58, J = 1.59, 1H); 8.31 (s, 1H); 8.42 (d, J = 2.85, 1H); 8.46 (d, J = 1.35, 1H).

### EXEMPLE 137 : 3-(2,4-Dichloro-phenyl)-6-[1-(2,2-dimethyl-propionyl)-1H-pyrazol-3-yl]-1,9-dimethyl-1,9-dihydro-pyrido[2,3-b]indol-2-one

On procède comme indiqué à l'ex99 ci-dessus avec le composé de l'Ex 39, 3-(2,4-Dichloro-phenyl)-1,9-dimethyl-6-(2H-pyrazol-3-yl)-1,9-dihydro-pyrido[2,3-b]indol-2-one et le chlorure de pivaloyle

RMN 1H DMSO-d6 (300 MHz) : 1.52 (s, 9H) ; 4.01 (s, 3H) ; 4.17 (s, 3H) ; 7.07 (s, 1H) ; 7.42-7.45 (m, 2H) ; 7.67-7.71 (m, 2H) ; 7.87 (d, J=8.5, 1H) ; 8.30 (s, 1H) ; 8.40-8.45 (m, 2H).
Tf = 245-246 °C

### EXEMPLE 138 :3-(2,4-Dichloro-phenyl)-6-(1-methanesulfonyl-1H-pyrazol-3-yl)-1,9-dimethyl-1,9-dihydro-pyrido[2,3-b]indol-2-one

On procède comme indiqué à l'ex99 ci-dessus avec le composé de l'Ex 39, 3-(2,4-Dichloro-phenyl)-1,9-dimethyl-6-(2H-pyrazol-3-yl)-1,9-dihydro-pyrido[2,3-b]indol-2-one et le methanesulfonyl chloride

RMN 1H DMSO-d6 (300 MHz) : 3.56 (s, 3H) ; 3.99 (s, 3H) ; 4.16 (s, 3H) ; 7.09 (s, 1H) ; 7.40-7.47 (m, 2H) ; 7.65-7.70 (m, 2H) ; 7.85 (d, J=8.5, 1H) ; 8.28 (s, 1H) ; 8.30 (d, J=2.8, 1H) ; 8.45 (s, 1H).
Tf= 267-268 °C (déc.)

### EXEMPLE 139 : 3-(2,4-Dichoro-phenyl)-6-[2-(1-hydroxy-1-methyl-ethyl)-thiazol-4-yl]-1,9-dimethyl-1,9-dihydro-pyrido[2,3-b]indol-2-one

On procède comme indiqué à l'exemple 21 ci-dessus à partir de composé de la prep.1.2, 6-(Bromoacétyl)-3-(2,4-dichlorophényl)-1,9-diméthyl-1,9-dihydro-2H-pyrido[2,3-b]indol-2-one et d'éthylthiooxamate.

RMN 1H DMSO-d6 (300 MHz) : 1.36 (t, J = 7.11, 3H); 4.02 (s, 3H); 4.20 (s, 3H) ; 4.42 (q, J = 7.11, 2H) ; 7.48 (m, 2H) ; 7.65 - 7.75 (m, 2H) ; 7.94 (dd, J = 8.61, J = 1:65, 1H) ; 8.31 (s, 1H); 8.43 (s, 1H); 8.52 (d, J = 1.44, 1H).

### B) : 3-(2,4-Dichloro-phenyl)-6-[2-(1-hydroxy-1-methyl-ethyl)-thiazol-4-yl]-1,9-dimethyl-1,9-dihydro-pyrido[2,3-b]indol-2-one

Dissoudre 131 mg (0.25 mmol) de composé A 4-[3-(2,4-Dichloro-phenyl)-1,9-dimethyl-2-oxo-2,9-dihydro-1H-pyrido[2,3-b]indol-6-yl]-thiazole-2-carboxylic acid ethyl ester dans 5 mL de Methylmagnesium bromide 1.4 M in toluene. Agiter à température ambiante pendant 3 jours. Concentrer à sec en adsorbant sur silice. Purifier sur colonne de silice éluée avec CH2Cl2 100% puis CH2Cl2/MeOH 3%. Reprendre le résidu dans MeOH, filtrer, laver avec MeOH.
Obtention de 54 mg de poudre beige clair.
Rendement : 42%
T°f : 268-269°C
MS : 497.95

RMN 1H DMSO-d6 (300 MHz) : 1.58 (s, 6H); 4.01 (s, 3H); 4.17 (s, 3H); 5.99 (s, 1H); 7.46 (m, 2H); 7.66 (m, 2H); 7.83 (s, 1H); 7.88 (d, J = 8.61, 1H); 8.28 (s, 1H); 8.44 (s, 1H).

### EXEMPLE 140 : 6-(2-Cyclohexyl-2H-pyrazol-3-yl)-3-(2,4-dichloro-phenyl)-1,9-dimethyl-1,9-dihydro-pyrido[2,3-b]indol-2-one

On procède comme à l'exemple 37 ci-dessus avec le composé de la prep. 1.10 , 3-(2,4-Dichlorophényl)-6-[3-(diméthylamino)prop-2-ènoyl]-1,9-diméthyl-1,9-dihydro-2H-pyrido[2,3-b]indol-2-one et le cyclohexylhydrazine hydrochloride.
T°f : 238-240°C
MS : 505.17

RMN 1H DMSO-d6 (300 MHz) : 1.17 (bs, 3H); 1.58 (bs, 1H); 1.73 (bs, 2H); 1.85 (bs, 4H); 4.03 (s, 3H); 4.10 (m, 1H); 4.20 (s, 3H); 6.28 (d, J = 1.56, 1H); 7.28 (d, J = 8.43, 1H); 7.40 - 7.55 (m, 3H); 7.68 (d, J = 1.83, 1H); 7.74 (d, J = 8.52, 1H); 7.93 (s, 1H); 8.31 (s, 1H).

### EXEMPLE 141 : 3-(4-Chloro-2-fluoro-phenyl)-1,9-dimethyl-6-(2H-pyrazol-3-yl)-1,9-dihydro-pyrido[2,3-b]indol-2-one

### A) Composé A : 3-(4-Chloro-2-fluoro-phenyl)-1,9-dimethyl-1,9-dihydro-pyrido[2,3-b]indol-2-one

On procède comme indiqué à la preparation 1.2B ci-dessus avec du chlorhydrate de Methyl-(1-methyl-1H-indol-2-yl)-amine (préparé selon les modes opératoires décrits dans WO 2004/041817) et du 2-(4-Chloro-2-fluoro-phenyl)-3-dimethylamino-acrylic acid methyl ester

### B) Composé B : 6-Acetyl-3-(4-chloro-2-fluoro-phenyl)-1,9-dimethyl-1,9-dihydropyrido[2,3-b]indol-2-one

On procède comme indiqué à a preparation 1.9B ci-dessus avec le Composé A 3-(4-Chloro-2-fluoro-phenyl)-1,9-dimethyl-1,9-dihydro-pyrido[2,3-b]indol-2-one et du acetyl chloride

RMN 1H DMSO-d6 (300 MHz) : 2.63 (s, 3H); 4.02 (s, 3H) ; 4.19 (s, 3H) ; 7.34 (dd, J=8.28, J = 2.19, 1H) ; 7.47 (dd, J = 10.05, J = 2.07, 1H); 7.55 (t, J = 8.20, 1H); 7.72 (d, J = 8.73, 1H); 7.90 (dd, J = 8.67, J = 1.68, 1H); 8.46 (s, 1H); 8.63 (d, J = 1.5, 1H).

### C) Composé C : 3-(4-Chloro-2-fluoro-phenyl)-6-(3-dimethylamino-acryloyl)-1,9-dimethyl-1,9-dihydro-pyrido[2,3-b]indol-2-one

On procède comme indiqué à la preparation 1.9C ci-dessus avec du composé B 6-Acetyl-3-(4-chloro-2-fluoro-phenyl)-1,9-dimethyl-1,9-dihydro-pyrido[2,3-b]indol-2-one et du réactif de Bredereck.
MS : 437.99

### D) 3-(4-Chloro-2-fluoro-phenyl)-1,9-dimethyl-6-(2H-pyrazol-3-yl)-1,9-dihydropyrido[2,3-b]indol-2-one

On procède comme indiqué à l'ex36 ci-dessus avec du composé C 3-(4-Chloro-2-fluoro-phenyl)-6-(3-dimethylamino-acryloyl)-1,9-dimethyl -1,9-dihydro-pyrido[2,3-b]indol-2-one et de l'hydrazine monohydrate.
T°f : 325-326°C
MS : 406.78

RMN 1H DMSO-d6 (300 MHz) : 4.01 (s, 3H); 4.15 (s, 3H); 6.72 (s, 1H); 7.34 (dd, J = 8.4, J = 1.8, 1H); 7.47 (dd, J = 9.9, J = 1.8, 1H); 7.55 (t, J = 8.4, 1H); 7.62 (m, 1H); 7.77 (bs, 2H); 8.32 (s, 2H); 12.80 - 13.20 (bs, 1H).

### EXEMPLE 142 : 3-(4-Chloro-2-fluoro-phenyl)-6-(1-ethyl-1H-pyrazol-3-yl)-1,9-dimethyl-1,9-dihydro-pyrido[2,3-b]indol-2-one

On procède comme à l'exemple 110 ci-dessus à partir de composé de l'ex 141, 3-(4-Chloro-2-fluoro-phenyl)-1,9-dimethyl-6-(2H-pyrazol-3-yl)-1,9-dihydro-pyrido[2,3-b]indol-2-one et l'iodoéthane.
T°f : 215-217°C
MS : 435.20

RMN 1H DMSO-d6 (300 MHz) : 1.42 (t, J = 7.23, 3H); 4.00 (s, 3H); 4.15 (s, 3H); 4.18 (m, 2H); 6.69 (d, J = 2.19, 1H); 7.33 (dd, J = 8.28, J = 1.83, 1H); 7.46 (dd, J = 10.02, J = 1.92, 1H); 7.51 - 7.65 (m, 2H); 7.76 (m, 2H); 8.30 (s, 1H); 8.36 (s, 1H).

### EXEMPLE 143 : 3-(2,4-Dichloro-phenyl)-6-[2-(2-methoxy-ethyl)-2H-pyrazol-3-yl]-1,9-dimethyl-1,9-dihydro-pyrido[2,3-b]indol-2-one

### A) Composé A : 3-(2,4-Dichloro-phenyl)-6-[2-(2-hydroxy-ethyl)-2H-pyrazol-3-yl]-1,9-dimethyl-1,9-dihydro-pyrido[2,3-b]indol-2-one

On procède comme à l'exemple 37 ci-dessus avec le composé de la prep. 1.10 , 3-(2,4-Dichlorophényl)-6-[3-(diméthylamino)prop-2-ènoyl]-1,9-diméthyl-1,9-dihydro-2H-pyrido[2,3-b]indol-2-one et la 2-hydroxyethylhydrazine
MS : 466.85

### B) 3-(2,4-Dichloro-phenyl)-6-[2-(2-methoxy-ethyl)-2H-pyrazol-3-yl]-1,9-dimethyl-1,9-dihydro-pyrido[2,3-b]indol-2-one

On procède comme à l'exemple 101 ci-dessus avec le composé A, 3-(2,4-Dichlorophenyl)-6-[2-(2-hydroxy-ethyl)-2H-pyrazol-3-yl]-1,9-dimethyl-1,9-dihydro-pyrido[2,3-b]indol-2-one et l'iodomethane.
T°f : 150-154°C
MS : 481.00

RMN 1H DMSO-d6 (300 MHz) : 3.11 (s, 3H); 3.68 (t, J = 5.52, 2H); 4.03 (s, 3H); 4.20 (s, 3H); 4.27 (t, J = 5.61, 2H); 6.35 (d, J = 1.8, 1H); 7.38 - 7.50 (m, 3H); 7.52 (d, J = 1.77, 1H); 7.68 (d, J = 1.83, 1H); 7.74 (d, J = 8.52, 1H); 8.02 (d, J = 1.38, 1H); 8.26 (s, 1H).

### EXEMPLE 144 :

### 3-(2,4-Dichloro-phenyl)-6-(1-ethoxymethyl-1H-pyrazol-3-yl)-1,9-dimethyl-1,9-dihydro-pyrido[2,3-b]indol-2-one

On procède comme à l'exemple 36 ci-dessus avec le composé de l'exemple 39, 3-(2,4-Dichloro-phenyl)-1,9-dimethyl-6-(1H-pyrazol-3-yl)-1,9-dihydro-pyrido[2,3-b]indol-2-one et le chloromethyl ethyl ether.

RMN 1H DMSO-d6 (300 MHz) : 1.09 (t, J=7.0, 3H); 3.52 (q, J=7.0, 2H); 4.02/4.03 (S/s, 3H); 4.17/4.20 (S/s, 3H); 5.45 (s, 2H); 6.51/6.80 (s/S, 1H); 7.45-7.78 (m, 5H); 7.94/8.10 (S/s, 1H), 8.22-8.34 (m, 2H).
T°f=183°C-189°C

### EXEMPLE 145 : 6-(1-Acetyl-1H-pyrazol-3-yl)-3-(2,4-dichloro-phenyl)-1,9-dimethyl-1,9-dihydro-pyrido[2,3-b]indol-2-one

On procède comme à l'exemple 99 ci-dessus avec le composé de l'exemple 39, , 3-(2,4-Dichloro-phenyl)-1,9-dimethyl-6-(1H-pyrazol-3-yl)-1,9-dihydro-pyrido[2,3-b]indol-2-one et le chlorure d'acetyle

RMN 1H DMSO-d6 (300 MHz) : 2.72 (s, 3H); 4.02 (s, 3H); 4.19 (s, 3H); 7.15 (d, J=2.85, 1H); 7.47 (m, 2H); 7.71 (m, 2H); 7.91 (d, J=8.55, 1H); 8.31 (s, 1H); 8.47 (m, 2H).
T°f= 256°C260°C (dec.)

### EXEMPLE 146 : 6-(2-Aminomethyl-thiazol-4-yl)-3-(2,4-dichloro-phenyl)-1,9-dimethyl-1,9-dihydro-pyrido[2,3-b]indol-2-one

### A) composé A : {4-[3-(2,4-Dichloro-phenyl)-1,9-dimethyl-2-oxo-2,9-dihydro-1H-pyrido[2,3-b]indol-6-yl]-thiazol-2-ylmethyl}-carbamic acid benzyl ester

On procède comme pour l'ex 8 ci-dessus à partir du composé de la prep.1.2, 6-Bromoacétyl)-3-(2,4-dichlorophényl)-1,9-diméthyl-1,9-dihydro-2H-pyrido[2,3-b]indol-2-one et le N-benzyloxycarbonylglycine thioamide

RMN 1H DMSO-d6 (300 MHz) : 4.01 (s, 3H); 4.17 (s, 3H); 4.55 (d, J= 6.15, 2H); 5.10 (s, 2H); 7.32-7.46 (m, 7H); 7.67 (m, 2H); 7.88 (m, 2H); 8.25 (s, 2H); 8.44 (s, 1H).

### B) 6-(2-Aminomethyl-thiazol-4-yl)-3-(2,4-dichloro-phenyl)-1,9-dimethyl-1,9-dihydro-pyrido[2,3-b]indol-2-one

Dissoudre 780 mg (1.3 mmol) de composé A {4-[3-(2,4-Dichloro-phenyl)-1,9-dimethyl-2-oxo-2,9-dihydro-1H-pyrido[2,3-b]indol-6-yl]-thiazol-2-ylmethyl}-carbamic acid benzyl ester dans 12 mL d'acide trifluoroacétique. Ajouter 8 mL (10.3 mmol) de thioanisole et agiter à température ambiante pendant 16 heures. Verser sur une solution saturée en K2CO3 et extraire à l'AcOEt. Adsorber sur silice la phase organique et purifier sur colonne de silice éluée avec de l'AcOEt puis avec AcOEt/MeOH/NH3 90/10/1.
Obtention de 100 mg de poudre jaune pâle.

RMN 1H DMSO-d6 (300 MHz) : 4.01 (s, 3H); 4.07 (s, 2H); 4.17 (s, 3H); 7.47 (m, 2H); 7.67 (m, 2H); 7.87 (m, 2H); 8.27 (s, 1H); 8.44 (s,1 1H).
T°f= 176°C-180°C

### EXEMPLE 147 : N-{4-[3-(2,4-Dichloro-phenyl)-1,9-dimethyl-2-oxo-2,9-dihydro-1H-pyrido[2,3-b]indol-6-yl]-thiazol-2-ylmethyl}-methanesulfonamide

On procède comme indiqué à l'ex99 ci-dessus avec le composé de l'ex147, 6-(2-Aminomethyl-thiazol-4-yl)-3-(2,4-dichloro-phenyl)-1,9-dimethyl-1,9-dihydro-pyrido[2,3-b]indol-2-one et le methanesulfonyl chloride

RMN 1H DMSO-d6 (300 MHz) : 3.03 (s, 3H); 4.02 (s, 3H); 4.19 (s, 3H); 4.54 (s, 2H); 7.47 (m, 2H); 7.69 (m, 2H); 7.90 (dd, J=1.8, J=8.7, 1H); 7.96 (s, 1H); 8.10 (bs, 1H); 8.28 (s, 1H); 8.46 (s, 1H).
T°f= 164°C-169°C

### EXEMPLE 148 : 3-(2,4-Dichloro-phenyl)-6-(2-methoxymethoxymethyl-thiazol-4-yl)-1,9-dimethyl-1,9-dihydro-pyrido[2,3-b]indol-2-one

On procède comme indiqué à l'ex10 ci-dessus avec le composé l'ex 9, 3-(2,4-Dichloro-phenyl)-6-(2-hydroxymethyl-thiazol-4-yl)-1,9-dimethyl-1,9-dihydropyrido[2,3-b]indol-2-one et le bromomethylmethylether.
Tf = 160-164 °C

RMN 1H DMSO-d6 (300 MHz) : 3.28 (s, 3H) ; 3.35 (s, 2H) ; 4.01 (s, 3H); 4,17 (s, 3H) ; 4.83 (m, 2H) ; 7.43-7.46 (m, 2H) ; 7.65-7.68 (m, 2H) ; 7.88-7.91 (dd, J=1.5, 8.6, 1H) ; 7.99 (s, 1H) ; 8.28 (s, 1H) ; 8.46 (d, J=1.2, 1H).

### EXEMPLE 149 : 6-(2-Cyclopropylmethoxymethyl-thiazol-4-yl)-3-(2,4-dichlorophenyl)-1,9-dimethyl-1,9-dihydro-pyrido[2,3-b]indol-2-one

On procède comme indiqué à l'ex10 ci-dessus avec le composé l'ex 9, 3-(2,4-Dichloro-pheny)-6-(2-hydroxymethyl-thiazol-4-yl)-1,9-dimethyl-1,9-dihydro-pyrido[2,3-b]indol-2-one et le bromomethylcyclopropane.

RMN 1H DMSO-d6 (300 MHz) : 0.20-0.25 (m, 2H) ; 0.49-0.52 (m, 2H) ; 1.03-1.11 (m, 1H) ; 3.43 (d, J=6.8, 2H) ; 4.01 (s, 3H) ; 4.17 (s, 3H) ; 4.83 (s, 2H) ; 7.43-7.46 (m, 2H) ; 7.65-7.68 (m, 2H) ; 7.89 (dd, J=1.6, J=8.6, 1H) ; 7.97 (s, 1H) ; 8.27 (s, 1H) ; 8.46 (d, J=1.4, 1H).

### EXEMPLE 150 : 3-(2,4-Dichoro-phenyl)-6-[2-(2-methoxy-ethylamino)-thiazol-4-yl]-1,9-dimethyl-1,9-dihydro-pyrido[2,3-b]indol-2-one

On procède comme pour l'ex 21 ci-dessus à partir du composé de la prep. 1.2, 6-(Bromoacétyl)-3-(2,4-dichlorophényl)-1,9-dimémyl-1,9-dihydro-2H-pyrido[2,3-b]indol-2-et la 1-(2-methoxyethyl)-2-thiourée.

RMN 1H DMSO-d6 (300 MHz) : 3.30 (s, 3H) ; 3.53 (m, 4H) ; 4.01 (s, 3H) ; 4.16 (s, 3H) ; 6.94 (s, 1H) ; 7.46 (m, 2H) ; 7.56-7.80 (m, 4H) ; 8.23 (s, 1H) ; 8.32 (s, 1H)

### EXEMPLE 151 : 3-(2,4-Dichloro-phenyl)-9-difluoromethyl-6-(2-ethyl-2H-pyrazol-3-yl)-1-methyl-1,9-dihydro-pyrido[2,3-b]indol-2-one

### A) composé A : 6-Acetyl-3-(2,4-dichloro-phenyl)-9-difluoromethyl-1-methyl-1,9-dihydro-pyrido[2,3-b]indol-2-one

Dissoudre 460 mg (1.19 mmol) du composé de la prep. 1.9A, 6-Acétyl-3-(2.4-dichlorophényl)-1-méthyl-1,9-dihydro-2H-pyrido[2,3-b]indol-2-one dans 7 mL de DMF et 2 mL d'eau. Ajouter 543 mg (1.67 mmol) de carbonate de césium et 419 mg (2.75 mmol) de sodium chlorodifluoroacetate. Chauffer à 100°C pendant 48 heures. Revenir à température ambiante. Verser le milieu réactionnel sur de l'eau, filtrer le précipité formé et laver à l'eau. Reprendre dans un mélange AcOEt/MeOH et adsorber sur silice. Purifier sur colonne de silice éluée avec un mélange AcOEt/cyclohexane 50/50 puis avec de l'AcOEt.

Obtention de 170 mg de poudre beige.

RMN 1H DMSO-d6 (300 MHz) : 2.65 (s, 3H) ; 3.87 (s, 3H) ; 7.50 (m, 2H) ; 7.73 (d, J=2.1, 1H); 7.89 (m, 2H) ; 8.40 (t, J=59, 1H) ; 8.51 (s, 1H) ; 8.69 (s, 1H).

### B) Composé B : 3-(2,4-Dichloro-phenyl)-9-difluoromethyl-6-((E)-3-dimethylamino -acryloyl)-1-methyl-1,9-dihydro-pyrido[2,3-b]indol-2-one

On procède comme pour la prep. 1.10 ci-dessus à partir du composé A : 6-Acetyl-3-(2,4-dichloro-phenyl)-9-difluoromethyl-1-methyl-1,9-dihydro-pyrido[2,3-b] indol-2-one et le réactif de Bredereck.

### C) 3-(2,4-Dichloro-phenyl)-9-difluoromethyl-6-(2-ethyl-2H-pyrazol-3-yl)-1-methyl-1,9-dihydro-pyrido[2,3-b]indol-2-one

On procède comme indiqué à l'ex 37 ci-dessus avec du composé B, 3-(2,4-Dichlorophenyl)-9-difluoromemyl-6-((E)-3-dimemylamino-acryloyl)-1-methyl-1,9-dihydropyrido[2,3-b]indol-2-one et l'éthyl hydrazine oxalate.

RMN 1H DMSO-d6 (300 MHz) : 1.29 (t, J=7.2, 3H); 3.88 (s, 3H) ; 4.17 (d, J=7.2, 2H) ;6.38 (s, 1H) ; 7.44 (m, 4H) ; 7.72 (d, J=2.1, 1H) ; 7.86 (d, J=8.1, 1H) ; 8.09 (d, J=1.5, 1H); 8.40 (t, J=60, 1H); 8.44 (s, 1H).

### EXEMPLE 152 : 3-(2,4-Dicholo-phenyl)-6-[2-(1-hydroxy-ethyl)-thiazol-4-yl]-1,9-dimethyl-1,9-dihydro-pyrido[2,3-b]indol-2-one

### A) Composé A : 4-[3-(2,4-Dichloro-phenyl)-1,9-dimethyl-2-oxo-2,9-dihydro-1H-pyrido[2,3-b]indol-6-yl]-thiazole-2-carbaldehyde

Mettre en suspension 258 mg (0.548 mmol) de composé de l'ex 9, 3-(2,4-Dichloro-pbenyl)-6-(2-hydroxymethyl-thiazol-4-yl)-1,9-dimethyl-1,9-dihydro-pyrido[2,3-b]indol-2-one dans 10 mL de CHCl3. Ajouter 177 mg (0.82 mmol) de pyridinium chlorochromate (PCC). Agiter à température ambiante pendant 3 heures. Déposer le milieu réactionnel directement sur une colonne de silice et purifier le mélange en éluant avec 100% AcOEt. Obtention de 163 mg de poudre jaune.
MS : 468.01

RMN 1H DMSO-d6 (300 MHz) : 4.02 (s, 3H); 4.20 (s, 3H); 7.47 (m, 2H); 7.69 (s, 1H); 7.74 (d, J = 8.64, 1H); 8.00 (d, J = 8.55, 1H); 8.30 (s, 1H); 8.59 (s, 2H); 10.03 (s, 1H).

### B) 3-(2,4-Dichloro-phenyl)-6-[2-(1-hydroxy-ethyl)-thiazol-4-yl]-1,9-dimethyl-1,9-dihydro-pyrido[2,3-b]indol-2-one

On procède comme indiqué à l'ex139 ci-dessus avec du composé A, 4-[3-(2,4-Dichloro-phenyl)-1,9-dimethyl-2-oxo-2,9-dihydro-1H-pyrido[2,3-b]indol-6-yl]-thiazole-2-carbaldehyde et le Methylmagnesium bromide
T°f : 280-285°C
MS : 484.3

RMN 1H DMSO-d6 (300 MHz) : 1.51 (d, J = 6.45, 3H); 4.00 (s, 3H); 4.16 (s, 3H); 4.99 (m, 1H); 6.15 (d, J = 4.89, 1H); 7.45 (m, 2H); 7.67 (m, 2H); 7.87 (m, 2H); 8.26 (s, 1H); 8.44 (d, J = 1.29, 1H).

### EXEMPLE 153 : 3-(2,4-Dichloro-phenyl)-6-(2-fluoromethyl-thiazol-4-yl)-1,9-dimethyl-1,9-dihydro-pyrido[2,3-b]indol-2-one

Dissoudre 150 mg (0.319 mmol) de composé de l'ex 9, 3-(2,4-Dichloro-phenyl)-6-(2-hydroxymethyl-thiazol-4-yl)-1,9-dimethyl-1,9-Mydro -pyrido[2,3-b]indol-2-one dans 3 mL de CH2Cl2. A 0°C, ajouter 51 µL de (diethylamino)sulfur trifluoride (DAST). Agiter à 0°C pendant 2 heures. Rajouter 100µL de DAST (présence de produit de départ) et laisser agiter à température ambiante pendant 16 heures supplémentaires. Ajouter H2O, extraire avec CH2C12, sécher sur MgS04, filtrer et concentrer à sec en adsorbant sur silice. Purifier sur colonne de silice éluée avec AcOEt/Cyclohexane 40% puis avec AcOEt/Cycloherane 30%. Reprendre le résidu dans MeOH, filtrer le précipité, le laver avec MeOH puis avec un minimum d'Et2O.
Obtention de 50 mg de poudre jaune pâle.
T°f: 259-260°C
MS : 471.95

RMN 1H DMSO-d6 (300 MHz) : 3.99 (s, 3H); 4.15 (s, 3H); 5.66 - 5.81 (m, 2H); 7.43 (m, 2H); 7.66 (m, 2H); 7.89 (d, J = 8.58, 1H); 8.11 (s, 1H); 8.26 (s, 1H); 8.47 (s, 1H).

### EXEMPLE 154 : 3-(2,4-Dichloro-phenyl)-6-(1-methoxymethyl-1H-[1,2,3]triazol-4-yl)-1,9-dimethyl-1,9-dihydro-pyrido[2,3-b]indol-2-one

Dissoudre 209 mg (0.49 mmol) de composé de l'ex 47; 3-(2,4-Dichloro-phenyl)-1,9-dimethyl-6-(1H-[1,2,3]triazol-4-yl)-1,9-dihydro-pyrido[2,3-b]indol-2-one dans 3 mL de DMF. Rajouter 30 mg (0.74 mmol) de NaH 60% et une pointe de spatule de dibenzo-18-crown-6. Ajouter ensuite 80 µL de bromomethyl methyl ether. Agiter à température ambiante pendant 16 heures. Verser le milieu réactionnel sur de l'eau puis extraire à l'AcOEt, sécher sur MgSO4, filtrer et évaporer à sec en adsorbant sur silice. Purifier sur colonne de silice éluée avec un gradient d'AcOEt/Cyclohexane 50% puis avec AcOEt/Cyclohexane 40% et enfin AcOEt 100%. Triturer le résidu dans le MeOH pour obtenir un solide beige.
Obtention de 28 mg de poudre beige
T°f :173-176°C
MS : 467.84

RMN 1H DMSO-d6 (300 MHz) : 3.28 (s, 3H); 3.99 (s, 3H); 4.16 (s, 3H); 5.64 (s, 2H); 7.44 (m, 2H); 7.66 (m, 1H); 7.70 (s, 1H); 7.78 (m, 1H); 8.26 (s, 1H); 8.31 (s, 1H); 8.40 (s, 1H).

### EXEMPLE 155 : 3-[3-(2,4-Dichloro-phenyl)-1,9-dimethyl-2-oxo-2,9-dihydro-1H-pyrido[2,3-b]indol-6-yl]-pyrazole-1-sulfonic acid dimethylamide

On procède comme indiqué à l'ex99 ci-dessus avec le composé de l'Ex 39, 3-(2,4-Dichloro-phenyl)-1,9-dimethyl-6-(2H-pyrazol-3-yl)-1,9-dihydro-pyrido[2,3-b]indol-2-one et le dimethyl sulfamoyl chloride.

RMN 1H DMSO : 2.92 (s, 6H) ; 4.03 (s, 3H) ; 4.20 (s, 3H) ; 7.09 (d, J=3, 1H) ; 7.47 (m, 2H) ; 7.70 (m, 2H) ; 7.85 (dd, J=1.5, J=8.6, 1H) ; 8.31 (m, 1H) ; 8.45 (s, 1H).

### EXEMPLE 156 : 3-(2,4-Dichloro-phenyl)-6-[2-(1-fluoro-1-methyl-ethyl)-thiazol-4-yl]-1,9-dimethyl-1,9-dihydro-pyrido[2,3-b]indol-2-one

On procède comme indiqué à à l'ex 153 ci-dessus avec le composé de l'Ex 139

### EXEMPLE 157 3-(2,4-Dichloro-phenyl)-6-[2-(2-hydroxy-ethyl)-2H-pyrazol-3-yl]-1,9-dimethyl-1,9-dihydro-pyrido[2,3-b]indol-2-one

On procède comme indiqué à à l'ex 37 ci-dessus avec le composé de la prep 1.10 et la 2-hydmxyethylhydrazine
T°f : 204-206°C
MS : 467.3

RMN 1H DMSO-d6 (300 MHz) : 3.75 (q, J = 5.7, 2H); 4.03 (s, 3H); 4.16 (m, 2H); 4.20 (s, 3H); 4.91 (t, J = 5.31, 1H); 6.35 (d, J = 1.74, 1H); 7.40 - 7.50 (m, 3H); 7.51 (d, J = 1.77, 1H); 7.69 (d, J = 1.89, 1H); 7.72 (m, 1H); 8.05 (d, J = 1.26, 1H); 8.26 (s, 1H).

### EXEMPLE 158 : 3-(2,4-Dichloro-phenyl)-1,9-dimethyl-6-{1-[2-(2-pyrrolidin-1-yl-ethoxy)-ethyl]-1H-pyrazol-3-yl}-1,9-dihydro-pyrido[2,3-b]indol-2-one

On procède comme indiqué à à l'ex 101 ci-dessus avec le composé de l'exemple 51 ci-dessus, 3-(2,4-Dichloro-phenyl)-6-[1-(2-hydroxy-ethyl)-1H-pyrazol-3-yl]-1,9-dimethyl-1,9-dihydro-pyrido[2,3-b]indol-2-one et la 1-(2-chloroethyl)pyrrolidine

### EXEMPLE 159 : 3-(2,4-Dichloro-phenyl)-6-[1-(2-methoxy-acetyl)-4-methyl-1H-pyrazol-3-yl]-1,9-dimethyl-1,9-dihydro-pyrido[2,3-b]indol-2-one

On procède comme indiqué à à l'ex 70 ci-dessus avec le composé de l'exemple 53 3-(2,4-Dichloro-phenylyl,9-dimethyl-6-(4-methyl-2H-pyrazol-3-yl)-1,9-dihydropyrido[2,3-b]indol-2-one et le méthoxyacétyl chloride

RMN 1H DMSO-d6 (300 MHz) : 2.3 (s, 3H) ; 3.42 (s, 3H) ; 4.03 (s, 3H) ; 4.20 (s, 3H) ; 4.92 (s, 2H) ; 7.43-7.50 (m, 2H) ; 7.68-7.75 (m, 3H) ; 8.25 (s, 1H) ; 8.30 (s, 1H) ; 8.38 (s, 1H).

### EXEMPLE 160 : 3-2,4-Dichloro-phenyl)-6-[1-(2-methoxy-acetyl)-1-pyrazol-3-yl]-1,9-dimethyl-1,9-dihydro-pyrido[2,3-b]indol-2-one

On procède comme indiqué à à l'ex 70 ci-dessus avec le composé de l'exemple 39 3-(2,4-Dichloro-phenyl)-1,9-dimethyl-(1H-pyrazol-3-yl)-1,9-dihydro-pyrido[2,3-b]indol-2-one et le methoxy acetyl chloride

RMN 1H DMSO-d6 (300 MHz) : 3.44 (s, 3H); 4.03 (s, 3H); 4.20 (s, 3H); 4.98 (s, 2H); 7.18 (d, J=2.7,1H); 7.48 (m, 2H); 7.70 (m, 2H); 7.90 (dd, J=1.5, J=8.6, 1H); 8.31 (s, 1H); 8.50 (m, 2H).
Tf= 243°C-247°C

### EXEMPLE 161 : 3-(2,4-Dichloro-phenyl)-9-difluoromethyl-6-(1-ethyl-1H-pyrazol-3-yl)-1-methyl-1,9-dihydro-pyrido[2,3-b]indol-2-one

### A) ComposéA : 3-(2,4-Dichloro-phenyl)-9-difluoromethyl-1-methyl-6-(1H-pyrazol-3-yl)-1,9-dihydro-pyrido[2,3-b]indol-2-one

On procède comme indiqué à à l'ex 40 ci-dessus avec le composé de l'exemple151B, 3-(2,4-Dichloro-phenyl)-9-difluoromethyl-6-((E)-3-dimethylamino-acryloyl)-1-methyl-1,9-dihydro-pyrido[2,3-b]indol-2-one et l'hydrazine mono hydrate.

RMN 1H DMSO-d6 (300 MHz) : 3.86 (s, 3H); 6.75 (s, 1H); 7.51 (m, 2H); 7.74 (m, 4H); 8.35 (t, J=57, 1H); 8.41 (s, 2H); 12.89 (s, 1H).

### B) 3-(2,4-Dichloro-phenyl)-9-difluoromethyl-6-(1-ethyl-1H-pyrazol-3-yl)-1-methyl-1,9-dihydro-pyrido[2,3-b]indol-2-one

On procède comme indiqué à à l'ex 110 ci-dessus avec le composé A, 3-(2,4-Dichloro-phenyl)-9-dinuoromethyl-1-methyl-6-(1H-pyrazol-3-yl)-1,9-dihydro-pyrido[2,3-b]indol-2-one et l'iodoéthane.

### EXEMPLE 162 : 2,2-Dimethyl-propionic acid 5-[3-(2,4-dichloro-phenyl)-1,9-dimethyl-2-oxo-2,9-dihydro-1H-pyrido[2,3-b]indol-6-yl]-2-methyl-2H-pyrazol-3-ylmethyl ester

On procède comme indiqué à à l'ex 70 ci-dessus avec le composé de l'exemple 82, 3-(2,4-Dichloro-phenyl)-6-(5-hydroxymethyl-1methyl-1H-pyrazol-3-yl)-1,9-dimethyl-1,9-dihydro-pyrido[2,3-b]indol-2-one et le chlorure de pivaloyle.

RMN 1H DMSO-d6 (300 MHz) : 1.17 (s, 9H); 3.86 (s, 3H); 4.01 (s, 3H); 4.16 (s, 3H); 5.18 (s, 2H); 6.76 (s, 1H); 7.47 (m, 2H); 7.62 (d, 1=8.4, 1H); 7.72 (m, 2H); 8.29 (m, 2H).
Tf=145°C-150°C

### EXEMPLE 163: 6-[1-(2,2-Dimethyl-propionyl)-4-methyl-1H-pyrazol-3-yl]-3-(4-fluoro-phenyl)-1,9-dimethyl-1,9-dihydro-pyrido[2,3-b]indol-2-one

On procède comme indiqué à à l'ex 70 ci-dessus avec le composé de l'exemple 53, 3-(4-Fluoro-phenyl)-1,9-dimethyl-6-(4-methyl-2H-pyrazol-3-yl)-1,9-dihydropyrido[2,3-b]indol-2-one et le chlorure de pivaloyle.

RMN 1H DMSO-d6 (300 MHz) : 1.48 (s, 9H) ; 2.28 (s, 3H) ; 4,00 (s, 3H) ; 4.13 (s, 3H) ; 7.16-7.22 (m, 2H) ; 7.66-7.79 (m, 4H) ; 8.22 (d, J=4.02, 2H) ; 8.50 (s, 1H)

### EXEMPLE 164 : 3-(2,4-Dichloro-phenyl)-1,9-dimethyl-6-[1-(3,3,3-trifluoro-propionyl)-1H-pyrazol-3-yl]-1,9-dihydro-pyrido[2,3-b]indol-2-one

On procède comme indiqué à à l'ex 70 ci-dessus avec le composé de l'exemple 39 3-(2,4-Dichloro-phenyl)-1,9-dimethyl-(1H-pyrazol-3-yl)-1,9-dihydro-pyrido[2,3-b]indol-2-one et le 3,3,3-trifluropropionyl chloride

RMN 1H DMSO-d6 (300 MHz) : 4.03 (s, 3H); 4.20 (s, 3H); 4.57 (q, J=12.3, 2H); 7.26 (d, J=2.9,1H); 7.48 (m, 2H); 7.71 (m, 2H); 7.94 (d, J=8.6, 1H); 8.30 (s, 1H); 8.56 (m, 2H).
Tf= 262°C-268°C (dec.)

Le tableau qui suit illustre les structures chimiques et les propriétés physiques de quelques exemples de composés selon l'invention.

**TABLEAU 1**

| | | | |
|---|---|---|---|
| 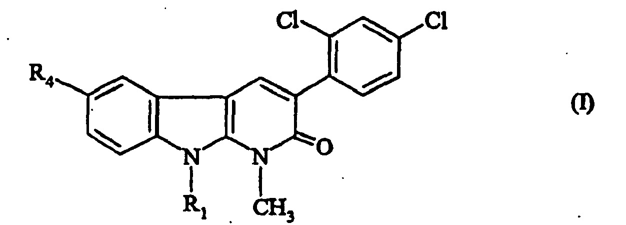 | | | |

| Composés N° | R₁ | R₄ | F°C RMN |
|---|---|---|---|
| 1 | H | 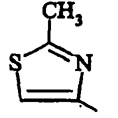 | > 350 (déc.) |
| 2 | H | 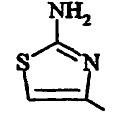 | > 350 (déc.) |
| 3 | H | 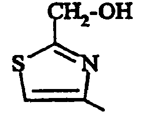 | RMN |
| 4 | H | 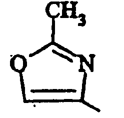 | RMN |
| 5 | H | 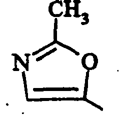 | 338-340 |
| 6 | -CH₃ | 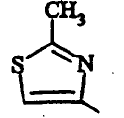 | 253-254 |
| 7 | -CH₃ | 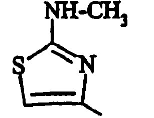 | RMN |
| 8 | -CH₃ | 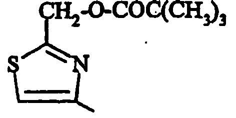 | 193-195 |
| 9 | -CH₃ | 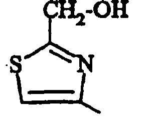 | 261-262 |
| 10 | -CH₃ | 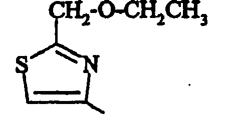 | RMN |
| 11 | -CH₃ | 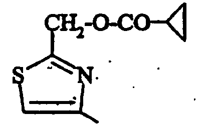 | RMN |
| 12 | -CH₃ | 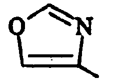 | 221-222 |
| 13 | -CH₃ | 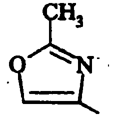 | 222-223 |
| 14 | -CH₃ | 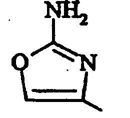 | 295-297 |
| 15 | -CH₃ | 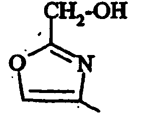 | RMN |
| 16 | -CH₃ | 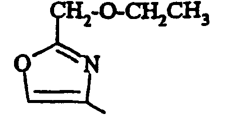 | RMN |
| 17 | -CH₃ | 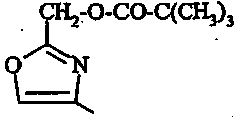 | RMN |
| 18 | -CH₃ | 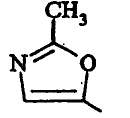 | 239-240 |
| 19 | -CH₃ | 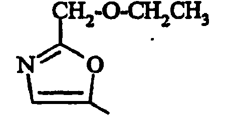 | 179-180 |
| 20 | -CH₃ | 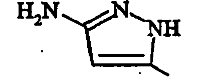 | 284-286 |
| 21 | -CH₃ | 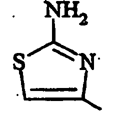 | 272-273 |
| 22 | H | 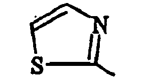 | RMN |
| 23 | -CH₃ | 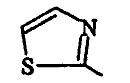 | |
| 24 | -CH₃ | 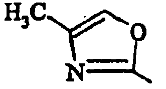 | 258-261 |
| 25 | -CH₃ | 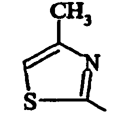 | 255-257 |
| 26 | -CH₃ | 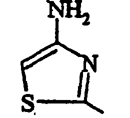 | RMN |
| 27 | -CH₃ | 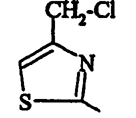 | 277-281 |
| 28 | -CH₃ | 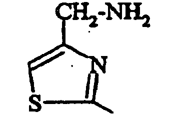 | 261-266 |
| 29 | -CH₃ | 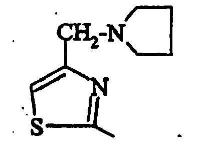 | 222-226 |
| 30 | -CH₃ | 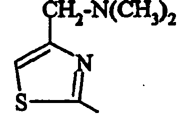 | RMN |
| 31 | -CH₃ | 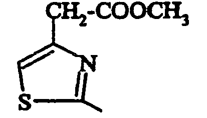 | 198-201 |
| 32 | -CH₃ | 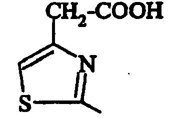 | 234-239 |
| 33 | -CH₃ | 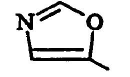 | 170-174 |
| 34 | -CH₃ | 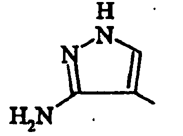 | 240-245 |
| 35 | H | 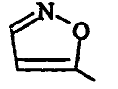 | 320-323 |
| 36 | H | 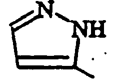 | 246-250 |
| 37 | H | 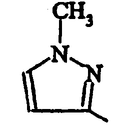 | 129-135 |
| 38 | H | 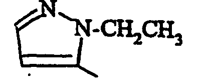 | 255-258 |
| 39 | -CH₃ | 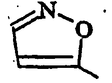 | 241-242 |
| 40 | -CH₃ | 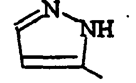 | 281-282 |
| 41 | -CH₃ | 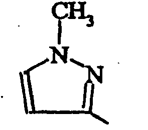 | 287-290 |
| 42 | -CH₃ | 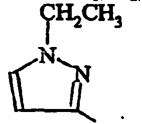 | 230-232 |
| 43 | -CH₃ | 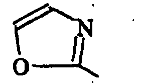 | 232-233 |
| 44 | -CH₃ | 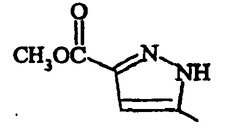 | 290-292 |
| 45 | -CH₃ | 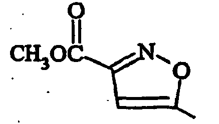 | 280-281 |
| 46 | -CH₃ | 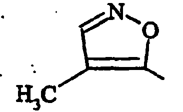 | 233-234 |
| 47 | -CH₃ | 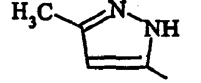 | 309-310 |
| 48 | -CH₃ | 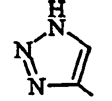 | 274-275 |
| 49 | -CH₃ | 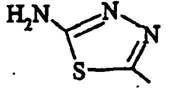 | 321-323 |
| 50 | -CH₃ | 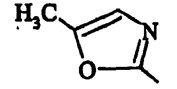 | 289-292 |

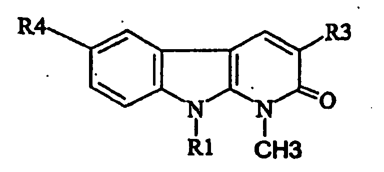

Le tableau qui suit illustre les structures chimiques et les propriétés physiques de quelques exemples de composés selon l'invention.

**TABLEAU II**

| | R1 | R4 | R3 | |
|---|---|---|---|---|
| 51 | -CH₃ | 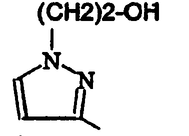 | 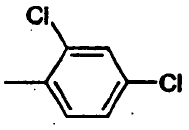 | RMN |
| 52 | -CH₃ | 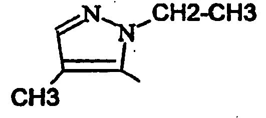 | 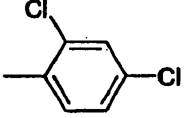 | RMN |
| 53 | CH₃ | 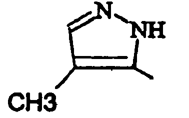 | 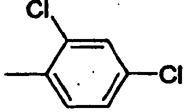 | RMN |
| 54 | CH₃ | 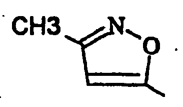 | 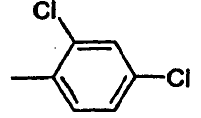 | RMN |
| 55 | CH₃ | 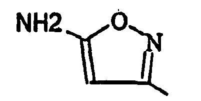 | 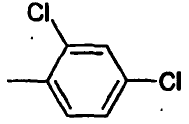 | RMN |
| 56 | CH₃ | 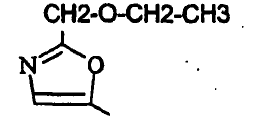 | 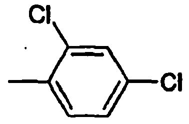 | RMN |
| 57 | CH₃ | 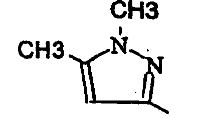 | 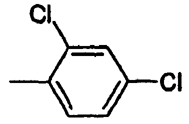 | RMN |
| 58 | CH₃ | 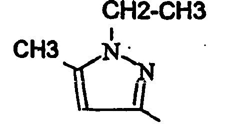 | 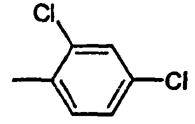 | RMN |
| 59 | CH₃ | 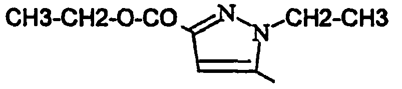 | 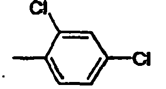 | RMN |
| 60 | CH₃ | 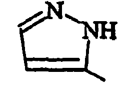 | 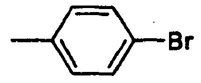 | RMN |
| 61 | CH₃ | 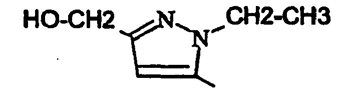 | 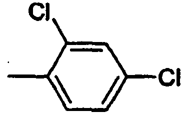 | RMN. |
| 62 | CH₃ | 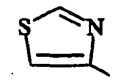 | 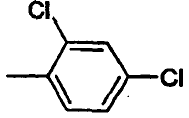 | RMN |
| 63 | CH₃ | 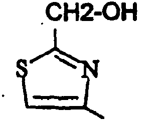 | 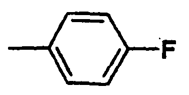 | RMN |
| 64 | CH₃ | 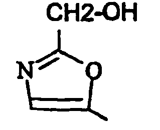 | 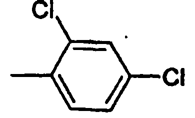 | RMN |
| 65 | CH₃ | 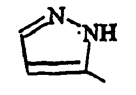 | 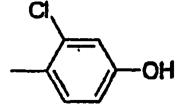 | RMN |
| 66 | CH₃ | 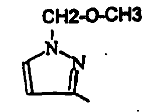 | 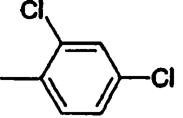 | RMN |
| 67 | CH₃ | 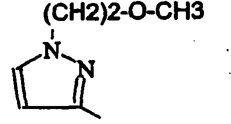 | 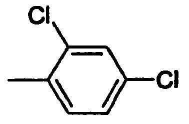 | RMN |
| 68 | CH₃ | 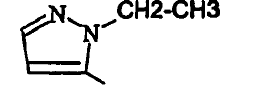 | 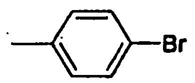 | RMN |
| 69 | CH₃ | 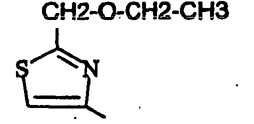 | 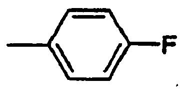 | RMN |
| 70 | CH₃ | 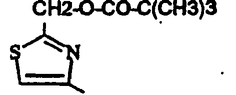 | 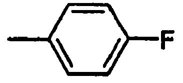 | RMN |
| 71 | CH₃ | 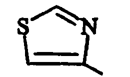 | 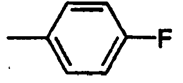 | RMN |
| 72 | CH₃ | 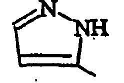 | 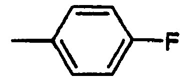 | RMN |
| 73 | CH₃ | 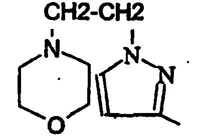 | 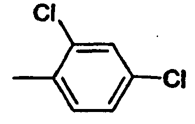 | RMN |
| 74 | CH₃ | 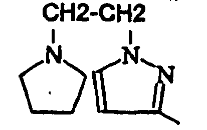 | 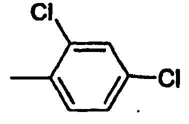 | RMN |
| 75 | CH₃ | 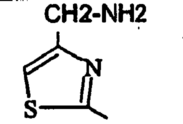 | 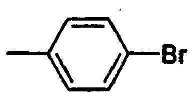 | RMN |
| 76 | CH₃ | 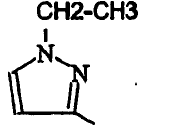 | 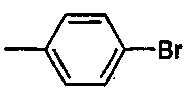 | RMN |
| 77 | CH₃ | 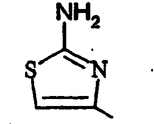 | 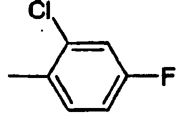 | RMN |
| 78 | CH₃ | 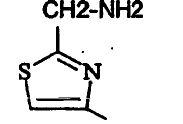 | 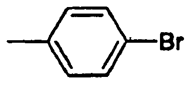 | RMN |
| 79 | CH₃ | 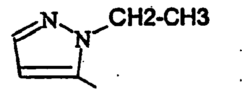 | 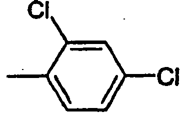 | RMN |
| 80 | CH₃ | 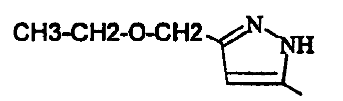 | 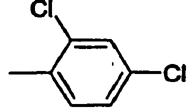 | RMN |
| 81 | CH₃ | 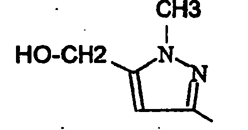 | 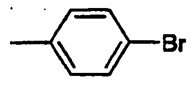 | RMN |
| 82 | CH₃ | 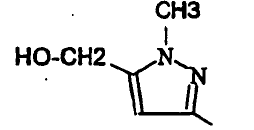 | 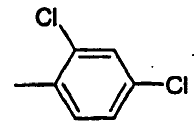 | RMN |
| 83 | CH₃ | 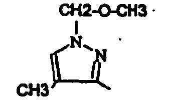 | 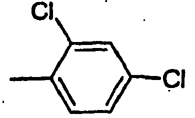 | RMN |
| 84 | CH₃ | 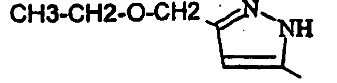 | 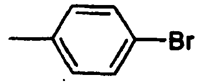 | RMN |
| 85 | CH₃ | 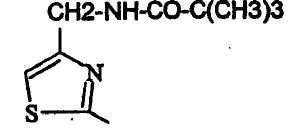 | 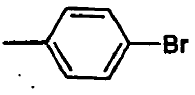 | RMN |
| 86 | CH₃ | 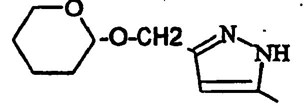 | 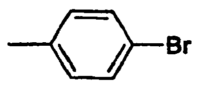 | RMN |
| 87 | CH₃ | 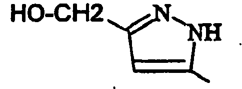 | 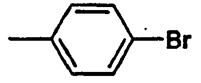 | RMN |
| 88 | CH₃ | 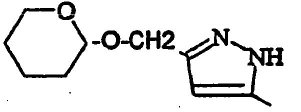 | 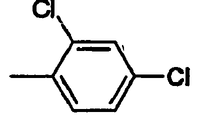 | RMN |
| 89 | CH₃ | 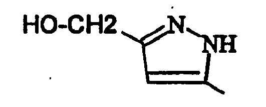 | 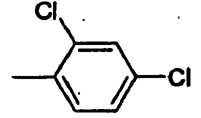 | RMN |
| 90 | CN | 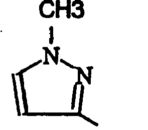 | 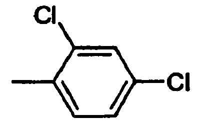 | RMN |
| 91 | CH₃ | 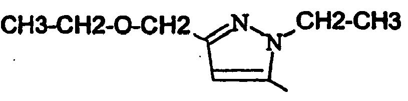 | 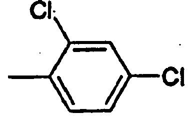 | RMN |
| 92 | CH₃ | 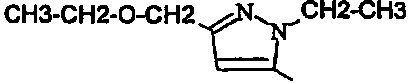 | 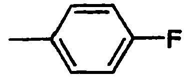 | RMN |
| 93 | CH₃ | 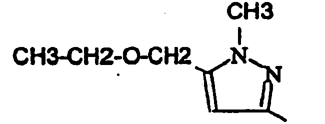 | 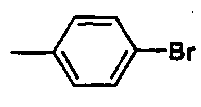 | RMN |
| 94 | CH₃ | 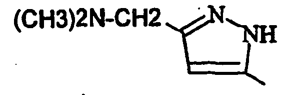 | 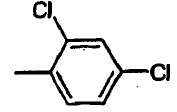 | RMN |
| 95 | CH₃ | 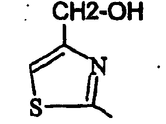 | 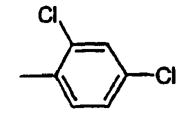 | RMN |
| 96 | CH₃ | 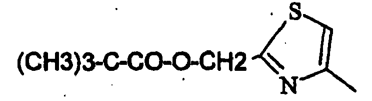 | 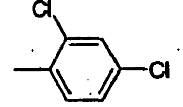 | RMN |
| 97 | CN | 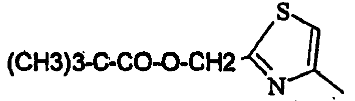 | 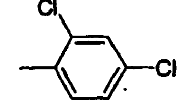 | RMN |
| 98 | CH₃ | 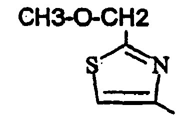 | 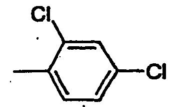 | RMN |
| 99 | CH₃ | 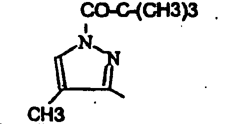 | 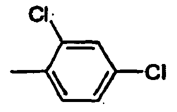 | RMN |
| 100 | CH₃ | 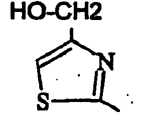 | 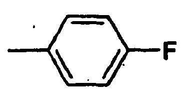 | RMN |
| 101 | CH₃ | 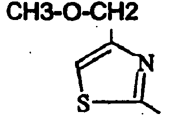 | 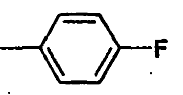 | RMN |
| 102 | CH₃ | 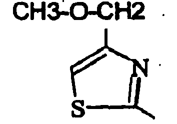 | 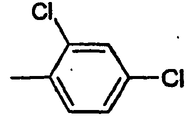 | RMN |
| 103 | CH₃ | 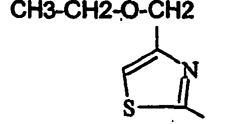 | 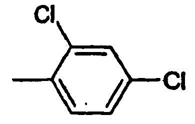 | RMN |
| 104 | CH₃ | 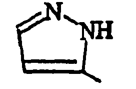 | 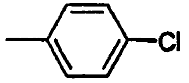 | RMN |
| 105 | CH₃ | 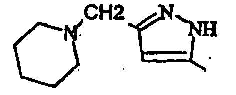 | 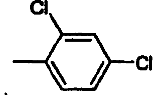 | RMN |
| 106 | CH₃ | 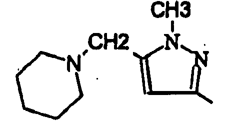 | 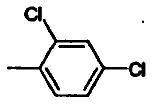 | RMN |
| 107 | CH₃ | 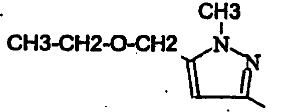 | 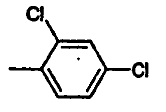 | RMN |
| 108 | CH₃ | 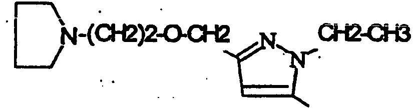 | 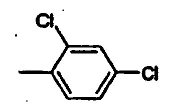 | RMN |
| 109 | H | 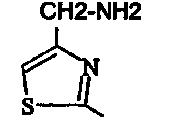 | 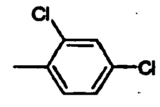 | RMN |
| 110 | CH₃ | 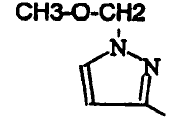 | 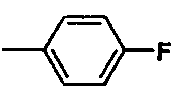 | RMN |
| 111 | CH₃ | 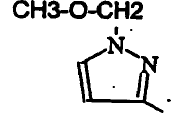 | 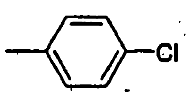 | RMN |
| 112 | CH₃ | 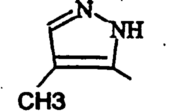 | 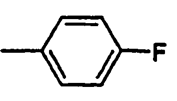 | RMN |
| 113 | CH₃ | 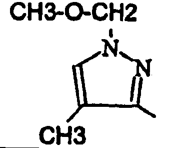 | 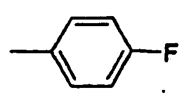 | RMN |
| 114 | CH₃ | 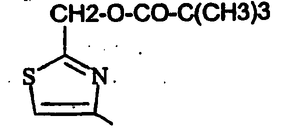 | 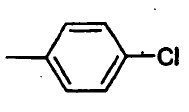 | RMN |
| 115 | CH₃ | 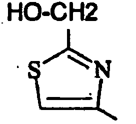 | 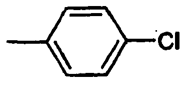 | RMN |
| 116 | CH₃ | 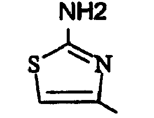 | 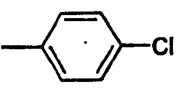 | RMN |
| 117 | CH₃ | 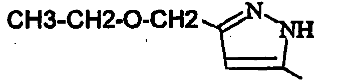 | 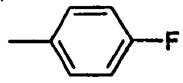 | RMN |
| 118 | CH₃ | 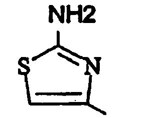 | 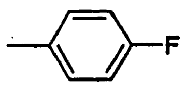 | RMN |
| 119 | CH₃ | 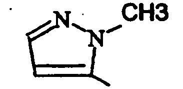 | 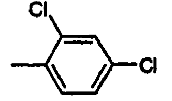 | RMN |
| 120 | CH₃ | 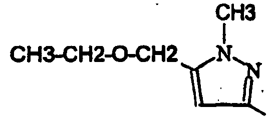 | 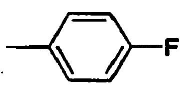 | RMN |
| 121 | CH₃ | 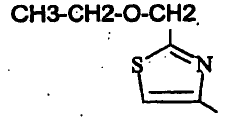 | 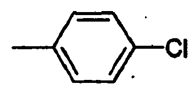 | RMN |
| 122 | CH₃ | 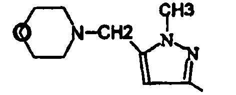 | 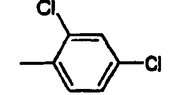 | RMN |
| 123 | CH₃ | 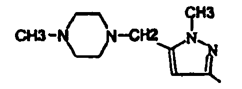 | 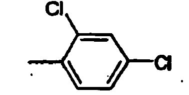 | RMN |
| 124 | CH₃ | 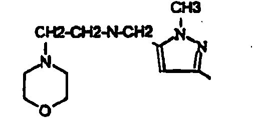 | 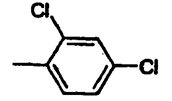 | RMN |
| 125 | CH₃ | 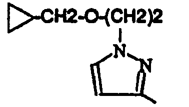 | 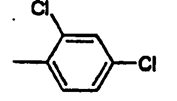 | RMN |
| 126 | CH₃ | 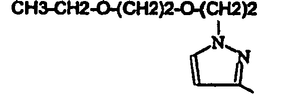 | 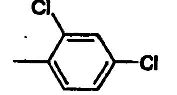 | RMN |
| 127 | CH₃ | 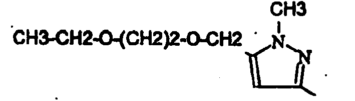 | 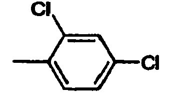 | RMN |
| 128 | CH₃ | 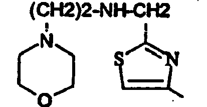 | 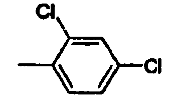 | RMN |
| 129 | CH₃ | 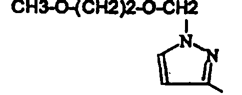 | 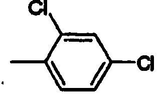 | RMN |
| 130 | CH₃ | 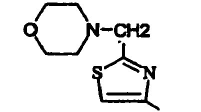 | 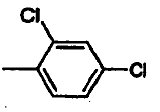 | RMN |
| 131 | CH₃ | 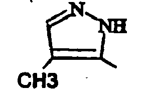 | 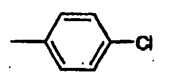 | RMN |
| 132 | CH₃ | 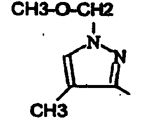 | 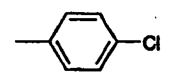 | RMN |
| 133 | CH₃ | 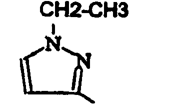 | 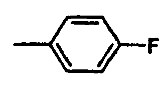 | RMN |
| 134 | CH₃ | 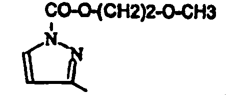 | 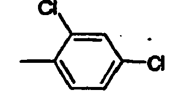 | RMN |
| 135 | CH₃ | 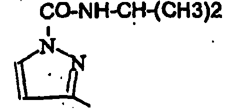 | 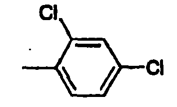 | RMN |
| 136 | CH₃ | 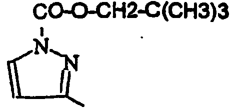 | 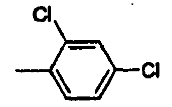 | RMN |
| 137 | CH₃ | 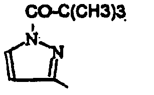 | 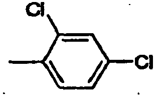 | RMN |
| 138 | CH₃ | 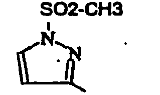 | 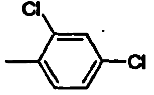 | RMN |
| 139 | CH₃ | 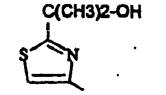 | 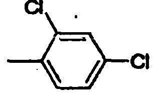 | RMN |
| 140 | CH₃ | 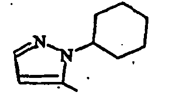 | 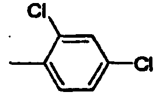 | RMN |
| 141 | CH₃ | 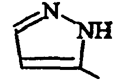 | 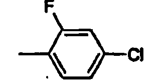 | RMN |
| 142 | CH₃ | 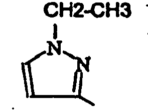 | 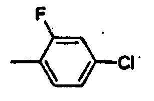 | RMN |
| 143 | CH₃ | 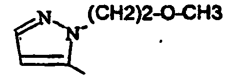 | 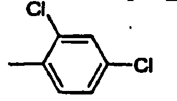 | RMN |
| 144 | CH₃ | 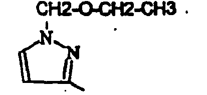 | 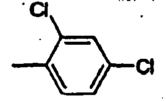 | RMN |
| 145 | CH₃ | 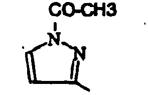 | 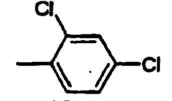 | RMN |
| 146 | CH₃ | 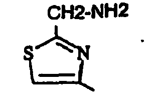 | 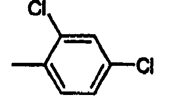 | RMN |
| 147 | CH₃ | 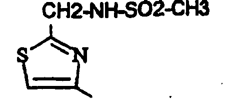 | 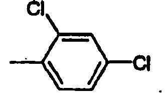 | RMN |
| 148 | CH₃ | 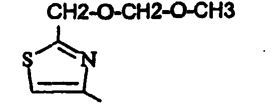 | 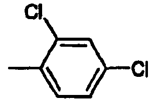 | RMN |
| 149 | CH₃ | 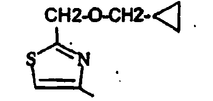 | 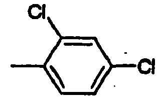 | RMN |
| 150 | CH₃ | 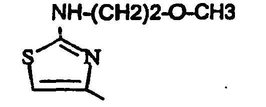 | 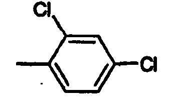 | RMN |
| 151 | CHF2 | 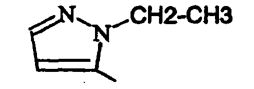 | 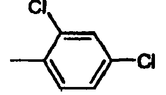 | RMN |
| 152 | CH₃ | 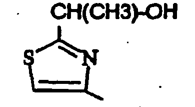 | 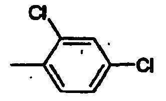 | RMN |
| 153 | CH₃ | 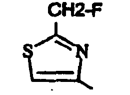 | 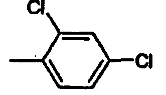 | RMN |
| 154 | CH₃ | 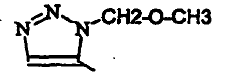 | 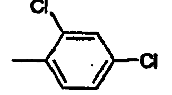 | RMN |
| 155 | CH₃ | 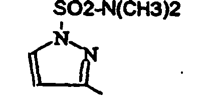 | 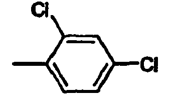 | RMN |
| 156 | CH₃ | 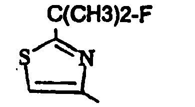 | 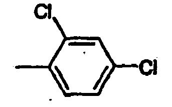 | RMN |
| 157 | CH₃ | 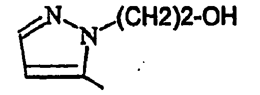 | 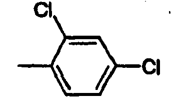 | RMN |
| 158 | CH₃ | 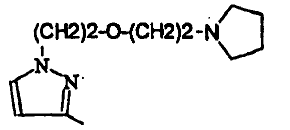 | 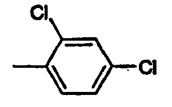 | RMN |
| 159 | CH₃ | 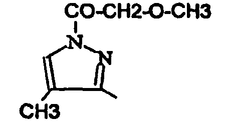 | 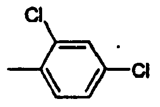 | RMN |
| 160 | CH₃ | 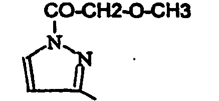 | 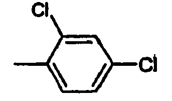 | RMN |
| 161 | CHF2 | 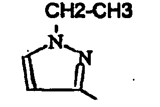 | 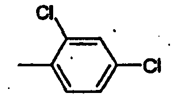 | RMN |
| 162 | CH₃ | 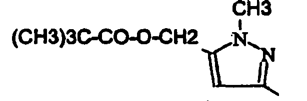 | 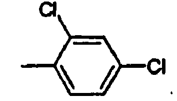 | RMN |
| 163 | CH₃ | 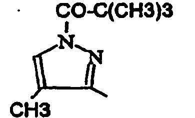 | 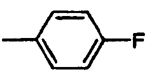 | RMN |
| 164 | CH₃ | 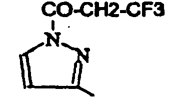 | 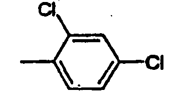 | RMN |

Les composés de formule (I) selon la présente invention ont été testés *in vitro* sur une lignée cellulaire humaine de cancer du sein : la lignée MDA-MB-231 disponible auprès de l'American Type Culture Collection (référence HTB26).

L'évaluation de l'effet antiprolifératif est effectuée selon J.M. Derocq et al., FEBS Letters, 1998, 425, 419-425 : on mesure le taux d'incorporation de la [3H]thymidine dans l'ADN des cellules traitées, après 96 heures d'incubation d'un composé de formule (I). La concentration inhibitrice 50 (CI₅₀) est définie comme la concentration qui inhibe la prolifération cellulaire de 50 %.

Les composés selon l'invention présentent une CI₅₀ généralement inférieure à 10 µM sur la lignée MDA-MB-231.

Les composés de formule (I) ont également été testés sur une autre lignée cellulaire humaine de cancer du sein, dite lignée multi-résistante MDR, (de l'anglais multi-drug-resistant) et appelée MDA-A₁. Cette lignée est décrite par E. Collomb, C. Dussert et P.M. Martin dans Cytometry, 1991, 12(1), 15-25.

Le terme "multi-résistant" qui qualifie cette lignée, signifie que ladite lignée est peu sensible d'une manière générale aux drogues de chimiothérapie communément utilisée et en particulier aux antimitotiques d'origine naturelle tels que le paclitaxel, la vincristine, la vinblastine.

Les composés selon l'invention présentent une CI₅₀ généralement inférieure à 10 µM sur la lignée multi-résistante MDA-A₁.

Ainsi, selon la présente invention, il apparaît que les composés de formule (I) inhibent la prolifération des cellules tumorales y compris celle des cellules présentant une multi-résistance. Il apparaît donc que les composés selon l'invention ont une activité anticancéreuse.

Ainsi selon un autre de ses aspects, l'invention a pour objet des médicaments qui comprennent un composé de formule (I), ou un sel d'addition de ce dernier à un acide pharmaceutiquement acceptable ou encore un hydrate ou un solvat du composé de formule (I).

Ces médicaments trouvent leur emploi en thérapeutique, notamment dans le traitement ou la prévention des maladies causées ou exacerbées par la prolifération des cellules tumorales.

Comme inhibiteur de la prolifération des cellules tumorales, ces composés sont utiles dans le traitement de tous types de néplasmes, de toutes origines, qu'ils soient solides ou non, bénins ou malins, primaires ou métastasiques, carcinomes, sarcomes, adénomes, adénocarcinomes, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage; cancer du foie, cancer de l'estomac, cancer des canaux biliaires, cancer de la vésicule biliaire, cancer du pancréas; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg selon l'âge du sujet à traiter ou le type de traitement : prophylactique ou curatif.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Selon la présente invention, le ou les composés de formule (I) peuvent être administrés en association avec un (ou plusieurs) principe(s) actif(s) anticancéreux, choisis parmi ceux décrits dans la littérature ou connus de l'homme du métier on peut citer en particulier des composés antitumoraux tels que les agents alkylants tels que les alkylsulfonates (busulfan), la dacarbazine, la procarbazine, les moutardes azotées (chlorméthine, melphalan, chlorambucil), cyclophosphamide, ifosfamide; les nitrosourées tels que la carmustine, la lomustine, la sémustine, la streptozocine; les alcaloïdes antinéoplasiques tels que la vincristine, la vinblastine ; les taxanes tel que le paclitaxel ou le taxotère ; les antibiotiques antinéoplasiques tels que l'actinomycine; les agents intercalants, les antimétabolites antinéoplasiques, les antagonistes des folates, le méthotrexate ; les inhibiteurs de la synthèse des purines; les analogues de la purine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside ; le bréquinar ; les inhibiteurs de topoisomérases tels que la camptothécine ou l'étoposide; les agonistes et antagonistes hormonaux anticancéreux incluant le tamoxifène ; les inhibiteurs de kinase, l'imatinib, le sutinib, le sorafenib ; les inhibiteurs de facteurs de croissance ; les antiinflammatoires tels que le pentosane polysulfate, les corticostéroïdes, la prednisone, la dexamethasone, les antracyclines incluant la doxorubicine, la bléomycine, la mitomycine et la méthramycine ; les complexes métalliques anticancéreux, les complexes du platine, le cisplatine, le carboplatine, l'oxaliplatine; l'interféron alpha, le triphénylthiophosphoramide, l'altrétamine ; les agents antiangiogéniques tel que le bévacizumab; les anticorps bloquants les facteurs de croissance ou leur récepteurs tels que le trastuzumab; les inhibiteurs du protéasome tel que le bortézomib ; la thalidomide ; les adjuvants d'immunothérapie; les vaccins.

La présente invention concerne l'utilisation d'un composé de formule (I) pour la préparation d'un médicament destiné au traitement du cancer du sein ; cancer du poumon ; cancer de l'intestin grêle; cancer du colon et du rectum ; cancer des voies respiratoires, de l'oropharynx et de l'hypopharynx ; cancer de l'oesophage; cancer du foie, cancer de l'estomac, cancer des canaux biliaires, cancer de la vésicule biliaire, cancer du pancréas; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de ta thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méninglomes ; tumeurs venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

## Revendications

1. Composé répondant à la formule (I) : 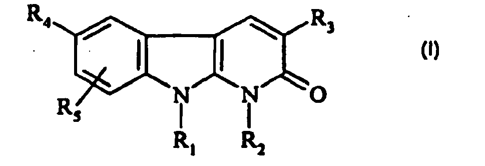 dans laquelle :
• R₁ représente un atome d'hydrogène ou un groupe (C₁-C₄)alkyle, CN, CF₃ ou CHF₂ ;
• R₂ représente un atome d'hydrogène ou un groupe (C₁-C₄)alkyle ;
• R₃ représente un phényle non substitué ou substitué une ou plusieurs fols par des substituants choisis indépendamment parmi un atome d'halogène, un groupe (C₁-C₄) alkyle, un groupe (C₁-C₄)alcoxy ;
• R₄ représente un radical hétérocyclique choisi parmi : 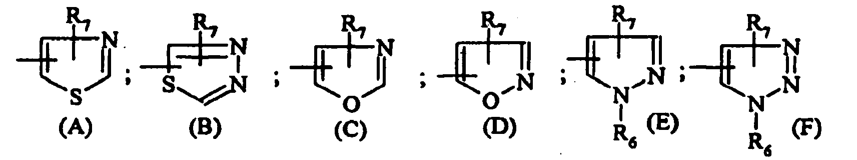
• R₅ représente un atome d'hydrogène, un atome d'halogène, un groupe (C₁-C₄)alkyle, un groupe (C₁-C₄)alcoxy ;
• R₈ est choisi parmi l'atome d'hydrogène,
un groupe -SO₂-R₁₂ ;
un groupe (C₁-C₆)alkyle, (C₁-C₆) mono ou perfluoro alkyle ;
un groupe -(CH₂)ₙ-NR₈R₉ ;
un groupe -(CH₂)ₙ-NH-CO-(C₁₋C₄)alkyle ;
un groupe -(CH₂)ₙ-NH-SO₂-(C₁-C₄)alkyle ;
un groupe -(CH₂)ₙ-NH-(CH₂)ₘ-NR₈R₉ ;
un groupe -(CH₂)ₙ-NH-(CH₂)ₘ-OR₁₀ ;
un groupe -(CH₂)ₙ-O-(CH₂)ₘ-NR₈R₉ ;
un groupe -(CH₂)ₙ-O-(CH₂)ₘ-O-(C₁-C₄)alkyle ;
un groupe -(CH₂)ₙHal ;
un groupe, -(C₁-C₆)alkyle-O-R₁₀ ;
un groupe -CO₂-(CH₂)ₘ-O-R₁₀ ;
un groupe -(CH₂)ₙ-COOR₁₁ ;
un groupe (C₁-C₈)alkylcarbonyle-, (C₁-C₆) mono ou perfluoro alkylcarbonyle-, CO-NH-R₁₀, -CO-(C₁-C₈) alkyle-O-(C₁-C₄)alkyle ;
• R₇ est choisi parmi l'atome d'hydrogène,
un groupe (C₁-C₆)alkyle, (C₁-C₈) mono ou perfluoro alkyle,
un groupe -(CH₂)ₙ-NR₈R₉,
un groupe -(CH₂)ₙ-NH-CO-(C₁-C₄)alkyle,
un groupe -(CH₂)ₙ-NH-SO₂-(C₁-C₄)alkyle,
un groupe-(CH₂)ₙ-NH-(CH₂)ₘ-NR₈R₉,
un groupe -(CH₂)ₙ-NH-(CH₂)ₘOR₁₀,
un groupe -(CH₂)ₙ-O-(CH₂)ₘ NR₈R₉
un groupe -(CH₂)ₙ-O-(CH₂)ₘ-O-(C₁-C₄)alkyle
un groupe -(CH₂)ₙHal,
un groupe, -(C₁-C₆)alkyle-OR₁₀,
un groupe -CO₂-(CH₂)ₘ-OR₁₀,
un groupe -(CH₂)ₙ-COOR₁₁;
un groupe (C₁-C₆)alkylcarbonyle-, (C₁-C₆) mono ou perfluoro alkylcarbonyle-, CO-NH-R₁₀, -CO-(C₁-C₈) alkyle-O-(C₁-C₄)alkyle
• R₈ et R₉ représentent chacun indépendamment l'un et l'autre un atome d'hydrogène ou un groupe (C₁-C₄)alkyle ou bien R₈ et R₉ ensemble avec l'atome d'azote auquel ils sont liés constituent un radical hétérocyclique choisi parmi la pyrrolidin-1-yle, la pipéridin-1-yle, la morpholin-4-yle ou la pipérazinyle éventuellement substituée sur son second atome d'azote ;
• R₁₀ représente un atome d'hydrogène, un groupe (C₁-C₄)alkyle, un groupe (C₁-C₄)alkylcarbonyle-, un groupe (C₃-C₆)cycloalkylcarbonyle-, un groupe (C₃-C₈) cycloalkyl(C₁-C₄)alkylcarbonyle-, un groupe (C₃-C₈)cycloalkyle, un groupe (C₅-C₆)hétérocycloalkyle, un groupe (C₁-C₆) mono ou perfluoro alkyle, un groupe (C₃-C₈)cycloalkyle (C₁-C₄)alkyle- ;
• R₁₁ représente un atome d'hydrogène ou un groupe (C₁-C₆)alkyle ;
• R₁₂ représente un groupe (C₁-C₆)alkyle, un groupe (C₁-C₆) mono ou perfluoro alkyle, un groupe cycloalkyle, un groupe cycloalkylalkyle-, un groupe (C₁-C₆) alkyle-O-(C₁-C₄) alkyle- ;
• m est 1, 2 ;
• n est 0, 1, 2 ;
• Hal représente un atome d'halogène;
à l'état de base ou de sel d'addition à un acide, ainsi qu'à l'état d'hydrate ou de solvat.

2. Composé répondant à la formule (I) selon la revendication 1 dans laquelle :
• R₁ représente un atome d'hydrogène ou un groupe (C₁-C₄)alkyle ;
• R₂ représente un atome d'hydrogène ou un groupe (C₁-C₄)alkyle ;
• R₃ représente un phényle non substitué ou substitué une ou plusieurs fois par des substituants choisis indépendamment parmi un atome d'halogène, un groupe (C₁-C₄) alkyle, un groupe (C₁-C₄)alcoxy ;
• R₄ représente un radical hétérocyclique choisi parmi: 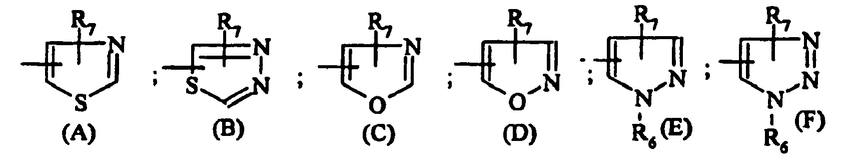
• R₅ représente un atome d'hydrogène, un atome d'halogène, un groupe (C₁-C₄) alkyle, un groupe (C₁-C₄)alcoxy ;
• R₆ représente un atome d'hydrogène ou un groupe (C₁-C₄)alkyle ;
• R₇ représente un atome d'hydrogène, un groupe (C₁-C₄)alkyle, un groupe -(CH₂)ₙ-NR₈R₉, un groupe -(CH₂)ₙHal, un groupe -CH₂-OR₁₀, un groupe -(CH₂)ₙ-COOR₁₁ ;
• R₈ et R₉ représentent chacun indépendamment l'un et l'autre un atome d'hydrogène ou un groupe (C₁-C₄)alkyle ou bien R₈ et R₉ ensemble avec l'atome d'azote auquel ils sont liés constituent un radical hétérocyclique choisi parmi la pyrrolidin-1-yle, la pipéridin-1-yle ou la morpholin-4-yle ;
• R₁₀ représente un atome d'hydrogène, un groupe (C₁-C₄)alkyle, un groupe (C₁-C₄)alkylcàrbonyle, un groupe (C₃-C₆)cycloalkylcarbonyle ;
• R₁₁ représente un atome d'hydrogène ou un groupe (C₁-C₄)alkyle;
• n est 0 ou 1 ;
• Hal représente un atome d'halogène ;
à l'état de base ou de sel d'addition à un acide, ainsi qu'à l'état d'hydrate ou de solvat.

3. Composé de formule (IA) selon la revendication 1 ou 2 dans laquelle R₄ représente un 1,3-thiazole substitué par R₇ et les substituants R₁ à R₇ sont tels que définis à la revendication 1, à l'état de base ou de sel d'addition à un acide, ainsi qu'à l'état d'hydrate ou de solvat.

4. Composé de formule (IB) selon la revendication 1 ou 2 dans laquelle R₄ représente un 1,3,4-thiadiazole substitué par R₇ et les substituants R₁ à R₇ sont tels que définis à la revendication 1, à l'état de base ou de sel d'addition à un acide, ainsi qu'à l'état d'hydrate ou de solvat.

5. Composé de formule (IC) selon la revendication 1 ou 2 dans laquelle R₄ représente un 1,3-oxazole substitué par R₇ et les substituants R₁ à R₇ sont tels que définis à la
revendication 1, à l'état de base ou de sel d'addition à un acide, ainsi qu'à l'état d'hydrate ou de solvat.

6. Composé de formule (ID) selon la revendication 1 ou 2 dans laquelle R₄ représente un isoxazole substitué par R₇ et les substituants R₁ à R₇ sont tels que définis à la revendication 1, à l'état de base ou de sel d'addition à un acide, ainsi qu'à l'état d'hydrate ou de solvat.

7. Composé de formule (IE) selon la revendication 1 ou 2 dans laquelle R₄ représente un 1*H*-pyrazole substitué par R₆ et R₇ et les substituant R₁ à R₇ sont tels que définis à la revendication 1, à l'état de base ou de sel d'addition à un acide, ainsi qu'à l'étant d'hydrate ou de solvat.

8. Composé de formule (IF) selon la revendication 1 ou 2 dans laquelle R₄ représente un 1,2,3-triazole substitué par R₆ et R₇ et les substituants R₁ à R₇ sont tels que définis à la revendication 1, à l'état de base ou de sel d'addition à un acide, ainsi qu'à l'état d'hydrate ou de solvat.

9. Composé de formule (I) selon la revendication 1 ou 2 dans laquelle :
• R₁ représente un atome d'hydrogène ou un méthyle ;
• R₂ représente un méthyle ;
• R₃ représente un 2,4-dichlorophényle ;
• R₄ représente un radical hétérocyclique choisi parmi : 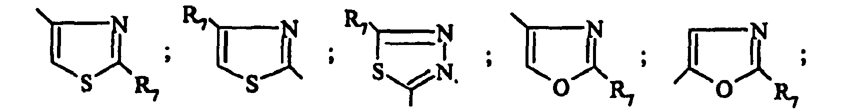 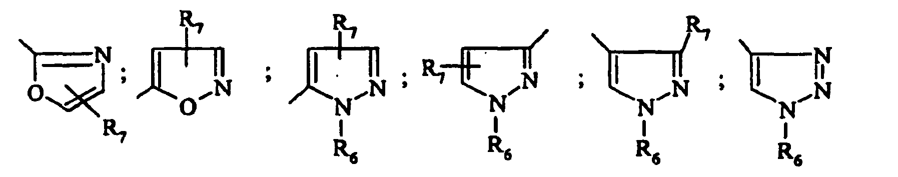
• R₅ représente un atome d'hydrogène ;
• R₆ représente un atome d'hydrogène, un méthyle ou un éthyle;
• R₇ représente un atome d'hydrogène, un méthyle, un amino, un méthylamino, un aminométhyle, un (diméthylamino)méthyle, un pyrrolidin-1-ylméthyle, un chlorométhyle, un hydroxyméthyle, un éthoxyméthyle, un [(2,2-diméthylpropanoyl)oxy]méthyle, un [(cyclopropylcarbonyl)oxy]méthyle, un méthoxycarbonyle, un 2-méthoxy-2-oxo éthyle, un carboxyméthyle;
à l'état de base ou de sel d'addition à un acide, ainsi qu'à l'état d'hydrate ou de solvat.

10. Composé de formule (I) selon la revendication 1 ou 2 dans laquelle :
• R₁ représente un atome d'hydrogène ou un méthyle ;
• R₂ représente un méthyle ;
• R₃ représente un 2,4-dichlorophényle ;
• R₄ représente :
. un 2-méthyl-1,3-thiazol-4-yle, un 2-amino-1,3-thiazol-4-yle, un 2-(méthylamino)-1,3-thiazol-4-yle, un 2-(hydroxyméthyl)-1,3-thiazol-4-yle, un 2-(éthoxyméthyl)-1,3-thiazol-4-yle, un 2-[[(2.2-diméthylpropanoyl)oxy]méthyl)-1,3-thiazol-4-yle, un 2-[(cyclopropylcarbonyl)oxy]méthyle ;
. un 1,3-thiazol-2-yle, un 4-méthyl-1,3-thiazol-2-yle, un 4-amino-1,3-thiazol-2-yle, un 4-(aminométhyl)-1,3-thiazol-2-yle, un 4-[(diméthylamino)méthyl]-1,3-thiazol-2-yle, un 4-(pyrrolidin-1-ylméthyl)-1,3-thiazol-2-yle, un 4-(chlorométhyl)-1,3-thiazol-2-yle, un 4-(2-méthoxy-2-oxoéthyl)-1,3-thiazol-2-yle, un 4-(carboxyméthyl)-1,3-thiazol-2-yle;
. un 1,3-oxazol-4-yle, un 2-méthyl-1.3-oxazol-4-yle, un 2-amino-1,3-oxazol-4-yle, un 2-(hydroxyméthyl)-1,3-oxazol-4-yle, un 2-(éthoxyméthyl)-1,3-oxazol-4-yle, un 2-[[(2,2-diméthylpropanoyl)oxy]méthyl]-1,3-oxazol-4-yle;
. un 1,3-oxazol-5-yle, un 2-méthyl-1,3-oxazol-5-yle, un 2-(éthoxyméthyl)-1,3-oxazol-5yle;
. un 1,3-oxazol-2-yle, un 4-méthyl-1,3-oxazol-2-yle, un 5-méthyl-1,3-oxazol-2-yle;
. un isoxazol-5-yle, un 4-méthylisoxazol-5-yle, un 3-(méthoxycarbonyl)isoxazol-5-yle;
. un 1*H*-pyrazol-5-yle, un 1-éthyl-1*H*-pyrazol-5-yle, un 3-(méthoxycarbonyl)-1*H*-pyrazol-5-yle, un 3-méthyl-1*H*-pyrazol-5-yle, un 3-amino-1*H*-pyrazol-5-yle ;
. un 1-méthyl-1*H*-pyrazol-3-yle, un 1-éthyl-1H-pyrazol-3-yle;
. un 3-amino-1*H*-pyrazol-4-yle ;
. un 5-amino-1,3,4-thiadiazol-2-yle ;
. un 1*H*-1,2,3-triazol-4-yle ;
- R₅ représente un atome d'hydrogène;
à l'état de base ou de sel d'addition à un acide, ainsi qu'à l'état d'hydrate ou de solvat.

11. Composé choisi parmi :
• 6-(2-Amino-1,3-thiazol-4-yl)-3-(2,4-dichlorophényl)-1-méthyl-1,9-dihydro-2H-pyrido[2,3-b]indol-2-one ;
• 3-(2,4-Dichlorophényl)-6-[2-(hydroxyméthyl)-1,3-thiazol-4-yl]-1-méthyl-1,9-dihydro-2H-pyrido[2,3-b]indol-2-one ;
• 3-(2,4-Dichlorophényl)-1,9-diméthyl-6-(2-méthyl-1,3-thiazol-4-yl)-1,9-dihydro-2H-pyrido[2,3-b]indol-2-one ;
• Pivalate de [4-[3-(2,4-dichlorophényl)-1,9-diméthyl-2-oxo-2,9-dihydro-1H-pyrido[2,3-b]indol-6-yl]-1,3-thiazol-2-yl]méthyle ;
• 3-(2,4-Dichlorophényl)-6-[2-(éthoxyméthyl)-1,3-thiazol-4-yl]-1,9-diméthyl-1,9-dihydro-2H-pyrido[2,3-b]indol-2-one ;
• 3-(2,4-Dichlorophényl)-1,9-diméthyl-6-(2-méthyl-1,3-oxazol-4-yl)-1,9-dihydro-2H-pyrido[2,3-b]indol-2-one ;
• 3-(2,4-Dichlorophényl)-1,9-diméthyl-6-(2-méthyl-1,3-oxazol-5-yl)-1,9-dihydro-2H-pyrido[2,3-b]indol-2-one ;
• 6-(3-Amino-1H-pyrazol-5-yl)-3-(2,4-dichlorophényl)-1,9-diméthyl-1,9-dihydro-2H-pyrido[2,3-b]indol-2-one ;
• 6-[4-(Aminométhyl)-1,3-thiazol-2-yl]-3-(2,4-dichlorophényl)-1,9-diméthyl-1,9-dihydro-2H-pyrido[2,3-b]indol-2-one ;
• 6-(3-Amino-1H-pyrazol-4-yl)-3-(2,4-dichlorophényl)-1,9-diméthyl-1,9-dihydro-2H-pyrido[2,3-b]indol-2-one ;
• 3-(2,4-Dichlorophenyl)-1,9-diméthyl-6-(1H-pyrazol-5-yl)-1,9-dihydro-2H-pyrido[2,3-b]indol-2-one;
• 3-(2,4-Dichloro-phenyl)-6-(1-methoxymethyl-1H-pyrazol-3-yl)-1,9-dimethyl-1,9-dihydro-pyrido[2,3-b]indol-2-one ;
• 3-(4-bromo-phenyl)-6-(1-ethyl-1H-pyrazol-3-yl)-1,9-dimethyl-1,9-dihydro-pyrido[2,3-b]indol-2-one ;
• 6-(2-Amino-thiazol-5-yl)-3-(2-chloro-4-fluoro-phenyl)-1,9-dimethyl-1,9-dihydropyrido[2,3-b]indol-2-one ;
• 3-(2,4-Dichloro-phenyl)-6-(2-methoxymethyl-thiazol-4-yl)-1,9-dimethyl-1,9-dihydropyrido[2,3-b]indol-2-one ;
• 3-(2,4-Dichloro-phenyl)-6-[1-(2,2-dimethyl-propionyl)-4-methyl-1H-pyrazol-3-yl]-1,9-dimethyl-1,9-dihydro-pyrido[2,3-b]indol-2-one ;
• 3-(2,4-Dichloro-phenyl)-6-(5-ethoxymethyl-1-methyl-1H-pyrazol-3-yl)-1,9-dimethyl-1,9-dihydro-pyrido[2,3-b]indol-2-one ;
• 3-(4-fluoro-phenyl)-6-(1-methoxymethyl-1H-pyrazol-3-yl)-1,9-dimethyl-1,9-dihydropyrido[2,3-b]indol-2-one ;
• 3-(4-Fluoro-phenyl)-6-(1-methoxymethyl-4-methyl-1H-pyrazol-3-yl)-1,9-dimethyl-1,9-dihydro-pyrido[2,3-b]indol-2-one ;
• 3-(4-Chloro-phenyl)-6-(2-ethoxymethyl-thiazol-4-yl)-1,9-dimethyl-1,9-dihydropyrido[2,3-b]inndol-2-one ;
• 3-(2,4-Dichloro-phenyl)-6-[1-(2,2-dimethyl-propionyl)-1H-pyrazol-3-yl]-1,9-dimethyl-1,9-dihydro-pyrido[2,3-b]indol-2-one ;
• 3-(2,4-Dichloro-phenyl)-6-(1-methoxymethyl-1H-[1,2,3]triazol-4-yl)-1,9-dimethyl-1,9-dihydro-pyrido[2,3-b]indol-2-one
à l'état de base ou de sel d'addition à un acide, ainsi qu'à l'état d'hydrate ou de solvat.

12. Composé de formule : 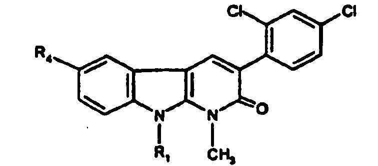 dans laquelle :
• R₁ représente H et R₄ est choisi parmi : 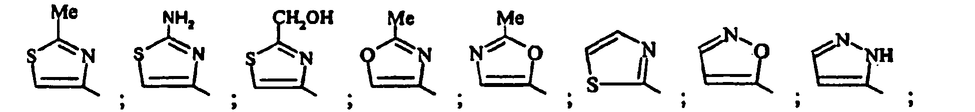 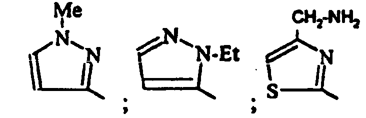 ou
• R₁ représente Me et R₄ est choisi parmi : 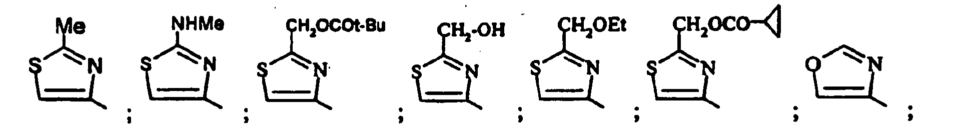 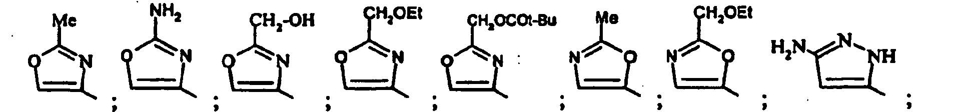 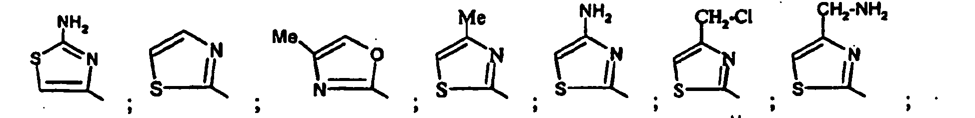 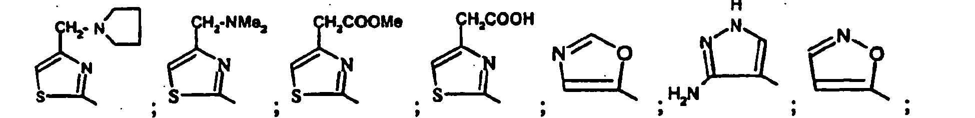 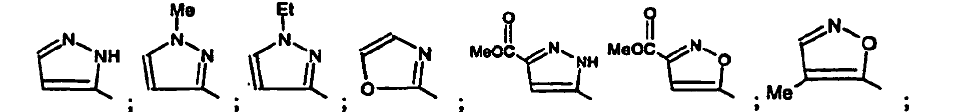 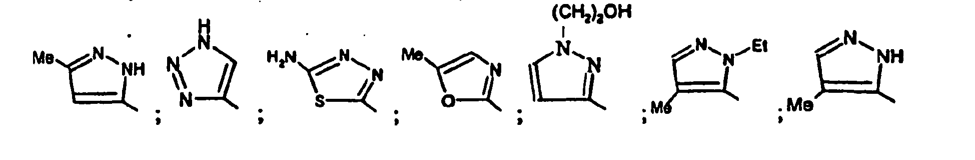 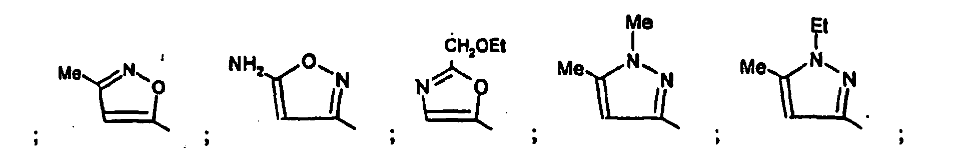 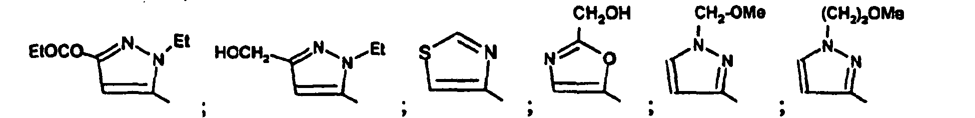 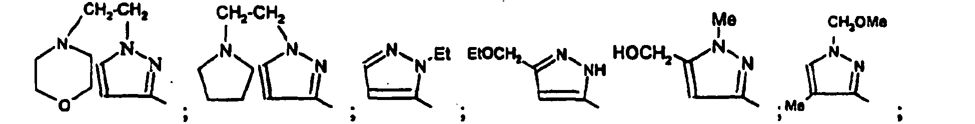 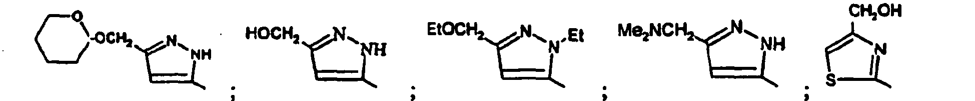 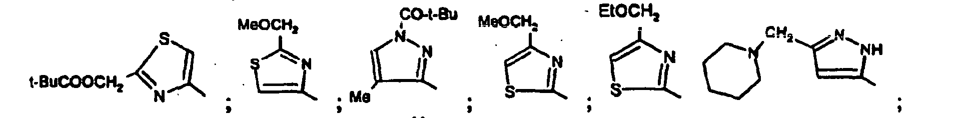 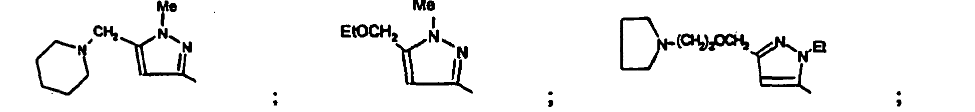 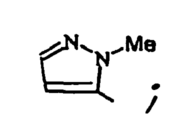 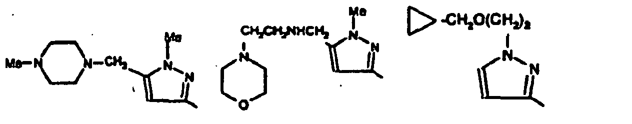 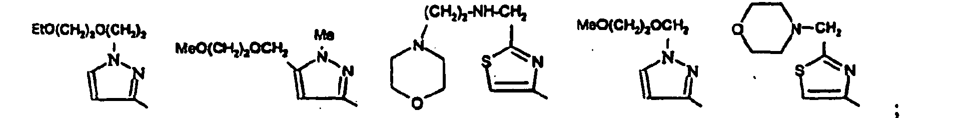 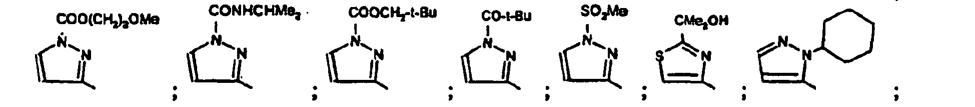 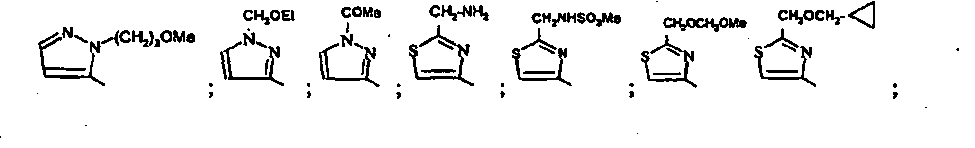 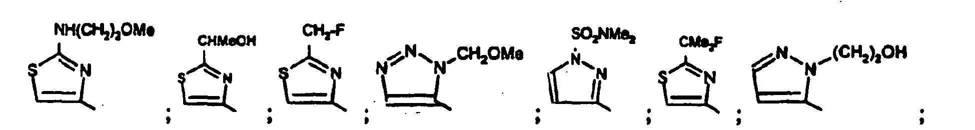 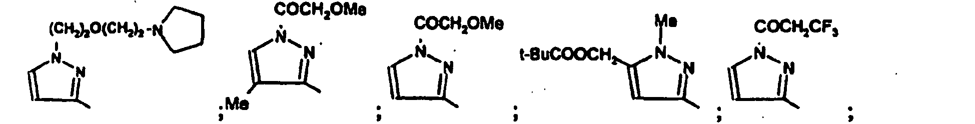
les abrévations suivantes étant utilisées : Me : méthyl ; Et : éthyl et t-Bu : tertio-butyl.

13. Composé selon la revendication 12 à l'état de base ou de sel d'addition à un acide, ainsi qu'à l'état d'hydrate ou de solvat.

14. Procédé de préparation des composés de formule (IA) selon la revendication 1 dans laquelle : 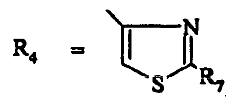 **caractérisé en ce que :**
on fait réagir un composé de formule : 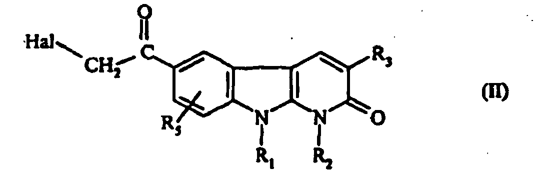 dans laquelle R₁, R₂, R₃ et R₅ sont tels que définis à la revendication 1 et Hal représente un atome d'halogène avec un composé de formule : 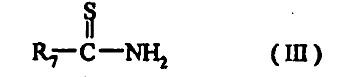 dans laquelle R₇ est tel que défini à la revendication 1.

15. Procédé de préparation des composés de formule (IA) selon la revendication 1 dans laquelle : 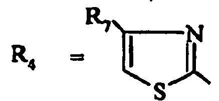 **caractérisé en ce que** :
on fait réagir un composé de formule : 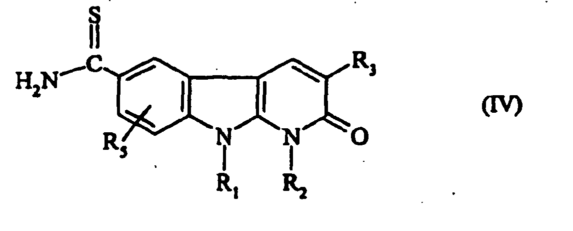 dans laquelle R₁, R₂, R₃ et R₅ sont tels que définis à la revendication 1 avec un composé de formule : 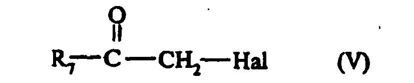 dans laquelle R₇ est tel que défini à la revendication 1 et Hal représente un atome d'halogène.

16. Procédé de préparation des composés de formule (IB) selon la revendication 1 dans laquelle : 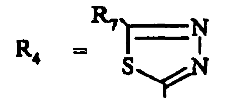 **caractérisé en ce que :**
on fait réagir un composé de formule : 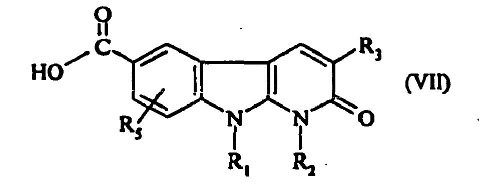 dans laquelle R₁, R₂, R₃ et R₅ sont tels que définis à la revendication 1, avec un composé de formule: 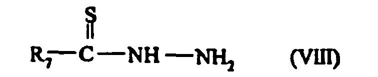 dans laquelle R₇ est tel que défini à la revendication 1.

17. Procédé de préparation des composés de formule (IC) selon la revendication 1 dans laquelle : 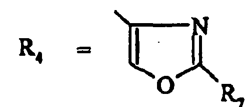 **caractérisé en ce que :**
on fait réagir un composé de formule : 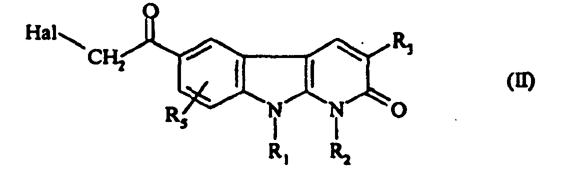 dans laquelle R₁, R₂, R₃ et R₅ sont tels que définis à la revendication 1, et Hal représente un atome d'halogène, avec un composé de formule : 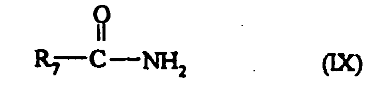 dans laquelle R₇ est tel que défini à la revendication 1.

18. Procédé de préparation des composés de formule (IC) selon la revendication 1 dans laquelle : 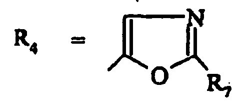 **caractérisé en ce que :**
A) on fait réagir un composé de formule : 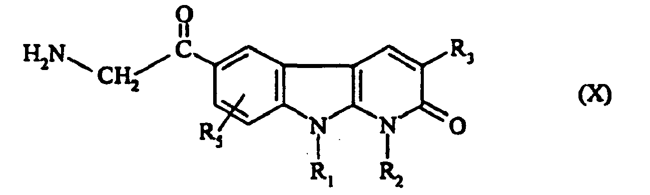 dans laquelle R₁, R₂, R₃ et R₅ sont tels que définis à la revendication 1, avec un dérivé fonctionnel d'un acide de formule : 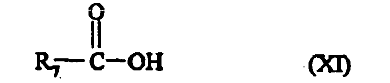 dans laquelle R₇ est tel que défini à la revendication 1 pour obtenir un composé de formule : 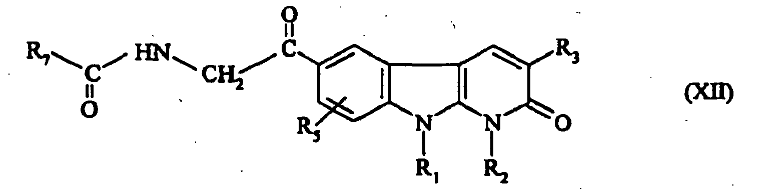
B) on cyclise le composé de formule (XII) ainsi obtenu par action d'un acide.

19. Procédé de préparation des composés de formule (IC) selon la revendication 1 dans laquelle : 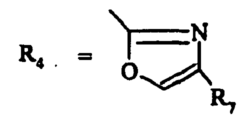 **caractérisé en ce que :**
on fait réagir un composé de formule : 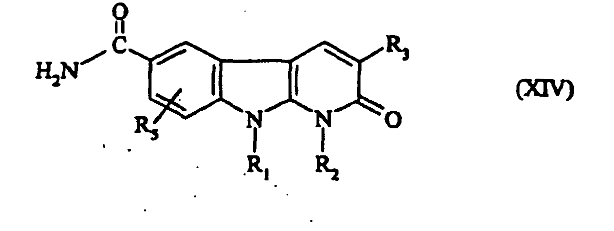 dans laquelle R₁, R₂, R₃ et R₅ sont tels que définis à la revendication 1, avec un composé de formule : 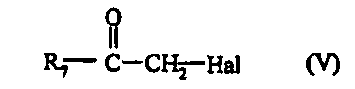 dans laquelle R₇ est tel que défini à la revendication 1 et Hal représente un atome d'halogène.

20. Procédé de préparation des composés de formule (IC) selon la revendication 1 dans laquelle : 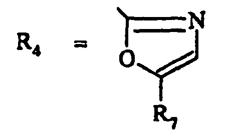 **caractérisé en ce que :**
on fait réagir un composé de formule : 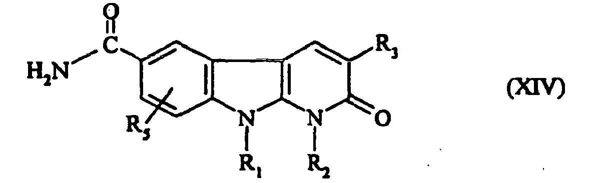 dans laquelle R₁, R₂, R₃ et R₅ sont tels que définis à la revendication 1, avec un composé de formule : 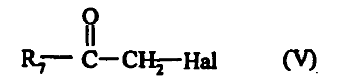 dans laquelle R₇ est tel que défini à la revendication 1 et Hal représente un atome d'halogène.

21. Procédé de préparation des composés de formule (ID) selon la revendication 1 dans laquelle : 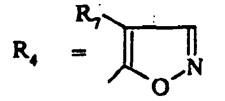 **caractérisé en ce que :**
on fait réagir un composé de formule : 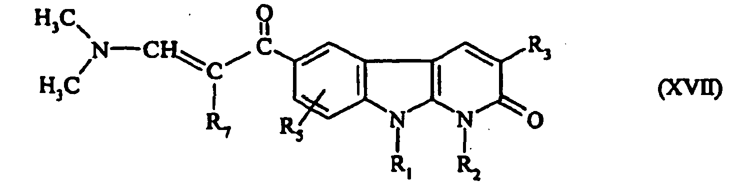 dans laquelle R₁, R₂, R₃, R₅ et R₇ sont tels que définis à la revendication 1, avec l'hydroxylamine.

22. Procédé de préparation des composés de formule (ID) selon la revendication 1 dans laquelle 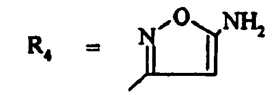 **caractérisé en ce que** l'on fait réagir un composé de formule (XX) : 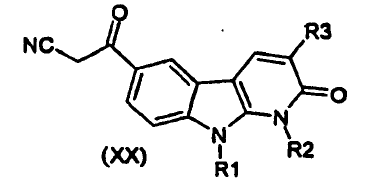 dans laquelle R₁, R₂ et R₃ sont tels que définis à la revendication 1, avec l'hydroxylamine.

23. Procédé de préparation des composés de formule (ID) selon la revendication 1 dans laquelle : 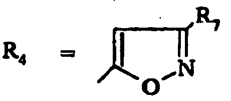 **caractérisé en ce que :**
on fait réagir un composé de formule : 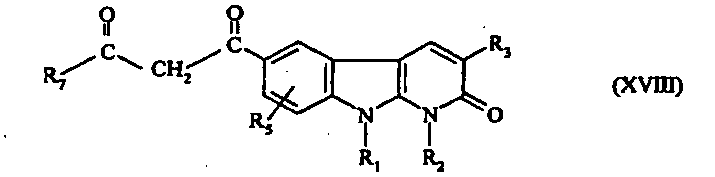 dans laquelle R₁, R₂, R₃, R₅ et R₇ sont tels que définis à la revendication 1, avec l'hydroxylamine.

24. Procédé de préparation des composés de formule (IE) selon la revendication 1 dans laquelle : 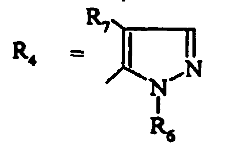 **caractérisé en ce que** :
on fait réagir un composé de formule : 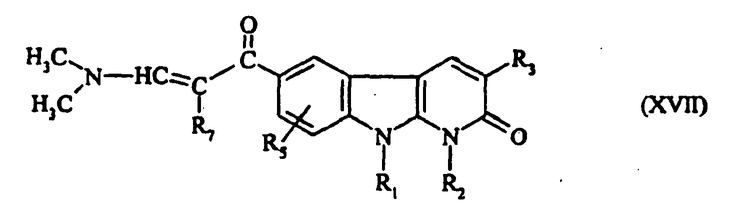 dans laquelle R₁, R₂, R₃, R₅ et R₇ sont tels que définis à la revendication 1, avec un composé de formule :
NH₂-NH-R₆ (XIX)
dans laquelle R₆ est tel que défini à la revendication 1.

25. Procédé de préparation des composés de formule (IE) selon la revendication 1 dans laquelle : 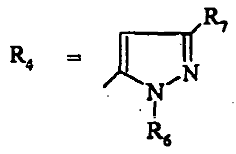 **caractérisé en ce que :**
on fait réagir un composé de formule : 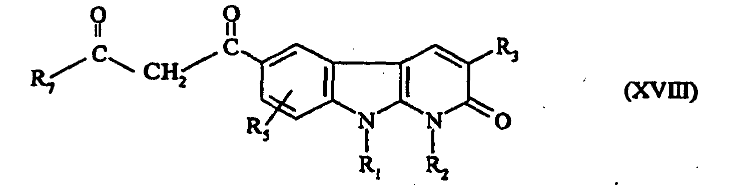 dans laquelle R₁, R₂, R₃, R₅ et R₇ sont tels que définis à la revendication 1, avec une hydrazine de formule :
NH₂-NH-R₆ (XIX)

26. Procédé de préparation des composés de formule (IF) selon la revendication 1 dans laquelle 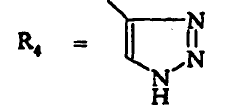 **caractérisé en ce que :**
on fait réagir un composé de formule (XXII): 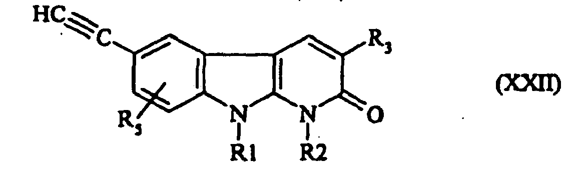 dans laquelle R₁, R₂, R₃ et R₅ sont tels que définis pour un composé de formule (I) à la revendication 1, avec l'azidure de sodium.

27. Médicament, **caractérisé en ce qu'**il comprend un composé de formule (I) selon l'une quelconque des revendications 1 à 13, ou un sel d'addition de ce composé à un acide pharmaceutiquement acceptable, ou encore un hydrate ou un solvat du composé de formule (I).

28. Composition pharmaceutique, **caractérisée en ce qu'**elle comprend un composé de formule (I) selon l'une quelconque des revendications 1 à 13, ou un sel pharmaceutiquement acceptable, un hydrate ou un solvat de ce composé, ainsi qu'au moins un excipient pharmaceutiquement acceptable.

29. Utilisation d'un composé de formule (I) selon l'une quelconque des revendications 1 à 13, pour la préparation d'un médicament destiné au traitement et à la prévention de maladies causées ou exacerbées par la prolifération des cellules tumorales.

30. Utilisation d'un composé de formule (I) selon l'une quelconque des revendications 1 à 13, pour la préparation d'un médicament destiné au traitement du cancer du sein ; cancer du poumon ; cancer de l'intestin grêle, cancer du colon et du rectum ; cancer des voies respiratoires, de l'oropharynx et de l'hypopharynx ; cancer de l'oesophage; cancer du foie, cancer de l'estomac, cancer des canaux biliaires, cancer de la vésicule biliaire, cancer du pancréas; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

## Claims

1. Compound corresponding to formula (I): 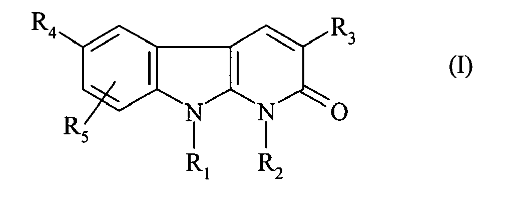 in which:
• R₁ is a hydrogen atom or a (C₁-C₄)alkyl, CN, CF₃ or CHF₂ group;
• R₂ is a hydrogen atom or a (C₁-C₄)alkyl group;
• R₃ is a phenyl which is unsubstituted or substituted one or more times which substituents selected independently from a halogen atom, a (C₁-C₄)alkyl group and a (C₁-C₄)alkoxy group;
• R₄ is a heterocyclic radical selected from: 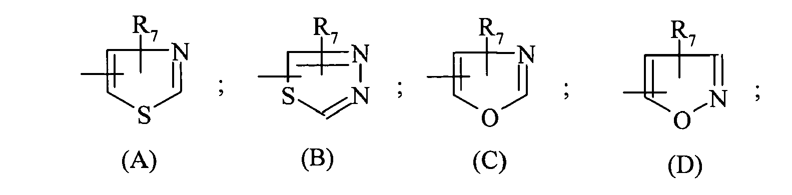 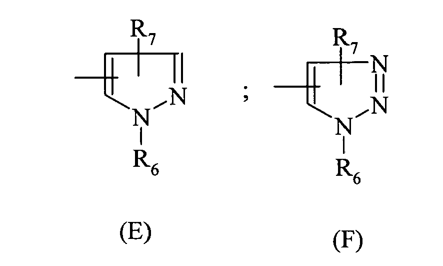
• R₅ is a hydrogen atom, a halogen atom, a (C₁-C₄)alkyl group or a (C₁-C₄) alkoxy group;
• R₆ is selected from a hydrogen atom,
a -SO₂-R₁₂ group,
a (C₁-C₆)alkyl or (C₁-C₆) mono or perfluoro alkyl group,
a -(CH₂)ₙ-NR₈R₉,
a -(CH₂)ₙ-NH-CO-(C₁-C₄)alkyl group,
a -(CH₂)ₙ-NH-SO₂-(C₁-C₄)alkyl group,
a -(CH₂)ₙ-NH-(CH₂)ₘ-NR₈R₉ group,
a -(CH₂)ₙ -NH-(CH₂)ₘ-OR₁₀ group,
a -(CH₂)ₙ-O-(CH₂)ₘ -NR₈R₉ group,
a -(CH₂)ₙ-O-(CH₂)ₘ-O-(C₁-C₄)alkyl group,
a -(CH₂)ₙHal group,
a -(C₁-C₆)alkyl-O-R₁₀ group,
a -CO₂-(CH₂)ₘ-O-R₁₀ group,
a -(CH₂)ₙ-COOR₁₁ group,
a (C₁-C₆)alkylcarbonyl-, (C₁-C₆) mono or perfluoro alkylcarbonyl-, -CO-NH-R₁₀, or -CO-(C₁-C₆)alkyl-O-(C₁-C₄)alkyl group;
• R₇ is selected from a hydrogen atom,
a (C₁-C₆)alkyl, or (C₁-C₆) mono or perfluoro alkyl group,
a -(CH₂)ₙ-NR₈R₉ group,
a -(CH₂)ₙ-NH-CO-(C₁-C₄)alkyl group,
a -(CH₂)ₙ-NH-SO₂-(C₁-C₄)alkyl group,
a -(CH₂)ₙ-NH-(CH₂)ₘ-NR₈R₉ group,
a -(CH₂)ₙ-NH-(CH₂)ₘ-OR₁₀ group,
a -(CH₂)ₙ-O-(CH₂)ₘ-NR₈R₉ group,
a -(CH₂)ₙ-O-(CH₂)ₘ-O-(C₁-C₄) alkyl group, a -(CH₂)ₙHal group,
a -(C₁-C₆)alkyl-O-R₁₀ group,
a -CO₂-(CH₂)ₘ-O-R₁₀ group,
a -(CH₂)ₙ-COOR₁₁ group,
a (C₁-C₆)alkylcarbonyl-, (C₁-C₆) mono or perfluoro alkylcarbonyl-, -CO-NH-R₁₀, or -CO-(C₁-C₆)alkyl-O-(C₁-C₄)alkyl group;
• R₈ and R₉ are each, independently of one another, a hydrogen atom or a (C₁-C₄)alkyl group or else R₈ and R₉, together with the nitrogen atom to which they are attached, constitute a heterocyclic radical selected from pyrrolidin-1-yl, piperidin-1-yl, morpholin-4-yl or piperazinyl optionally substituted on its second nitrogen atom;
• R₁₀ is a hydrogen atom, a (C₁-C₄)alkyl group, a (C₁-C₄)alkylcarbonyl- group, a (C₃-C₆)cycloalkylcarbonyl- group, a (C₃-C₆)cycloalkyl(C₁-C₄)alkylcarbonyl- group, a (C₃-C₆)cycloalkyl group, a (C₅-C₆)heterocycloalkyl group, a (C₁-C₆) mono or perfluoro alkyl group, or a (C₃-C₆)cycloalkyl(C₁-C₄)alkyl group;
• R₁₁ is a hydrogen atom or a (C₁-C₆)alkyl group;
• R₁₂ is a (C₁-C₆)alkyl group, a (C₁-C₆) mono ou perfluoro alkyl group, a cycloalkyl group, a cycloalkylalkyl- group, or a (C₁-C₆)alkyl-O-(C₁-C₄)alkyl- group;
• m is 1 or 2
• n is 0, 1 or 2;
• Hal is a halogen atom;
in the form of a base or of an addition salt with an acid, and also in the form of a hydrate or of a solvate.

2. Compound corresponding to formula (I) according to Claim 1, in which:
• R₁ is a hydrogen atom or a (C₁-C₄) alkyl group;
• R₂ is a hydrogen atom or a (C₁-C₄) alkyl group;
• R₃ is a phenyl which is unsubstituted or substituted one or more times with substituents selected independently from a halogen atom, a (C₁-C₄) alkyl group and a (C₁-C₄) alkoxy group;
• R₄ is a heterocyclic radical selected from: 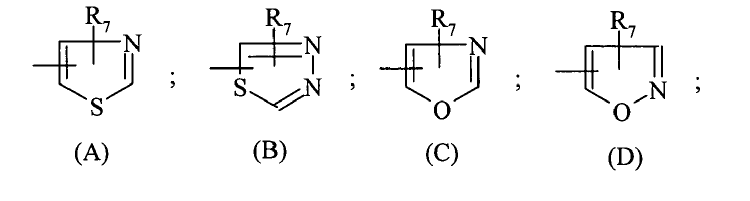 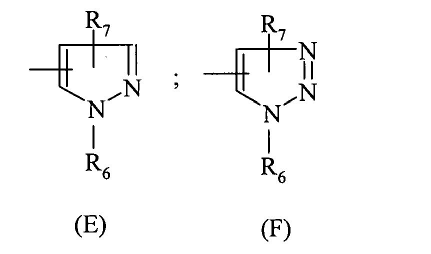
• R₅ is a hydrogen atom, a halogen atom, a (C₁-C₄) alkyl group or a (C₁-C₄) alkoxy group;
• R₆ is a hydrogen atom or a (C₁-C₄) alkyl group;
• R₇ is a hydrogen atom, a (C₁-C₄) alkyl group, a -(CH₂)ₙ-NR₈R₉ group, a -(CH₂)ₙHal group, a -CH₂-OR₁₀ group or a -(CH₂)ₙ-COOR₁₁ group;
• R₈ and R₉ are each, independently of one another, a hydrogen atom or a (C₁-C₄) alkyl group or else R₈ and R₉, together with the nitrogen atom to which they are attached, constitute a heterocyclic radical selected from pyrrolidin-1-yl, piperidin-1-yl or morpholin-4-yl;
• R₁₀ is a hydrogen atom, a (C₁-C₄) alkyl group, a (C₁-C₄) alkylcarbonyl group or a (C₃-C₆) cycloalkylcarbonyl group;
• R₁₁ is a hydrogen atom or a (C₁-C₄) alkyl group;
• n is 0 or 1;
• Hal is a halogen atom;
in the form of a base or of an addition salt with an acid, and also in the form of a hydrate or of a solvate.

3. Compound of formula (IA) according to Claim 1 or 2, in which R₄ is a 1,3-thiazole substituted with R₇ and the substituents R₁ to R₇ are as defined in Claim 1, in the form of a base or of an addition salt with an acid, and also in the form of a hydrate or of a solvate.

4. Compound of formula (IB) according to Claim 1 or 2, in which R₄ is a 1,3,4-thiadiazole substituted with R₇ and the substituents R₁ to R₇ are as defined in Claim 1, in the form of a base or of an addition salt with an acid, and also in the form of a hydrate or of a solvate.

5. Compound of formula (IC) according to Claim 1 or 2, in which R₄ is a 1,3-oxazole substituted with R₇ and the substituents R₁ to R₇ are as defined in Claim 1, in the form of a base or of an addition salt with an acid, and also in the form of a hydrate or of a solvate.

6. Compound of formula (ID) according to Claim 1 or 2, in which R₄ is an isoxazole substituted with R₇ and the substituents R₁ to R₇ are as defined in Claim 1, in the form of a base or of an addition salt with an acid, and also in the form of a hydrate or of a solvate.

7. Compound of formula (IE) according to Claim 1 or 2, in which R₄ is a 1*H*-pyrazole substituted with R₆ and R₇ and the substituents R₁ to R₇ are as defined in Claim 1, in the form of a base or of an addition salt with an acid, and also in the form of a hydrate or of a solvate.

8. Compound of formula (IF) according to Claim 1 or 2, in which R₄ is a 1,2,3-triazole substituted with R₆ and R₇ and the substituents R₁ to R₇ are as defined in Claim 1, in the form of a base or of an addition salt with an acid, and also in the form of a hydrate or of a solvate.

9. Compound of formula (I) according to Claim 1 or 2 in which:
• R₁ is a hydrogen atom or a methyl;
• R₂ is a methyl;
• R₃ is a 2,4-dichlorophenyl;
• R₄ is a heterocyclic radical selected from: 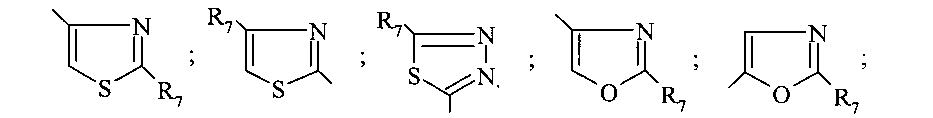 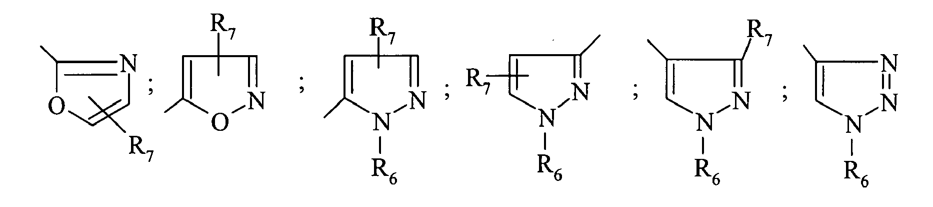
• R₅ is a hydrogen atom;
• R₆ is a hydrogen atom, a methyl or an ethyl;
• R₇ is a hydrogen atom, a methyl, an amino, a methylamino, an aminomethyl, a (dimethylamino)methyl, a pyrrolidin-1-ylmethyl, a chloromethyl, a hydroxymethyl, an ethoxymethyl, a [(2,2-dimethylpropanoyl)oxy]methyl, a [(cyclopropylcarbonyl)oxy]methyl, a methoxycarbonyl, a 2-methoxy-2-oxoethyl or a carboxymethyl;
in the form of a base or of an addition salt with an acid, and also in the form of a hydrate or of a solvate.

10. Compound of formula (I) according to Claim 1 or 2, in which:
• R₁ is a hydrogen atom or a methyl;
• R₂ is a methyl;
• R₃ is a 2,4-dichlorophenyl;
• R₄ is:
. a 2-methyl-1,3-thiazol-4-yl, a 2-amino-1,3-thiazol-4-yl, a 2-(methylamino)-1,3-thiazol-4-yl, a 2-(hydroxymethyl)-1,3-thiazol-4-yl, a 2-(ethoxymethyl)-1,3-thiazol-4-yl, a 2-[[(2,2-dimethylpropanoyl)oxy]methyl]-1,3-thiazol-4-yl, or a 2-[(cyclopropylcarbonyl)oxy]methyl;
. a 1,3-thiazol-2-yl, a 4-methyl-1,3-thiazol-2-yl, a 4-amino-1,3-thiazol-2-yl, a 4-(aminomethyl)-1,3-thiazol-2-yl, a 4-[(dimethylamino)methyl]-1,3-thiazol-2-yl, a 4-(pyrrolidin-1-ylmethyl)-1,3-thiazol-2-yl, a 4-(chloromethyl)-1,3-thiazol-2-yl, a 4-(2-methoxy-2-oxoethyl)-1,3-thiazol-2-yl, or a 4-(carboxymethyl)-1,3-thiazol-2-yl;
. a 1,3-oxazol-4-yl, a 2-methyl-1,3-oxazol-4-yl, a 2-amino-1,3-oxazol-4-yl, a 2-(hydroxymethyl)-1,3-oxazol-4-yl, a 2-(ethoxymethyl)-1,3-oxazol-4-yl, or a 2-[[(2,2-dimethylpropanoyl)oxy]methyl]-1,3-oxazol-4-yl;
. a 1,3-oxazol-5-yl, a 2-methyl-1,3-oxazol-5-yl, or a 2-(ethoxymethyl)-1,3-oxazol-5-yl;
. a 1,3-oxazol-2-yl, a 4-methyl-1,3-oxazol-2-yl, or a 5-methyl-1,3-oxazol-2-yl;
. an isoxazol-5-yl, a 4-methylisoxazol-5-yl, or a 3-(methoxycarbonyl)isoxazol-5-yl;
. a 1*H*-pyrazol-5-yl, a 1-ethyl-1*H*-pyrazol-5-yl, a 3-(methoxycarbonyl)-1*H*-pyrazol-5-yl, a 3-methyl-1*H*-pyrazol-5-yl, or a 3-amino-1*H*-pyrazol-5-yl;
. a 1-methyl-1*H*-pyrazol-3-yl, or a 1-ethyl-1H-pyrazol-3-yl;
. a 3-amino-1*H*-pyrazol-4-yl;
. a 5-amino-1,3,4-thiadiazol-2-yl;
. a 1*H* -1,2,3-triazol-4-yl;
• R₅ is a hydrogen atom;
in the form of a base or of an addition salt with an acid, and also in the form of a hydrate or of a solvate.

11. Compound selected from:
• 6-(2-amino-1,3-thiazol-4-yl)-3-(2,4-dichlorophenyl)-1-methyl-1,9-dihydro-2H-pyrido[2,3-b]indol-2-one;
• 3-(2,4-dichlorophenyl)-6-[2-(hydroxymethyl)-1,3-thiazol-4-yl]-1-methyl-1,9-dihydro-2H-pyrido[2,3-b]indol-2-one;
• 3-(2,4-dichlorophenyl)-1,9-dimethyl-6-(2-methyl-1,3-thiazol-4-yl)-1,9-dihydro-2H-pyrido[2,3-b]indol-2-one;
• [4-[3-(2,4-dichlorophenyl)-1,9-dimethyl-2-oxo-2,9-dihydro-1H-pyrido[2,3-b]indol-6-yl]-1,3-thiazol-2-yl]methyl pivalate;
• 3-(2,4-dichlorophenyl)-6-[2-(ethoxymethyl)-1,3-thiazol-4-yl]-1,9-dimethyl-1,9-dihydro-2H-pyrido[2,3-b]indol-2-one;
• 3-(2,4-dichlorophenyl)-1,9-dimethyl-6-(2-methyl-1,3-oxazol-4-yl)-1,9-dihydro-2H-pyrido[2,3-b]indol-2-one;
• 3-(2,4-dichlorophenyl)-1,9-dimethyl-6-(2-methyl-1,3-oxazol-5-yl)-1,9-dihydro-2H-pyrido[2,3-b]indol-2-one;
• 6-(3-amino-1H-pyrazol-5-yl)-3-(2,4-dichlorophenyl)-1,9-dimethyl-1,9-dihydro-2H-pyrido[2,3-b]indol-2-one;
• 6-[4-(aminomethyl)-1,3-thiazol-2-yl]-3-(2,4-dichlorophenyl)-1,9.-dimethyl-1,9-dihydro-2H-pyrido[2,3-b]indol-2-one;
• 6-(3-amino-1H-pyrazol-4-yl)-3-(2,4-dichlorophenyl)-1,9-dimethyl-1,9-dihydro-2H-pyrido[2,3-b]indol-2-one;
• 3-(2,4-dichlorophenyl)-1,9-dimethyl-6-(1H-pyrazol-5-yl)-1,9-dihydro-2H-pyrido[2,3-b]indol-2-one;
• 3-(2,4-dichlorophenyl)-6-(1-methoxymethyl-1H-pyrazol-3-yl)-1,9-dimethyl-1,9-dihydropyrido[2,3-b]indol-2-one;
• 3-(4-bromophenyl)-6-(1-ethyl-1H-pyrazol-3-yl)-1,9-dimethyl-1,9-dihydropyrido[2,3-b]indol-2-one;
• 6-(2-aminothiazol-5-yl)-3-(2-chloro-4-fluorophenyl)-1,9-dimethyl-1,9-dihydropyrido[2,3-b]indol-2-one;
• 3-(2,4-dichlorophenyl)-6-(2-methoxymethylthiazol-4-yl)-1,9-dimethyl-1,9-dihydropyrido[2,3-b]indol-2-one;
• 3-(2,4-dichlorophenyl)-6-[1-(2,2-dimethylpropionyl)-4-methyl-1H-pyrazol-3-yl]-1,9-dimethyl-1,9-dihydropyrido[2,3-b]indol-2-one;
• 3-(2,4-dichlorophenyl)-6-(5-ethoxymethyl-1-methyl-1H-pyrazol-3-yl)-1,9-dimethyl-1,9-dihydropyrido[2,3-b]indol-2-one;
• 3-(4-fluorophenyl)-6-(1-methoxymethyl-1H-pyrazol-3-yl)-1,9-dimethyl-1,9-dihydropyrido[2,3-b]indol-2-one;
• 3-(4-fluorophenyl)-6-(1-methoxymethyl-4-methyl-1H-pyrazol-3-yl)-1,9-dimethyl-1,9-dihydropyrido[2,3-b]indol-2-one;
• 3-(4-chlorophenyl)-6-(2-ethoxymethylthiazol-4-yl)-1,9-dimethyl-1,9-dihydropyrido[2,3-b]indol-2-one;
• 3-(2,4-dichlorophenyl)-6-[1-(2,2-dimethylpropionyl)-1H-pyrazol-3-yl]-1,9-dimethyl-1,9-dihydropyrido[2,3-b]indol-2-one;
• 3-(2,4-dichlorophenyl)-6-(1-methoxymethyl-1H-[1,2,3]triazol-4-yl)-1,9-dimethyl-1,9-dihydropyrido[2,3-b]indol-2-one in the form of a base or of an addition salt with an acid, and also in the form of a hydrate or of a solvate.

12. Compound of formula: 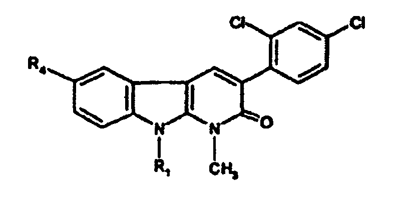 in which:
• R₁ represents H and R₄ is selected from: 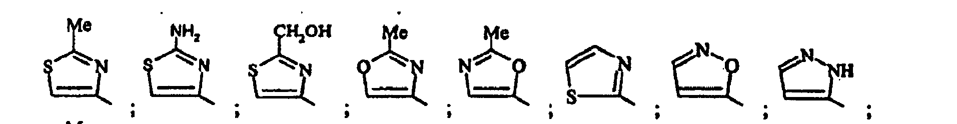 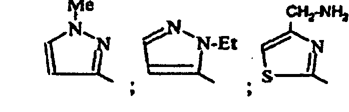 or
• R₁ represents Me and R₄ is selected from: 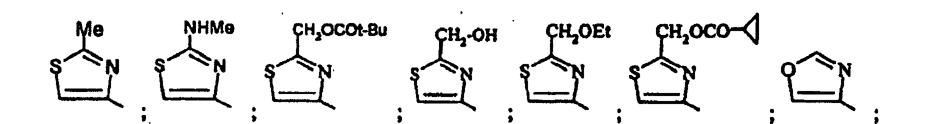 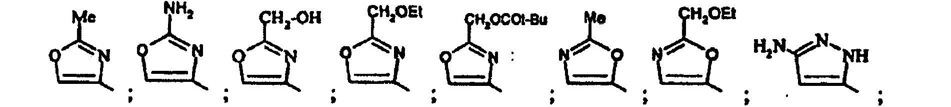 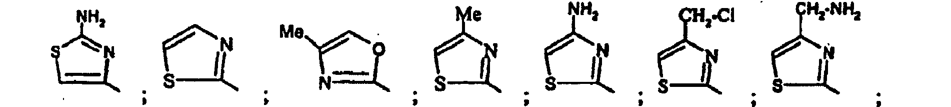 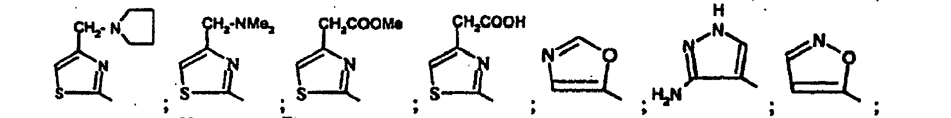 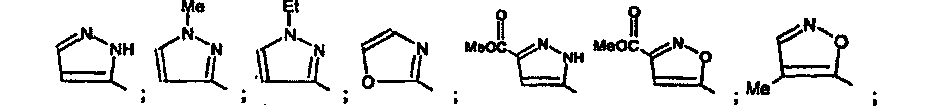 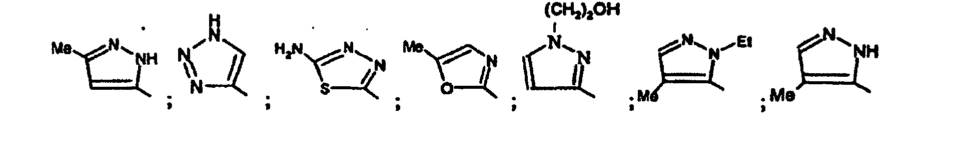 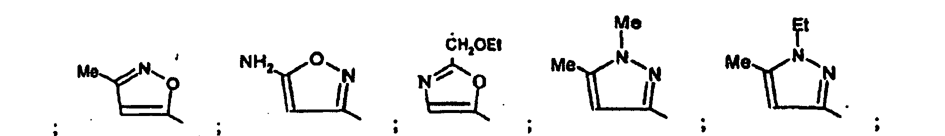 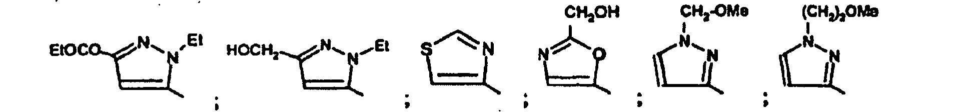 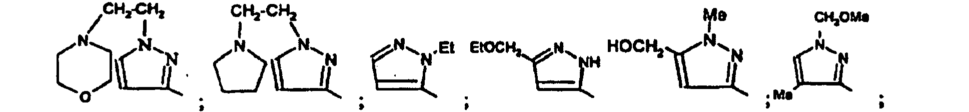 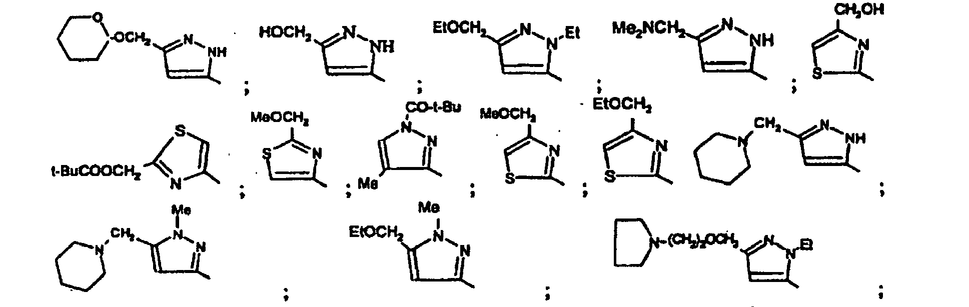 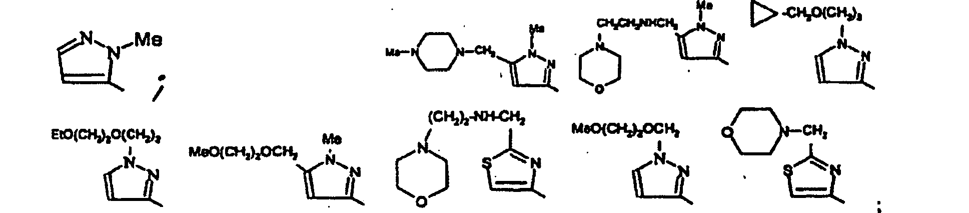 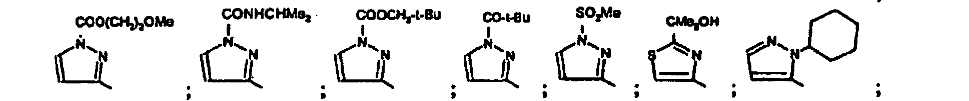 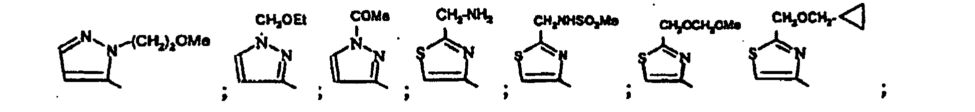 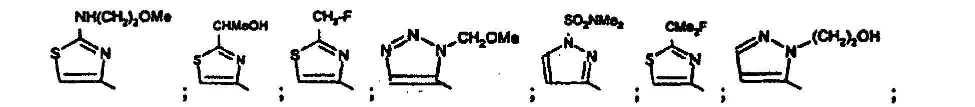 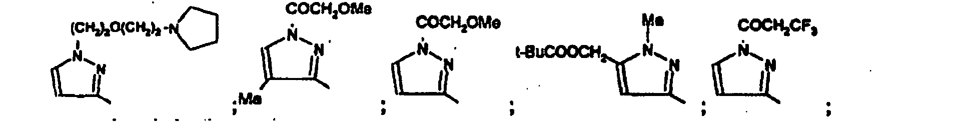 the following abbreviations being used: Me: methyl; Et: ethyl and t-Bu: tert-butyl.

13. Compound according to Claim 12, in the form of a base or of an addition salt with an acid, and also in the form of a hydrate or of a solvate.

14. Process for preparing the compounds of formula (IA) according to Claim 1 in which: **characterized in that**:
a compound of formula: 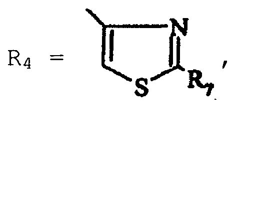 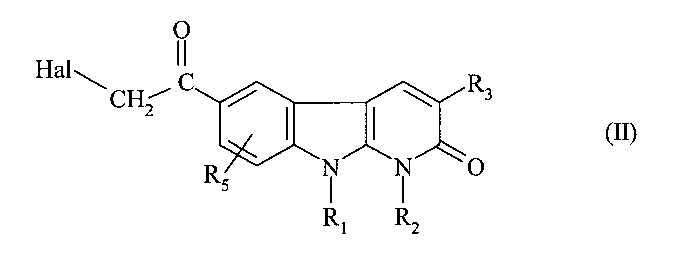
in which R₁, R₂, R₃ and R₅ are as defined in Claim 1 and Hal is a hydrogen atom, is reacted with a compound of formula: 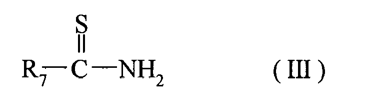 in which R₇ is as defined in Claim 1.

15. Process for preparing the compounds of formula (IA) according to Claim 1 in which 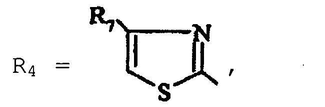 **characterized in that** a compound of formula: 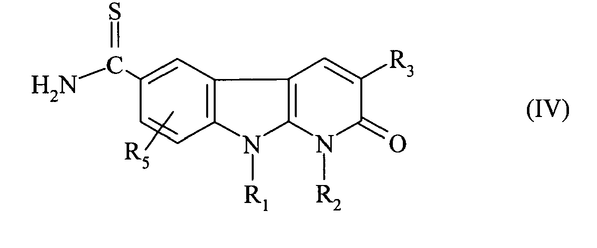 in which R₁, R₂, R₃ and R₅ are as defined in Claim 1, is reacted with a compound of formula: 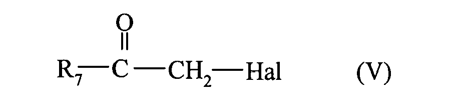 in which R₇ is as defined in Claim 1 and Hal is a halogen atom.

16. Process for preparing the compounds of formula (IB) according to Claim 1 in which 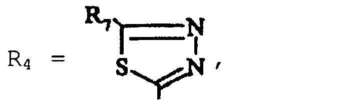 **characterized in that**
a compound of formula: 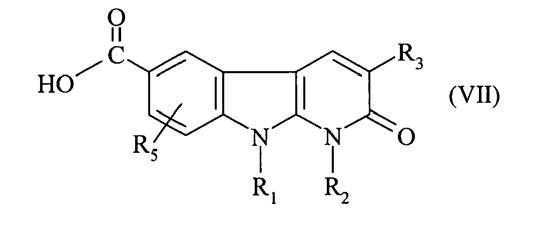
in which R₁, R₂, R₃ and R₅ are as defined in Claim 1, is reacted with a compound of formula: 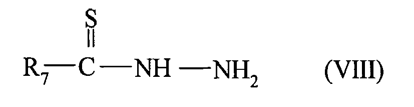 in which R₇ is as defined in Claim 1.

17. Process for preparing the compounds of formula (IC) according to Claim 1 in which 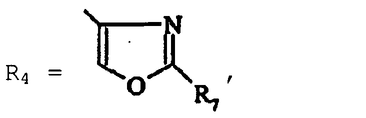 **characterized in that**
a compound of formula: 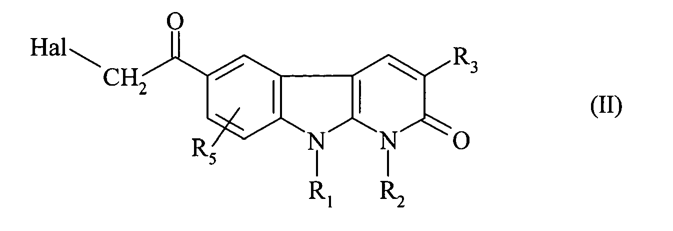 in which R₁, R₂, R₃ and R₅ are as defined in Claim 1 and Hal is a halogen atom, is reacted with a compound of formula: 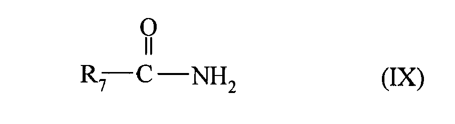 in which R₇ is as defined in Claim 1.

18. Process for preparing the compounds of formula (IC) according to Claim 1 in which 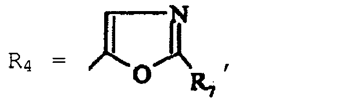 **characterized in that**
A) a compound of formula: 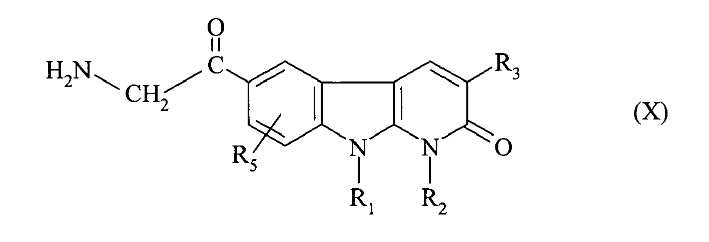 in which R₁, R₂, R₃ and R₅ are as defined in Claim 1, is reacted with a functional derivative of an acid of formula: 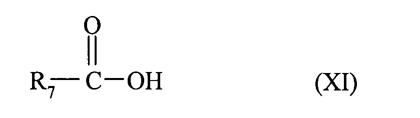 in which R₇ is as defined in Claim 1, so as to obtain a compound of formula: 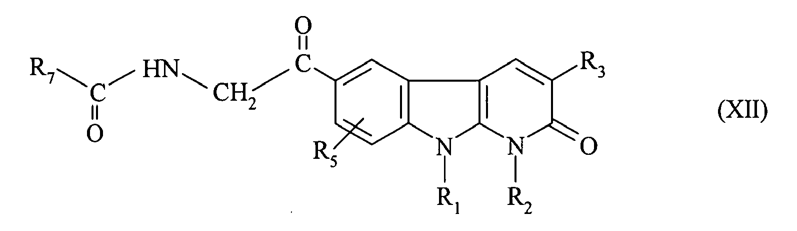
B) the compound of formula (XII) thus obtained is cyclized through the action of an acid.

19. Process for preparing the compounds of formula (IC) according to Claim 1 in which 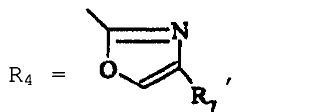 **characterized in that**
a compound of formula: 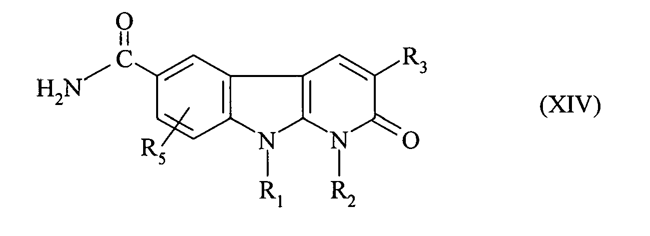 in which R₁, R₂, R₃ and R₅ are as defined in Claim 1, is reacted with a compound of formula: 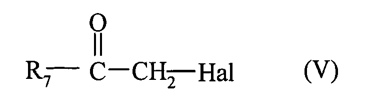 in which R₇ is as defined in Claim 1 and Hal is a halogen atom.

20. Process for preparing the compounds of formula (IC) according to Claim 1 in which 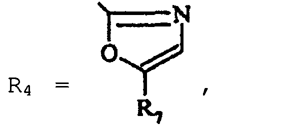 **characterized in that** a compound of formula: 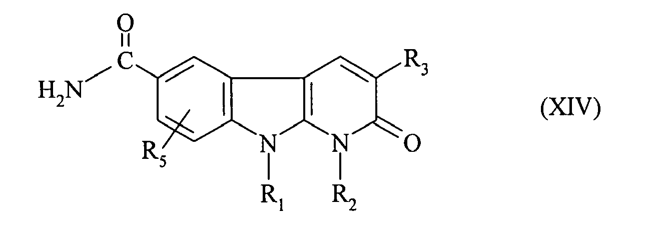 in which R₁, R₂, R₃ and R₅ are as defined in Claim 1, is reacted with a compound of formula: 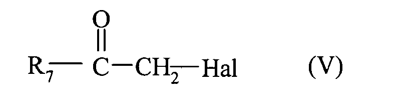 in which R₇ is as defined in Claim 1 and Hal is a halogen atom.

21. Process for preparing the compounds of formula (ID) according to Claim 1 in which 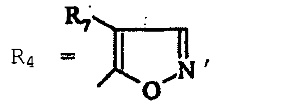 **characterized in that**
a compound of formula: 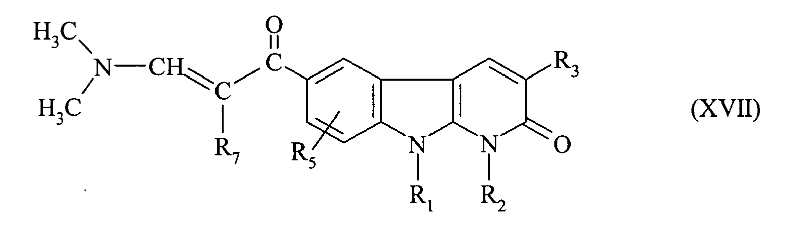 in which R₁, R₂, R₃, R₅ and R₇ are as defined in Claim 1, is reacted with hydroxylamine.

22. Process for preparing the compounds of formula (ID) according to Claim 1 in which 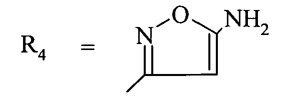 **characterized in that** a compound of formula (XX): 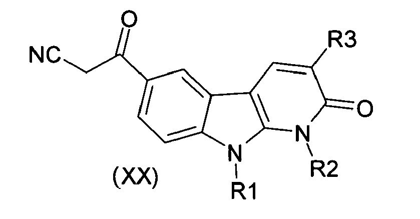 in which R₁, R₂ and R₃ are as defined in Claim 1, is reacted with hydroxylamine.

23. Process for preparing the compounds of formula (ID) according to Claim 1 in which 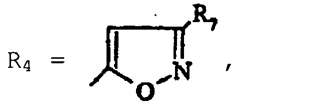 **characterized in that**
a compound of formula: 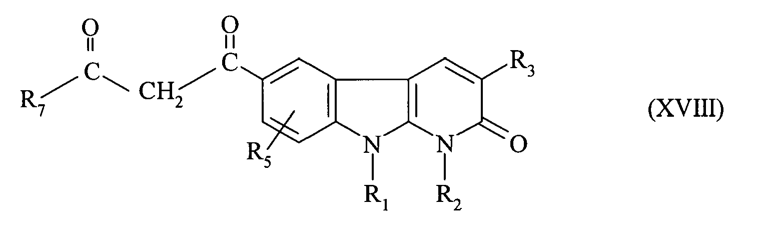 in which R₁, R₂, R₃, R₅ and R₇ are as defined in Claim 1, is reacted with hydroxylamine.

24. Process for preparing the compounds of formula (IE) according to Claim 1 in which 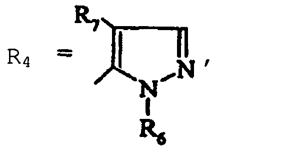 **characterized in that**
a compound of formula: 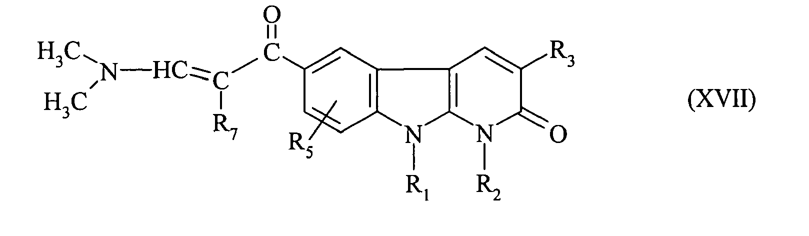 in which R₁, R₂, R₃, R₅ and R₇ are as defined in Claim 1, is reacted with a compound of formula:
NH₂-NH-R₆ (XIX)
in which R₆ is as defined in Claim 1.

25. Process for preparing the compounds of formula (IE) according to Claim 1 in which 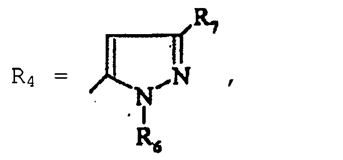 **characterized in that**
a compound of formula: 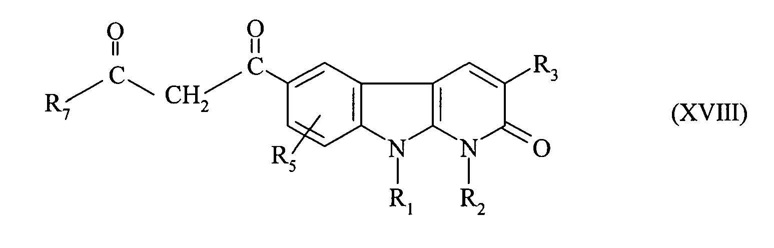 in which R₁, R₂, R₃, R₅ and R₇ are as defined in Claim 1, is reacted with a hydrazine of formula:
NH₂-NH-R₆ (XIX)

26. Process for preparing the compounds of formula (IF) according to Claim 1 in which **characterized in that** 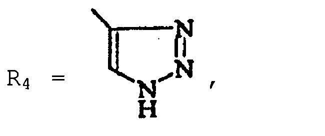 a compound of formula (XXII): 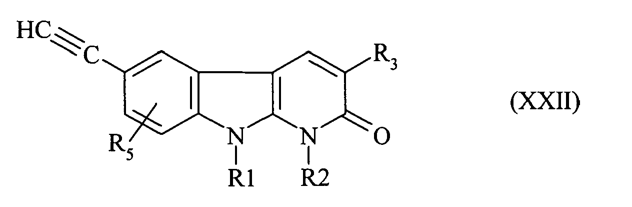 in which R₁, R₂, R₃ and R₅ are as defined for a compound of formula (I) in Claim 1, is reacted with sodium azide.

27. Medicament, **characterized in that** it comprises a compound of formula (I) according to any one of Claims 1 to 13, or an addition salt of this compound with a pharmaceutically acceptable acid, or else a hydrate or a solvate of the compound of formula (I).

28. Pharmaceutical composition, **characterized in that** it comprises a compound of formula (I) according to any one of Claims 1 to 13, or a pharmaceutically acceptable salt, a hydrate or a solvate of this compound, and also at least one pharmaceutically acceptable excipient.

29. Use of a compound of formula (I) according to any one of Claims 1 to 13, for the preparation of a medicament for use in the treatment and prevention of diseases caused or exacerbated by tumour cell proliferation.

30. Use of a compound of formula (I) according to any one of Claims 1 to 13, for the preparation of a medicament for use in the treatment of breast cancer; lung cancer; cancer of the small intestine, cancer of the colon and rectum; cancer of the respiratory pathways, of the oropharynx and of the hypopharynx; oesophageal cancer; liver cancer, stomach cancer, cancer of the bowel ducts, gallbladder cancer, pancreatic cancer; cancer of the urinary tracts, including kidney, urothelium and bladder; cancers of the female genital tract, including uterine cancer, cervical cancer, ovarian cancer, choriocarcinoma and trophoblastoma; cancers of the male genital tract, including prostate cancer, cancer of the seminal vesicles, testicular cancer, germinal cell tumours; cancers of the endocrine glands, including cancer of the thyroid, of the pituitary gland, of the adrenal glands; skin cancers, including haemangiomas, melanomas, sarcomas, including Kaposi's sarcoma; brain tumours, tumours of the nerves, of the eyes, of the meninges, including astrocytomas, gliomas, glioblastomas, retinoblastomas, neurinomas, neuroblastomas, schwannomas, meningiomas; tumours originating from malignant haematopoietic tumours, including leukaemias, chloromas, plasmacytomas, mycosis fongoides, T-cell leukaemia or lymphoma, non-Hodgkin's lymphoma, malignant haemopathies, myelomas.

## Patentansprüche

1. Verbindung der Formel (I): 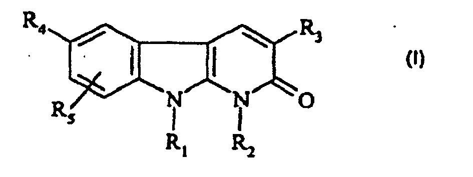 worin:
• R₁ für ein Wasserstoffatom oder eine (C₁-C₄)-Alkyl-, CN-, CF₃- oder CHF₂-Gruppe steht;
• R₂ für ein Wasserstoffatom oder eine (C₁-C₄)-Alkylgruppe steht;
• R₃ für unsubstituiertes oder ein- oder mehrfach durch Substituenten, die unabhängig unter einem Halogenatom, einer (C₁-C₄)-Alkylgruppe und einer (C₁-C₄)-Alkoxygruppe ausgewählt sind, substituiertes Phenyl steht;
• R₄ für einen heterocyclischen Rest steht, der unter: 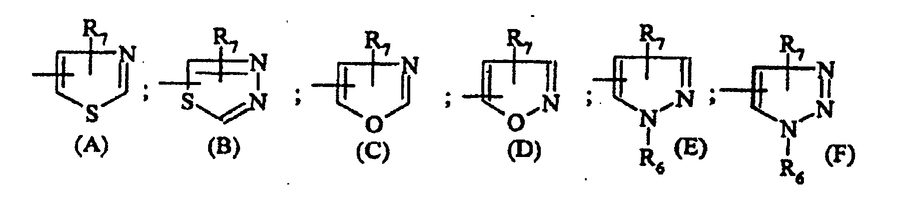 ausgewählt ist;
• R₅ für ein Wasserstoffatom, ein Halogenatom, eine (C₁-C₄) -Alkylgruppe oder eine (C₁-C₄)-Alkoxygruppe steht;
• R₆ unter einem Wasserstoffatom;
einer -SO₂-R₁₂-Gruppe;
einer (C₁-C₆)-Alkyl- oder mono- oder perfluorierten (C₁-C₆)-Alkylgruppe;
einer -(CH₂)ₙ-NR₈R₉-Gruppe;
einer -(CH₂)ₙ-NH-CO-(C₁-C₄)-Alkylgruppe;
einer -(CH₂)ₙ-NH-SO₂-(C₁-C₄)-Alkylgruppe;
einer -(CH₂)ₙ-NH-(CH₂)ₘ-NR₈R₉-Gruppe;
einer -(CH₂)ₙ-NH-(CH₂)ₘ-OR₁₀-Gruppe;
einer -(CH₂)ₙ-O-(CH₂)ₘ-NR₈R₉-Gruppe;
einer -(CH₂)ₙ-O-(CH₂)ₘ-O-(C₁-C₄)-Alkylgruppe;
einer -(CH₂)ₙHal-Gruppe;
einer (C₁-C₆)-Alkyl-OR₁₀-Gruppe;
einer -CO₂-(CH₂)ₘ-OR₁₀-Gruppe;
einer -(CH₂)ₙ-COOR₁₁-Gruppe;
einer (C₁-C₆)-Alkylcarbonyl-, mono- oder perfluorierten (C₁-C₆)-Alkylcarbonyl-, CO-NH-R₁₀- oder -CO-(C₁-C₆)-Alkyl-O-(C₁-C₄)-alkylgruppe; ausgewählt ist;
• R₇ unter einem Wasserstoffatom,
einer (C₁-C₆)-Alkyl- oder mono- oder perfluorierten (C₁-C₆)-Alkylgruppe;
einer -(CH₂)ₙ-NR₈R₉-Gruppe;
einer -(CH₂)ₙ-NH-CO-(C₁-C₄)-Alkylgruppe;
einer -(CH₂)ₙ-NH-SO₂-(C₁-C₄)-Alkylgruppe;
einer -(CH₂)ₙ-NH-(CH₂)ₘ-NR₈R₉-Gruppe;
einer -(CH₂)ₙ-NH-(CH₂)ₘ-OR₁₀-Gruppe;
einer -(CH₂)ₙ-O-(CH₂)ₘ-NR₈R₉-Gruppe;
einer -(CH₂)ₙ-O-(CH₂)ₘ-O-(C₁-C₄)-Alkylgruppe;
einer -(CH₂)ₙHal-Gruppe;
einer (C₁-C₆)-Alkyl-OR₁₀-Gruppe;
einer -CO₂-(CH₂)ₘ-OR₁₀-Gruppe;
einer -(CH₂)ₙ-COOR₁₁-Gruppe;
einer (C₁-C₆)-Alkylcarbonyl-, mono- oder perfluorierten (C₁-C₆)-Alkylcarbonyl-, CO-NH-R₁₀- oder -CO-(C₁-C₆)-Alkyl-O-(C₁-C₄)-alkylgruppe;
ausgewählt ist;
• R₈ und R₉ jeweils unabhängig voneinander für ein Wasserstoffatom oder eine (C₁-C₄)-Alkylgruppe stehen oder R₈ und R₉ auch gemeinsam mit dem Stickstoffatom, an das sie gebunden sind, einen heterocyclischen Rest bilden, der unter Pyrrolidin-1-yl, Piperidin-1-yl, Morpholin-4-yl oder Piperazinyl, das gegebenenfalls am zweiten Stickstoffatom substituiert ist, ausgewählt ist;
• R₁₀ für ein Wasserstoffatom, eine (C₁-C₄)-Alkylgruppe, eine (C₁-C₄)-Alkylcarbonylgruppe, eine (C₃-C₆)-Cycloalkylcarbonylgruppe, eine (C₃-C₆)-Cycloalkyl-(C₁-C₄)-alkylcarbonylgruppe, eine (C₃-C₆)-Cycloalkylgruppe, eine (C₃-C₆)-Heterocycloalkylgruppe, eine mono- oder perfluorierte (C₁-C₆)-Alkylgruppe oder eine (C₃-C₆)-Cycloalkyl-(C₁-C₄)-alkylgruppe steht;
• R₁₁ für ein Wasserstoffatom oder eine (C₁-C₆)-Alkylgruppe steht;
• R₁₂ für eine (C₁-C₆)-Alkylgruppe, eine mono- oder perfluorierte (C₁-C₆)-Alkylgruppe; eine Cycloalkylgruppe, eine Cycloalkylalkylgruppe oder eine (C₁-C₆)-Alkyl-O-(C₁-C₄)-alkylgruppe steht;
• m gleich 1 oder 2 ist;
• n gleich 0, 1 oder 2 ist;
• Hal für ein Halogenatom steht;
in Basen- oder Säureadditionssalzform sowie in Hydrat- oder Solvatform.

2. Verbindung der Formel (I) nach Anspruch 1, worin:
• R₁ für ein Wasserstoffatom oder eine (C₁-C₄)-Alkylgruppe steht;
• R₂ für ein Wasserstoffatom oder eine (C₁-C₄)-Alkylgruppe steht;
• R₃ für unsubstituiertes oder ein- oder mehrfach durch Substituenten, die unabhängig unter einem Halogenatom, einer (C₁-C₄)-Alkylgruppe und einer (C₁-C₄) -Alkoxygruppe ausgewählt sind, substituiertes Phenyl steht;
• R₄ für einen heterocyclischen Rest steht, der unter: 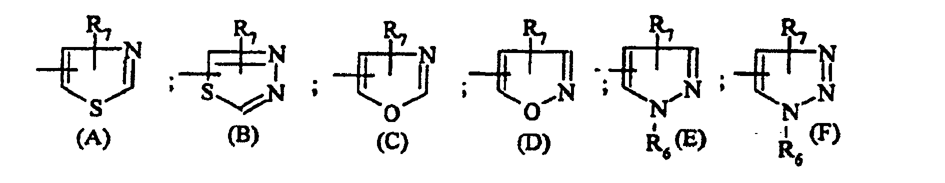 ausgewählt ist;
• R₅ für ein Wasserstoffatom, ein Halogenatom, eine (C₁-C₄)-Alkylgruppe oder eine (C₁-C₄)-Alkoxygruppe steht;
• R₆ für ein Wasserstoffatom oder eine (C₁-C₄)-Alkylgruppe steht;
• R₇ für ein Wasserstoffatom, eine (C₁-C₄)-Alkylgruppe, eine - (CH₂)ₙ-NR₈R₉-Gruppe, eine -(CH₂)ₙHal-Gruppe, eine -CH₂-OR₁₀-Gruppe oder eine -(CH₂)ₙ-COOR₁₁-Gruppe steht;
• R₈ und R₉ jeweils unabhängig voneinander für ein Wasserstoffatom oder eine (C₁-C₄)-Alkylgruppe stehen oder R₈ und R₉ auch gemeinsam mit dem Stickstoffatom, an das sie gebunden sind, einen heterocyclischen Rest bilden, der unter Pyrrolidin-1-yl, Piperidin-1-yl oder Morpholin--4-yl ausgewählt ist;
• R₁₀ für ein Wasserstoffatom, eine (C₁-C₄)-Alkylgruppe, eine (C₁-C₄)-Alkylcarbonylgruppe, oder eine (C₃-C₆)-Cycloalkylcarbonylgruppe steht;
• R₁₁ für ein Wasserstoffatom oder eine (C₁-C₄)-Alkylgruppe steht;
• n gleich 0 oder 1 ist;
• Hal für ein Halogenatom steht;
in Basen- oder Säureadditionssalzform sowie in Hydrat- oder Solvatform.

3. Verbindung der Formel (IA) nach Anspruch 1 oder 2, worin R₄ für ein durch R₇ substituiertes 1,3-Thiazol steht und die Substituenten R₁ bis R₇ die in Anspruch 1 angegebene Bedeutung besitzen, in Basen- oder Säureadditionssalzform sowie in Hydrat- oder Solvatform.

4. Verbindung der Formel (IB) nach Anspruch 1 oder 2, worin R₄ für ein durch R₇ substituiertes 1,3,4-Thiadiazol steht und die Substituenten R₁ bis R₇ die in Anspruch 1 angegebene Bedeutung besitzen, in Basen- oder Säureadditionssalzform sowie in Hydrat- oder Solvatform.

5. Verbindung der Formel (IC) nach Anspruch 1 oder 2, worin R₄ für ein durch R₇ substituiertes 1,3-Oxazol steht und die Substituenten R₁ bis R₇ die in Anspruch 1 angegebene Bedeutung besitzen, in Basen- oder Säureadditionssalzform sowie in Hydrat- oder Solvatform.

6. Verbindung der Formel (ID) nach Anspruch 1 oder 2, worin R₄ für ein durch R₇ substituiertes Isoxazol steht und die Substituenten R₁ bis R₇ die in Anspruch 1 angegebene Bedeutung besitzen, in Basen- oder Säureadditionssalzform sowie in Hydrat- oder Solvatform.

7. Verbindung der Formel (IE) nach Anspruch 1 oder 2, worin R₄ für ein durch R₆ und R₇ substituiertes 1*H-*Pyrazol steht und die Substituenten R₁ bis R₇ die in Anspruch 1 angegebene Bedeutung besitzen, in Basen- oder Säureadditionssalzform sowie in Hydrat- oder Solvatform.

8. Verbindung der Formel (IF) nach Anspruch 1 oder 2, worin R₄ für ein durch R₆ und R₇ substituiertes 1,2,3-Triazol steht und die Substituenten R₁ bis R₇ die in Anspruch 1 angegebene Bedeutung besitzen, in Basen- oder Säureadditionssalzform sowie in Hydrat- oder Solvatform.

9. Verbindung der Formel (I) nach Anspruch 1 oder 2, worin:
• R₁ für ein Wasserstoffatom oder Methyl steht;
• R₂ für Methyl steht;
• R₃ für 2,4-Dichlorphenyl steht;
• R₄ für einen heterocyclischen Rest steht, der unter: 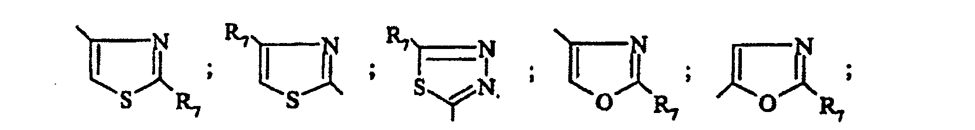 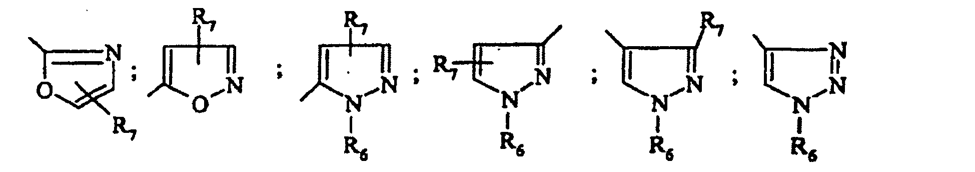 ausgewählt ist;
• R₅ für ein Wasserstoffatom steht;
• R₆ für ein Wasserstoffatom, Methyl oder Ethyl steht;
• R₇ für ein Wasserstoffatom, Methyl, Amino, Methylamino, Aminomethyl, (Dimethylamino)-methyl, Pyrrolidin-1-ylmethyl, Chlormethyl, Hydroxymethyl, Ethoxymethyl, [(2,2-Dimethylpropanoyl)oxy]methyl, [(Cyclopropylcarbonyl)-oxy]methyl, Methoxycarbonyl, 2-Methoxy-2-oxoethyl oder Carboxymethyl steht;
in Basen- oder Säureadditionssalzform sowie in Hydrat- oder Solvatform.

10. Verbindung der Formel (I) nach Anspruch 1 oder 2, worin:
• R₁ für ein Wasserstoffatom oder Methyl steht;
• R₂ für Methyl steht;
• R₃ für 2,4-Dichlorphenyl steht;
• R₄ für:
. 2-Methyl-1,3-thiazol-4-yl, 2-Amino-1,3-thiazol-4-yl, 2-(Methylamino)-1,3-thiazol-4-yl, 2-(Hydroxymethyl)-1,3-thiazol-4-yl, 2-(Ethoxymethyl)-1,3-thiazol-4-yl, 2-[[(2,2-Dimethylpropanoyl)oxy]methyl]-1,3-thiazol-4-yl oder 2-[(Cyclopropylcarbonyl)oxy]methyl;
1,3-Thiazol-2-yl, 4-Methyl-1,3-thiazol-2-yl, 4-Amino-1,3-thiazol-2-yl, 4-(Aminomethyl)-1,3-thiazol-2-yl, 4-[(Dimethylamino)methyl]-1,3-thiazol-2-yl, 4-(Pyrrolidin-1-ylmethyl)-1,3-thiazol-2-yl, 4-(Chlormethyl)-1,3-thiazol-2-yl, 4-(2-Methoxy-2-oxoethyl)-1,3-thiazol-2-yl oder 4-(Carboxymethyl)-1,3-thiazol-2-yl;
. 1,3-Oxazol-4-yl, 2-Methyl-1,3-oxazol-4-yl, 2-Amino-1,3-oxazol-4-yl, 2-(Hydroxymethyl)-1,3-oxazol-4-yl, 2-(Ethoxymethyl)-1,3-oxazol-4-yl, oder 2-[[(2,2-Dimethylpropanoyl)oxy]methyl]-1,3-oxazol-4-yl;
. 1,3-Oxazol-5-yl, 2-Methyl-1,3-oxazol-5-yl oder 2-(Ethoxymethyl)-1,3-oxazol-5-yl;
. 1,3-Oxazol-2-yl, 4-Methyl-1,3-oxazol-2-yl oder 5-Methyl-1,3-oxazol-2-yl;
. Isoxazol-5-yl, 4-Methylisoxazol-5-yl oder 3-(Methoxycarbonyl)isoxazol-5-yl;
. 1*H*-Pyrazol-5-yl, 1-Ethyl-1*H*-pyrazol-5-yl, 3-(Methoxycarbonyl)-1*H*-pyrazol-5-yl, 3-Methyl-1*H-*pyrazol-5-yl oder 3-Amino-1*H*-pyrazol-5-yl;
. 1-Methyl-1*H*-pyrazol-3-yl oder 1-Ethyl-1*H-*pyrazol-3-yl;
. 3-Amino-1*H*-pyrazol-4-yl;
. 5-Amino-1,3,4-thiadiazol-2-yl oder
. 1*H*-1,2,3-Triazol-4-yl
steht;
• R₅ für ein Wasserstoffatom steht;
in Basen- oder Säureadditionssalzform sowie in Hydrat- oder Solvatform.

11. Verbindung, ausgewählt unter:
• 6-(2-Amino-1,3-thiazol-4-yl)-3-(2,4-dichlorphenyl)-1-methyl-1,9-dihydro-2*H*-pyrido[2,3-b]-indol-2-on;
• 3-(2,4-Dichlorphenyl)-6-[2-(hydroxymethyl)-1,3-thiazol-4-yl]-1-methyl-1,9-dihydro-2*H*-pyrido[2,3-b]indol-2-on;
• 3-(2,4-Dichlorphenyl)-1,9-dimethyl-6-(2-methyl-1,3-thiazol-4-yl)-1,9-dihydro-2*H*-pyrido[2,3-b]-indol-2-on;
• Pivalinsäure[4-[3-(2,4-dichlorphenyl)-1,9-dimethyl-2-oxo-2,9-dihydro-1*H*-pyrido[2,3-b]indol-6-yl]-1,3-thiazol-2-yl]methylester;
• 3-(2,4-Dichlorphenyl)-6-[2-(ethoxymethyl)-1,3-thiazol-4-yl]-1,9-dimethyl-1,9-dihydro-2*H-*pyrido[2,3-b]indol-2-on;
• 3-(2,4-Dichlorphenyl)-1,9-dimethyl-6-(2-methyl-1,3-oxazol-4-yl)-1,9-dihydro-2*H*-pyrido[2,3-b]-indol-2-on;
• 3-(2,4-Dichlorphenyl)-1,9-dimethyl-6-(2-methyl-1,3-oxazol-5-yl)-1,9-dihydro-2*H*-pyrido[2,3-b]-indol-2-on;
• 6-(3-Amino-1*H*-pyrazol-5-yl)-3-(2,4-dichlorphenyl)-1,9-dimethyl-1,9-dihydro-2*H*-pyrido[2,3-b]-indol-2-on;
• 6-[4-(Aminomethyl)-1,3-thiazol-2-yl]-3-(2,4-dichlorphenyl)-1,9-dimethyl-1,9-dihydro-2*H*-pyrido-[2,3-b]indol-2-on;
• 6-(3-Amino-1*H*-pyrazol-4-yl)-3-(2,4-dichlorphenyl)-1,9-dimethyl-1,9-dihydro-2*H*-pyrido[2,3-b]-indol-2-on;
• 3-(2,4-Dichlorphenyl)-1,9-dimethyl-6-(1*H-*pyrazol-5-yl)-1,9-dihydro-2*H-*pyrido[2,3-b]indol-2-on;
• 3-(2,4-Dichlorphenyl)-6-(1-methoxymethyl-1H-pyrazol-3-yl)-1,9-dimethyl-1,9-dihydropyrido[2,3-b]indol-2-on;
• 3-(4-Bromphenyl)-6-(1-ethyl-1H-pyrazol-3-yl)-1,9-dimethyl-1,9-dihydropyrido[2,3-b]indol-2-on;
• 6-(2-Aminothiazol-5-yl)-3-(2-chlor-4-fluorphenyl)-1,9-dimethyl-1,9-dihydropyrido[2,3-b]-indol-2-on;
• 3-(2,4-Dichlorphenyl)-6-(2-methoxymethylthiazol-4-yl)-1,9-dimethyl-1,9-dihydropyrido[2,3-b]indol-2-on;
• 3-(2,4-Dichlorphenyl)-6-[1-(2,2-dimethylpropionyl)-4-methyl-1H-pyrazol-3-yl]-1,9-dimethyl-1,9-dihydropyrido[2,3-b]indol-2-on;
• 3-(2,4-Dichlorphenyl)-6-(5-ethoxymethyl-1-methyl-1H-pyrazol-3-yl)-1,9-dimethyl-1,9-dihydropyrido[2,3-b]indol-2-on;
• 3-(4-Fluorphenyl)-6-(1-methoxymethyl-1H-pyrazol-3-yl)-1,9-dimethyl-1,9-dihydropyrido[2,3-b]indol-2-on;
• 3-(4-Fluorphenyl)-6-(1-methoxymethyl-4-methyl-1H-pyrazol-3-yl)-1,9-dimethyl-1,9-dihydropyrido-[2,3-b]indol-2-on;
• 3-(4-Chlorphenyl)-6-(2-ethoxymethylthiazol-4-yl)-1,9-dimethyl-1,9-dihydropyrido[2,3-b]indol-2-on;
• 3-(2,4-Dichlorphenyl)-6-[1-(2,2-dimethylpropionyl)-1H-pyrazol-3-yl]-1,9-dimethyl-1,9-dihydropyrido[2,3-b]indol-2-on und
• 3-(2,4-Dichlorphenyl)-6-(1-methoxymethyl-1H-[1,2,3]triazol-4-yl)-1,9-dimethyl-1,9-dihydropyrido[2,3-b]indol-2-on;
in Basen- oder Säureadditionssalzform sowie in Hydrat- oder Solvatform.

12. Verbindung der Formel: 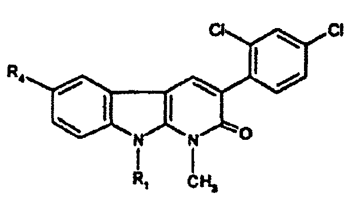 worin:
• R₁ für H steht und R₄ unter: 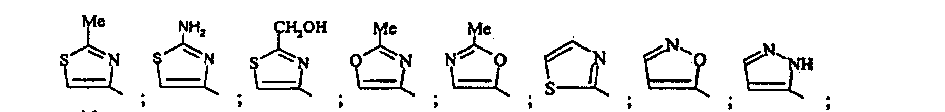 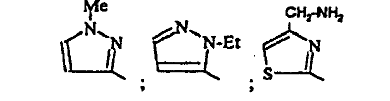 ausgewählt ist oder
• R₁ für Me steht und R₄ unter: 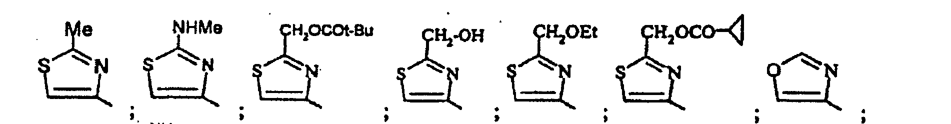 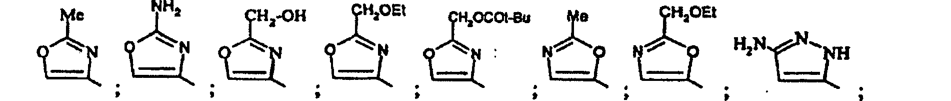 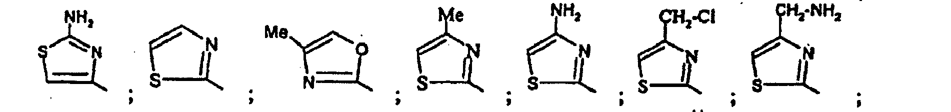 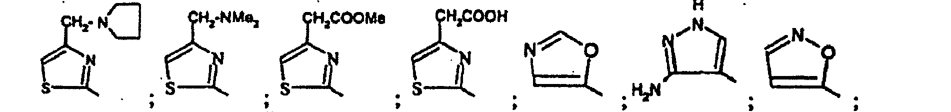 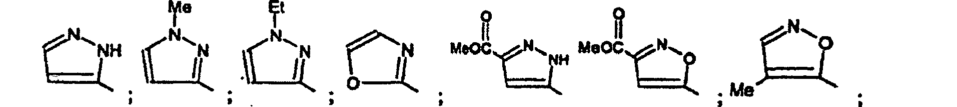 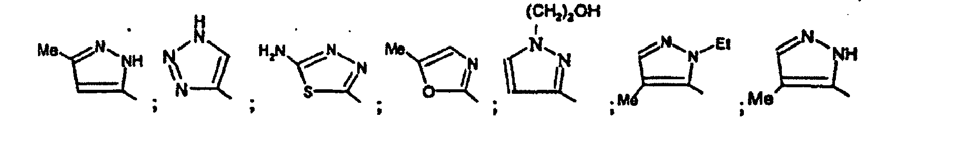 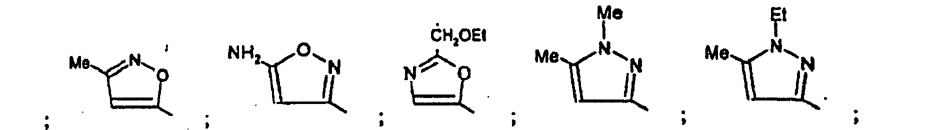 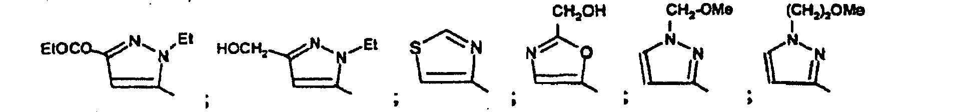 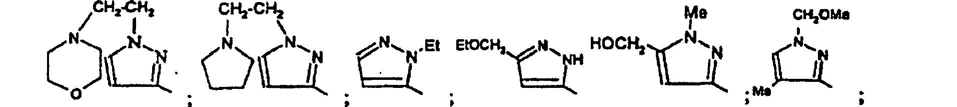 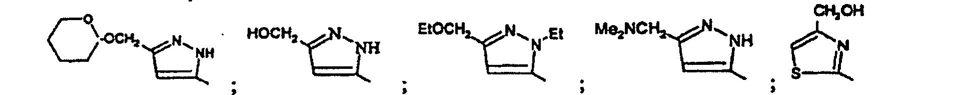 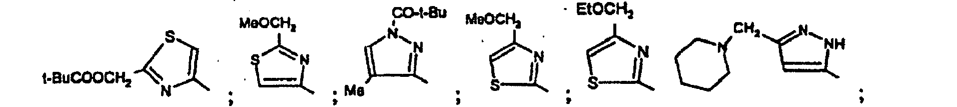 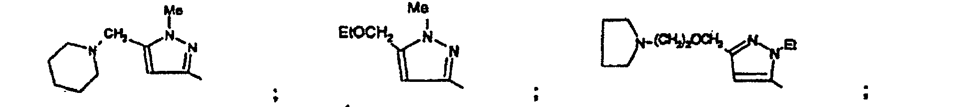 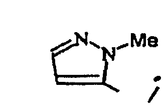 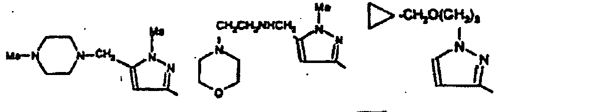 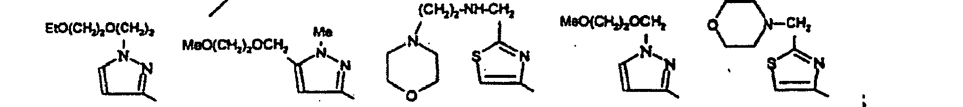 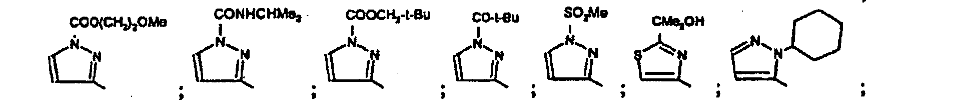 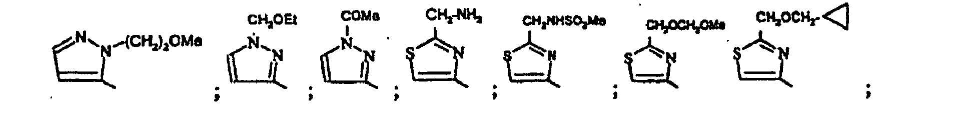 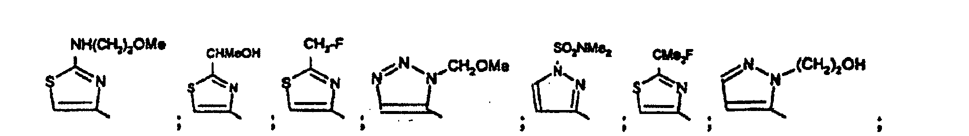 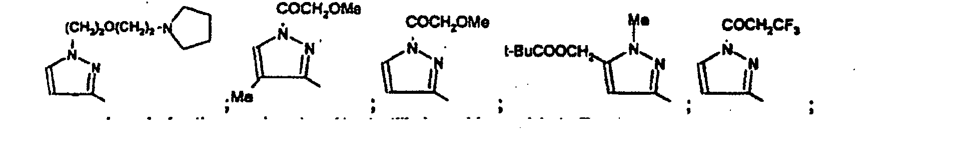 ausgewählt ist;
wobei die folgenden Abkürzungen verwendet werden: Me: Methyl; Et: Ethyl und t-Bu: tert-Butyl.

13. Verbindung nach Anspruch 12 in Basen- oder Säureadditionssalzform sowie in Hydrat- oder Solvatform.

14. Verfahren zur Herstellung der Verbindungen der Formel (IA) nach Anspruch 1, worin: 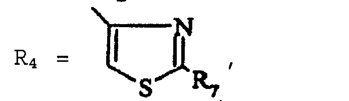 **dadurch gekennzeichnet, daß** man:
eine Verbindung der Formel: 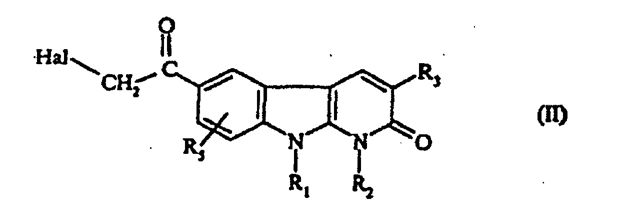
worin R₁, R₂, R₃ und R₅ die in Anspruch 1 angegebene Bedeutung besitzen und Hal für ein Halogenatom steht, mit einer Verbindung der Formel: 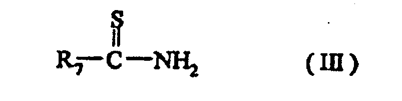 worin R₇ die in Anspruch 1 angegebene Bedeutung besitzt, umsetzt.

15. Verfahren zur Herstellung der Verbindungen der Formel (IA) nach Anspruch 1, worin: 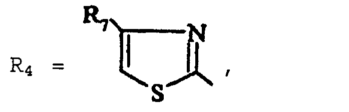 **dadurch gekennzeichnet, daß** man:
eine Verbindung der Formel: 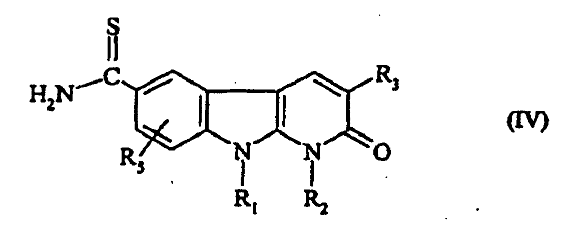
worin R₁, R₂, R₃ und R₅ die in Anspruch 1 angegebene Bedeutung besitzen, mit einer Verbindung der Formel: 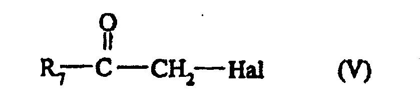 worin R₇ die in Anspruch 1 angegebene Bedeutung besitzt und Hal für ein Halogenatom steht, umsetzt.

16. Verfahren zur Herstellung der Verbindungen der Formel (IB) nach Anspruch 1, worin: 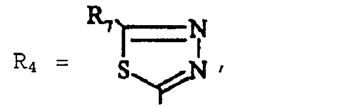 **dadurch gekennzeichnet, daß** man:
eine Verbindung der Formel: 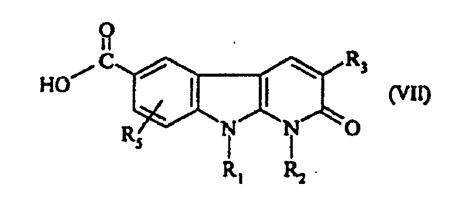
worin R₁, R₂, R₃ und R₅ die in Anspruch 1 angegebene Bedeutung besitzen, mit einer Verbindung der Formel: 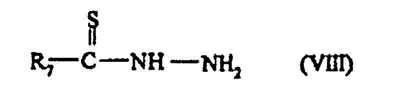 worin R₇ die in Anspruch 1 angegebene Bedeutung besitzt, umsetzt.

17. Verfahren zur Herstellung der Verbindungen der Formel (IC) nach Anspruch 1, worin: 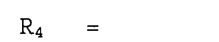 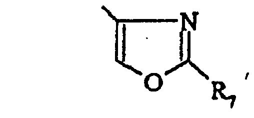 **dadurch gekennzeichnet, daß** man:
eine Verbindung der Formel: 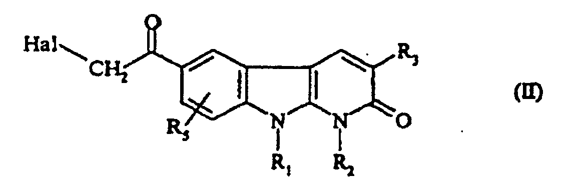 worin R₁, R₂, R₃ und R₅ die in Anspruch 1 angegebene Bedeutung besitzen und Hal für ein Halogenatom steht, mit einer Verbindung der Formel: 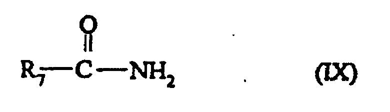 worin R₇ die in Anspruch 1 angegebene Bedeutung besitzt, umsetzt.

18. Verfahren zur Herstellung der Verbindungen der Formel (IC) nach Anspruch 1, worin: 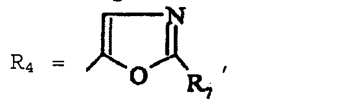 **dadurch gekennzeichnet, daß** man:
A) eine Verbindung der Formel: 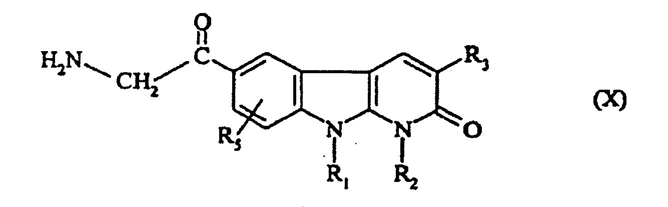 worin R₁, R₂, R₃ und R₅ die in Anspruch 1 angegebene Bedeutung besitzen, mit einem funktionellen Derivat einer Säure der Formel: 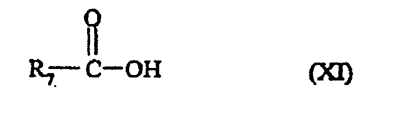 worin R₇ die in Anspruch 1 angegebene Bedeutung besitzt, zu einer Verbindung der Formel: 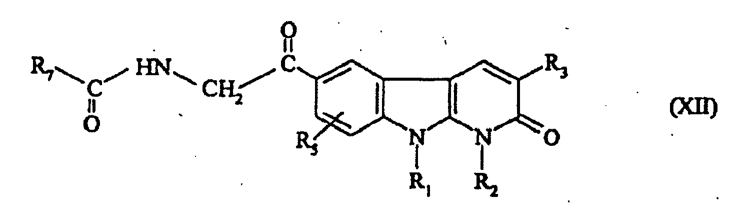 umsetzt und
B) die so erhaltene Verbindung der Formel (XII) durch Einwirkung einer Säure cyclisiert.

19. Verfahren zur Herstellung der Verbindungen der Formel (IC) nach Anspruch 1, worin: 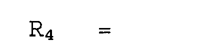 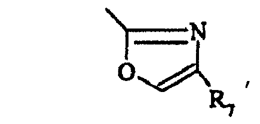 **dadurch gekennzeichnet, daß** man:
eine Verbindung der Formel: 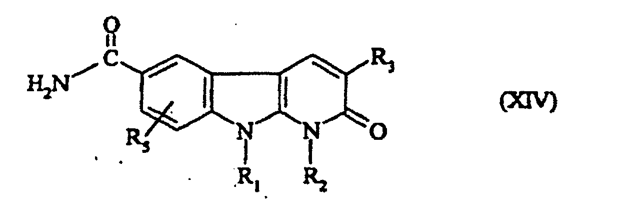 worin R₁, R₂, R₃ und R₅ die in Anspruch 1 angegebene Bedeutung besitzen, mit einer Verbindung der Formel: 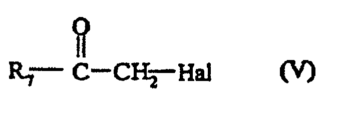 worin R₇ die in Anspruch 1 angegebene Bedeutung besitzt und Hal für ein Halogenatom steht, umsetzt.

20. Verfahren zur Herstellung der Verbindungen der
Formel (IC) nach Anspruch 1, worin: 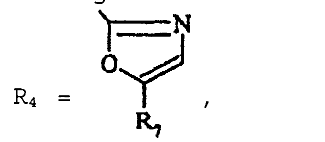 **dadurch gekennzeichnet, daß** man: eine Verbindung der Formel: 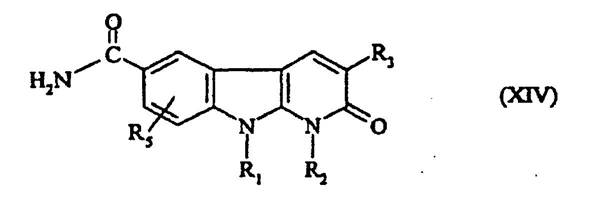 worin R₁, R₂, R₃ und R₅ die in Anspruch 1 angegebene Bedeutung besitzen, mit einer Verbindung der Formel: 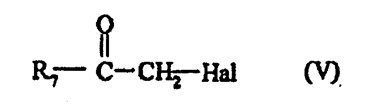 worin R₇ die in Anspruch 1 angegebene Bedeutung besitzt und Hal für ein Halogenatom steht, umsetzt.

21. Verfahren zur Herstellung der Verbindungen der Formel (ID) nach Anspruch 1, worin: 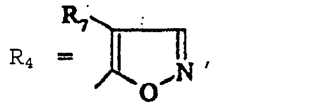 **dadurch gekennzeichnet, daß** man:
eine Verbindung der Formel: 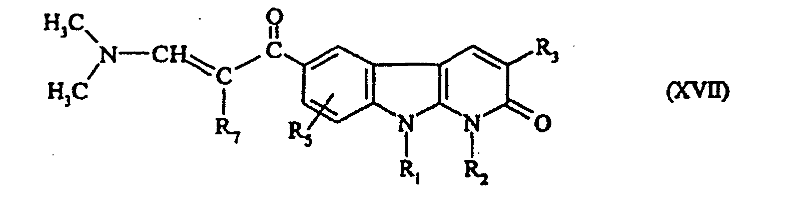
worin R₁, R₂, R₃, R₅ und R₇ die in Anspruch 1 angegebene Bedeutung besitzen, mit Hydroxylamin umsetzt.

22. Verfahren zur Herstellung der Verbindungen der Formel (ID) nach Anspruch 1, worin 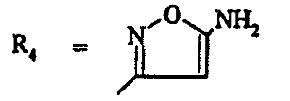 **dadurch gekennzeichnet, daß** man eine Verbindung der Formel (XX) : 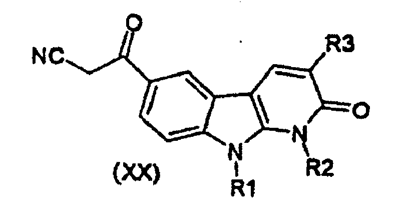 worin R₁, R₂ und R₃ die in Anspruch 1 angegebene Bedeutung besitzen, mit Hydroxylamin umsetzt.

23. Verfahren zur Herstellung der Verbindungen der Formel (ID) nach Anspruch 1, worin: 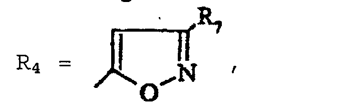 **dadurch gekennzeichnet, daß** man:
eine Verbindung der Formel: 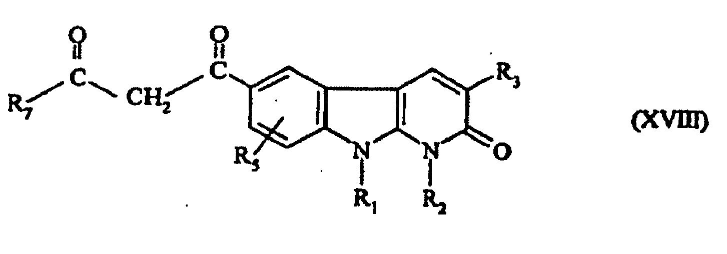
worin R₁, R₂, R₃, R₅ und R₇ die in Anspruch 1 angegebene Bedeutung besitzen, mit Hydroxylamin umsetzt.

24. Verfahren zur Herstellung der Verbindungen der Formel (IE) nach Anspruch 1, worin: 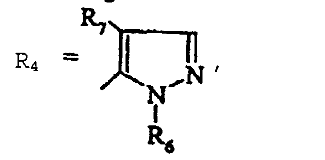 **dadurch gekennzeichnet, daß** man:
eine Verbindung der Formel: 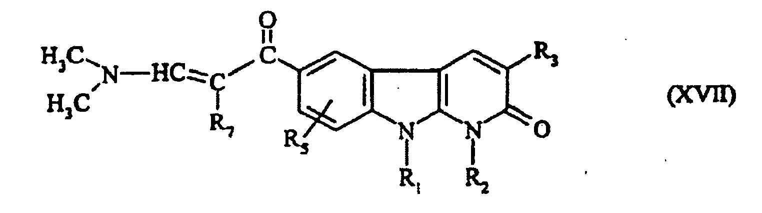
worin R₁, R₂, R₃, R₅ und R₇ die in Anspruch 1 angegebene Bedeutung besitzen, mit einer Verbindung der Formel:
**NH₂-NH-R₆** **(XIX)**
worin R₆ die in Anspruch 1 angegebene Bedeutung besitzt, umsetzt.

25. Verfahren zur Herstellung der Verbindungen der Formel (IE) nach Anspruch 1, worin: R₄ = 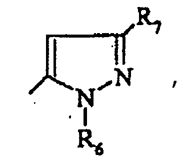 **dadurch gekennzeichnet, daß** man:
eine Verbindung der Formel: 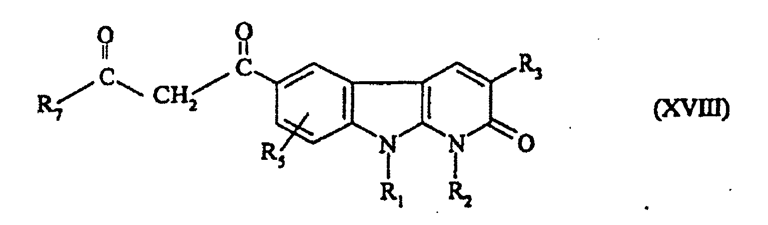 worin R₁, R₂, R₃, R₅ und R₇ die in Anspruch 1 angegebene Bedeutung besitzen, mit einem Hydrazin der Formel:
**NH₂-NH-R₆** **(XIX)**

26. Verfahren zur Herstellung der Verbindungen der Formel (IF) nach Anspruch 1, worin: R₄ = 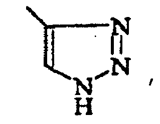 **dadurch gekennzeichnet, daß** man:
eine Verbindung der Formel (XXII): 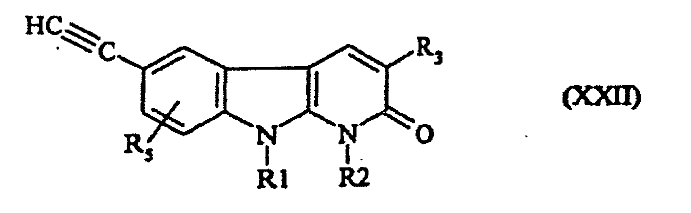 worin R₁, R₂, R₃ und R₅ wie für eine Verbindung der Formel (I) in Anspruch 1 definiert sind, mit Natriumazid umsetzt.

27. Arzneimittel, **dadurch gekennzeichnet, daß** es eine Verbindung der Formel (I) nach einem der Ansprüche 1 bis 13 oder ein Additionssalz dieser Verbindung mit einer pharmazeutisch annehmbaren Säure oder auch ein Hydrat oder ein Solvat der Verbindung der Formel (I) enthält.

28. Pharmazeutische Zusammensetzung, **dadurch gekennzeichnet, daß** sie eine Verbindung der Formel (I) nach einem der Ansprüche 1 bis 13 oder ein pharmazeutisch annehmbares Salz, ein Hydrat oder ein Solvat dieser Verbindung sowie mindestens einen pharmazeutisch annehmbaren Hilfsstoff enthält.

29. Verwendung einer Verbindung der Formel (I) nach einem der Ansprüche 1 bis 13 zur Herstellung eines Arzneimittels zur Behandlung und Prävention von durch die Proliferation von Tumorzellen verursachten oder verschlimmerten Erkrankungen.

30. Verwendung einer Verbindung der Formel (I) nach einem der Ansprüche 1 bis 13 zur Herstellung eines Arzneimittels zur Behandlung von Brustkrebs; Lungenkrebs; Dünndarmkrebs; Kolon- und Rektumkrebs; Krebs der Atemwege, des Oropharynx und des Hypopharynx; Speiseröhrenkrebs; Leberkrebs, Magenkrebs, Gallengangskrebs, Gallenblasenkrebs, Bauchspeicheldrüsenkrebs; Krebserkrankungen der Harnwege einschließlich Niere, Urothel und Blase; Krebserkrankungen des weiblichen Genitaltrakts einschließlich Uteruskrebs, Gebärmutterhalskrebs, Eierstockkrebs, Chorionkarzinom und Trophoblastom; Krebserkrankungen des männlichen Genitaltrakts einschließlich Prostatakrebs, Samenblasenkrebs, Hodenkrebs und Keimzellentumoren; Krebserkrankungen der endokrinen Drüsen einschließlich Schilddrüsenkrebs, Hypophysenkrebs und Nebennierenkrebs; Krebserkrankungen der Haut einschließlich Hämangiomen, Melanomen und Sarkomen einschließlich Kaposi-Sarkom; Tumoren des Gehirns, der Nerven, der Augen und der Meningen einschließlich Astrozytomen, Gliomen, Glioblastomen, Retinoblastomen, Neurinomen, Neuroblastomen, Schwannomen und Meningiomen; Tumoren, die sich aus hämatopoetischen malignen Tumoren ergeben, einschließlich Leukämien, Chloromen, Plasmazytomen, Pilzmykose, Lymphom oder T-Zellenleukämie, non-Hodgkin-Lymphom, malignen Hämopathien und Myelomen.
